# FASCICULE DE BREVET EUROPEEN

(11) **EP 2 432 799 B1**
(45) Date de publication et mention de la délivrance du brevet: **10.09.2014**
(21) Numéro de dépôt: 10723079.9
(22) Date de dépôt: 20.05.2010
(51) Int. Cl.: C07J 53/00, C07J 71/00, A61K 31/58, A61K 31/575, A61P 35/00

(54) **DERIVES DES CYCLOARTANONES AVEC ACTIVITE ANTICANCEREUSE**
CYCLOATANON-DERIVATE MIT WIRKUNG GEGEN KREBSERKRANKUNGEN
CYCLOARTANONE DERIVATIVES WITH ANTI-CANCER ACTIVITY

(30) Priorité: 20.05.2009 FR 0953385
(43) Date de publication de la demande: 28.03.2012
(73) Titulaire: Pierre Fabre Médicament, 92100 Boulogne-Billancourt (FR); Centre National de la Recherche Scientifique (CNRS), 75016 Paris (FR)
(72) Inventeur: LONG, Christophe, F-81570 Vielmur Sur Agout (FR); GUMINSKI, Yves, F-81090 Lagarrigue (FR); DERGUINI, Fadila, F-31000 Toulouse (FR); BECK, Joséphine, F-31400 Toulouse (FR); CANTAGREL, Frédéric, F-33400 Talence (FR)
(74) Mandataire: Regimbeau
(86) Numéro de dépôt international: PCT/EP2010/057006
(87) Numéro de publication internationale: WO 2010/133687

(56) Documents cités:
- KIKUCHI, TAKASHI ET AL: "Cancer Chemopreventive Effects of Cycloartane-Type and Related Triterpenoids in in Vitro and in Vivo Models" JOURNAL OF NATURAL PRODUCTS , 70(6), 918-922 CODEN: JNPRDF; ISSN: 0163-3864, 2007, XP002567886
- GRANDO, ROGERIO ET AL: "Antineoplastic 31-norcycloartanones from Solanum cernuum Vell" 2008, ZEITSCHRIFT FUER NATURFORSCHUNG, C: JOURNAL OF BIOSCIENCES , 63(7/8), 507-514 CODEN: ZNCBDA; ISSN: 0939-5075 , XP8118808 page 510; figure 1; composé 2 page 511; tableau II; composé 2
- RAKHIMOV K D ET AL: "TRITERPENE GLYCOSIDES FROM THALICTRUM-FOETIDUM L. AND THALICTRUM-MINUS L. AND THEIR ANTITUMOR ACTIVITY" 1987, Pharmaceutical Chemistry Journal, VOL. 21, NR. 12, PAGE(S) 848-850 , XP8118816 ISSN: 0023-1134 page 849; tableau 1; composé III

## Description

La présente invention concerne de nouveaux dérivés pouvant être utiles en tant que médicament, notamment dans le traitement d'une maladie proliférative telle qu'un cancer. J. Nat. Prod. 70(6) pages 918-922 décrit des dérives des cycloartanes et leur activité anti-cancereuse.

Ainsi, la présente invention a pour objet un composé de formule (I) suivante: 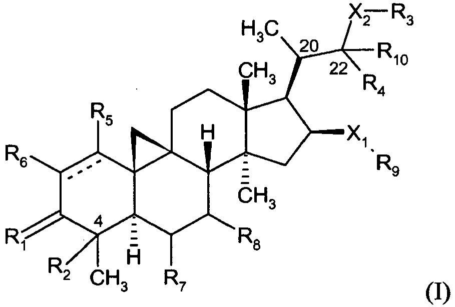 ou un sel pharmaceutiquement acceptable de celui-ci,
pour lequel : 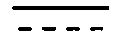 désigne une liaison simple ou une liaison double,
- X₁ et X₂ représentent, indépendamment l'un de l'autre, un atome d'oxygène ou de soufre, et notamment un atome d'oxygène,
- R₁ représente un atome d'oxygène, un atome de soufre ou un groupe N-OR₁₁ ou N-NHCO-NH₂,
- R₂ représente un atome d'hydrogène ou un groupe OR₁₂ ou SR₁₂,
- R₃ représente un atome d'hydrogène, -SO₂R₅₅, -CH₂OCH₂CH₂SiR₆₁R₆₂R₆₃ ou un groupe -CO-(C₁-C₆)alkyle, tel que -COCH₃, ou -CO-(C₂-C₆)alcényle, ledit groupe étant éventuellement substitué par un atome d'halogène ou un groupe COOH, ou - NR₅₆R₅₇, avec par exemple R₅₆ = R₅₇ = H ,
- R₄ représente une chaîne : 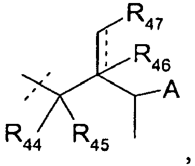 où :
   ■ 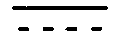 représente une liaison simple ou une liaison double,
   ■ R₄₄ et R₄₅ forment ensemble un groupe =O,
   ■ R₄₆ représente un atome d'hydrogène et R₄₇ représente un atome d'hydrogène, un groupe (C₁-C₆)alcoxy, -NH-OR₄₉ ou un hétérocycle à 5 ou 6 chaînons lié au reste de la molécule par l'intermédiaire d'un atome d'azote, tel qu'un imidazolyle, lorsque 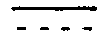 représente une liaison simple, ou R₄₆ est absent et R₄₇ représente un atome d'hydrogène lorsque 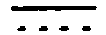 représente une liaison double, et
      ■ A représente un groupe -CHO, -COOH, -CH₂A, ou -CH₂OCH₂A₁ avec :
         ∘ A₁ représentant un atome d'hydrogène, un atome d'halogène, -OH, -OSO₂R₁₃, -N₃, (C₁-C₆)alcoxy éventuellement substitué par un ou plusieurs groupes -OH ; (C₂-C₆)alcénoxy, -OCH₂OR₆₆, -Z₁C(X)R₁₄, -Z₃C(X)Z₄R₁₆, -NH-OR₁₇, -OSiR₁₉R₂₀R₂₁, -OP(O)(OR₂₂)(OR₂₃), -NR₂₄R₂₅, un hétérocycle à 5 ou 6 chaînons ou un résidu de sucre, un ou plusieurs groupes hydroxy dudit résidu de sucre étant éventuellement substitués par un groupe acétyle ou -P(O)(OH)₂,
         o R₄₈ et R₄₉ représentant, indépendamment l'un de l'autre, un atome d'hydrogène, un groupe (C₁-C₆)alkyle, aryle ou aryl-(C₁-C₆)alkyle,
         o R₆₆ représentant un groupe -CO-((C₁-C₆)alkyle) ou (C₁-C₆)alkyle éventuellement substitué par un groupe SiR₆₇R₆₈R₆₉, et
         o R₆₇, R₆₈ et R₆₉ représentant, indépendamment les uns des autres, un groupe (C₁-C₆)alkyle,
- R₅ et R₆ représentent chacun un atome d'hydrogène lorsque -̅-̅-̅-̅ représente une liaison double, ou
   R₅ et R₆ représentent chacun, indépendamment l'un de l'autre, un atome d'hydrogène ou un groupe OR₄₈, tel que OH, ou R₅ et R₆ forment ensemble, avec les atomes de carbone qui les portent, un cycle époxyde, lorsque -̅-̅-̅-̅ représente une liaison simple,
- R₇ représente un atome d'hydrogène ou un groupe OR₄₉, tel que OH,
- R₈ représente un atome d'hydrogène, ou
   R₇ et R₈ forment ensemble, avec les atomes de carbone qui les portent, un cycle époxyde,
- R₉ représente un groupe -CO-(C₁-C₆)alkyle,
- R₁₀ représente un atome d'hydrogène, ou
   R₁₀ et R₃ forment ensemble une liaison, c'est-à-dire que la liaison entre l'atome de carbone 22 et X₂ est une double liaison, avec :
   ■ R₁₁, R₂₆, R₂₈, R₃₀, R₃₁, R₃₆, R₃₇, R₄₁, R₄₂, R₄₃, R₄₈, R₄₉ et R₅₀ représentant, indépendamment les uns des autres, un atome d'hydrogène ou un groupe (C₁-C₆)alkyle, (C₂-C₆)alcényle, aryle ou aryl-(C₁-C₆)alkyle,
   ■ R₁₂ représentant un atome d'hydrogène ou un groupe (C₁-C₆)alkyle ou (C₂-C₆)alcényle, et notamment un atome d'hydrogène,
   ■ R₁₃ et R₅₅ représentent, indépendamment l'un de l'autre, un groupe -OH, (C₁-C₆)alcoxy, aryle, -NR₃₀R₃₁, (C₁-C₆)alkyl-aryle, ou un groupe (C₁-C₆)alkyle éventuellement substitué par un groupe -NR₃₀R₃₁,
   ■ R₁₄ représentant un groupe (C₁-C₆)alkyle, (C₂-C₆)alcényle, aryle, (C₁-C₆)alkyl-aryle ou aryl-(C₁-C₆)alkyle, ledit groupe étant éventuellement substitué par un groupe choisi parmi un atome d'halogène, un groupe -NR₃₂-[(CH₂)ₐ-NR₃₃]_{d}-[(CH₂)_{b}-NR₃₄-(CH₂)_{c}-NR₃₅]e-R₅₂, -P(O)(OH)₂ ou -COOH, avec a, b et c représentant un nombre entier compris entre 1 et 5 et d et e représentant chacun 0 ou 1,
   ■ R₁₅ et R₁₆ représentant, indépendamment l'un de l'autre, un atome d'hydrogène ou un groupe (C₁-C₆)alkyle, (C₂-C₆)alcényle, aryle, (C₁-C₆)alkyl-aryle ou aryl-(C₁-C₆)alkyle, ledit groupe étant éventuellement substitué par un groupe choisi parmi un atome d'halogène, un groupe -NR³²-[(CH₂)ₐ-NR₃₃]_{d}-[(CH₂)_{b}-NR₃₄₋(CH₂)_{c}-N-R₃₅]ₑ-R₅₂ ou -COOH, avec a, b, c, d et e tels que définis ci-dessus,
   ■ R₁₇ et R₁₈ représentant, indépendamment l'un de l'autre, un atome d'hydrogène ou un groupe (C₁-C₆)alkyle, (C₂-C₆)alcényle, aryle ou aryl-(C₁-C₆)alkyle,
   ■ R₁₉, R₂₀, R_{2]}, R₅₈, R₅₉, R₆₀, R₆₁, R₆₂ et R₆₃ représentant, indépendamment les uns des autres, un groupe (C₁-C₆)alkyle, (C₂-C₆)alcényle ou aryle,
   ■ R₂₂ et R₂₃, identiques ou différents, et notamment identiques, représentant un atome d'hydrogène ou un groupe (C₁-C₆)alkyle ou (C₂-C₆)alcényle, ledit groupe étant éventuellement substitué par un groupe -OC(O)-(C₁-C₆)alkyle, NR₃₆R₃₇ et - N⁺R₃₈R₃₉R₄₀,
      ou éventuellement R₂₂ et R₂₃ formant ensemble, avec les atomes d'oxygène qui les portent et l'atome de phosphore, un cycle, notamment à 5 ou 6 chaînons,
   ■ R₂₄ et R₂₅, représentant, indépendamment l'un de l'autre, un atome d'hydrogène ou un groupe -CO-(C₁-C₆)alkyle, -CO-(C₂-C₆)alcényle, (C₂-C₆)alcényle, (C₃-C₇)cycloalkyle ou (C₁-C₆)alkyle éventuellement substitué par un groupe NR₄₁R₄₂, ou
      R₂₄ et R₂₅ formant ensemble, avec l'atome d'azote qui les porte, un hétérocycle à 5 ou 6 chaînons, ledit hétérocycle pouvant comporter, en plus de l'atome d'azote portant R₂₄ et R₂₅, un ou plusieurs hétéroatomes choisis parmi l'atome d'azote, d'oxygène et de soufre, et étant éventuellement substitué par un groupe (C₁-C₆)alkyle,
   ■ R₂₇ représentant un groupe aryle, (C₁-C₆)alkyle ou (C₂-C₆)alcényle, par exemple (C₁-C₆)alkyle ou (C₂-C₆)alcényle, ledit groupe étant éventuellement substitué par un ou plusieurs atomes d'halogène,
   ■ R₂₉ représentant un groupe (C₁-C₆)alkyle, (C₂-C₆)alcényle, aryle ou aryl-(C₁-C₆)alkyle,
   ■ R₃₂, R₃₃, R₃₄, R₃₅, R₅₂, R₅₃, R₅₄, R₅₆ et R₅₇ représentant, indépendamment les uns des autres, un atome d'hydrogène ou un groupe (C₁-C₆)alkyle, (C₂-C₆)alcényle, - CO-(C₁-C₆)alkyle, -CO-(C₂-C₆)alcényle, -CO₂-(C₁-C₆)alkyle ou -CO₂-(C₂-C₆)alcényle, par exemple R₅₂ représentant un atome d'hydrogène),
   ■ R₃₈, R₃₉ et R₄₀ représentant, indépendamment les uns des autres, un groupe (C₁-C₆)alkyle ou (C₂-C₆)alcényle,
   ■ X représentant O, S ou NR₅₀, et notamment O,
   ■ Z₁, Z₂, Z₃ et Z₄ représentant, indépendamment les uns des autres, O ou NR₄₃, ou Z₂R₁₅ et/ou Z₄R₁₆ représentant, indépendamment l'un de l'autre, un hétérocycle à 5 ou 6 chaînons éventuellement substitué par un groupe (C₁-C₆)alkyle, l'hétérocycle comportant au moins un atome d'azote par lequel il est lié au reste de la molécule.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Par « atome d'halogène », on entend, au sens de la présente invention, les atomes de fluor, de chlore, de brome et d'iode.

Par « atomes de carbone non consécutifs », on entend, au sens de la présente invention, des atomes de carbone qui ne sont pas liés les uns aux autres.

Par groupement « (C₁-C₆)alkyle », on entend, au sens de la présente invention, une chaîne hydrocarbonée saturée, linéaire ou ramifiée, comportant 1 à 6, en particulier 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par groupement « (C₂-C₆)alcényle », on entend, au sens de la présente invention, une chaîne hydrocarbonée, linéaire ou ramifiée, comportant au moins une double liaison et comportant 2 à 6 atomes de carbone. A titre d'exemple, on peut citer les groupes éthényle ou allyle.

Par groupement « (C₁-C₆)alcoxy », on entend, au sens de la présente invention, un groupe (C₁-C₆)alkyle, tel que défini ci-dessus, lié à la molécule par l'intermédiaire d'un atome d'oxygène. A titre d'exemple, on peut citer les groupes méthoxy, éthoxy, propoxy, isopropoxy, butoxy ou encore tert-butoxy.

Par groupement « (C₂-C₆)alcénoxy », on entend, au sens de la présente invention, un groupe (C₂-C₆)alcényle, tel que défini ci-dessus, lié au reste de la molécule par l'intermédiaire d'un atome d'oxygène. A titre d'exemple, on peut citer le groupe -OCH₂CH=CH₂.

Par groupement « (C₃-C₇)cycloalkyle », on entend, au sens de la présente invention, une chaîne hydrocarbonée saturée cyclique, comportant 3 à 7 atomes de carbone cycliques. A titre d'exemple, on peut citer les groupes cyclopropyle, cyclopentyle, cyclohexyle et cycloheptyle.

Par « aryle », on entend, au sens de la présente invention, un groupement aromatique, comportant notamment de 5 à 10 atomes de carbone, et comprenant un où plusieurs cycles accolés, comme par exemple un groupement phényle ou naphtyle. Avantageusement, il s'agit du phényle.

Par « aryl-(C₁-C₆)alkyle » ou « aralkyle », on entend, au sens de la présente invention, un groupe aryle, tel que défini ci-dessus, lié à la molécule par l'intermédiaire d'une chaîne (C₁-C₆)alkyle, telle que définie ci-dessus. A titre d'exemple, on peut citer le groupe benzyle.

Par « (C₁-C₆)alkyl-aryle », on entend, au sens de la présente invention, un groupe (C₁-C₆)alkyle, tel que défini ci-dessus, lié à la molécule par l'intermédiaire d'un groupe aryle, telle que défini ci-dessus. A titre d'exemple, on peut citer le groupe méthyl-phényle encore appelé tolyle.

Par « hétérocycle à 5 ou 6 chaînons », on entend, au sens de la présente invention, un cycle à 5 ou 6 chaînons, saturé, insaturé ou aromatique, et contenant un ou plusieurs, avantageusement 1 à 4, encore plus avantageusement 1 ou 2, hétéroatomes, tels que par exemple des atomes de soufre, azote ou oxygène. Il peut s'agir notamment du groupe pyrrolidinyle, pipéridinyle, pipérazinyle, morpholinyle, dihydrofuranyle, tétrahydrofuranyle, imidazolyle ou triazolyle.

Par «polycycle à 10 à 15 chaînons », on entend, au sens de la présente invention, un système polycyclique hydrocarboné comprenant au moins 2, par exemple 2 ou 3, cycles accolés, chaque cycle pouvant être saturé, insaturé ou aromatique et contenir éventuellement un ou plusieurs, par exemple 0 ou 1, hétéroatome tel qu'un atome de soufre, d'azote ou d'oxygène. Il s'agira notamment d'un système bi- ou tricyclique associant des cycles à 5, 6 ou 7 chaînons comprenant notamment une fonction acétale. Avantageusement, le polycycle aura la structure suivante : 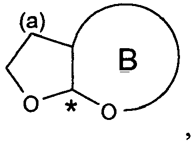 où B représente un système mono- ou bi-cyclique saturé, insaturé ou aromatique comprenant éventuellement un ou plusieurs, avantageusement 0 ou 1, hétéroatome supplémentaire notamment choisi parmi O, S et N. La liaison avec le reste de la molécule se fait alors au niveau du carbone marqué par une étoile (*) et le carbone marqué d'un (a) étant avantageusement substitué par un groupe méthyle. D'autres substitutions peuvent bien évidemment être envisagées comme indiqué ci-dessus. Ainsi, il pourra s'agir notamment des cycles suivants :

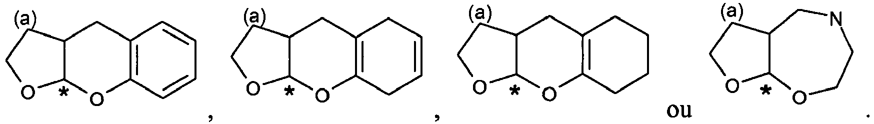

Par « époxyde », on entend, au sens de la présente invention, un cycle

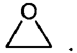

Par « sucre », on entend notamment, au sens de la présente invention, l'érythrose, le thréose, le ribose, l'arabinose, le xylose, le lyxose, l'allose, l'altrose, le glucose, le mannose, le gulose, l'idose, le galactose, le talose, l'érythrulose, le ribulose, le xylulose, le psicose, le fructose, le sorbose, le sophrose, ou encore le tagatose, sous forme D ou L.

Par « résidu de sucre », on entend, au sens de la présente invention, qu'une molécule de sucre, par exemple substituée ou non sustituée, telle que définie ci-dessus, est liée au reste de la molécule par l'intermédiaire de l'atome d'oxygène situé en position anomérique, et éventuellement par l'intermédiaire d'un second atome d'oxygène.

Par « inositol », on entend, au sens de la présente invention, un cycle hydrocarboné saturé à 6 chaînons substitué sur chaque atome de carbone par un groupe OH.

Par « résidu d'inositol », on entend, au sens de la présente invention, qu'une molécule d'inositol, telle que définie ci-dessus, est liée au reste de la molécule par l'intermédiaire de l'un de ses atomes d'oxygène.

En particulier, R₃ représente un atome d'hydrogène, ou un groupe -CO-(C₁-C₆)alkyle, tel que -COCH₃, ou -CO-(C₂-C₆)alcényle, ledit groupe étant éventuellement substitué par un groupe NH₂

Le groupement méthyle situé en position (4) peut par exemple être situé du même côté du cycle à 6 chaînons que l'atome d'hydrogène voisin.

Par ailleurs, le carbone (20) est par exemple de configuration (S).

R₁ est par exemple choisi parmi un atome d'oxygène, un groupe N-OH, -NOMe, -N-OBn et-N-NHCO-NH2_{,} par exemple R₁ est un atome d'oxygène.

R₂ représente par exemple un atome d'hydrogène ou un groupe OR₁₂, et plus particulièrement un atome d'hydrogène.

Avantageusement, R₅ et R₆ représentent chacun un atome d'hydrogène et -̅-̅-̅-̅ représente une liaison double.

R₈ représente, par exemple, un atome d'hydrogène et R₇ et représentera un atome d'hydrogène ou un groupe OH, et notamment un atome d'hydrogène.

R₉ représente un groupe -CO-(C₁-C₆)alkyle, tel qu'un groupe -COCH₃.

Avantageusement, X₂-R₃ représente un groupe -OH ou -OC(O)CH₃ et R₁₀ représente un atome d'hydrogène ou R₃ et R₁₀ forment ensemble une liaison. Notamment, X₂-R₃ représentera un groupe -OH ou -OC(O)CH₃ et R₁₀ représentera un atome d'hydrogène.

Ainsi, les composés de l'invention répondront avantageusement à la formule (Ia) ou (Ib) suivante : 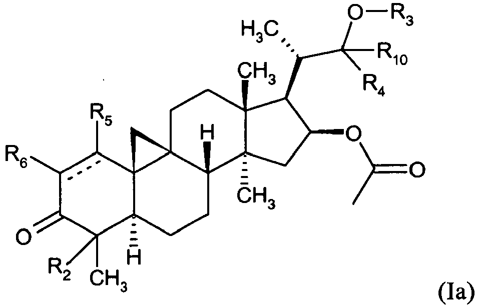 ou 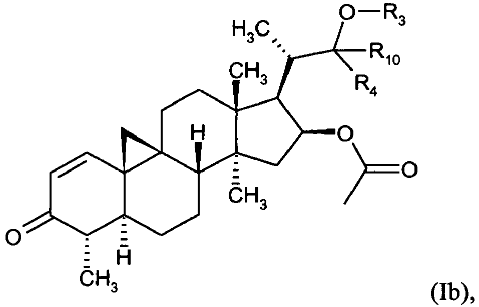 pour lesquelles R₂, R₃, R₄, R₅, R₆ et R₁₀ sont tels que définis ci-dessus.

Selon un premier mode de réalisation particulier de l'invention, R₄ représente, dans la formule (I), (Ia) ou (Ib) précédente, une chaîne : 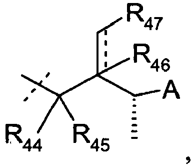 où:
- 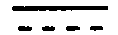 représente une liaison simple ou une liaison double, avec R₄₄ à R₄₇ et A tels que définis précédemment.

En particulier, R₄₆ est absent et R₄₇ représente un atome d'hydrogène et -̅-̅-̅-̅ représente une liaison double.

Par ailleurs, A peut représenter un groupe -CH₂A₁.

Ainsi, R₄ représente avantageusement la chaîne suivante : 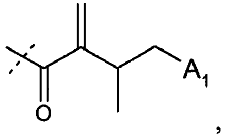 telle qu'une chaîne 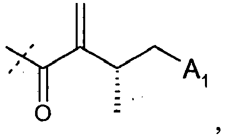 avec A₁ tel que défini ci-dessus.

Avantageusement, A₁ représente un groupe -OH, -OSO₂R₁₃, -N₃, (C₁-C₆)alcoxy, -Z₁C(X)R₁₄, -Z₃C(X)Z₄R₁₆, -OSiR₁₉R₂₀R₂₁, -OP(O)(OR₂₂)(OR₂₃), -NR₂₄R₂₅, un hétérocycle à 5 ou 6 chaînons ou un résidu de sucre.

A₁ peut notamment représenter un groupe :
■ -OH ; (C₁-C₆)alcoxy tel que méthoxy ; -OSiR₁₉R₂₀R₂₁ tel que -OSiMe₃ ou -OSitBuMe₂ ; -OSO₂R₁₃ tel que -OSO₃H, -OSO₂CH₃, -OSO₂-C₆H₄-CH₃ ou - OSO₂NMe₂ ; -OP(O)(OR₂₂)(OR₂₃) tel que -OP(O)O⁻(OCH₂CH₂N⁺Me₃), - OP(O)(OCH₂OC(O)CH₃), -OPO₃H₂, -OP(O)(OEt)₂, ou -OP(O)(OH)(OCH₂CH₂NH₂) ; un résidu de sucre tel qu'un résidu de glucose ;
■ -Z₁C(X)R₁₄, en particulier -OC(O)R₁₄, tel que -OC(O)CH₃, -OC(O)CH₂NMe₂, - OC(O)CH₂NH₂, -OC(O)CH₂Cl, -OC(O)-C₆H₄-COOH, -OC(O)CH₂CH₂COOH, -OC(O)CH₂NHCO₂tBu, -OC(O)CH₂NH(CH₂)₃NH(CH₂)₄NH(CH₂)₃NH₂ ou -OC(O)CH₂NBoc(CH₂)₃NBoc(CH₂)₄NBoc(CH₂)₃NHBoc (avec Boc = -COOtBu) ;
■ -Z₃C(X)Z₄R₁₆, en particulier -OC(O)NHR₁₆ (carbamates) et -OC(O)OR₁₆ (carbonates), tel que -OC(O)NHPh, -OC(O)NHCH₂CH₂NMe₂ ou 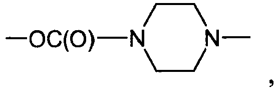 ou -OC(O)-diméthylaniline (groupement de formule - OC(O)C₆H₄-NMe₂) ;
■ -N₃ ; ou -NR₂₄R₂₅ tel que -NMe₂, morpholinyle, N-méthyl-pipérazinyle ou - N(COOtBu)(CH₂CH₂NMe₂).
Ainsi, A₁ pourra être choisi parmi OH, -OCH₃, -OSiMe₃, -OSitBuMe₂, - OSO₃H, -OSO₂CH₃,-OSO₂-C₆H₄-CH₃,-OSO₂NMe₂, -OP(O)O⁻(OCH₂CH₂N⁺Me₃), - OP(O)(OCH₂OC(O)CH₃), -OPO₃H₂, -OP(O)(OEt)₂, -OP(O)(OH)(OCH₂CH₂NH₂), un résidu de glucose, -OC(O)CH₃, -OC(O)CH₂NMe₂, -OC(O)CH₂NH₂, -OC(O)CH₂Cl, - OC(O)-C₆H₄-COOH, -OC(O)CH₂CH₂COOH, -OC(O)CH₂NHCO₂tBu, -OC(O)NHPh, -OC(O)NHCH₂CH₂NMe₂, NMe₂, -OC(O)CH₂NH(CH₂)₃NH(CH₂)₄NH(CH₂)₃NH₂, -OC(O)CH₂NBoc(CH₂)₃NBoc(CH₂)₄NBoc(CH₂)₃NHBoc, N(COOtBu)(CH₂CH₂NMe₂) et 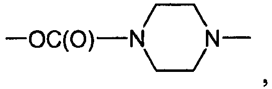 ou encore -OC(O)C₆H₄-NMe₂.

Selon un mode de réalisation particulier de l'invention, les composés de l'invention pourront être des composés de formule (I) ou des sels pharmaceutiquement acceptables de ceux-ci, pour lesquels :
- 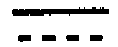 désigne une liaison simple ou une liaison double,
- X₁ et X₂ représentent un atome d'oxygène,
- R₁ représente un atome d'oxygène, ou un groupe -N-OR₁₁ ou -N-NHCO-NH₂, par exemple un atome d'oxygène,
- R₂ représente un atome d'hydrogène ou un groupe -OH, par exemple un atome d'hydrogène,
- R₃ représente un atome d'hydrogène, ou un groupe -SO₃H, -CH₂OCH₂CH₂Si(CH₃)₃, -COCH₃, -C(O)CH₂Cl, -CO(CH₂)₂COOH, -CO(CH₂)NHCOO(C₁-C₆)alkyle tel que -CO(CH₂)NHCOOtBu,
- R₄ représente:
   ■ une chaîne : 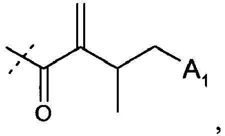 telle qu'une chaîne 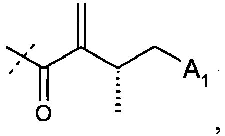 avec A₁ représentant un atome d'hydrogène ou un groupe -OH ; -COOH, (C₁-C₆)alcoxy tel que méthoxy ; -OCH₂CH=CH₂ ; - OCH₂CH(OH)CH₂OH; -OCH₂OCOCH₃ ; -OSiMe₃, - OCH₂OCH₂CH₂SiMe₃; -OSitBuMe₂ , -OSO₃H ; -OSO₂CH₃; -OSO₂-C₆H₄-CH₃; -OSO₂NMe₂; -OP(O)O⁻(OCH₂CH₂N⁺Me³); - OP(O)(OCH₂OC(O)CH₃)₂ ; -OPO₃H₂ ; -OP(O)(OEt)₂ ; -OP(O)(OH)(OCH₂CH₂NH₂) ; un résidu de sucre tel qu'un résidu de glucose; -OC(O)CH₃ ; -OC(O)CH₂NMe₂ ; -OC(O)CH₂NH₂ ; -OC(O)CH₂Cl ; -OC(O)-C₆H₄-COOH ; -OC(O)CH₂CH₂COOH ; -OC(O)CH₂NHCO₂tBu ; -OC(O)(CH₂)₂N(C₂H₅)₂; -OC(O)CH=CH₂ ; - OC(O)CH₂NH(CH₂)₃NH(CH₂)₄NH(CH₂)₃NH₂ ; -OC(O)CH₂NBoc(CH₂)₃NBoc(CH₂)₄NBoc(CH₂)₃NHBoc (avec Boc = - COOtBu), -OC(O)CH₂OPO₃H₂; -OC(O)NHPh ; - OC(O)NHCH₂CH₂NMe₂ ; 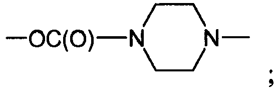 -OC(O)-diméthylaniline (-OC(O)-C₆H₄-NMe₂) ; NMe₂, morpholinyle, -*N*-méthyl- pipérazinyle ou -N(COO*t*Bu)(CH₂CH₂NMe₂),
- R₅ et R₆ représentent chacun un atome d'hydrogène lorsque 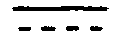 représente une liaison double, ou
   R₅ et R₆ représentent chacun, indépendamment l'un de l'autre, un atome d'hydrogène ou un groupe -OH, ou R₅ et R₆ forment ensemble, avec les atomes de carbone qui les portent, un cycle époxyde, lorsque 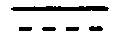 représente une liaison simple,
- R₇ représente un atome d'hydrogène ou un groupe -OH,
- R₈ représente un atome d'hydrogène, ou
   R₇ et R₈ forment ensemble, avec les atomes de carbone, qui les portent un cycle époxyde,
- R₉ représente un groupe -CO-(C₁-C₆)alkyle, par exemple -CO-CH₃,
- R₁₀ représente un atome d'hydrogène, ou
   R₁₀ et R₃ forment ensemble une liaison, la liaison entre C₂₂ et X₂ est alors une double liaison,
   avec :
   R₁₁ représentant un atome d'hydrogène, un groupe (C₁-C₆)alkyle, par exemple -CH₃, aryle ou aryl-(C₁-C₆)alkyle, par exemple benzyle.

Selon un autre mode de réalisation particulier de l'invention, les composés de l'invention pourront être des composés de formule (I) ou des sels pharmaceutiquement acceptables de ceux-ci, pour lesquels :
- 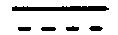 représente une liaison double ou une liaison simple,
- X₁ et X₂ représentent chacun un atome d'oxygène,
- R₁ représente un atome d'oxygène, un groupe N-OH, N-O-(C₁-C₆)alkyle tel que N-OCH₃, N-O-(C₁-C₆)alkyl-aryle tel que N-OBn, ou N-NHCO-NH₂,
- R₂ représente un atome d'hydrogène,
- R₃ représente un atome d'hydrogène ou un groupe CO-(C₁-C₆)alkyle tel que CO-CH₃,
- R₄ représente un groupe choisi parmi :
   ■ 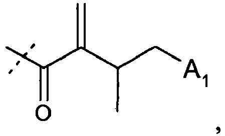 tel que 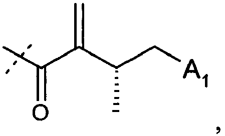 où A₁ représente un groupe -OH ; (C₁-C₆)alcoxy tel que méthoxy ; -OSiMe₃ ; - OSitBuMe₂ ; -OSO₃H ; OSO₂CH₃; -OSO₂-C₆H₄-CH₃; -OSO₂NMe₂; - OP(O)O⁻(OCH₂CH₂N⁺Me₃); -OP(O)(OCH₂OC(O)CH₃); -OPO₃H₂; - OP(O)(OEt)₂; -OP(O)(OH)(OCH₂CH₂NH₂); un résidu de sucre tel qu'un résidu de glucose; -OC(O)CH₃; -OC(O)CH₂NMe₂₃; -OC(O)CH₂NH₂; - OC(O)CH₂Cl; -OC(O)-C₆H₄-COOH; -OC(O)NHPh; - OC(O)NHCH₂CH₂NMe₂; -OC(O)CH₂CH₂COOH; -OC(0)CH₂NHCO₂tBu; - NMe₂; -N(COOtBu)(CH₂CH₂NMe₂; - OC(O)CH₂NH(CH₂)₃NH(CH₂)₄NH(CH₂)₃NH₂; - OC(O)CH₂NBoc(CH₂)₃NBoc(CH₂)₄NBoc(CH₂)₃NHBoc (avec Boc = COOtBu) ; ou 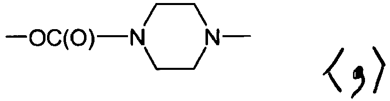
- R₅ et R₆ représentent chacun un atome d'hydrogène lorsque 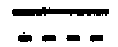 représente une liaison double, ou
   R₅ et R₆ représentent, indépendamment l'un de l'autre, un atome d'hydrogène ou un groupe OH, ou forment ensemble, avec les atomes de carbone qui les portent, un cycle époxyde, lorsque 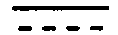 représente une liaison simple,
- R₇ représente un atome d'hydrogène ou un groupe OH et R₈ représente un atome d'hydrogène, ou
   R₇ et R₈ forment ensemble, avec les atomes de carbone qui les portent, un cycle époxyde,
- R₉ représente un groupe CO-(C₁-C₆)alkyle tel que CO-CH₃, et
- R₁₀ représente un atome d'hydrogène, ou
   R₁₀ et R₃ forment ensemble une liaison.

En particulier, le composé de formule (I) de l'invention pourra être choisi parmi les composés 1-4, 6-8, 11-31, 35-39, 41, 43-46, 49, 52-76, 78-74 et 89-92 tels qu'exemplifiés dans les exemples de l'invention qui suivent.

La présente invention a également pour objet un composé de formule (I) tel que défini ci-dessus ou un sel pharmaceutiquement acceptable de celui-ci pour son utilisation en tant que médicament, destiné notamment au traitement d'une maladie proliférative telle qu'un cancer (notamment par inhibition du protéasome cellulaire).

La présente invention concerne donc également l'utilisation d'un composé de formule (I) tel que défini ci-dessus ou un sel pharmaceutiquement acceptable de celui-ci pour la fabrication d'un médicament, destiné notamment au traitement d'une maladie proliférative telle qu'un cancer.

La présente invention a également pour objet une composition pharmaceutique comprenant au moins un composé de formule (I) tel que défini ci-dessus ou un sel pharmaceutiquement acceptable de celui-ci et au moins un véhicule pharmaceutiquement acceptable.

Les compositions pharmaceutiques selon l'invention peuvent être formulées pour une administration orale, sublinguale, sous-cutanée, intramusculaire, intraveineuse, transdermique, locale ou rectale, destinée aux mammifères, y compris l'homme.

L'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les composés de l'invention en tant que principes actifs peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

La composition pharmaceutique selon l'invention peut comprendre en outre au moins un autre principe actif tel qu'un agent anticancéreux.

La présente invention a également pour objet une composition pharmaceutique comprenant :
(i) au moins un composé de formule (I) tel que défini ci-dessus ou un sel pharmaceutiquement acceptable de celui-ci, et
(ii) au moins un autre principe actif, tel qu'un agent anticancéreux,
en tant que produits de combinaison pour une utilisation simultanée, séparée ou étalée dans le temps.

La présente invention a également pour objet une composition pharmaceutique selon l'invention telle que définie précédemment pour son utilisation en tant que médicament, destiné notamment au traitement d'une maladie proliférative telle qu'un cancer.

Les composés de l'invention ont été préparés par extraction à partir des feuilles du genre Neoboutonia, et en particulier de l'espèce *Neoboutonia melleri* (Euphorbiaceae), plante originaire du Cameroun, ou par hémisynthèse, selon des réactions de fonctionnalisation bien connues de l'homme du métier, à partir des deux composés suivants obtenus par extraction comme décrit ultérieurement, le second composé ayant été nommé neoboutomellerone : 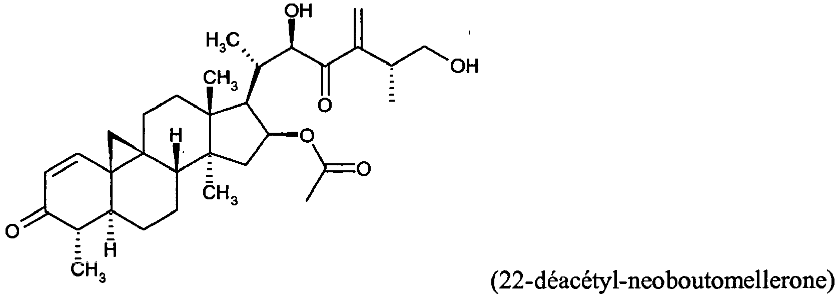 et

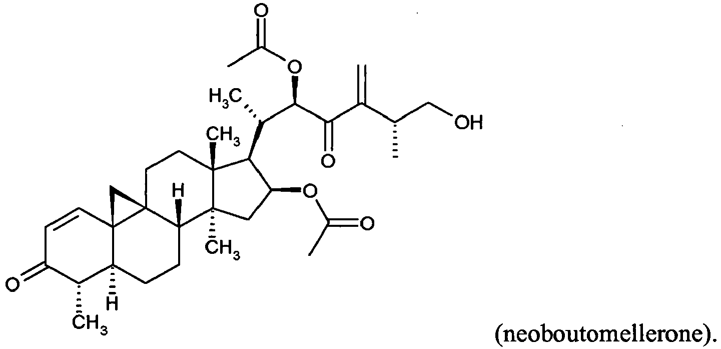

La présente invention sera mieux comprise à la lumière des exemples non limitatifs qui suivent.

### EXEMPLES :

Les abréviations suivantes ont été utilisées dans les exemples qui suivent :

| | |
|---|---|
| ACN | Acétonitrile |
| Boc | *tert*-Butyloxycarbonyle |
| CCM | Chromatographie sur Couche Mince |
| DBU | 1,8-Diazabicyclo[5.4.0]undéc-7-ène |
| DCC | Dicyclohexylcarbodiimide |
| DCM | Dichlorométhane |
| DIAD | Diisopropylazodicarboxylate |
| DMAP | Diméthylaminopyridine |
| DMF | Diméthylformamide |
| DMSO | Diméthylsulfoxyde |
| EDC | 1-Ethyl-3-(3-diméthylaminopropyl)carbodiimide |
| Gly | Glycine |
| HPLC | Chromatographie Liquide Haute Pérformance |
| NMG | N-méthyl-D-glucamine |
| Rdt | Rendement |
| Rf | Rapport frontal |
| RMN | Résonance Magnétique Nucléaire |
| rt | Température ambiante |
| TBAF | Fluorure de tétra-n-butylammonium |
| TBDMS | *tert*-Butyldiméthylsilyle |
| TBTU | Tétrafluoroborate de O-benzotriazole-1-yl-N,N,N',N'-tétraméthyl uronium |
| Tf | Triflate |
| THF | Tétrahydro furane |

### 1. Obtention des composés de l'invention

### 1.1. Par extraction à partir de Neoboutonia melleri

1 kg de feuilles de *Neoboutonia melleri* (Euphorbiaceae) est extrait par 15 litres de dichlorométhane à température ambiante pendant 24 heures. Après filtration, le marc de plante est à nouveau extrait avec du dichorométhane dans les mêmes conditions. Les filtrats sont réunis et amenés à sec sous pression réduite à l'aide d'un évaporateur rotatif. L'extrait dichlorométhanique obtenu (45 g, R=4.5%) est ensuite repris dans 1 litre de dichlorométhane auquel est rajouté 100 g de charbon actif. La solution ainsi obtenue est mise 1 heure sous agitation puis filtrée. Le filtrat est ensuite évaporé à sec sous pression réduite (32 g). Cet extrait déchlorophyllé est partitionné par liquide/liquide avec du méthanol et du cyclohexane avec un peu d'eau pour obtenir 2 phases non miscibles. Les 2 phases obtenues sont évaporées à sec : extrait méthanolique EM (15 g) et extrait hexanique EH (17g). Seul l'extrait EM sera utilisé pour l'isolement des cycloartanes. L'EM est purifié dans un premier temps par Chromatographie Liquide Moyenne Pression (CLMP) sur silice. Une colonne de 600 g est utilisée et éluée avec un mélange isocratique acétate d'éthyle/cyclohexane 50/50. Après analyse des fractions obtenues par Chromatographie sur Couche Mince (CCM) (éluant dichlorométhane/méthanol 97/3), 4 fractions sont obtenues : EM1 (6 g), EM2 (5.5 g), EM3 (1.5 g) et EM4 (1 g).

La fraction la moins polaire (EM1) est purifiée par chromatographie liquide haute performance (HPLC) préparative sur phase inverse. Un mélange eau/acétonitrile est utilisé comme phase éluante avec un gradient linéaire de 20/80 à 100% d'acétonitrile. On obtient dans l'ordre d'élution 7 (10 mg, 0.001% poids sec), 18 (0.1 mg, 0.00001%), 10 (0.4 mg, 0.00004%), 9 (6 mg, 0.0006%), 8 (7 mg, 0.0007%), 5 (3.5 mg, 0.000351%).

Les fractions de polarité moyenne (EM2 et EM3) sont aussi purifiées par HPLC préparative sur C18 en utilisant un gradient linéaire eau/acétonitrile 45/55 à 100%. On obtient à partir de la fraction EM2 l'un des cycloartanes majoritaires 2 (2.5 g, 0.25%) et 14 (1.5 mg, 0.00015%), 11 (10.5 mg, 0.00105%). A partir de EM3, le deuxième cycloartane majoritaire 1 (600 mg, 0.06%) est purifié, ainsi que les produits 12 (2 mg, 0.0002%) et 3 (15 mg, 0.0015%).

Enfin la fraction la plus polaire (EM4), a permis d'obtenir après purification par HPLC préparative (gradient eau/acétonitrile 50/50 à 100%) : 15 (0.5 mg, 0.00005%), 6 (1 mg, 0.0001%), 16 (0.5 mg, 0.00005%), 4 (17 mg, 0.0017%) et 13 (0.2 mg, 0.00002%).

Les produits ainsi obtenus sont décrits ci-dessous

### ◆exemple 1 : 22-déacétyl- neoboutomellerone

| | |
|---|---|
| 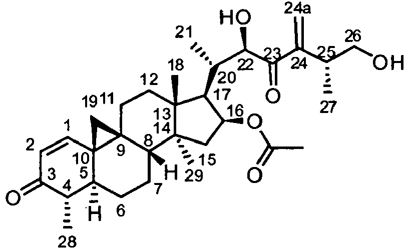 | Formule Chimique : C₃₂H₄₆O₆ |
| | Masse Exacte : 526,33 |
| | Poids moléculaire : 526,70 |

¹H RMN (500MHz, CD₃CN) δ = 6.94 (1H, d, J = 10.1 Hz, H-1), 6.12 (1H, s, H-24a'), 5.99 (1H, d, J = 0.9 Hz, H-24a"), 5.89 (1H, d, J = 9.8 Hz, H-2), 5.20 (1H, td, J = 7.5 Hz, J = 4.6 Hz, H-16), 4.72 (1H, dd, J = 6.1 Hz, J = 1.8 Hz, H-22), 3.54 (1H, d, J = 6.1 Hz, OH-22), 3.56 (1H, dt, J = 10.6 Hz, J = 6.0 Hz, H-26'), 3.41 (1H, dt, J = 10.6 Hz, J = 6.0 Hz, H-26"), 2.83 (1H, sxt, J = 6.6 Hz, H-25), 2.67 (1H, t, J = 5.6 Hz, OH-26), 2.46 (1H, dqd, J = 10.7 Hz, J = 6.7 Hz, J = 2.1 Hz, H-20), 2.42 (1H, dd, J = 10.7 Hz, J = 7.0 Hz, H-17), 2.22 (1H, dd, J = 13.9 Hz, J = 8.1 Hz, H-15'), 2.17 (1H, dq, J = 12.8 Hz, J = 6.7 Hz, H-4), 2.03 (3H, s, H-16b), 1.98 - 2.07 (2H, m, H-8, 11'), 1.95 - 1.98 (1H, m, H-5), 1.60 - 1.74 (3H, m, H-6', 12', 12"), 1.50 - 1.59 (1H, m, H-11"), 1.41 - 1.50 (1H, m, H-7'), 1.38 (1H, dd, J = 14.0 Hz, J = 4.0 Hz, H-15"), 1.24 (1H, d, J = 4.3 Hz, H-19'), 1.15 - 1.26 (4H, m, H-7"), 1.17 (3H, s, H-18), 1.05 (3H, d, J = 7.0 Hz, H-27), 1.03 (3H, d, J = 6.7 Hz, H-28), 0.96 (3H, s, H-29), 0.94 (3H, qd, J = 12.5 Hz, J = 3.7 Hz, H-6"), 0.64 (3H, d, J = 6.1 Hz, H-21), 0.57 (1H, d, H-19")
¹³C RMN (126MHz, CD₃CN) δ = 205.6 (C-23), 202.4 (C-3), 171.3 (C-16a), 155.6 (C-1), 149.1 (C-24), 128.4 (C-2), 126.5 (C-24a), 77.2 (C-16), 75.8 (C-22), 66.6 (C-26), 51.4 (C-17), 48.3 (C-14), 47.6 (C-4), 46.9 (C-15), 46.7 (C-13), 45.3 (C-8), 43.6 (C-5), 37.6 (C-25), 36.3 (C-20), 33.1 (C-12), 32.9 (C-10), 28.1 (C-11), 27.7 (C-19), 27.2 (C-9), 24.3 (C-6), 24.3 (C-7), 22.1 (C-16b), 20.1 (C-29), 18.5 (C-21), 17.2 (C-27), 12.3 (C-21), 11.3 (C-28)

### ◆ exemple 2 : neoboutomellerone

| | |
|---|---|
| 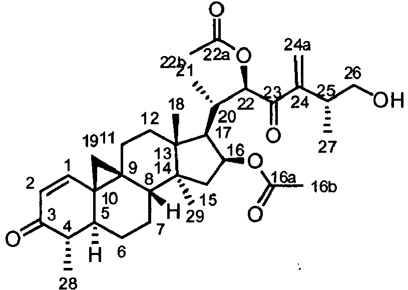 | Formule Chimique : C₃₄H₄₈O₇ |
| | Masse Exacte : 568,34 |
| | Poids moléculaire : 568,74 |

¹H RMN (500MHz, CD₃CN) δ = 6.94 (1H, d, J = 10.1 Hz, H-1), 6.04 (1H, s, H-24a'), 5.89 (1H, d, J = 1.2 Hz, H-24a"), 5.90 (1H, d, J = 9.8 Hz, H-2), 5.53 (1H, d, J = 2.4 Hz, H-22), 5.09 (1H, td, J = 7.7 Hz, J = 4.4 Hz, H-16), 3.53 (1H, dt, J = 10.8 Hz, J = 5.9 Hz, H-26'), 3.39 (1H, dt, J = 10.5 Hz, J = 6.1 Hz, H-26"), 2.77 (1H, sxt, J = 6.7 Hz, H-25), 2.68 (1H, t, J = 5.8 Hz, OH-26), 2.54 - 2.65 (1H, dqd, J = 11.1 Hz, J = 6.9 Hz, J = 2.3 Hz, H-20), 2.29 (1H, dd, J = 11.0 Hz, J = 7.6 Hz, H-17), 2.14 - 2.22 (2H, m, H-4, 15'), 2.09 (3H, s, H-22b), 2.03 (3H, s, H-16b), 1.98 - 2.05 (2H, m, H-8, 11'), 1.95 - 1.98 (1H, m, H-5), 1.63 - 1.77 (3H, m, H-6', 12', 12"), 1.51 - 1.61 (1H, m, H-11 "), 1.40 - 1.50 (1H, m, H-7'), 1.33 - 1.40 (1H, ddq, J = 14.0 Hz, J = 4.6 Hz, J = 0.9 Hz, H-15"), 1.24 (1H, d, J = 4.3 Hz, H-19'), 1.18 (3H, s, H-18), 1.14 - 1.23 (1H, m, H-7"), 1.03 (3H, d, J = 7.0 Hz, H-27), 1.02 (3H, d, H-28), 0.95 (3H, d, J = 0.6 Hz, H-29), 0.88 - 0.99 (1H, m, H-6"), 0.85 (3H, d, J = 7.0 Hz, H-21), 0.58 (1H, d, J = 4.6 Hz, H-19")
¹³C RMN (126MHz, CD₃CN) δ = 202.4 (C-3), 199.6 (C-23), 171.7 (C-22a), 171.3 (C-16a), 155.5 (C-1), 150.3 (C-24), 128.4 (C-2), 124.6 (C-24a), 78.5 (C-22), 76.6 (C-16), 66.4 (C-26), 51.3 (C-17), 48.4 (C-14), 47.6 (C-4), 46.8 (C-13), 46.7 (C-15), 45.2 (C-8), 43.6 (C-5), 37.9 (C-25), 33.2 (C-20), 33.0 (C-12), 32.9 (C-10), 28.1 (C-11), 27.7 (C-19), 27.2 (C-9), 24.3 (C-7, 6), 22.1 (C-16b), 20.9 (C-22b), 20.0 (C-29), 18.2 (C-18), 17.2 (C-27), 13.3 (C-21), 11.3 (C-28)

### ◆ exemple 3: Diastéréoisomère du cycle A du 22-déacétyl-neoboutomellerone

| | |
|---|---|
| 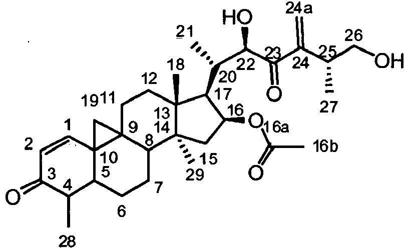 | Formule Chimique : C₃₂H₄₆O₆ |
| | Masse Exacte : 526,33 |
| | Poids moléculaire : 526,70 |

¹H RMN (500MHz, CD₃CN) δ = 6.49 (1H, d, J = 10.1 Hz, H-1), 6.13 (1H, s, H-24a'), 5.98 (1H, d, J = 0.9 Hz, H-24a"), 5.87 (1H, d, J = 10.1 Hz, H-2), 5.24 (1H, td, J = 7.4 Hz, J = 5.0 Hz, H-16), 4.72 (1H, br. s., H-22), 3.55 (1H, br. s., OH-22), 3.55 (1H, dd, J = 10.4 Hz, J = 6.7 Hz, H-26'), 3.41 (1H, dd, J = 10.5 Hz, J = 6.6 Hz, H-26"), 2.83 (1H, sxt, J = 6.6 Hz, H-25), 2.69 (1H, br. s., OH-26), 2.42 - 2.51 (1H, dqd, J = 11.0 Hz, J = 6.4 Hz, J = 2.1 Hz, H-20), 2.43 (1H, dd, J = 11.0 Hz, J = 7.3 Hz, H-17), 2.07 - 2.26 (4H, m, H-4, 8, 11', 15'), 2.04 (3H, s, H-16b), 1.98 - 2.07 (1H, m, H-6'), 1.91 (1H, m, H-5), 1.81 (1H, td, J = 13.3 Hz, J = 4.9 Hz, H-12'), 1.59 (1H, ddd, J = 13.1 Hz, J = 5.3 Hz, J = 1.7 Hz, H-12"), 1.33 - 1.44 (2H, m, H-11", 15"), 1.31 (1H, d, J = 4.9 Hz, H-19'), 1.21 - 1.30 (2H, m, H-7', 7"), 1.12 (3H, s, H-18), 1.09 - 1.19 (1H, m, H-6"), 1.04 (3H, d, J = 6.7 Hz, H-28), 1.04 (3H, d, J = 7.3 Hz, H-27), 0.94 (3H, d, J = 0.9 Hz, H-29), 0.91 (1H, d, J = 5.2 Hz, H-19"), 0.65 (3H, d, J = 6.4 Hz, H-21)
¹³C RMN (126MHz, CD₃CN) δ = 205.6 (C-23), 201.0 (C-3), 171.3 (C-16a), 157.3 (C-1), 149.1 (C-24), 126.5 (C-2), 126.5 (C-24a), 77.2 (C-16), 76.0 (C-22), 66.6 (C-26), 50.6 (C-17), 48.8 (C-14), 48.5 (C-4), 46.8 (C-13), 44.7 (C-15), 41.5 (C-8), 40.4 (C-5), 37.6 (C-25), 36.4 (C-20), 35.0 (C-10), 33.0 (C-12), 31.9 (C-6), 31.6 (C-19), 30.4 (C-11), 27.7 (C-9), 22.1 (C-16b), 21.3 (C-7), 19.1 (C-29), 17.2 (C-27), 15.6 (C-18), 12.7 (C-21), 12.6 (C-28)

### ◆ exemnle 4 : Diastéréoisomère du cycle A du neoboutomellerone

| | |
|---|---|
| 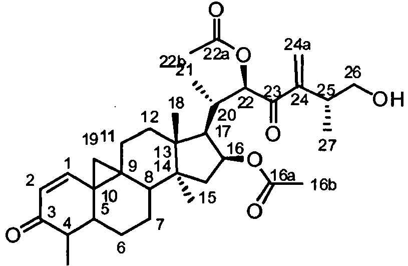 | Formule Chimique : C₃₄H₄₈O₇ |
| | Masse Exacte : 568,34 |
| | Poids moléculaire : 568,74 |

¹H RMN (500MHz, CD₃CN) δ = 6.49 (1H, d, J = 10.1 Hz, H-1), 6.04 (1H, s, H-24a'), 5.88 (1H, d, J = 0.9 Hz, H-24a"), 5.88 (1H, d, J = 10.1 Hz, H-2), 5.53 (1H, d, J = 2.1 Hz, H-22), 5.13 (1H, td, J = 7.6 Hz, J = 4.9 Hz, H-16), 3.53 (1H, dt, J = 10.6 Hz, J = 5.8 Hz, H-26'), 3.39 (1H, dt, J = 10.7 Hz, J = 6.1 Hz, H-26"), 2.76 (1H, sxt, J = 6.4 Hz, H-25), 2.72 (1H, t, J = 5.6 Hz, OH-26), 2.56 - 2.66 (1H, dqd, J = 10.9 Hz, J = 7.0 Hz, J = 2.1 Hz, H-20), 2.28 (1H, dd, J = 11.0 Hz, J = 7.6 Hz, H-17), 2.15 - 2.26 (1H, m, H-4), 2.09 (3H, s, H-22b), 2.07 - 2.16 (2H, m, H-11', 15'), 2.04 (3H, s, H-16b), 1.97 - 2.05 (1H, m, H-6'), 1.88 - 1.93 (1H, m, H-5), 1.83 (1H, td, J = 13.3 Hz, J = 4.9 Hz, H-12'), 1.63 (1H, ddd, J = 13.1 Hz, J = 5.2 Hz, J = 1.5 Hz, H-12"), 1.41 (1H, ddd, J = 14.3 Hz, J = 4.9 Hz, J = 1.8 Hz, H-11"), 1.35 (1H, dd, J = 13.4 Hz, J = 4.3 Hz, H-15"), 1.30 (1H, d, J = 5.2 Hz, H-19'), 1.20 - 1.29 (1H, m, H-7'), 1.10 - 1.18 (2H, m, H-6", 7"), 1.13 (3H, s, H-18), 1.04 (3H, d, J = 6.4 Hz, H-28), 1.02 (3H, d, J = 7.0 Hz, H-27), 0.92 (3H, s, H-29), 0.88 - 0.96 (1H, m, H-19), 0.85 (3H, d, J = 7.0 Hz, H-21)
¹³C RMN (126MHz, CD₃CN) δ = 200.9 (C-3), 199.6 (C-23), 171.6 (C-22a), 171.2 (C-16a), 157.2 (C-1), 150.3 (C-24), 126.5 (C-2), 124.6 (C-24a), 78.6 (C-22), 76.6 (C-16), 66.4 (C-26), 50.5 (C-17), 48.8 (C-14), 48.4 (C-4), 46.9 (C-13), 44.5 (C-15), 41.4 (C-8), 40.3 (C-22), 37.9 (C-25), 34.9 (C-10), 33.2 (C-20), 32.9 (C-12), 31.9 (C-6), 31.6 (C-19), 30.4 (C-11), 27.7 (C-10), 22.1 (C-16b), 21.3 (C-7), 20.9 (C-22b), 18.9 (C-29), 17.2 (C-27), 15.4 (C-18), 13.7 (C-21), 12.6 (C-28)

### ◆ exemple 5 (comparatif) : neoboutomellerone tronqué

| | |
|---|---|
| 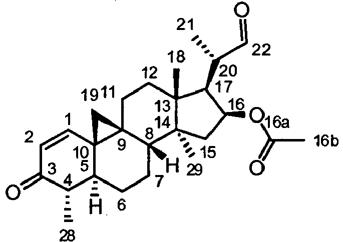 | Formule Chimique : C₂₆H₃₆O₄ |
| | Masse Exacte : 412,26 |
| | Poids moléculaire : 412,56 |

¹H RMN (500MHz, CD₃CN) δ = 9.59 (1H, d, J = 1.8 Hz, H-22), 6.95 (1H, d, J = 10.1 Hz, H-1), 5.91 (1H, d, J = 9.8 Hz, H-2), 5.25 (1H, td, J = 8.2 Hz, J = 5.5 Hz, H-16), 2.87 (1H, dqd, J = 11.0 Hz, J = 7.3 Hz, J = 2.0 Hz, H-20), 2.39 (1H, dd, J = 11.0 Hz, J = 8.2 Hz, H-17), 2.16 - 2.21 (1H, m, H-4), 2.04 - 2.11 (2H, m, H-8a, 15'), 1.96 - 2.04 (2H, m, H-5a, 11'), 1.90 (3H, s, H-16b), 1.70 - 1.78 (1H, m, H-12'), 1.58 - 1.70 (3H, m, H-6', 11", 12"), 1.42 - 1.51 (1H, m, H-7'), 1.33 ( 1 H, ddq, J = 13.4 Hz, J = 5 .6 Hz, J = 1.1 Hz, H-15"), 1.27 (1H, d, J = 4.3 Hz, H-19'), 1.19 - 1.26 (1H, m, H-7"), 1.17 (3H, s, H-18), 1.09 (3H, d, J = 7.3 Hz, H-21), 1.02 (3H, d, J = 6.7 Hz, H-28), 0.96 (3H, d, J = 0.9 Hz, H-29), 0.89 - 1.00 (1H, m, H-6"), 0.56 (1H, d, J = 4.3 Hz, H-19")
¹³C RMN (126MHz, CD₃CN) δ = 205.2 (C-22), 202.3 (C-3), 170.9 (C-16a), 155.4 (C-1), 128.5 (C-2), 75.1 (C-16), 50.7 (C-17), 48.4 (C-14), 47.6 (C-4), 46.5 (C-13), 45.3 (C-15), 45.1 (C-20), 44.3 (C-8), 43.3 (C-5), 33.0 (C-10), 32.6 (C-12), 28.0 (C-11), 27.3 (C-9), 26.9 (C-19), 24.1 (C-6), 24.0 (C-7), 21.3 (C-16b), 19.4 (C-29), 18.4 (C-18), 13.4 (C-21), 11.3 (C-28)

### ◆ exemple 6 : 6-hydroxy- neoboutomellerone

| | |
|---|---|
| 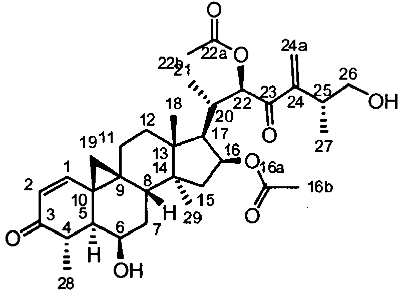 | Formule Chimique : C₃₄H₄₈O₈ |
| | Masse Exacte : 584,33 |
| | Poids moléculaire : 584,74 |

¹H RMN (500MHz, CD₃CN) δ = 6.84 (1H, d, J = 9.8 Hz, H-1), 6.06 (1H, s, H-24a'), 5.90 (1H, d, J = 0.9 Hz, H-24a"), 5.82 (1H, d, J = 9.8 Hz, H-2), 5.55 (1H, d, J = 2.1 Hz, H-22), 5.09 (1H, td, J = 7.6 Hz, J = 4.3 Hz, H-16), 4.05 (1H, br. s., H-6), 3.54 (1H, dd, J = 10.2 Hz, J = 6.3 Hz, H-26'), 3.39 (1H, dd, J = 10.2 Hz, J = 6.6 Hz, H-26"), 2.77 (1H, sxt, J = 6.6 Hz, H-25), 2.69 (1H, br. s., OH-26), 2.57 - 2.65 (2H, m, OH-6, H-20), 2.48 (1H, dq, J = 13.0 Hz, J = 6.8 Hz, H-4), 2.25 - 2.36 (2H, m, H-8, 17), 2.18 - 2.25 (1H, m, H-11'), 2.22 (1H, dd, J = 14.0 Hz, J = 7.9 Hz, H-15'), 2.10 (3H, s, H-22b), 2.04 (3H, s, H-16b), 1.93 - 1.95 (1H, m, H-5), 1.81 (1H, d, J = 3.4 Hz, H-19'), 1.64 - 1.79 (2H, m, H-12', 12"), 1.52 (1H, dt, J = 13.4 Hz, J = 4.7 Hz, H-7'), 1.43 (1H, dd, J = 13.1 Hz, J = 1.8 Hz, H-7"), 1.37 (1H, dd, J = 13.7 Hz, J = 4.9 Hz, H-15"), 1.33 - 1.41 (1H, m, H-11"), 1.24 (3H, s, H-18), 1.14 (3H, d, J = 6.7 Hz, H-28), 1.03 (3H, d, J = 7.0 Hz, H-27), 1.00 (3H, s, H-29), 0.86 (3H, d, J = 7.0 Hz, H-21), 0.68 (1H, d, J = 3.4 Hz, H-19")
¹³C RMN (126MHz, CD₃CN) δ = 203.4 (C-3), 199.5 (C-23), 171.7 (C-22a), 171.4 (C-16a), 156.2 (C-1), 150.3 (C-24), 127.5 (C-2), 124.7 (C-24a), 78.5 (C-22), 76.8 (C-16), 66.4 (C-26), 65.6 (C-6), 51.5 (C-17), 51.4, 50.5, 47.7 (C-14), 47.5 (C-15), 47.1 (C-13), 46.6 (C-5), 44.9 (C-4), 41.1 (C-8), 37.9 (C-25), 33.2 (C-20), 33.1 (C-12), 33.0 (C-19), 32.7 (C-7), 30.8 (C-10), 28.1 (C-9), 27.7 (C-11), 22.1 (C-16b), 20.9 (C-22b), 20.7 (C-29), 19.4 (C-18), 17.2 (C-27), 13.2 (C-21), 11.0 (C-28)

### ◆ exemple 7 : 22-déacétyl, 26-déhydroxy-neoboutomellerone

| | |
|---|---|
| 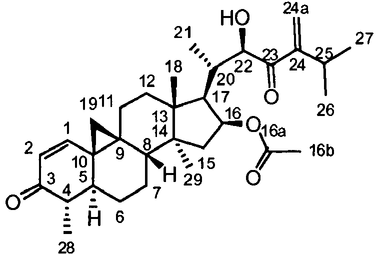 | Formule Chimique : C₃₂H₄₆O₅ |
| | Masse Exacte : 510,33 |
| | Poids moléculaire : 510,70 |

¹H RMN (500MHz, CD₃CN) δ = 6.94 (1H, d, J = 9.8 Hz, H-1), 6.03 (1H, s, H-24a'), 5.94 (1H, d, J = 1.2 Hz, H-24a"), 5.89 (1H, d, J = 10.1 Hz, H-2), 5.20 (1H, td, J = 7.6 Hz, J = 4.4 Hz, H-16), 4.71 (1H, d, J = 5.8 Hz, H-22), 3.54 (1H, d, J = 5.8 Hz, OH-22), 2.79 - 2.91 (1H, m, J = 6.9 Hz, J = 0.9 Hz, H-25), 2.38 - 2.49 (2H, m, H-17, 20), 2.22 (1H, dd, J = 13.9 Hz, J = 7.8 Hz, H-15'), 2.16 - 2.21 (1H, m, H-4), 2.03 (3H, s, H-16b), 1.98 - 2.06 (2H, m, H-8, 11'), 1.94 - 1.98 (1H, m, H-5), 1.61 - 1.74 (3H, m, H-6', 12', 12"), 1.50 - 1.59 (1H, m, H-11"), 1.41 - 1.49 (1H; m, H-7'), 1.38 (1H, dd, J = 13.9 Hz, J = 4.4 Hz, H-15"), 1.24 (1H, d, J = 4.3 Hz, H-19'), 1.18 - 1.26 (1H, m, H-7"), 1.17 (3H, s, H-18), 1.10 (3H, d, J = 7.0 Hz, H-26), 1.03 (3H, d, J = 6.7 Hz, H-28), 1.02 (3H, d, J = 6.7 Hz, H-27), 0.96 (3H, d, J = 0.6 Hz, H-29), 0.94 (1H, qd, J = 12.5 Hz, J = 3.7 Hz, H-6"), 0.64 (3H, d, J = 6.4 Hz, H-21), 0.57 (1H, d, J = 4.3 Hz, H-19")
¹³C RMN (126MHz, CD₃CN) δ = 205.4 (C-23), 202.5 (C-3), 171.3 (C-16a), 155.7 (C-1), 153.1 (C-24), 128.3 (C-2), 124.3 (C-24a), 77.3 (C-16), 75.8 (C-22), 51.4 (C-17), 48.3 (C-14), 47.6 (C-4), 46.9 (C-15), 46.7 (C-13), 45.3 (C-8), 43.6 (C-5), 36.2 (C-20), 33.1 (C-12), 33.0 (C-10), 29.5 (C-25), 28.1 (C-11), 27.7 (C-19), 27.2 (C-9), 24.3 (C-7), 24.3 (C-6), 22.6 (C-27), 22.1 (C-16b), 22.0 (C-26), 20.1 (C-29), 18.5 (C-18), 12.3 (C-21), 11.3 (C-28)

### ◆ exemple 8 : 26-déhydroxy-neoboutomellerone

| | |
|---|---|
| 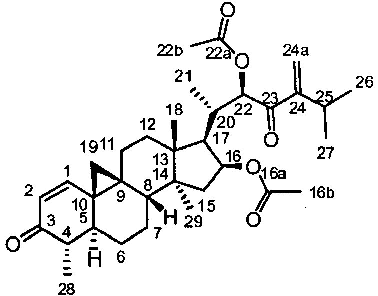 | Formule Chimique : C₃₄H₄₈O₆ |
| | Masse Exacte : 552,35 |
| | Poids moléculaire : 552,74 |

¹H RMN (500MHz, CD₃CN) δ = 6.94 (1H, d, J = 9.8 Hz, H-1), 5.94 (1H, s, H-24a'), 5.90 (1H, d, J = 10.1 Hz, H-2), 5.83 (1H, d, J = 1.2 Hz, H-24a"), 5.51 (1H, d, J = 2.4 Hz, H-22), 5.09 (1H, td, J = 7.6 Hz, J = 4.3 Hz, H-16), 2.73 - 2.86 (1H, m, J = 7.0 Hz, J = 0.9 Hz, H-25), 2.50 - 2.61 (1H, dqd, J = 11.0 Hz, J = 7.0 Hz, J = 2.1 Hz, H-20), 2.29 (1H, dd, J = 11.0 Hz, J = 7.3 Hz, H-17), 2.16 - 2.22 (2H, m, H-4, 15'), 2.09 (3H, s, H-22b), 2.02 (3H, s, H-16b), 1.98 - 2.06 (2H, m, H-8, 11'), 1.94 - 1.98 (1H, m, H-5), 1.62 - 1.77 (3H, m, H-6', 12', 12"), 1.51 - 1.61 (1H, m, H-11"), 1.40 - 1.49 (1H, m, H-7'), 1.36 (1H, ddd, J = 14.0 Hz, J = 4.3 Hz, J = 0.9 Hz, H-15"), 1.24 (1H, d, J = 4.6 Hz, H-19'), 1.18 (3H, s, H-18), 1.13 - 1.22 (1H, m, H-7"), 1.07 (3H, d, J = 6.7 Hz, H-26), 1.02 (3H, d, J = 7.0 Hz, H-28), 1.00 (3H, d, J = 7.0 Hz, H-27), 0.95 (3H, s, H-29), 0.94 (1H, qd, J = 12.5 Hz, J = 4.0 Hz, H-6"), 0.85 (3H, d, J = 7.0 Hz, H-21), 0.58 (1H, d, H-19")
¹³C RMN (126MHz, CD₃CN) δ = 202.5 (C-3), 199.5 (C-23), 171.7 (C-22a), 171.3 (C-16a), 155.6 (C-1), 154.3 (C-24), 128.4 (C-2), 122.3 (C-24a), 78.5 (C-22), 76.6 (C-16), 51.3 (C-17), 48.4 (C-14), 47.6 (C-4), 46.8 (C-13), 46.7 (C-15), 45.2 (C-8), 43.6 (C-5), 33.0 (C-20), 32.9 (C-12), 29.7 (C-25), 28.1 (C-11), 27.7 (C-19), 27.2, 24.3 (C-7, 6), 22.4 (C-27), 22.1 (C-16b), 21.6 (C-26), 20.9 (C-22b), 20.0 (C-29), 18.2 (C-18), 13.2 (C-21), 11.3 (C-28)

### ◆ exemple 9 (comparatif): 26-déhydroxy, 24,25-déhydro-nor neoboutomellerone

| | |
|---|---|
| 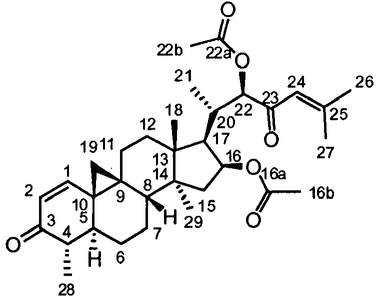 | Formule Chimique : C₃₃H₄₆O₆ |
| | Masse Exacte : 538,33 |
| | Poids moléculaire : 538,71 |

¹H RMN (500MHz, CD₃CN) δ = 6.94 (1H, d, J = 9.8 Hz, H-1), 6.14 (1H, spt, J = 1.3 Hz, H-24), 5.90 (1H, d, J = 9.8 Hz, H-2), 5.10 (1H, td, J = 7.8 Hz, J = 4.6 Hz, H-16), 4.86 (1H, d, J = 1.8 Hz, H-22), 2.56 - 2.64 (1H, dqd, J = 10.9 Hz, J = 7.0 Hz, J = 1.5 Hz, H-20), 2.12 (3H, d, J = 1.2 Hz, H-27), 2.10 (3H, s, H-22b), 2.09 (3H, s, H-16b), 2.04 - 2.22 (4H, m, H-4, 8a, 15', 17), 1.95 - 2.03 (2H, m, H-5, 11'), 1.98 (3H, m, H-26), 1.63 - 1.73 (3H, m, H-6', 12', 12"), 1.53 - 1.63 (1H, m, H-11"), 1.42 - 1.51 (1H, m, H-7'), 1.38 (1H, dd, J = 13.7 Hz, J = 4.6 Hz, H-15"), 1.26 (1H, d, J = 4.3 Hz, H-19'), 1.20 (3H, s, H-18), 1.17 - 1.24 (1H, m, H-7"), 1.02 (3H, d, J = 6.7 Hz, H-28), 0.94 (3H, d, J = 0.9 Hz, H-29), 0.89 - 0.99 (1H, m, H-6"), 0.86 (3H, d, J = 7.0 Hz, H-21), 0.57 (1H, d, J = 4.3 Hz, H-19")
¹³C RMN (126MHz, CD₃CN) δ = 202.6 (C-3), 196.9 (C-23), 171.7 (C-22a), 171.3 (C-16a), 159.8 (C-25), 155.7 (C-1), 128.4 (C-2), 120.5 (C-24), 81.5 (C-22), 76.1 (C-16), 51.1 (C-17), 47.6 (C-4), 46.7 (C-13), 46.1 (C-15), 44.9 (C-8), 43.5 (C-5), 32.7 (C-12), 28.1 (C-26), 28.0 (C-11), 27.4 (C-19), 24.2 (C-6), 24.2 (C-7), 21.8 (C-16b), 21.2 (C-22b), 21.0 (C-27), 19.8 (C-29), 18.1 (C-18), 13.2 (C-21), 11.3 (C-28)

### ◆ exemple 10 (comparatif): 22-déacétyl, 26-déhydroxy, 24,25-déhydro-, nor neoboutomellerone

| | |
|---|---|
| 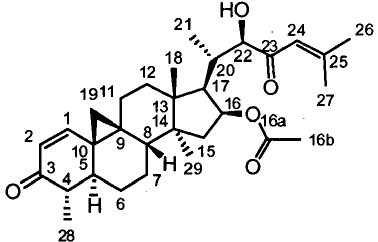 | Formule Chimique : C₃₁H₄₄O₅ |
| | Masse Exacte : 496,32 |
| | Poids moléculaire : 496,68 |

¹H RMN (500MHz, CD₃CN) δ = 6.94 (1H, d, J = 10.1 Hz, H-1), 6.17 (1H, spt, J = 1.3 Hz, H-24), 5.90 (1H, d, J = 10.1 Hz, H-2), 5.20 (1H, td, J = 7.9 Hz, J = 4.7 Hz, H-16), 4.00 (1H, dd, J = 4.9 Hz, J = 1.2 Hz, H-22), 3.55 (1H, d, J = 5.2 Hz, OH-22), 2.44 - 2.53 (1H, dqd, J = 11.3 Hz, J = 7.0 Hz, J = 1.8 Hz, H-20), 2.35 (1H, dd, J = 11.0 Hz, J = 7.6 Hz, H-17), 2.17 (3H, d, J = 1.2 Hz, H-27), 2.16 - 2.20 (1H, m, H-4), 2.06 (3H, s, H-16b), 2.04 - 2.13 (2H, m, H-8, 15'), 1.96 - 2.02 (2H, m, H-5, 11'), 1.94 (3H, m, H26), 1.61 - 1.73 (3H, m, H-6', 12', 12"), 1.53 - 1.61 (1H, m, H-11"), 1.43 - 1.50 (1H, m, H-7'), 1.39 (1H, dd, J = 13.6 Hz, J = 5.0 Hz, H-15"), 1.26 (1H, d, J = 4.6 Hz, H-19'), 1.20 - 1.30 (1H, m, H-7"), 1.20 (3H, s, H-18), 1.03 (3H, d, J = 6.7 Hz, H-28), 0.96 (3H, d, J = 0.6 Hz, H-29), 0.91 - 1.00 (1H, m, H-6"), 0.65 (3H, d, J = 6.7 Hz, H-21), 0.57 (1H, d, J = 4.3 Hz, H-19")
¹³C RMN (126MHz, CD₃CN) δ = 202.5 (C-23, 3), 171.3 (C-16a), 159.9 (C-25), 155.7 (C-1), 128.4 (C-2), 120.3 (C-24), 79.0 (C-22), 76.5 (C-16), 51.2 (C-17), 48.4 (C-14), 47.6 (C-4); 46.6 (C-13), 46.3 (C-15), 45.0 (C-8), 43.5 (C-5), 34.4 (C-20), 32.9 (C-12), 28.1 (C-11), 28.1 (C-26), 27.4 (C-19), 24.3 (C-6), 24.2 (C-7), 21.8 (C-16b), 21.3 (C-27), 19.9 (C-29), 18.3 (C-18), 12.2 (C-21), 11.3 (C-28)

### ◆ exemple 11 : 1,2-dihydro-neoboutomellerone

| | |
|---|---|
| 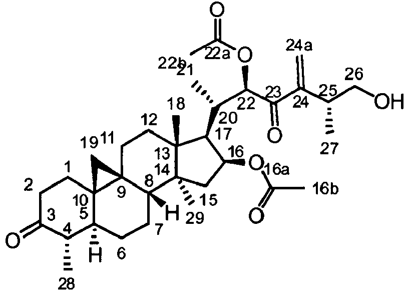 | Formule Chimique : C₃₄H₅₀O₇ |
| | Masse Exacte : 570,36 |
| | Poids moléculaire : 570,76 |

¹H RMN (500MHz, CD₃CN) δ = 6.05 (1H, s, H-24a'), 5.90 (1H, d, J = 0.9 Hz, H-24a"), 5.53 (1H, d, J = 2.1 Hz, H-22), 5.08 (1H, td, J = 7.6 Hz, J = 4.3 Hz, H-16), 3.53 (1H, dt, J = 10.6 Hz, J = 5.8 Hz, H-26'), 3.39 (1H, dt, J = 10.5 Hz, J = 6.1 Hz, H-26"), 2.77 (1H, sxt, J = 6.7 Hz, H-25), 2.68 (1H, t, J = 5.6 Hz, OH-26), 2.55 - 2.64 (1H, dqd, J = 10.7 Hz, J = 6.7 Hz, J = 1.8 Hz, H-20), 2.43 (1H, td, J = 13.6 Hz, J = 6.4 Hz, H-2'), 2.23 - 2.32 (3H, m, H-2", 4, 17), 2.20 (1H, dd, J = 14.0 Hz, J = 7.9 Hz, H-15'), 2.09 (3H, s, H-22b), 2.07 - 2.13 (1H, m, H-11'), 2.03 (3H, s, H-16b), 1.77 - 1.87 (1H, m, H-1'), 1.63 - 1.75 (4H, m, H-6', 8, 12', 12"), 1.50 - 1.62 (2H, m, H-1", 5), 1.36 (1H, dd, J = 14.5 Hz, J = 4.1 Hz, H-15"), 1.23 (3H, s, H-18), 1.20 - 1.34 (2H, m, H-7', 11"), 1.10 (1H, qd, J = 12.8 Hz, J = 2.7 Hz, H-7"), 1.03 (3H, d, J = 7.0 Hz, H-27), 0.97 (3H, s, H-29), 0.91 (3H, d, J = 6.7 Hz, H-28), 0.84 (3H, d, J = 7.0 Hz, H-21), 0.75 (1H, qd, J = 12.6 Hz, J = 2.4 Hz, H-6"), 0.65 (1H, d, J = 3.7 Hz, H-19'), 0.47 (1H, d, H-19")
¹³C RMN (126MHz, CD₃CN) δ = 213.2 (C-3), 199.6 (C-23), 171.7 (C-22a), 171.3 (C-16a), 150.3 (C-24), 124.6 (C-24a), 78.5 (C-22), 76.9 (C-16), 66.4 (C-26), 51.4 (C-17), 50.5 (C-4), 48.6 (C-8), 48.0 (C-14), 47.6 (C-15), 47.0 (C-5), 46.8 (C-13), 41.5 (C-2), 37.9 (C-25), 33.5 (C-1), 33.3 (C-12), 33.2 (C-20), 30.3 (C-10), 28.0 (C-19), 27.4 (C-11), 26.5 (C-6), 26.2 (C-7), 25.4 (C-9), 22.1 (C-16b), 20.9 (C-22b), 20.6 (C-29), 19.3 (C-18), 17.2 (C-27), 13.2 (C-21), 11.2 (C-28)

### ◆ exemple 12 : 6.7-époxy-neoboutomellerone

| | |
|---|---|
| 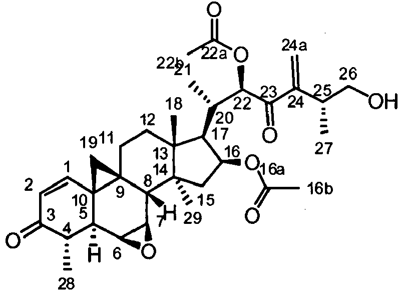 | Formule Chimique : C₃₄H₄₆O₈ |
| | Masse Exacte: 582,32 |
| | Poids moléculaire : 582,72 |

¹H RMN (500MHz, CD₃CN) δ = 6.94 (1H, d, J = 10.1 Hz, H-1), 6.06 (1H, s, H-24a'), 5.91 (1H, d, J = 10.1 Hz, H-2), 5.90 (1H, s, H-24a"), 5.54 (1H, d, J = 2.1 Hz, H-22), 5.16 (1H, td, J = 7.7 Hz, J = 4.7 Hz, H-16), 3.53 (1H, dd, J = 10.4 Hz, J = 6.4 Hz, H-26'), 3.39 (1H, dd, J = 10.5 Hz, J = 6.6 Hz, H-26"), 3.15 (1H, dd, J = 4.3 Hz, J = 1.5 Hz, H-6), 3.00 (1H, dd, J = 4.3 Hz, J = 1.8 Hz, H-7), 2.83 (1H, d, J = 1.2 Hz, H-8), 2.77 (1H, sxt, J = 6.7 Hz, H-25), 2.70 (1H, br. s., OH-26), 2.59 - 2.68 (1H, dqd, J = 10.8 Hz, J = 7.0 Hz, J = 2.1 Hz, H-20), 2.55 (1H, dq, J = 12.5 Hz, J = 7.0 Hz, H-4), 2.45 (1H, d, J = 11.3 Hz, H-5), 2.33 (1H, dd, J = 13.4 Hz, J = 7.9 Hz, H-15'), 2.28 (1H, dd, J = 10.8 Hz, J = 7.8 Hz, H-17), 2.08 (3H, s, H-22b), 2.06 (3H, s, H-16b), 2.02 - 2.05 (2H, m, H-11', 19'), 1.51 - 1.73 (3H, m, H-12', 12", 15"), 1.40 (1H, dd, J = 15.9 Hz, J = 3.1 Hz, H-11"), 1.23 (3H, d, J = 7.0 Hz, H-28), 1.20 (3H, s, H-18), 1.03 (3H, d, J = 7.0 Hz, H-27), 0.94 (3H, s, H-29), 0.86 (3H, d, J = 7.0 Hz, H-21), 0.03 (1H, d, J = 4.0 Hz, H-19")
¹³C RMN (126MHz, CD₃CN) δ = 202.1 (C-3), 199.6 (C-23), 171.7 (C-22a), 171.3 (C-16a), 154.2 (C-1), 150.3 (C-24), 128.4 (C-2), 124.7 (C-24a), 78.6 (C-22), 76.3 (C-16), 66.4 (C-26), 55.0 (C-7), 52.8 (C-6), 49.7 (C-17), 47.3 (C-14), 47.1 (C-13), 45.6 (C-4), 44.7 (C-15), 41.5 (C-5), 38.9 (C-8), 38.0 (C-25), 33.2 (C-20), 32.1 (C-12), 31.8 (C-10), 27.4 (C-11), 27.2 (C-9), 22.1 (C-16b, 19), 22.1, 20.9 (C-22b), 19.6 (C-29), 17.2 (C-27), 15.2 (C-18), 13.7 (C-21), 11.3 (C-28), 1.8

### ◆ exemple 13 : Diastéréoisomère sur la chaîne latérale du neoboutomellerone

| | |
|---|---|
| 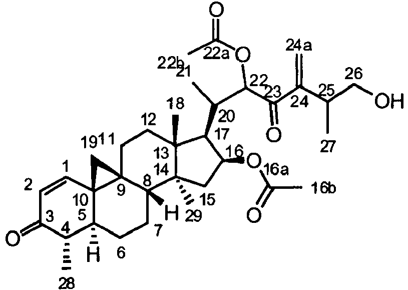 | Formule Chimique : C₃₄H₄₈O₇ |
| | Masse Exacte : 568,34 |
| | Poids moléculaire : 568,74 |

¹H RMN (500MHz, CDCl₃) δ = 6.82 (1H, d, J = 9.8 Hz, H-1), 6.11 (1H, s, H-24a'), 5.98 (1H, d, J = 10.1 Hz, H-2), 5.83 (1H, d, J = 0.9 Hz, H-24a"), 5.52 (1H, d, J = 2.1 Hz, H-22), 5.07 (1H, td, J = 7.7 Hz, J = 4.4 Hz, H-16), 3.56 (2H, t, J = 6.0 Hz, H-26), 2.96 (1H, sxt, J = 6.7 Hz, H-25), 2.55 - 2.63 (1H, m, H-20), 2.21 - 2.33 (2H, m, H-15', 17), 2.14 - 2.22 (1H, m, H-4), 2.14 (3H, s, H-22b), 2.06 (3H, s, H-16b), 1.91 - 2.03 (3H, m, H-5, 8, 11'), 1.62 - 1.77 (3H, m, H-6', 12', 12"), 1.48 - 1.59 (1H, m, H-11"), 1.41 - 1.48 (1H, m, H-7'), 1.30 (1H, dd, J = 14.2 Hz, J = 4.7 Hz, H-15"), 1.18 (1H, d, J = 4.0 Hz, H-19'), 1.15 - 1.21 (1H, m, H-7"), 1.16 (3H, s, H-18), 1.09 (3H, d, J = 7.0 Hz, H-27), 1.08 (3H, d, J = 6.7 Hz, H-28), 0.93 (3H, s, H-29), 0.89 (3H, d, J = 7.0 Hz, H-21), 0.86 - 0.89 (1H, m, H-6"), 0.55 (1H, d, J = 4.6 Hz, H-19")
¹³C RMN (126MHz, CDCl₃) δ = 202.0 (C-3), 198.6 (C-23), 170.7 (C-22a), 170.1 (C-16a), 153.7 (C-1), 149.2 (C-24), 128.3 (C-2), 124.3 (C-24a), 77.8 (C-22), 75.9 (C-16), 66.8 (C-26), 50.3 (C-17), 47.4 (C-14), 46.9 (C-4), 46.0 (C-15), 45.6 (C-13), 44.3 (C-8), 42.5 (C-5), 37.9 (C-25), 32.2 (C-20), 32.1 (C-12), 31.9 (C-10), 27.4 (C-11), 27.0 (C-19), 23.5 (C-7), 23.4 (C-6), 21.7 (C-16b), 20.7 (C-22b), 19.6 (C-29), 17.8 (C-18), 15.5 (C-27), 12.7 (C-21), 10.8 (C-28)

### ◆ exemple 14 : 1,2-dihydro- 22-déacétyl-neoboutomellerone

| | |
|---|---|
| 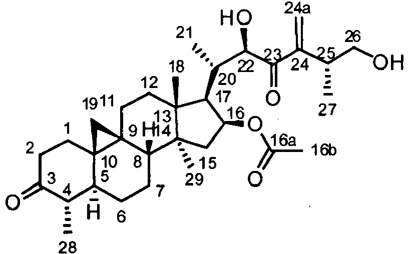 | Formule Chimique : C₃₂H₄₈O₆ |
| | Masse Exacte : 528,35 |
| | Poids moléculaire : 528,72 |

¹H RMN (500MHz, CD₃CN) δ = 6.12 (1H, s, H-24a'), 5.99 (1H, d, J = 0.9 Hz, H-24a"), 5.20 (1H, td, J = 7.6 Hz, J = 4.4 Hz, H-16), 4.72 (1H, dd, J = 6.1 Hz, J = 2.1 Hz, H-22), 3.56 (1H, dt, J = 10.7 Hz, J = 5.8 Hz, H-26'), 3.54 (1H, d, J = 6.1 Hz, OH-22), 3.41 (1H, dt, J = 10.5 Hz, J = 6.0 Hz, H-26"), 2.83 (1H, sxt, J = 6.7 Hz, H-25), 2.67 (1H, t, J = 5.8 Hz, OH-26), 2.34 - 2.50 (3H, m, H-2', 17, 20), 2.19 - 2.34 (3H, m, H-2", 4, 15'), 2.05 - 2.13 (1H, m, H-11'), 2.03 (3H, s, H-16b), 1.77 - 1.87 (1H, tdd, J = 13.5 Hz, J = 3.7 Hz, J = 1.2 Hz, H-1'), 1.62 - 1.75 (4H, m, H-6', 8, 12', 12"), 1.50 - 1.62 (2H, m, H-1", 5), 1.35 - 1.41 (1H, ddq, J = 14.2 Hz, J = 4.4 Hz, J = 0.9 Hz, H-15"), 1.21 - 1.37 (2H, m, H-7', 11"), 1.22 (3H, s, H-18), 1.12 (1H, qd, J = 12.8 Hz, J = 2.7 Hz, H-7"), 1.05 (3H, d, J = 7.3 Hz, H-27), 0.97 (3H, d, J = 0.6 Hz, H-29), 0.91 (3H, d, J = 6.7 Hz, H-28), 0.75 (1H, qd, J = 12.5 Hz, J = 2.4 Hz, H-6"), 0.61 - 0.67 (1H, m, H-19'), 0.64 (3H, d, J = 6.4 Hz, H-21), 0.47 (1H, d, J = 4.0 Hz, H-19")
¹³C RMN (126MHz, CD₃CN) 5 = 213.3 (C-3), 205.6 (C-23), 171.3 (C-16a), 149.1 (C-24), 126.5 (C-24a), 77.5 (C-16), 75.8 (C-22), 66.7 (C-26), 51.5 (C-17), 50.5 (C-4), 48.6 (C-8), 47.9 (C-14), 47.8 (C-15), 47.0 (C-5), 46.7 (C-13), 41.5 (C-2), 37.6 (C-25), 36.3 (C-20), 33.5 (C-1), 33.5 (C-12), 30.3 (C-10), 28.0 (C-19), 27.4 (C-11), 26.5 (C-6), 26.2 (C-7), 25.4 (C-9), 22.1 (C-16b), 20.7 (C-29), 19.5 (C-18), 17.2 (C-27), 12.2 (C-21), 11.2 (C-28)

### ◆ exemple 15 : 1-hydroxy-2-hydro-22-déacétyl-neoboutomellerone

| | |
|---|---|
| 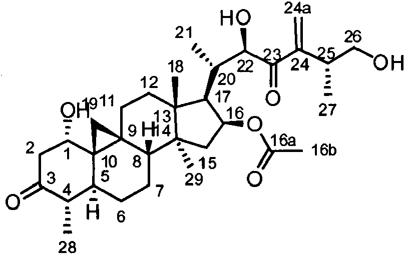 | Formule Chimique : C₃₂H₄₈O₇ |
| | Masse Exacte : 544,34 |
| | Poids moléculaire : 544,72 |

¹H RMN (500MHz, CD₃CN) δ = 6.12 (1H, s, H-24a'), 5.99 (1H, d, J = 0.9 Hz, H-24a"), 5.21 (1H, td, J = 7. 5 Hz, J = 4.3 Hz, H-16), 4.72 (1H, dd, J = 5.3 Hz, J = 1.7 Hz, H-22), 3.81 (1H, t, J = 3.1 Hz, H-1), 3.55 (2H, d, J = 6.1 Hz, OH-22), 3.50 - 3.61 (1H, m, H-26'), 3.34 - 3.45 (1H, m, H-26"), 2.83 (1H, sxt, J = 6.4 Hz, H-25), 2.80 (1H, br. s., OH-1), 2.69 (1H, br. s., OH-26), 2.64 (1H, dd, J = 14.0 Hz, J = 3.7 Hz, H-2'), 2.41 - 2.50 (1H, dqd, J = 11.0 Hz, J = 6.7 Hz, J = 2.1 Hz, H-20), 2.41 (1H, dd, J = 11.0 Hz, J = 7.0 Hz, H-17), 2.20 - 2.34 (4H, m, H-2", 4,11', 15'), 2.10 - 2.14 (1H, m, H-5), 2.03 (3H, s, H-16b), 1.70 - 1.79 (1H, m, H-6'), 1.64 - 1.71 (3H, m, H-8, 12', 12"), 1.25 - 1.42 (3H, m, H-7', 11", 15"), 1.21 (3H, s, H-18), 1.12 (1H, qd, J = 12.8 Hz, J = 2.1 Hz, H-7"), 1.05 (3H, d, J = 7.0 Hz, H-27), 1.01 (3H, s, H-29), 0.92 (3H, d, J = 6.4 Hz, H-28), 0.80 (1H, qd, J = 12.6 Hz, J = 2.4 Hz, H-6"), 0.73 (1H, d, J = 4.3 Hz, H-19'), 0.64 (3H, d, H-21), 0.48 (1H, d, J = 4.3 Hz, H-19")
¹³C RMN (126MHz, CD₃CN) δ = 212.2 (C-3), 205.6 (C-23), 171.3 (C-16a), 149.1 (C-24), 126.5 (C-24a), 77.5 (C-16), 75.8 (C-22), 74.0 (C-1), 66.7 (C-26), 51.5 (C-17), 50.5 (C-4), 49.3 (C-2), 48.7 (C-8), 47.9 (C-14), 47.9 (C-15), 46.6 (C-13), 39.5 (C-5), 37.6 (C-25), 36.3 (C-20), 34.0 (C-10), 33.3 (C-12), 28.1 (C-19), 26.5 (C-11), 26.2 (C-7), 26.1 (C-6), 26.0 (C-9), 22.1 (C-16b), 20.7 (C-29), 19.6 (C-18), 17.2 (C-27), 12.2 (C-21), 11.0 (C-28)

### ◆ exemple 16 : 6-hydroxy-22-déacétyl-neoboutomellerone

| | |
|---|---|
| 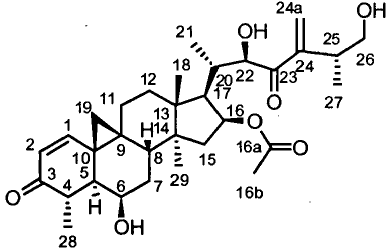 | Formule Chimique : C₃₂H₄₆O₇ |
| | Masse Exacte : 542,32 |
| | Poids moléculaire : 542,70 |

¹H RMN (500MHz, CDCl₃) δ = 6.74 (1H, d, J = 10.1 Hz, H-1), 6.16 (1H, s, H-24a'), 6.01 (1H, s, H-24a"), 5.92 (1H, d, J = 9.8 Hz, H-2), 5.33 (1H, td, J = 7.1 Hz, J = 4.4 Hz, H-16), 4.72 (1H, s, H-22), 4.16 (1H, br. s., H-6), 3.62 (2H, d, J = 6.1 Hz, H-26), 3.57 (1H, d, J = 5.5 Hz, OH-22), 2.94 (1H, sxt, J = 6.4 Hz, H-25), 2.52 - 2.59 (1H, m, H-4), 2.47 - 2.54 (1H, m, H-17), 2.39 - 2.47 (1H, m, H-20), 2.35 (1H, dd, J = 13.7 Hz, J = 7.9 Hz, H-15'), 2.27 (1H, dd, J = 12.7 Hz, J = 4.4 Hz, H-8), 2.15 - 2.24 (1H, m, H-11'), 2.07 (3H, s, H-16b), 1.96 (1H, dd, J = 12.8 Hz, J = 2.4 Hz, H-5), 1.82 (1H, d, J = 3.1 Hz, H-19'), 1.65 - 1.79 (2H, m, H-12', 12"), 1.56 (1H, td, J = 12.9 Hz, J = 4.4 Hz, H-7'), 1.47 (1H, t, J = 13.1 Hz, H-7"), 1.35 (1H, dd, J = 15.1 Hz, J = 4.4 Hz, H-15"), 1.26 - 1.39 (1H, m, H-11"), 1.21 (3H, br. s., H-18), 1.21 (3H, d, J = 7.3 Hz, H-28), 1.11 (3H, d, J = 7.0 Hz, H-27), 1.01 (3H, s, H-29), 0.68 (1H, d, J = 3.1 Hz, H-19"), 0.65 (3H, d, J = 6.4 Hz, H-21)
¹³C RMN (126MHz, CDCl₃) δ = 204.4 (C-23), 202.7 (C-3), 170.2 (C-16a), 154.6 (C-1), 147.9 (C-24), 127.3 (C-2), 125.8 (C-24a), 77.1 (C-16), 74.8 (C-22), 74.8, 67.0 (C-26), 65.6 (C-6), 50.9, 50.4 (C-17), 46.9 (C-15), 46.7 (C-14), 46.1 (C-13), 45.7 (C-5), 43.8 (C-4), 40.2 (C-8), 36.6 (C-25), 35.8 (C-20), 32.7 (C-19), 32.3 (C-12), 31.9 (C-7), 29.7 (C-10), 27.2 (C-11), 25.4 (C-9), 21.8 (C-16b), 20.3 (C-29), 19.3 (C-18), 16.5 (C-27), 11.5 (C-21), 10.8 (C-28)

### 1.2. Par hémisynthèse

### ◆ exemple 1 : 22-déacétyl- neoboutomellerone

Le composé 1 peut être obtenu par extraction à partir des feuilles de *Neoboutonia melleri* comme décrit ci-dessus ou être préparé par synthèse à partir de neoboutomellerone comme décrit ci-dessous.

Dans un tube scellé, 100 mg (0.178 mmol) de composé 2 sont dissous (dans 3.2 ml) dans un alcool utilisé comme solvant tel que l'isopropanol ou le tertiobutanol. On rajoute ensuite du carbonate de potassium (123 mg, 0.889 mmol, 5 éq.) et 0.8 ml d'eau. Le tube est scellé et la réaction est laissée 72 heures à 40°C.

La réaction est diluée avec de l'acétate d'éthyle, la phase organique est séparée puis lavée successivement avec de l'eau et de la saumure. Le produit est purifié sur colonne de gel de silice et élué par un mélange cyclohexane / acétate d'éthyle de 60 / 40, pour conduire à l'isolement du composé 1 avec un rendement de 70%.

La même réaction réalisée à 70 °C en utilisant le triflate de scandium (20%, 18 mg, 0.036 mmol) dans un mélange eau / tertiobutanol (1 ml / 0.2 ml) conduit au même composé 1 avec une conversion de 50 %.

### ◆ exemple 17 : 26-acétyl-22-déacétyl-neoboutomellerone

| | |
|---|---|
| 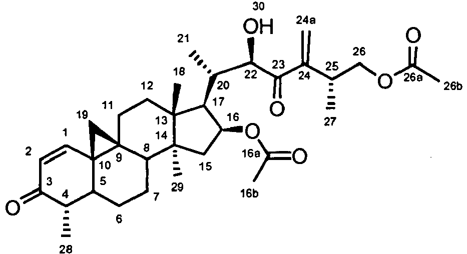 | Formule Chimique : C₃₄H₄₈O₇ |
| | Masse Exacte : 568,34 |
| | Poids moléculaire : 568,74 |

Protocole A : 200 mg (0.380 mmol) de composé 1 sont dissous dans 16 ml de DCM. La réaction est refroidie à 0°C, puis 1 éq. (0.380 mmol, 45 µl) de lutidine est ajouté, suivi de 0.75 éq. (0.285 mmol, 27 µl) d'anhydride acétique et enfin 5 mg de DMAP (10 %).Après 1h, la réaction est hydrolysée à l'eau. La phase organique est lavée à l'eau, par une solution de sulfate de cuivre, de l'eau puis de la saumure. Après séchage sur sulfate de sodium et évaporation du solvant, on recueille 240 mg de brut réactionnel. Le produit est purifié sur colonne de gel de silice et élué par un mélange cyclohexane / acétate d'éthyle de 80 / 20. On obtient 94.6 mg (58 %) de produit 17 (Rf: 0.68 - cyclohexane / acétate d'éthyle 50 / 50), et 76 mg (38 %) de produit de départ 1 (Rf: 0.31 - cyclohexane / acétate d'éthyle 50 / 50).
Protocole B: 200 mg (0.352 mmol) de composé 2 sont dissous dans 25 ml d'acétonitrile. A cette solution, sont rajoutés 195 mg (4 éq., 1.4 mmol) de carbonate de potassium, et 11 mg (0.1 éq., 0.035 mmol) de bromure de tétrabutylammonium. La réaction est laissée sous agitation à 40°C pendant 1 nuit, puis le milieu réactionnel est filtré. Le produit est purifié sur colonne de gel de silice dans les même conditions que précédemment et conduit à 136 mg (69%) de composé 17 (Rf: 0.68 - cyclohexane / acétate d'éthyle 50 / 50).
   ¹H RMN (500MHz, CD3CN) δ = 6.94 (1H, d, J = 10.1 Hz, H-1), 6.17 (1H, s, H-24aa), 6.04 (1H, d, J = 0.6 Hz, H-24ab), 5.89 (1H, d, J = 9. 8 Hz, H-2), 5.20 (1H, td, J = 7.5 Hz, J = 4.6 Hz, H-16), 4.72 (1H, dd, J = 6.3 Hz, J = 1.7 Hz, H-22), 3.99 - 4.10 (2H, m, H-26<">, 26<'>), 3.52 (1H, d, J = 6.1 Hz, H-30), 3.05 (1H, sxt, J = 6.8 Hz, H-25), 2.37 - 2.49 (2H, m, H-20, 17), 2.22 (1H, dd, J = 13.9 Hz, J = 7.8 Hz, H-15<'>), 2.15 - 2.21 (1H, m, H-4), 2.03 (3H, s, H-16b), 1.98 - 2.07 (2H, m, H-8, 11 <'>), 1.97 (3H, s, H-26b), 1.94 - 1.98 (1H, m, H-5), 1.60 - 1.74 (3H, m, H-6<'>, 12<">, 12<'>), 1.50 - 1.59 (1H, m, H-11<">), 1.41 - 1.49 (1H, m, H-7<'>), 1.38 (1H, dd, J = 13.6 Hz, J = 4.4 Hz, H-15<">), 1.24 (1H, d, J = 4.6 Hz, H-19<'>), 1.17 - 1.23 (1H, m, H-7<">), 1.17 (3H, s, H-18), 1.09 (3H, d, J = 7.0 Hz, H-27), 1.03 (3H, d, J = 7.0 Hz, H-28), 0.96 (3H, s, H-29), 0.94 (1H, qd, J = 12.8 Hz, J = 4.0 Hz, H-6<">), 0.64 (3H, d, J = 6.1 Hz, H-21), 0.57 (1H, d, J = 4.3 Hz, H-19<">)
   ¹³C RMN (126MHz, CD3CN) δ = 205.0 (C-23), 202.4 (C-3), 171.6 (C-26a), 171.3 (C-16a), 155.6 (C-1), 148.0 (C-24), 128.4 (C-2), 127.4 (C-24a), 77.3 (C-16), 75.8 (C-22), 68.0 (C-26), 51.4 (C-17), 48.3 (C-14), 47.6 (C-4), 46.9 (C-15), 46.7 (C-13), 45.3 (C-8), 43.6 (C-5), 36.4 (C-20), 34.7 (C-25), 33.1 (C-12), 32.9 (C-10), 28.1 (C-11), 27.7 (C-19), 27.2 (C-9), 24.3 (C-6), 24.3 (C-7), 22.1 (C-16b), 21.1 (C-26b), 20.1 (C-29), 18.5 (C-18), 17.3 (C-27), 12.3 (C-21), 11.3 (C-28)

### ◆ exemple 18 : 26-acétyl- neoboutomellerone

| | |
|---|---|
| 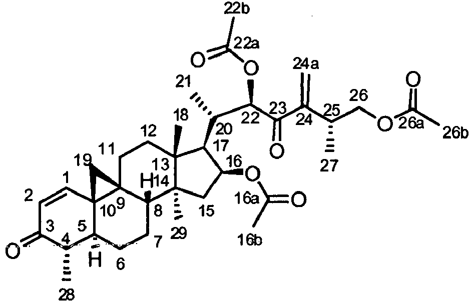 | Formule Chimique : C₃₆H₅₀O₈ |
| | Masse Exacte : 610,35 |
| | Poids moléculaire : 610,78 |

Protocole : 50 mg (0.088 mmol) de 2 sont dissous dans 4 ml de DCM. La réaction est refroidie à 0°C, puis 300 µl (3.7 mmol) de pyridine sont ajoutés, suivi de 10 éq. (0.88 mmol, 60 µl) de chlorure d'acétyle. La réaction est laissée sous agitation une nuit. L'excès de chlorure d'acétyle est neutralisé par du méthanol, et les solvants sont évaporés. Le brut réactionnel est purifié sur colonne de gel de silice et élué par un mélange cyclohexane / acétate d'éthyle de 70 / 30, et conduit à 40.52 mg (75%) de composé 18 (Rf : 0.68 - cyclohexane / acétate d'éthyle 50 / 50).
¹H RMN (500MHz, CD₃CN) δ = 6.94 (1H, d, J = 10.1 Hz, H-1), 6.10 (1H, s, H-24a'), 5.96 (1H, d, J = 0.6 Hz, H-24a"), 5.90 (1H, d, J = 10.1 Hz, H-2), 5.52 (1H, d, J = 2.1 Hz, H-22), 5.09 (1H, td, J = 7.6 Hz, J = 4.6 Hz, H-16), 3.96 - 4.09 (2H, m, H-26', 26"), 3.00 (1H, sxt, J = 6.9 Hz, H-25), 2.53 - 2.63 (1H, dqd, J = 11.0 Hz, J = 7.0 Hz, J = 2.1 Hz, H-20), 2.30 (1H, dd, J = 11.0 Hz, J = 7.6 Hz, H-17), 2.14 - 2.24 (2H, m, H-15', 4), 2.09 (3H, s, H-22b), 2.03 (3H, s, H-16b), 1.99 - 2.06 (2H, m, H-11', 8), 1.97 (3H, s, H-26b), 1.95 - 1.98 (1H, m, H-5), 1.63 - 1.77 (3H, m, H-6', 12', 12"), 1.52 - 1.62 (1H, m, H-11"), 1.41 - 1.49 (1H, m, H-7'), 1.37 (1H, dd, J = 14.5 Hz, J = 4.1 Hz, H-15"), 1.24 (1H, d, J = 4.6 Hz, H-19'), 1.19 (3H, s, H-18), 1.14 - 1.22 (1H, m, H-7"), 1.07 (3H, d, J = 7.0 Hz, H-27), 1.03 (3H, d, J = 6.7 Hz, H-28), 0.95 (3H, s, H-29), 0.89 - 0.99 (1H, m, H-6"), 0.85 (3H, d, J = 7.0 Hz, H-21), 0.58 (1H, d, H-19")
¹³C RMN (126MHz, CD₃CN) δ = 202.4 (C-3), 198.9 (C-23), 171.6 (C-22a), 171.6 (C-26a), 171.3 (C-16a), 155.5 (C-1), 149.2 (C-24), 128.4 (C-2), 125.6 (C-24a), 78.3 (C-22), 76.7 (C-16), 67.8 (C-26), 51.3 (C-17), 48.4 (C-14), 47.6 (C-4), 46.9 (C-13), 46.7 (C-15), 45.2 (C-8), 43.6 (C-5), 34.9 (C-25), 33.2 (C-20), 32.9 (C-12), 28.1 (C-11), 27.6 (C-19), 27.2 (C-9), 24.3 (C-6, 7), 22.1 (C-16b), 21.1 (C-26b), 20.9 (C-22b), 20.0 (C-29), 18.3 (C-18), 17.3 (C-27), 13.3 (C-21), 11.3 (C-28)

### ◆ exemple 19 : 26-méthoxy- neoboutomellerone

| | |
|---|---|
| 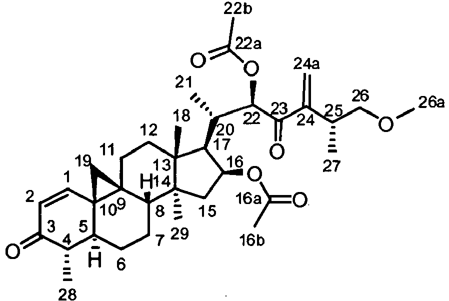 | Formule Chimique : C₃₅H₅₀O₇ |
| | Masse Exacte : 582,36 |
| | Poids moléculaire : 582,77 |

Protocole : 51 mg (0.089 mmol) de 2 sont dissous dans 1 ml de DCM. A 0°C, 70 µl (3.5 éq, 0.31 mmol) de 2-6-(di-*tert*-butyl)-pyridine puis 69 mg (3 éq, 0.27 mmol) de triflate d'argent sont ajouté et le milieu réactionnel est protégé de la lumière. On ajoute enfin, 94 µl (6 éq, 0.54 mmol) d'iodométhane. Après 23h, le milieu réactionnel est filtré sur célite®, le filtrat est lavée successivement avec une solution d'acide chlorhydrique 4%, une solution d'hydrogénocarbonate de sodium et de la saumure. Après séchage sur sulfate de sodium et évaporation des solvants, on recueille 240 mg de brut réactionnel. Le produit est purifié sur colonne de gel de silice et élué par un gradient de cyclohexane / acétate d'éthyle de 100/0 à 0 / 100. Le produit 19 (Rf: 0.73 - cyclohexane / acétate d'éthyle 60 / 40) est finalement obtenu avec 77 % de rendement (40.5 mg).
¹H RMN (500MHz, CD₃CN) δ = 6.94 (1H, d, J = 10.1 Hz, H-1), 6.01 (1H, s, H-24a'), 5.90 (1H, d, J = 10.1 Hz, H-2), 5.87 (1H, d, J = 0.9 Hz, H-24a"), 5.51 (1H, d, J = 2.1 Hz, H-22), 5.09 (1H, td, J = 7.7 Hz, J = 4.4 Hz, H-16), 3.40 (1H, dd, J = 9.3 Hz, J = 6.9 Hz, H-26'), 3.25 (3H, s, H-26a), 3.26 (1H, dd, J = 9.3 Hz, J = 6.6 Hz, H-26"), 2.90 (1H, sxt, J = 7.0 Hz, H-25), 2.53 - 2.64 (1H, dqd, J = 11.0 Hz, J = 7.0 Hz, J = 2.1 Hz, H-20), 2.28 (1H, dd, J = 11.1 Hz, J = 7.5 Hz, H-17), 2.15 - 2.22 (2H, m, H-15', 4), 2.09 (3H, s, H-22b), 1.98 - 2.05 (2H, m, H-11', 8), 2.02 (3H, s, H-16b), 1.94 - 1.98 (1H, m, H-5), 1.62 - 1.76 (3H, m, H-6', 12', 12"), 1.52 - 1.62 (1H, m, H-11"), 1.41 - 1.49 (1H, m, H-7'), 1.36 (1H, dd, J = 13.9 Hz, J = 3.5 Hz, H-15"), 1.24 (1H, d, J = 4.6 Hz, H-19'), 1.15 - 1.22 (1H, m, H-7"), 1.18 (3H, s, H-18), 1.04 (3H, d, J = 7.0 Hz, H-28), 1.02 (3H, d, J = 6.4 Hz, H-27), 0.95 (3H, s, H-29), 0.94 (1H, qd, J = 12.5 Hz, J = 3.7 Hz, H-6"), 0.85 (3H, d, J = 6.7 Hz, H-21), 0.58 (1H, d, J = 4.3 Hz, H-19")
¹³C RMN (126MHz, CD₃CN) δ = 202.4 (C-3), 199.3 (C-23), 171.7 (C-22a), 171.3 (C-16a), 155.5 (C-1), 150.3 (C-24), 128.4 (C-2), 124.7 (C-24a), 78.4 (C-22), 76.6 (C-26), 76.6 (C-16), 58.8 (C-26a), 51.3 (C-17), 48.4 (C-14), 47.6 (C-4), 46.8 (C-13), 46.7 (C-15), 45.2 (C-8), 43.6 (C-5), 36.1 (C-25), 33.0 (C-20), 32.9 (C-12), 28.1 (C-11), 27.6 (C-19), 27.2 (C-9), 24.3 (C-7, 6), 22.1 (C-16b), 20.9 (C-22b), 20.0 (C-29), 18.2 (C-18), 17.7 (C-27), 13.2 (C-21), 11.3 (C-28)

### ◆ exemple 20 : 26-N-phénylcarbamate- neoboutomellerone

| | |
|---|---|
| 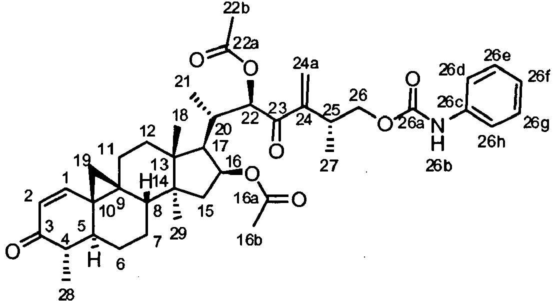 | Formule Chimique : C₄₁H₅₃O₈ |
| | Masse Exacte : 687,38 |
| | Poids moléculaire : 687,86 |

Protocole : 53 mg (0.093 mmol) de 2 sont solubilisés dans 1 mL de dichlorométhane sous azote. 6 mg (0.047 mmol, 0.5 eq.) de DMAP, 11 µL (0.102 mmol, 1.1 eq.) d'isocyanate de phényle et 11 µL (0.102 mmol, 1.1 eq.) de triéthylamine sont ajoutés et le milieu réactionnel est agité à température ambiante. Après 22h, le milieu réactionnel est dilué dans l'acétate d'éthyle et la phase organique est lavée successivement avec une solution d'HCl 4%, une solution saturée d'hydrogénocarbonate de sodium et une solution saturée de chlorure de sodium. La phase organique est séchée sur MgSO₄, filtrée et concentrée à l'évaporateur rotatif. Le produit est purifié par chromatographie sur silice (éluant : Cyclohexane/AcOEt :10/0 à 0/10). Un solide blanc est obtenu avec un rendement de 47% (30 mg).
¹H RMN (500MHz, CD₃CN) δ = 7.68 (1H, br. s., H-26b), 7.41 (2H, d, J = 7.9 Hz, H-26d, 26h), 7.29 (2H, t, J = 8.5 Hz, H-26g, 26e), 7.03 (1H, tt, J = 7.3 Hz, J = 1.2 Hz, H-26f), 6.94 (1H, d, J = 10.1 Hz, H-1), 6.15 (1H, s, H-24a'), 6.02 (1H, d, J = 0.6 Hz, H-24a"), 5.90 (1H, d, J = 9.8 Hz, H-2), 5.54 (1H, d, J = 2.1 Hz, H-22), 5.09 (1H, td, J = 7.7 Hz, J = 4.4 Hz, H-16), 4.13 (1H, dd, J = 10.7 Hz, J = 7.0 Hz, H-26'), 4.06 (1H, dd, J = 10.7 Hz, J = 6.4 Hz, H-26"), 3.07 (1H, sxt, J = 7.0 Hz, H-25), 2.54 - 2.66 (1H, dqd, J = 10.9 Hz, J = 7.0 Hz, J = 2.3 Hz, H-20), 2.30 (1H, dd, J = 11.0 Hz, J = 7.3 Hz, H-17), 2.15 - 2.22 (2H, m, H-15', 4), 2.10 (3H, s, H-22b), 2.03 (3H, s, H-16b), 1.98 - 2.06 (2H, m, H-8, 11'), 1.94 - 1.98 (1H, m, H-5), 1.61 - 1.76 (3H, m, H-6', 12', 12"), 1.50 - 1.61 (1H, m, H-11"), 1.44 (1H, s, H-7'), 1.37 (1H, dd, J = 13.9 Hz, J = 4.1 Hz, H-15"), 1.24 (1H, d, J = 4.6 Hz, H-19'), 1.18 (3H, s, H-18), 1.14 - 1.22 (1H, m, H-7"), 1.11 (3H, d, J = 7.0 Hz, H-27), 1.02 (3H, d, J = 6.7 Hz, H-28), 0.95 (3H, s, H-29), 0.88 - 1.00 (1H, m, H-6"), 0.85 (3H, d, J = 7.0 Hz, H-21), 0.57 (1H, d, J = 4.6 Hz, H-19")
¹³C RMN (126MHz, CD₃CN) δ = 202.4 (C-3), 198.8 (C-23), 171.8 (C-22a), 171.3 (C-16), 155.5 (C-1), 149.2 (C-24), 139.9 (C-26c), 129.9 (C-26e, 26g), 128.4 (C-2), 125.7 (C-24a), 123.9 (C-26f), 119.5 (C-26d, 26h), 78.5 (C-22), 76.7 (C-16), 68.5 (C-26), 51.3 (C-17), 48.3 (C-14), 47.6 (C-4), 46.8 (C-13), 46.7 (C-15), 45.2 (C-8), 43.6 (C-5), 35.0 (C-25), 33.4 (C-20), 32.9 (C-12), 28.1 (C-11), 27.6 (C-19), 27.2 (C-9), 24.3 (C-6, 7), 22.1 (C-16b), 21.0 (C-22b), 20.0 (C-29), 18.3 (C-18), 17.1 (C-27), 13.4 (C-21), 11.3 (C-28)

### ◆ exemples 21 & 22 : 26-N-(N-méthylpiperazine)carbamate-neoboutomellerone (21) et chlorhydrate de 26-N-(N-méthylpiperazine)carbamate-neoboutomellerone (22)

| | |
|---|---|
| 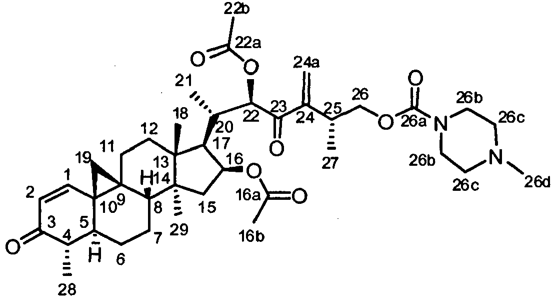 | 21 |
| | Formule Chimique : C₄₀H₅₈N₂O₈ |
| | Masse Exacte : 694,42 |
| | Poids moléculaire : 694,90 |
| 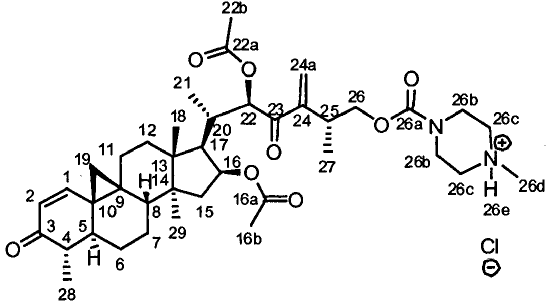 | 22 |
| | Formule Chimique : C₄₀H₅₉ClN₂O₈ |
| | Masse Exacte : 730.40 |
| | Poids moléculaire : 731,36 |

Protocole : 200 mg de 2 (0.380 mmol) sont solubilisés dans 1 mL d'acétonitrile anhydre. 210 mg (1.520 mmol, 4 eq.) de carbonate de potassium, 151 mg (0.760 mmol, 2 eq.) de chlorhydrate du chlorure de carbonyle de la 4-méthylpiperazine et 12 mg (0.035 mmol, 0.1 eq.) de bromure de tétrabutyle ammonium sont ajoutés. Le milieu réactionnel est agité pendant 28 heures à température ambiante. Le milieu réactionnel est filtré et concentré à l'évaporateur rotatif. Le résidu est purifié par chromatographie sur gel de silice (éluant : gradient DCM/MeOH : 98/2 à 95/5). Deux produits sont isolés : le carbamate attendu 21 (70 mg, 29%) et le produit 17 avec un rendement de (65 mg, 33%). 3 mL d'HCl 0.1 M sont ajoutés à 44 mg de carbamate 21 et le milieu réactionnel est agité à température ambiante pendant 1 heure. Le milieu réactionnel est ensuite lyophilisé afin d'obtenir 43 mg chlorhydrate 22 (93%).

### exemple 21 :

¹H NMR (500MHz, ACETONITRILE-d₃) δ = 6.94 (1H, d, J = 9.8 Hz, H-1), 6.11 (1H, s, H-24aa), 5.96 (1H, s, H-24ab), 5.90 (1H, d, J = 9.8 Hz, H-2), 5.53 (1H, d, J = 2.4 Hz, H-22), 5.08 (1H, td, J = 7.6 Hz, J = 4.6 Hz, H-16), 4.02 - 4.09 (1H, m, H-26<'>), 3.99 (1H, dd, J = 10.6 Hz, J = 6.2 Hz, H-26<">), 3.33 - 3.39 (4H, m, H-26b), 2.99 (1H, sxt, J = 6.8 Hz, H-25), 2.54 - 2.61 (1H, m, H-20), 2.29 (1H, dd, J = 11.0 Hz, J = 7.6 Hz, H-17), 2.21 - 2.28 (4H, m, H-26c,), 2.20 (3H, s, H-26d), 2.14 - 2.19 (2H, m, H-15<'>, 4), 2.09 (3H, s, H-22b), 2.03 (3H, s, H-16b), 1.95-2.01 (3H, m, H-11<'>, 8a, 5a), 1.64 - 1.75 (3H, m, H-6<'>, 12), 1.52 - 1.60 (1H, m, H-11<">), 1.41 - 1.48 (1H, m, H-7<">), 1.33 - 1.40 (1H, m, H-15<">), 1.22 - 1.26 (1H, m, H-19<">), 1.19 - 1.22 (1H, m, H-7<'>), 1.18 (3H, s, H-18), 1.08 (2H, d, J = 7.3 Hz, H-27), 1.02 (2H, d, J = 6.7 Hz, H-28), 0.95 (3H, s, H-29), 0.87 - 0.93 (1H, m, H-6<">), 0.84 (3H, d, J = 7.0 Hz, H-21), 0.58 (1H, d, J = 4.3 Hz, H-19<'>)
¹³C NMR (126MHz, ACETONITRILE-d₃) δ = 202.4 (C-3), 198.8 (C-23), 171.6 (C-22a), 171.2 (C-16a), 155.9 (C-26a), 155.5 (C-1), 149.3 (C-24), 128.4 (C-2), 125.7 (C-24a), 78.3 (C-22), 76.7 (C-16), 68.7 (C-26), 55.5 (C-26c), 51.3 (C-17), 48.4 (C-14), 47.6 (C-4), 46.8 (C-13), 46.7 (C-15), 46.4 (C-8), 45.2 (C-26d), 44.6 (C-26b), 43.6 (C-5), 35.4 (C-25), 33.3 (C-20), 33.0 (C-12), 32.9 (C-10), 28.1 (C-11), 27.6 (C-19), 27.2 (C-9), 24.3 (C-6, 7), 22.1 (C-16b), 20.9 (C-22b), 20.0 (C-29), 18.3 (C-18), 17.2 (C-27), 13.3 (C-21), 11.3 (C-28)

### exemple 22 :

¹H NMR (500MHz, DMSO-d₆) δ = 9.97 (1H, br. s, H-26e), 6.97 (1H, d, J = 10.1 Hz, H-1), 6.11 (2H, d, J = 10.8 Hz, H-24ab, 24aa), 5.90 (1H, d, J = 10.0 Hz, H-2), 5.43 - 5.49 (1H, m, H-22), 5.03 (1H, td, J = 7.5 Hz, J = 4.2 Hz, H-16), 3.91 - 4.09 (4H, m, H-26, 26b), 3.36-3.44 (1H, m, H-26c), 3.13 (2H, br. s., H-26b), 2.90 - 3.02 (3H, m, H-25, 26c), 2.77 (3H, br. s., H-26d), 2.46 - 2.48 (1H, m, H-20), 2.22 (1H, dd, J = 11.0 Hz, J = 7.5 Hz, H-17), 2.12 - 2.16 (1H, m, H-4, 15<'>), 2.11 (5H, s, H-22b), 2.06 (3H, s, H-16b), 1.94 - 2.02 (3H, m, H-11<'>, 8a), 1.89 (1H, td, J = 12.5 Hz, J = 4.2 Hz, H-5a), 1.50 - 1.66 (5H, m, H-12, 6<">, 11<">), 1.35 - 1.44 (1H, m, H-7<'>), 1.30 (1H, dd, J = 13.9 Hz, J = 3.7 Hz, H-15 <">), 1.24 (1H, d, J = 4.0 Hz, H-19<'>), 1.14 - 1.18 (1H, m, H-7<">), 1.13 (3H, s, H-18), 1.04 (3H, d, J = 7.0 Hz, H-27), 0.98 (3H, d, J = 6.7 Hz, H-28), 0.92-0.94 (1H, m, H-6<">), 0.90 (3H, s, H-29), 0.79 (3H, d, J = 7.0 Hz, H-21), 0.58 (1H, d, J = 4.2 Hz, H-19<">)
¹³C NMR (126MHz, DMSO-d₆) δ = 200.6 (C-3), 197.1 (C-23), 170.2 (C-22a), 169.8 (C-16a), 154.7 (C-26a), 154.0 (C-1), 147.5 (C-24), 127.3 (C-2), 125.1 (C-24a), 76.6 (C-22), 75.1 (C-16), 68.3 (C-26), 52.0 (C-26c), 49.7 (C-17), 47.0 (C-14), 46.1 (C-4), 45.4 (C-13), 45.3 (C-15), 43.3 (C-8), 42.4 (C-26b), 42.0 (C-26d, 5), 33.1 (C-25), 31.9 (C-20), 31.7 (C-10), 31.6 (C-12), 26.6 (C-9), 26.3 (C-11), 25.9 (C-19), 22.9 (C-6), 22.8 (C-7), 21.4 (C-16b), 20.4 (C-22b), 19.1 (C-29), 17.5 (C-18), 16.6 (C-27), 12.4 (C-21), 10.7 (C-28)

### ◆ exemples 23 & 24 : 26-N-(N-méthylpiperazine)carbamate-22-déacétyl,neoboutomellerone (23) et chlorhydrate de 26-N-(N-méthylpiperazine)carbamate-22-déacétyl,neoboutomellerone (24)

| | |
|---|---|
| 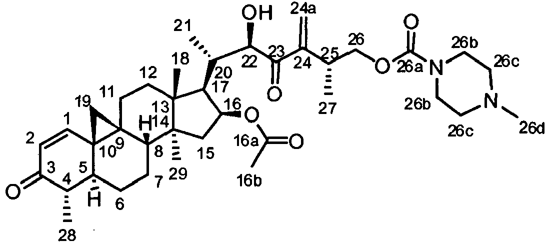 | 23 |
| | Formule Chimique : C₃₈H₅₆N₂O₇ |
| | Masse Exacte : 652.41 |
| | Poids moléculaire : 652.86 |
| 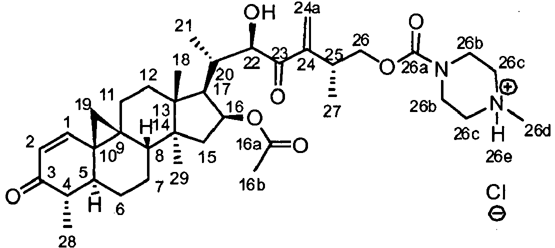 | 24 |
| | Formule Chimique : C₃₈H₅₇ClN₂O₇ |
| | Masse Exacte : 688.39 |
| | Poids moléculaire : 689.32 |

Protocole : 200 mg de 1 (0.38 mmol) sont solubilisés dans l'acétonitrile anhydre. 194 mg (1.408 mmol, 4 eq.) de carbonate de potassium, 140 mg (0.704 mmol, 2 eq.) de chlorhydrate du chlorure de carbonyle de la 4-méthylpiperazine et 11 mg (0.035 mmol, 0.1 eq.) de bromure de tétrabutyle ammonium sont ajoutés. Le milieu réactionnel est agité pendant 90 heures à température ambiante. Le milieu réactionnel est filtré et concentré à l'évaporateur rotatif. Le résidu est purifié par chromatographie sur gel de silice (éluant : DCM/MeOH : 95/5). Deux produits sont isolés : le carbamate 23 (87 mg, 35%), obtenu sous forme d'un solide blanc et le produit de départ 1 (96 mg, 48%). 3 mL d'HCl 0.1 M sont ajoutés à 69 mg de carbamate 23 et le milieu réactionnel est agité à température ambiante pendant 1 heure. Le milieu réactionnel est ensuite lyophilisé afin d'obtenir 66 mg de chlorhydrate 24 (90%).

### exemple 23 :

¹H NMR (500MHz, ACETONITRILE-d₃) δ = 6.94 (1H, d, J = 9.8 Hz, H-1), 6.18 (1H, s, H-24aa), 6.04 (1H, d, J = 0.6 Hz, H-24ab), 5.89 (1H, d, J = 10.1 Hz, H-2), 5.20 (1H, td, J = 7.5 Hz, J = 4.6 Hz, H-16), 4.72 (1H, br. s., H-22), 4.05 (2H, d, J = 6.4 Hz, H-26), 3.54 (1H, d, J = 5.2 Hz, H-30), 3.32 - 3.40 (4H, m, H-26b), 3.00 - 3.10 (1H, m, H-25), 2.39 - 2.49 (2H, m, H-20, 17), 2.23 - 2.28 (4H, m, H-26c), 2.20 - 2.23 (1H, m, H-15<'>), 2.20 (3H, s, H-26d), 2.16 - 2.19 (1H, m, H-4), 2.03 (3H, s, H-16b), 1.96 - 2.02 (3H, m, H-11<'>, 8a, 5a), 1.58 - 1.74 (3H, m, H-6<">, 12), 1.51 - 1.58 (1H, m, H-11<">), 1.42 - 1.49 (1H, m, H-7<">), 1.32 - 1.41 (1H, m, H-15<">), 1.23 (1H, d, J = 4.3 Hz, H-19<">), 1.18 - 1.22 (1H, m, H-7<'>), 1.17 (3H, s, H-18), 1.10 (3H, d, J = 7.3 Hz, H-27), 1.03 (3H, d, J = 6.7 Hz, H-28), 0.96 (3H, s, H-29), 0.88-0.95 (1H, m, H-6<'>), 0.64 (3H, d, J = 6.1 Hz, H-21), 0.57 (1H, d, J = 4.6 Hz, H-19<'>)
¹³C NMR (126MHz, ACETONITRILE-d₃) δ = 204.9 (C-23), 202.4 (C-3), 171.2 (C-16a), 156.0 (C-26a), 155.6 (C-1), 148.1 (C-24), 128.4 (C-2), 127.5 (C-24a), 77.3 (C-16), 75.8 (C-22), 68.8 (C-26), 55.5 (C-26c), 51.4 (C-17), 48.3 (C-14), 47.6 (C-4), 46.9 (C-15), 46.7 (C-13), 46.4 (C-26d), 45.3 (C-8), 44.6 (C-26b), 43.6 (C-5), 36.4 (C-20), 35.3 (C-25), 33.1 (C-12), 32.9 (C-10), 28.1 (C-11), 27.7 (C-19), 27.2 (C-9), 24.3 (C-6), 24.3 (C-7), 22.1 (C-16b), 20.1 (C-29), 18.6 (C-18), 17.2 (C-27), 13.8, 12.3 (C-21), 11.3 (C-28)

### exemple 24 :

¹H NMR (500MHz, DMSO-d₆) δ = 10.30 (1H, br. s., H-26e), 6.97 (1H, d, J = 10.1 Hz, H-1), 6.10 (1H, s, H-24aa), 6.03 (1H, s, H-24ab), 5.90 (1H, d, J = 10.1 Hz, H-2), 5.15 (1H, dd, J = 7.4 Hz, J = 4.5 Hz, H-16), 4.79 (1H, d, J = 6.1 Hz, H-30), 4.60 (1H, d, J = 3.7 Hz, H-22), 3.96 - 4.08 (4H, m, H-26, 26b), 3.34 - 3.42 (2H, m, H-26c), 3.09 - 3.21 (2H, m, H-26b), 2.89 - 3.03 (3H, m, H-25, 26c), 2.76 (3H, br. s., H-26d), 2.29 - 2.39 (2H, m, H-20, 17), 2.07 - 2.16 (2H, m, H-4, 15<'>), 2.02 (3H, s, H-16b), 1.93 - 2.00 (2H, m, H-11<'>, 8a), 1.90 (1H, td, J = 12.5 Hz, J = 4.4 Hz, H-5a), 1.50 - 1.64 (4H, m, H-12, 6<'>, 11<">), 1.37 - 1.44 (1H, m, H-7<'>), 1.27 - 1.34 (1H, m, H-15<'>), 1.24 (1H, d, J = 4.3 Hz, H-19<'>), 1.13 - 1.19 (1H, m, H-7<">), 1.11 (3H, s, H-18), 1.05 (3H, d, J = 7.0 Hz, H-27), 0.98 (3H, d, J = 6.7 Hz, H-28), 0.93 (2H, t, J = 7.3 Hz, H-6<'>), 0.90 (3H, s, H-29), 0.64 (3H, d, J = 6.4 Hz, H-21), 0.55 - 0.59 (1H, m, H-19<">)
¹³C NMR (126MHz, DMSO-d₆) δ = 203.5 (C-23), 200.7 (C-3), 170.0 (C-16a), 154.8 (C-1), 154.0 (C-26a), 147.4 (C-24), 127.3 (C-2), 125.0 (C-24a), 75.3 (C-16), 74.2 (C-22), 68.5 (C-26), 51.9 (C-26c), 49.6 (C-17), 47.0 (C-14), 46.2 (C-4), 45.4 (C-15), 45.2 (C-13), 43.3 (C-8), 42.0 (C-26d, 5), 40.5 (C-26b), 34.4 (C-20), 33.2 (C-25), 31.8 (C-10, 12), 31.6, 26.7 (C-11), 26.3 (C-9), 26.0 (C-19), 22.9 (C-6), 22.8 (C-7), 21.5 (C-16b), 19.2 (C-29), 17.7 (C-18), 16.6 (C-27), 11.8 (C-21), 10.8 (C-28)

### ◆ exemples 25 : 26-N-(4-N,N-diméthylaniline)carbamate-neoboutomellerone

| | |
|---|---|
| 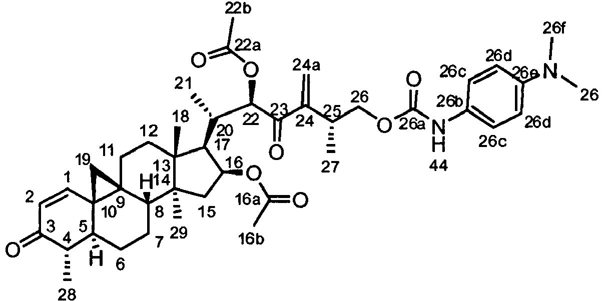 | Formule Chimique : C₄₃H₅₈N₂O₈ |
| | Masse Exacte : 730,42 |
| | Poids moléculaire : 730,93 |

Protocole : 100 mg (0.176 mmol) de 2 sont solubiliséq dans 1 mL de dichlorométhane anhydre sous azote. 11 mg (0.080 mmol, 0.5 eq.) de DMAP, 43 mg (0.264 mmol, 1.5 eq.) d'isocyanate de diméthylaminophényle et 40 µL (0.264 mmol, 1.5 eq.) de triéthylamine sont ajoutés et le milieu réactionnel est agité à température ambiante. Après 24h, 1 équivalent d'isocyanate de diméthylaminophényle (m= 29 mg) est ajouté et le milieu réactionnel est agité à température ambiante pendant 18 heures. Le milieu réactionnel est dilué dans l'acétate d'éthyle et la phase organique est lavée successivement avec une solution d'HCl 4%, une solution saturée d'hydrogénocarbonate de sodium et une solution saturée de chlorure de sodium. La phase organique est séchée sur MgSO₄, filtrée et concentrée à l'évaporateur rotatif. Le produit est purifié par chromatographie sur silice (éluant : Cyclohexane/AcOEt : 6/4). Un solide blanc 25 est obtenu avec un rendement de 79% (101 mg).
¹H NMR (500MHz, ACETONITRILE-d₃) δ = 7.32 (1H, br. s, H-44), 7.20 (2H, d, J = 8.2 Hz, H-26c), 6.94 (1H, d, J = 9.8 Hz, H-1), 6.71 (2H, d, J = 9.1 Hz, H-26d), 6.13 (1H, s, H-24aa), 5.99 (1H, s, H-24ab), 5.90 (1H, d, J = 10.1 Hz, H-2), 5.55 (1H, d, J = 2.1 Hz, H-22), 5.10 (1H, td, J = 7.6 Hz, J = 4.3 Hz, H-16), 4.11 (1H, dd, J = 10.6 Hz, J = 7.1 Hz, H-26<'>), 4.03 (1H, dd, J = 10.6 Hz, J = 6.3 Hz, H-26<">), 3.05 (1H, sxt, J = 6.9 Hz, H-25), 2.86 (6H, s, H-26f), 2.56 - 2.65 (1H, m, H-20), 2.30 (1H, dd, J = 11.0 Hz, J = 7.6 Hz, H-17), 2.14 - 2.22 (2H, m, H-4, 15<'>), 2.10 (3H, s, H-22b), 2.03 (3H, s, H-16b), 1.96 - 2.01 (3H, m, H-11<'>, 8a, 5a), 1.62 - 1.76 (3H, m, H-6<'>, 12), 1.56 (1H, qd, J = 8.7 Hz, J = 6.3 Hz, H-11<">), 1.41 - 1.49 (1H, m, H-7<'>), 1.37 (1H, dd, J = 13.9 Hz, J = 3.8 Hz, H-15<">), 1.24 (1H, d, J = 4.3 Hz, H-19<'>), 1.20 - 1.23 (1H, m, H-7<">), 1.18 (3H, s, H-18), 1.10 (3H, d, J = 7.0 Hz, H-27), 1.03 (3H, d, J = 7.0 Hz, H-28), 0.96 (3H, s, H-29), 0.88 - 0.94 (1H, m, H-6<">), 0.86 (3H, d, J = 6.7 Hz, H-21), 0.57 (1H, d, J = 4.3 Hz, H-19<">)
¹³C NMR (126MHz, ACETONITRILE-d₃) δ = 202.4 (C-3), 198.9 (C-23), 171.8 (C-22a), 171.3 (C-16a), 155.5 (C-1), 155.1 (C-26a), 149.4 (C-24), 148.8 (C-26e), 129.4 (C-26b), 128.5 (C-2), 125.5 (C-24a), 121.8 (C-26c, 26c), 114.2 (C-26d, 26d), 78.5 (C-22), 76.7 (C-16), 68.3 (C-26), 51.4 (C-17), 48.4 (C-14), 47.7 (C-4), 46.9 (C-13), 46.8 (C-15), 45.2 (C-8), 43.6 (C-5), 41.3 (C-26f, 26f), 35.3 (C-25), 33.4 (C-20), 33.0 (C-12), 33.0 (C-10), 28.1 (C-11), 27.7 (C-19), 27.3 (C-9), 24.3 (C-7, 6), 22.1 (C-16b), 21.0 (C-22b), 20.0 (C-29), 18.3 (C-18), 17.2 (C-27),13.4 (C-21), 11.3 (C-28)

### ◆ exemple 26 : 26-N-(4-N,N-diméthylaniline)carbamate-22-déacétyl,neoboutomellerone

| | |
|---|---|
| 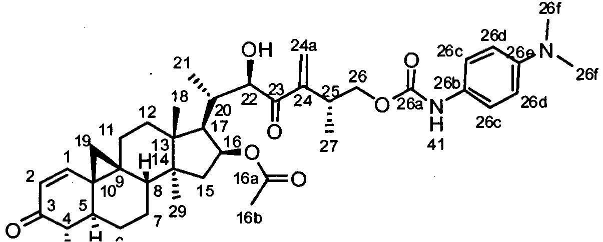 | Formule Chimique : C₄₁H₅₆N₂O₇ |
| | Masse Exacte : 688,41 |
| | Poids moléculaire : 688,89 |

Protocole : 100 mg (0.19 mmol) de 2 sont solubilisés dans 1 mL de dichlorométhane anhydre sous azote. 12 mg (0.095 mmol, 0.5 eq.) de DMAP, 76 mg (0.475 mmol, 2.5 eq.) d'isocyanate de diméthylaminophényle et 40 µL (0.285 mmol, 1.5 eq.) de triéthylamine sont ajoutés et le milieu réactionnel est agité à température ambiante. Après 22h, le milieu réactionnel est dilué dans l'acétate d'éthyle et la phase organique est lavée successivement avec une solution d'HCl 4%, une solution saturée d'hydrogénocarbonate de sodium et une solution saturée de chlorure de sodium. La phase organique est séchée sur MgSO₄, filtrée et concentrée à l'évaporateur rotatif. Le produit est purifié par chromatographie sur silice (éluant : Cyclohexane/AcOEt :7/3). Un solide blanc 26 est obtenu avec un rendement de 60% (79 mg).
¹H NMR (500MHz, ACETONITRILE-d₃) δ = 7.32 (1H, br. s, H-41a), 7.19 (2H, br. s., H-26c), 6.93 (1H, d, J = 10.1 Hz, H-1), 6.71 (2H, d, J = 8.9 Hz, H-26d), 6.19 (1H, s, H-24aa), 6.09 (1H, d, J = 16.2 Hz, H-24ab), 5.89 (1H, d, J = 9.8 Hz, H-2), 5.21 (1H, td, J = 7.5 Hz, J = 4.9 Hz, H-16), 4.72 (1H, dd, J = 6.1 Hz, J = 1.5 Hz, H-22), 4.14 (1H, dd, J = 10.7 Hz, J = 6.5 Hz, H-26<'>), 4.08 (1H, dd, J = 10.6 Hz, J = 6.3 Hz, H-26<">), 3.53 (1H, d, J = 6.2 Hz, H-30), 3.08 (1H, sxt, J = 6.9 Hz, H-25), 2.86 (6H, s, H-26f), 2.40-2.51 (2H, m, H-20, 17), 2.14 - 2.25 (2H, m, H-4, 15<'>), 2.03 - 2.07 (1H, m, H-11<'>), 2.02 (3H, s, H-16b), 1.97 - 2.01 (2H, m, H-8a, 5a), 1.60 - 1.72 (3H, m, H-6<'>, 12), 1.54 (1H, qd, J = 8.8 Hz, J = 5.9 Hz, H-11<">), 1.42 - 1.48 (1H, m, H-7<'>), 1.38 (1H, dd, J = 14.6 Hz, J = 4.5 Hz, H-15<">), 1.24 (1H, d, J = 4.6 Hz, H-19<'>), 1.19 - 1.21 (1H, m, H-7<">), 1.17 (3H, s, H-18), 1.13 (3H, d, J = 7.0 Hz, H-27), 1.03 (3H, d, J = 7.0 Hz, H-28), 0.96 (3H, s, H-29), 0.90 - 0.94 (1H, m, H-6<">), 0.65 (3H, d, J = 6.1 Hz, H-21), 0.57 (1H, d, J = 4.3 Hz, H-19<">)
¹³C NMR (126MHz, ACETONITRILE-d₃) δ = 205.0 (C-23), 202.4 (C-3), 171.3 (C-16a), 155.6 (C-26a, 1), 155.2, 148.8 (C-26e), 148.2 (C-24), 129.5 (C-26b), 128.4 (C-2), 127.3 (C-24a), 121.9 (C-26c, 26c), 114.2 (C-26d, 26d), 77.3 (C-22), 75.9 (C-16), 68.3 (C-26), 51.5 (C-17), 48.4 (C-14), 47.7 (C-4), 46.9 (C-15), 46.7 (C-13), 45.4 (C-8), 43.7 (C-5), 41.3 (C-26f, 26f), 36.4 (C-25), 35.2 (C-20), 33.2 (C-12), 33.0 (C-10), 28.2 (C-11), 27.8 (C-19), 27.3 (C-9), 24.4 (C-7, 6), 22.1 (C-16b), 20.2 (C-29), 18.6 (C-18), 17.3 (C-27), 12.4 (C-21), 11.3 (C-28)

### ◆ exemples 27 & 28 : 3-anti oxime- neoboutomellerone (27) et 3-syn oxime-neoboutomellerone (28)

| | |
|---|---|
| 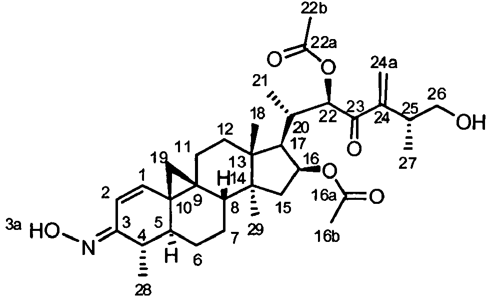 | 27 |
| | Formule Chimique : C₃₄H₄₉O₇ |
| | Masse Exacte : 583,35 |
| | Poids moléculaire : 583,76 |
| 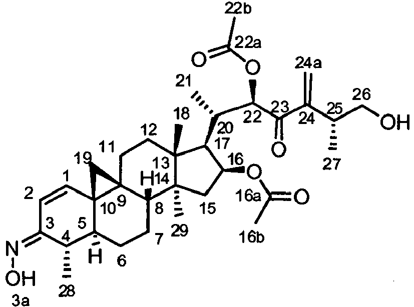 | 28 |
| | Formule Chimique : C₃₄H₄₉O₇ |
| | Masse Exacte : 583,35 |
| | Poids moléculaire : 583,76 |

Protocole : 28.40 mg (0.05 mmol) de 2 sont solubilisés dans 700 µL d'un mélange Dioxane/Méthanol (1/1) et 20.84 mg (0.15 mmol, 6 eq.) d'hydroxylamine hydrochloré en solution dans 200 µL d'eau est ajouté. Après 20 heures d'agitation à température ambiante, Le milieu réactionnel est dilué dans le dichlorométhane et filtré sur célite. Après concentration du filtrat à l'évaporateur rotatif, le produit est purifié par chromatographie sur gel de silice (éluant : DCM/MeOH :100/0 à 98/2) afin d'obtenir 27 (5 mg, 2%) et 28 (2.88 mg, 1%).

### exemple 27 :

¹H RMN (500MHz, CD₃CN) δ = 8.29 (1H, s, H-3a), 6.70 (1H, d, J = 10.4 Hz, H-2), 6.21 (1H, d, J = 10.1 Hz, H-1), 6.04 (1H, s, H-24a'), 5.89 (1H, d, J = 0.9 Hz, H-24a"), 5.53 (1H, d, J = 2.1 Hz, H-22), 5 .08 (1H, td, J = 7.7 Hz, J = 4.4 Hz, H-16), 3.53 (1H, dt, J = 10.8 Hz, J = 5.6 Hz, H-26'), 3.39 (1H, dt, J = 10.9 Hz, J = 5.7 Hz, H-26"), 2.77 (1H, sxt, J = 6.7 Hz, H-25), 2.69 (1H, t, J = 5.6 Hz, OH-26), 2.53 - 2.64 (1H, dqd, J = 11.2 Hz, J = 2.1 Hz, H-20), 2.28 (1H, dd, J = 11.0 Hz, J = 7.6 Hz, H-17), 2.24 (1H, dq, J = 11.6 Hz, J = 6.7 Hz, H-4), 2.15 (1H, m, H-15'), 2.09 (3H, s, H-22b), 2.02 (3H, s, H-16b), 1.96 - 2.04 (2H, m, H-11', 8), 1.61 - 1.76 (4H, m, H-6', 12', 12", 5), 1.50 - 1.60 (1H, m, H-11"), 1.40 - 1.49 (1H, m, H-7'), 1.35 (1H, dd, J = 14.0 Hz, J = 4.0 Hz, H-15"), 1.17 (3H, s, H-18), 1.13 - 1.20 (1H, m, H-7"), 1.07 (3H, d, J = 6.7 Hz, H-28), 1.03 (3H, d, J = 7.0 Hz, H-27), 1.00 (1H, d, J = 4.6 Hz, H-19'), 0.93 (3H, s, H-29), 0.84 (3H, d, J = 7.0 Hz, H-21), 0.78 - 0.90 (1H, m, H-6"), 0.41 (1H, d, J = 4.3 Hz, H-19")
¹³C RMN (126MHz, CD₃CN) δ = 199.6 (C-23), 171.7 (C-22a), 171.3 (C-16a), 157.7 (C-3), 150.3 (C-24), 143.3 (C-1), 124.6 (C-24a), 116.4 (C-2), 78.5 (C-22), 76.6 (C-16), 66.4 (C-26), 51.2 (C-17), 48.4 (C-14), 46.8 (C-13), 46.6 (C-15), 44.7 (C-8), 43.4 (C-5), 39.7 (C-4), 37.9 (C-25), 33.2 (C-20), 33.0 (C-12), 32.7 (C-10), 28.1 (C-11), 27.5 (C-19), 26.7 (C-9), 24.2 (C-7), 23.7 (C-6), 22.1 (C-16b), 20.9 (C-22b), 19.9 (C-29), 18.1 (C-18), 17.2 (C-27), 13.3 (C-21), 13.1 (C-28)

### exemple 28 :

¹H RMN (500MHz, CD₃CN) δ = 8.44 (1H, br. s., H-3a), 6.25 (1H, d, J = 9.8 Hz, H-1), 6.11 (1H, d, J = 10.1 Hz, H-2), 6.04 (1H, s, H-24a'), 5.89 (1H, d, J = 0.9 Hz, H-24a"), 5.53 (1H, d, J = 2.1 Hz, H-22), 5.08 (1H, td, J = 7.6 Hz, J = 4.3 Hz, H-16), 3.53 (1H, dt, J = 10.7 Hz, J = 6.0 Hz, H-26'), 3.39 (1H, dt, J = 10.7 Hz, J = 6.1 Hz, H-26"), 2.77 (1H, sxt, J = 6.7 Hz, H-25), 2.69 (1H, t, J = 5.8 Hz, OH-26), 2.54 - 2.65 (1H, dqd, J = 11.0 Hz, J = 7.0 Hz, J = 2.3 Hz, H-20), 2.41 (1H, dq, J = 11.0 Hz, J = 6.7 Hz, H-4), 2.29 (1H, dd, H-17), 2.16 - 2.21 (1H, m, H-15'), 2.09 (3H, s, H-22b), 2.08 - 2.12 (1H, m, H-11'), 2.02 (3H, s, H-16b), 1.82 - 1.88 (2H, m, H-6', 8), 1.65 - 1.77 (2H, m, H-12', 12"), 1.61 (1H, td, J = 11.4 Hz, J = 4.1 Hz, H-5), 1.36 (1H, dd, J = 13.6 Hz, J = 4.4 Hz, H-15"), 1.33 - 1.41 (1H, m, H-7'), 1.29 (3H, d, J = 6.4 Hz, H-28), 1.24 - 1.33 (1H, m, H-11"), 1.18 (3H, s, H-18), 1.14 - 1.21 (1H, m, H-7"), 1.03 (3H, d, J = 7.0 Hz, H-27), 0.97 (3H, s, H-29), 0.82 - 0.84 (1H, m, H-19'), 0.84 (3H, d, J = 7.0 Hz, H-21), 0.73 (1H, qd, J = 13.1 Hz, J = 3.1 Hz, H-6"), 0.26 (1H, d, H-19")
¹³C RMN (126MHz, CD₃CN) δ = 199.6 (C-23), 171.7 (C-22a), 171.3 (C-16a), 160.5 (C-3), 150.3 (C-24), 140.7 (C-1), 126.3 (C-2), 124.7 (C-24a), 78.5 (C-22), 76.8 (C-16), 66.4 (C-26), 51.3 (C-17), 48.2 (C-14), 47.2 (C-15), 47.0 (C-13), 46.9 (C-8), 44.7 (C-5), 37.9 (C-25), 37.7 (C-4), 33.2 (C-20), 33.2 (C-12), 28.6 (C-19), 28.5 (C-11), 25.3 (C-7), 25.0 (C-6), 22.1 (C-16b), 20.9 (C-22b), 20.3 (C-29), 18.9 (C-18), 17.2 (C-27), 16.0 (C-28), 13.3 (C-21)

### ◆ exemples 29, 30 et 31 : 3-anti O-méthyloxime- neoboutomellerone (29), 3-anti O-méthyloxime-24a-O-methylhydroxylamine neoboutomellerone (30) et 24a-O-methylhydroxylamine neoboutomellerone (31).

| | |
|---|---|
| 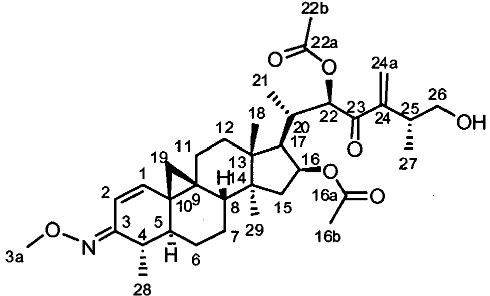 | 29 |
| | Formule Chimique : C₃₅H₅₁O₇ |
| | Masse Exacte : 597,37 |
| | Poids moléculaire : 597,78 |
| 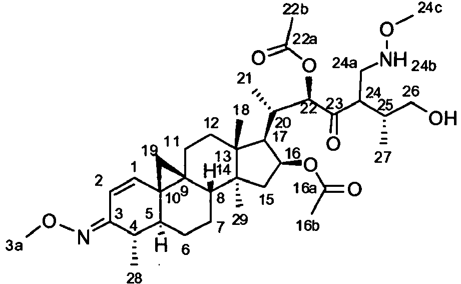 | 30 |
| | Formule Chimique : C₃₆H₅₆N₂O₈ |
| | Masse Exacte : 644,40 |
| | Poids moléculaire : 644,84 |
| 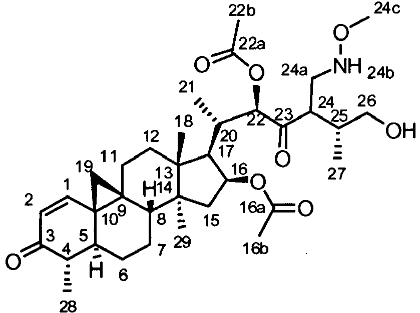 | 32 |
| | Formule Chimique : C₃₅H₅₃NO₈ |
| | Masse Exacte : 615,38 |
| | Poids moléculaire : 615,80 |

Protocole : 21.7 mg (0.26 mmol, 4 eq.) de chlorhydrate de O-methylhydoxylamine et 38 mg (0.286 mmol, 4.4 eq.) d'acétate de sodium sont dissous dans 1.3 mL de méthanol. 37 mg (0.065 mmol) de 2 sont ensuite ajoutés. Après 10 heures d'agitation à température ambiante, le milieu réactionnel est dilué dans l'acétate d'éthyle et la phase organique est lavée avec de l'eau distillée. La phase aqueuse est extraite 3 fois à l'acétate d'éthyle et les phases organiques rassemblées sont lavées avec une solution saturée de chlorure de sodium et séchées sur MgSO₄. Après concentration à l'évaporateur rotatif, le produit est purifié par chromatographie sur gel de silice (éluant : Cyclohexane/AcOEt : 8/2 à 4/6) afin d'obtenir les produits 29 (24 mg, 62%), 30 (3.2 mg, 8%) et 31 (0.75 mg, 2%).

### exemple 29 :

¹H RMN (500MHz, CD₃CN) δ = 6.61 (1H, d, J = 10.1 Hz, H-2), 6.24 (1H, d, J = 10.1 Hz, H-1), 6.04 (1H, s, H-24a'), 5.89 (1H, d, J = 0.9 Hz, H-24a"), 5.53 (1H, d, J = 2.1 Hz, H-22), 5.08 (1H, td, J = 7.6 Hz, J = 4.6 Hz, H-16), 3.78 (3H, s, H-3a), 3.53 (1H, dt, J = 10.7 Hz, J = 6.0 Hz, H-26'), 3.39 (1H, dt, J = 10.4 Hz, J = 6.3 Hz, H-26"), 2.76 (1H, sxt, J = 6.8 Hz, H-25), 2.68 (1H, t, J = 5.8 Hz, OH-26), 2.54 - 2.64 (1H, m, H-20), 2.20 - 2.33 (2H, m, H-4, 17), 2.13 - 2.18 (1H, m, H-15'), 2.02 (3H, s, H-16b), 1.96 - 2.05 (2H, m, H-8, 11'), 1.97 (3H, s, H-22b), 1.60 - 1.76 (4H, m, H-6', 5, 12', 12"), 1.50 - 1.59 (1H, m, H-11"), 1.40 - 1.49 (1H, m, H-7'), 1.35 (1H, dd, J = 14.2 Hz, J = 4.1 Hz, H-15"), 1.16 - 1.21 (1H, m, H-7"), 1.17 (3H, s, H-18), 1.09 (3H, d, J = 6.7 Hz, H-28), 1.01 (1H, d, J = 4.6 Hz, H-19'), 1.03 (3H, d, J = 7.0 Hz, H-27), 0.92 (3H, s, H-29), 0.79 - 0.88 (1H, m, H-6"), 0.84 (3H, d, J = 7.0 Hz, H-21), 0.41 (1H, d, J = 4.6 Hz, H-19")
¹³C RMN (126MHz, CD₃CN) δ = 199.6 (C-23), 171.7 (C-22a), 171.3 (C-16a), 157.4 (C-3), 150.3 (C-24), 144.4 (C-1), 124.6 (C-24a), 116.7 (C-2), 78.5 (C-22), 76.6 (C-16), 66.4 (C-26), 61.8 (C-3a), 51.2 (C-17), 48.4 (C-14), 46.8 (C-13), 46.6 (C-15), 44.7 (C-8), 43.4 (C-5), 39.7 (C-4), 37.9 (C-25), 33.2 (C-20), 33.0 (C-12), 32.7 (C-10), 28.1 (C-11), 27.5 (C-19), 26.8 (C-9), 24.2 (C-7), 23.7 (C-6), 22.1 (C-16b), 21.2 (C-22b), 19.9 (C-29), 18.0 (C-18), 17.2 (C-27), 13.3 (C-21), 13.1 (C-28)

### exemple 30 :

¹H NMR (500MHz, CD₃CN) δ = 6.61 (1H, d, J = 10.4 Hz, H-2), 6.24 (1H, d, J = 10.1 Hz, H-1), 6.20 (1H, br. s., H-24b), 5.09 (1H, td, J = 7.6 Hz, J = 4.6 Hz, H-16), 4.99 (1H, d, J = 0.6 Hz, H-22), 3.78 (3H, s, H-3a), 3.46 - 3.53 (1H, m, H-26'), 3.39 - 3.46 (1H, m, H-26"), 3.37 (3H, s, H-24c), 3.25 (1H, td, J = 8.1 Hz, J = 4.6 Hz, H-24), 3.07 - 3.17 (1H, m, J = 14.3 Hz, H-24a'), 2.94 - 3.03 (1H, m, J = 9.8 Hz, H-24a"), 2.83 (1H, t, J = 4.7 Hz, OH-26), 2.70 - 2.80 (1H, m, H-20), 2.22 - 2.30 (1H, m, H-4), 2.19 (1H, dd, J = 11.3 Hz, J = 7.6 Hz, H-17), 2.14 - 2.16 (1H, m, H-15'), 2.11 (3H, s, H-22b), 2.08 (3H, s, H-16b), 2.04 (1H, dd, J = 9.3 Hz, J = 7.2 Hz, H-8), 1.92 - 1.94 (1H, m, H11'),1.82 - 1.89 (1H, m, H-25), 1.52 - 1.76 (5H, m, H-12', 12", 5, 11', 6'), 1.42 - 1.51 (1H, m, H-7'), 1.36 (1H, dd, J = 13.6 Hz, J = 4.1 Hz, H-15"), 1.19 (3H, s, H-18), 1.17 - 1.23 (1H, m, H-7"), 1.09 (3H, d, J = 6.7 Hz, H-28), 1.02 (1H, d, J = 4.0 Hz, H-19'), 0.90 (3H, s, H-29), 0.90 (3H, d, J = 6.7 Hz, H-27), 0.85 - 0.92 (1H, m, H-6"), 0.86 (3H, d, J = 6.7 Hz, H-21), 0.40 (1H, d, J = 4.3 Hz, H-19")
¹³C NMR (126MHz, CD₃CN) δ = 210.8 (C-23), 172.4 (C-22a), 171.5 (C-16a), 157.4 (C-3), 144.3 (C-1), 116.8 (C-2), 82.3 (C-22), 76.2 (C-16), 64.9, 61.8 (C-3a), 61.3 (C-24c), 52.8 (C-24a), 51.2 (C-17), 48.5 (C-14), 48.3 (C-24), 46.7 (C-13), 46.3 (C-15), 44.2 (C-19), 43.2 (C-5), 39.6 (C-4), 36.6 (C-25), 32.8 (C-10), 32.7 (C-12), 30.4 (C-20), 28.0 (C-11), 27.1 (C-19), 26.8 (C-9), 24.0 (C-7), 23.6 (C-6), 22.1 (C-16b), 21.0 (C-22b), 19.7 (C-29), 17.7 (C-18), 16.3 (C-27), 13.4 (C-21), 13.1 (C-28)

### exemple 31 :

¹H NMR (500MHz, CD₃CN) δ = 6.94 (1H, d, J = 10.1 Hz, H-1), 6.20 (1H, dd, J = 9.8 Hz, J = 5.2 Hz, H-24b), 5.90 (1H, d, J = 10.1 Hz, H-2), 5.10 (1H, td, J = 7.8 Hz, J = 4.6 Hz, H-16), 5.00 (1H, d, J = 0.6 Hz, H-22), 3.49 (1H, dq, J = 10.8 Hz, J = 5.4 Hz, H-26'), 3.38 - 3.46 (1H, m, H-26"), 3.37 (3H, s, H-24c), 3.21 - 3.29 (1H, m, H-24), 3.13 (1H, ddd, J = 12.9 Hz, J = 8.8 Hz, J = 4.3 Hz, H-24a'), 2.99 (1H, ddd, J = 13.0 Hz, J = 8.3 Hz, J = 4.9 Hz, H-24a"), 2.84 (1H, t, J = 5.3 Hz, OH-26), 2.71 - 2.80 (1H, m, H-20), 2.16 - 2.23 (3H, m, H-4, 15', 17), 2.11 (3H, s, H-22b), 2.08 (3H, s, H-16b), 2.06 - 2.10 (1H, m, H-8), 1.93 - 1.98 (2H, m, H-5, 11'), 1.83 - 1.90 (1H, m, H-25), 1.63 - 1.75 (3H, m, H-6', 12', 12"), 1.55 - 1.63 (2H, m, H-11"), 1.41 - 1.50 (1H, m, H-7'), 1.37 (1H, dd, J = 14.0 Hz, J = 4.6 Hz, H-15"), 1.26 (1H, d, J = 4.3 Hz, H-19'), 1.20 (3H, s, H-18), 1.18 - 1.22 (1H, m, H-7"), 1.02 (3H, d, J = 6.7 Hz, H-28), 0.93 (3H, s, H-29), 0.90 (3H, d, J = 7.0 Hz, H-27), 0.87 (3H, d, J = 7.0 Hz, H-21), 0.56 (1H, d, J = 4.6 Hz, H-19")
¹³C NMR (126MHz, CD₃CN) δ = 210.8 (C-23), 202.4 (C-3), 172.4 (C-22a), 171.5 (C-16a), 155.5 (C-1), 128.4 (C-2), 82.3 (C-22), 76.2 (C-16), 64.9 (C-26), 61.3 (C-24c), 52.8 (C-24a), 51.3 (C-17), 48.5 (C-14), 48.3 (C-24), 47.6 (C-4), 46.7 (C-13), 46.4 (C-15), 44.7 (C-8), 43.4 (C-5), 36.7 (C-25), 33.0 (C-10), 32.7 (C-12), 30.4 (C-20), 28.0 (C-11), 27.7 (C-9), 27.3 (C-19), 24.2 (C-7), 24.1 (C-6), 22.1 (C-16b), 21.0 (C-22b), 19.8 (C-29), 17.9 (C-18), 16.3 (C-27), 13.3 (C-21), 11.3 (C-28)

### ◆ exemples 32,33 & 34 (comparatifs) :

| | |
|---|---|
| 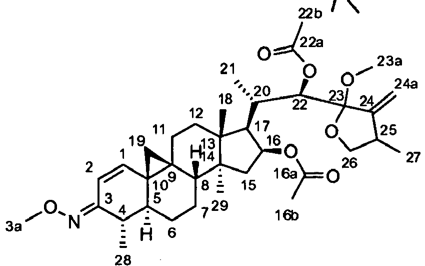 | **32** |
| | Formule Chimique : C₃₆H₅₃NO₇ |
| | Masse Exacte : 611.38 |
| | Poids moléculaire : 611.81 |
| 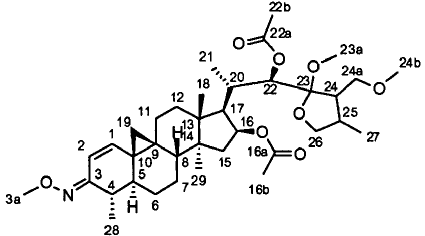 | **33** |
| | Formule Chimique : C₃₇H₅₇NO₈ |
| | Masse Exacte : 643.41 |
| | Poids moléculaire : 643.85 |
| 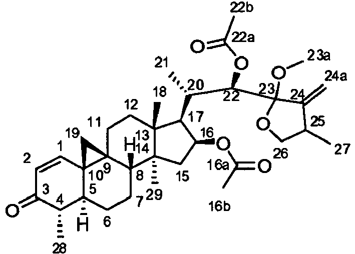 | **34** |
| | Formule Chimique : C₃₅H₅₀O₇ |
| | Masse Exacte : 582.36 |
| | Poids moléculaire : 582.77 |

Protocole : A 114 mg de composé 2 solubilisés dans un mélange de MeOH et de dioxane (1/1, v = 2.8 mL) est ajouté 50 mg de chlorhydrate de O-méthylhydroxylamine. Après 30 minutes d'agitation à température ambiante, le milieu réactionnel est dilué dans l'acétate d'éthyle et la phase organique est lavée avec de l'eau distillée. La phase aqueuse est extraite 3 fois à l'acétate d'éthyle et les phases organiques rassemblées sont lavées avec une solution saturée de chlorure de sodium et séchées sur MgSO₄. Après concentration à l'évaporateur rotatif, le résidu brut est purifié par chromatographie sur gel de silice (éluant : Cyclohexane/AcOEt : 8/2 à 5/5) afin d'obtenir les produits 32 (13 mg, 11%), 33 (4.5 mg, 4%) et 34 (18 mg, 15%).

### exemple 32 :

¹H NMR (500MHz, CD₃CN) δ = 6.60 (1H, d, J = 10.1 Hz, H-2), 6.23 (1H, d, J = 10:1 Hz, H-1), 5.55 (1H, td, J = 7.9 Hz, J = 4.9 Hz, H-16), 5.13 (1H, d, J = 3.1 Hz, H-24a'), 4.97 (1H, d, J = 3.1 Hz, H-24a"), 4.91 (1H, s, H-22), 4.21 (1H, t, J = 8.2 Hz, H-26'), 3.77 (3H, s, H-3a), 3.42 (1H, dd, J = 9.2 Hz, J = 7.9 Hz, H-26"), 3.09 (3H, s, H-23a), 2.73 - 2.82 (1H, m, H-25), 2.64 - 2.73 (1H, m, H-20), 2.23 (1H, dq, J = 13.1 Hz, J = 6.7 Hz, H-4), 1.97 - 2.05 (2H, m, H-15', 8), 1.94 - 1.97 (6H, m, H-16b, 22b), 1.81 - 1.91 (2H, m, H-11', 17), 1.65 - 1.74 (2H, m, H-5, H6'), 1.53 - 1.65 (3H, m, H-12', 12", 11"), 1.42 - 1.50 (1H, m, H-7'), 1.31 (1H, dd, J = 14.2 Hz, J = 5.0 Hz, H-15"), 1.16 (3H, s, H-18), 1.13 - 1.18 (1H, m, H-7"), 1.10 (3H, d, J = 6.7 Hz, H-21), 1.08 (3H, d, J = 6.7 Hz, H-28), 1.03 (3H, d, J = 6.7 Hz, H-27), 1.02 (1H, d, J = 3.0 Hz, H-19'), 0.83 - 0.90 (1H, m, H-6"), 0.81 (3H, s, H-29), 0.38 (1H, d, J = 4.6 Hz, H-19")
¹³C NMR (126MHz, CD₃CN) δ = 171.5 (C-16a), 171.4 (C-22a), 157.4 (C-3), 154.3 (C-24), 144.4 (C-1), 116.7 (C-2), 110.5 (C-23), 109.3 (C-24a), 78.2 (C-22), 75.1 (C-16), 75.0 (C-26), 61.8 (C-3a), 52.9 (C-17), 49.8 (C-23a), 48.5 (C-14), 46.6 (C-13), 45.3 (C-15), 43.9 (C-8), 43.1 (C-5), 39.6 (C-4), 38.0 (C-25), 32.9 (C-12), 32.8 (C-10), 31.8 (C-20), 28.1 (C-11), 27.0 (C-9), 26.8 (C-19), 23.9 (C-7), 23.6 (C-6), 21.7 (C-16b), 21.3 (C-22b), 19.6 (C-29), 17.4 (C-18), 16.2 (C-27), 13.8 (C-21), 13.1 (C-28)

### exemple 33 :

¹H NMR (500MHz, CD₃CN) δ = 6.60 (1H, d, J = 10.1 Hz, H-2), 6.23 (1H, d, J = 10.1 Hz, H-1), 5.35 (1H, td, J = 7.9 Hz, J = 4.9 Hz, H-16), 4.89 (1H, s, H-22), 4.06 (1H, t, J = 8.1Hz, H-26<'>), 3.77 (3H, s, H-3a), 3.54 (1H, dd, J = 9,8 Hz, J = 4.6 Hz, H-24a'), 3.29 - 3.37 (2H, m, H-26", 24a"), 3.23 (3H, s, H-24b), 3.15 (3H, s, H-23a), 2.47 - 2.59 (1H, m, H-20), 2.23 (1H, dq, J = 13.1 Hz, 6.4 Hz, H-4), 2.16 - 2.19 (1H, m, H-25), 2.06 (3H, s, H-22b), 1.99 - 2.08 (2H, m, H-15', 8), 1.97 (3H, s, H-16b), 1.77 - 1.92 (3H, m, H-11', 24, 17), 1.49 - 1.75 (5H, m, H-11", 6', 12", 12', 5), 1.39 - 1.49 (1H, m, H-7'), 1.28 (1H, dd, J = 13.4 Hz, J = 4.3 Hz, H-15"), 1.14 - 1.23 (1H, m, H-7"), 1.14 (3H, s, H-18), 1.08 (3H, d, J = 6.4 Hz, H-28), 1.05 (3H, d, J = 7.0 Hz, H-21), 1.02 (1H, d, J = 4.6 Hz, H-19'), 1.03 (3H, d, J = 6.4 Hz, H-27), 0.83 (3H, s, H-29), 0.81 - 0.88 (1H, m, H-6"), 0.38 (1H, d, J = 4.6 Hz, H-19")
¹³C NMR (126MHz, CD₃CN) δ = 171.8 (C-22a), 171.4 (C-16a), 157.4 (C-3), 144.4 (C-1), 116.8 (C-2), 109.6 (C-23), 75.7 (C-26), 75.6 (C-16), 75.4 (C-22), 73.2 (C-24a), 61.8 (C-3a), 59.1 (C-24b), 53.3 (C-24), 52.8 (C-17), 50.1 (C-23a), 45.7 (C-15), 43.9 (C-8), 43.1 (C-5), 39.6 (C-4), 38.6 (C-25), 33.0 (C-12), 32.7 (C-10), 31.5 (C-20), 28.1 (C-11), 26.8 (C-19), 23.9 (C-7), 23.6 (C-6), 21.8 (C-16b), 21.2 (C-22b), 19.7 (C-29), 17.6 (C-27), 17.4 (C-18), 14.0 (C-21), 13.1 (C-28)

### exemple 34 :

¹H NMR (500MHz, CD₃CN) δ = 6.93 (1H, d, J = 10.1 Hz, H-1), 5.89 (1H, d, J = 9.8 Hz, H-2), 5.57 (1H, td, J = 7.9 Hz, J = 4.9 Hz, H-16), 5.13 (1H, d, J = 3.1 Hz, H-24a'), 4.97 (1H, d, J = 3.4 Hz, H-24a"), 4.91 (1H, s, H-22), 4.22 (1H, t, J = 8.1 Hz, H-26'), 3.42 (1H, dd, J = 9.2 Hz, J = 7.9 Hz, H-26"), 3.09 (3H, s, H-23a), 2.73 - 2.82 (1H, m, H-25), 2.65 - 2.74 (1H, m, H-20), 2.10 - 2.15 (1H, m, H-4), 1.97 - 2.05 (2H, m, H-15', 8), 1.93 - 1.97 (8H, m, H-16b, 22b, 11', 5), 1.85 (1H, dd, J = 11.4 Hz, J = 7.8 Hz, H-17), 1.62 - 1.70 (3H, m, H-6', 12', 12"), 1.53 - 1.62 (1H, m, H-11"), 1.40 - 1.50 (1H, m, H-7'), 1.32 (1H, dd, J = 13.6 Hz, J = 4.4 Hz, H-15"), 1.25 (1H, d, J = 4.3 Hz, H-19'), 1.16 - 1.23 (1H, m, J = 7.2 Hz, J = 7.2 Hz, H-7"), 1.18 (3H, s, H-18), 1.11 (3H, d, J = 6.7 Hz, H-21), 1.04 (3H, d, J = 6.7 Hz, H-27), 1.02 (3H, d, J = 6.7 Hz, H-28), 0.90 - 1.00 (1H, m, H-6"), 0.85 (3H, s, H-29), 0.55 (1H, d, J = 4.6 Hz, H-19")
¹³C NMR (126MHz, CD₃CN) δ = 202.4 (C-3), 171.5 (C-16a), 171.4 (C-22a), 155.6 (C-1), 154.3 (C-24), 128.4 (C-2), 110.5 (C-23), 109.3 (C-24a), 78.2 (C-22), 75.1 (C-16), 75.0 (C-26), 52.9 (C-17), 49.8 (C-23a), 48.5 (C-14), 47.6 (C-4), 46.6 (C-13), 45.4 (C-15), 44.5 (C-8), 43.4 (C-5), 38.0 (C-25), 32.9 (C-12), 31.7 (C-20), 28.1 (C-11), 27.4 (C-10), 27.1 (C-19), 24.3 (C-9), 24.2 (C-7), 24.0 (C-6), 21.7 (C-16b), 21.3 (C-22b), 19.7 (C-29), 17.7 (C-18), 16.2 (C-27), 13.8 (C-21), 11.3 (C-28)

### ◆ exemple 35 : 3-semi carbazone- neoboutomellerone

| | |
|---|---|
| 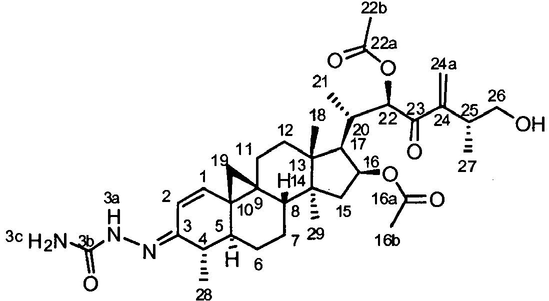 | Formule Chimique : C₃₅H₅₁NO₇ |
| | Masse Exacte : 625,37 |
| | Poids moléculaire : 625,80 |

Protocole : 49 mg (0.44 mmol, 5 eq.) de chlorhydrate de semi-carbazide et 67 mg (0.48 mmol, 5.5 eq.) d'acétate de sodium sont dissous dans 2 mL de méthanol. 51 mg (0.089 mmol) de 2 sont ensuite ajoutés et le milieu réactionnel est agité à température ambiante. Après 20h, le milieu réactionnel est dilué dans l'acétate d'éthyle et la phase organique est lavée avec de l'eau distillée. La phase aqueuse est extraite 3fois à l'acétate d'éthyle et les phases organiques rassemblées sont séchées sur MgSO₄. Après concentration à l'évaporateur rotatif, le produit est purifié par chromatographie sur gel de silice (éluant : DCM/MeOH : 97.5/2.5 à 92.5/7.5) afin d'obtenir un solide blanc (36 mg, 66%).
¹H RMN (500MHz, CD₃CN) δ = 8.04 (1H, s, NH-3a), 6.41 (1H, d, J = 10.4 Hz, H-2), 6.33 (1H, d, J = 10.1 Hz, H-1), 6.04 (1H, s, H-24a'), 5.89 (1H, d, J = 0.9 Hz, H-24a"), 5.53 (1H, d, J = 2.1 Hz, H-22), 5.08 (1H, td, J = 7.7 Hz, J = 4.4 Hz, H-16), 3.53 (1H, dd, J = 10.7 Hz, J = 6.1 Hz, H-26'), 3.39 (1H, dd, J = 10.4 Hz, J = 6.4 Hz, H-26"), 2.76 (1H, sxt, J = 6.8 Hz, H-25), 2.54 - 2.64 (1H, dqd, J = 10.9 Hz, J = 6.9 Hz, J = 2.1 Hz, H-20), 2.23 - 2.33 (2H, m, H-17, 4), 2.12 - 2.17 (1H, m, H-15'), 2.09 (3H, s, H-22b), 2.03 (3H, s, H-16b), 1.96 - 2.11 (2H, m, H-11', 8), 1.64 - 1.77 (4H, m, H-5, 12', 12", 6'), 1.53 - 1.64 (1H, m, H-11"), 1.41 - 11.50 (1H, m, H-7'), 1.35 (1H, dd, J = 13.9 Hz, J = 3.8 Hz, H-15"), 1.17 - 1.24 (1H, m, H-7"), 1.17 (3H, s, H-18), 1.12 (3H, d, J = 6.7 Hz, H-28), 1.06 (1H, d, J = 4.6 Hz, H-19'), 1.03 (3H, d, J = 7.0 Hz, H-27), 0.92 (3H, s, H-29), 0.85 - 0.95 (1H, m, H-6"), 0.84 (3H, d, J = 7.0 Hz, H-21), 0.46 (1H, d, J = 4.3 Hz, H-19")
¹³C RMN (126MHz, CD₃CN) δ = 199.6 (C-23), 171.7 (C-22a), 171.3 (C-16a), 158.4 (C-3b), 150.3 (C-24), 150.2 (C-3), 145.4 (C-1), 124,7 (C-24a), 116.4 (C-2), 78.5 (C-22), 76.6 (C-16), 66.4 (C-26), 51.2 (C-17), 48.4 (C-14), 46.8 (C-13), 46.6 (C-15), 44.6 (C-8), 43.3 (C-5), 41.2 (C-4), 37.9 (C-25), 33.2 (C-20), 33.1 (C-10), 33.0 (C-12), 28.1 (C-11), 27.7 (C-19), 27.1 (C-9), 24.1 (C-7), 23.9 (C-6), 22.1 (C-16b), 20.9 (C-22b), 19.9 (C-29), 18.0 (C-18), 17.2 (C-27), 13.3 (C-21), 13.2 (C-28)

### ◆ exemples 36 & 37 : 3-anti O-benzyloxime- neoboutomellerone (36) et 3-anti O-benzyloxime-24a-O-benzylhydroxylamine neoboutomellerone (37)

| | |
|---|---|
| 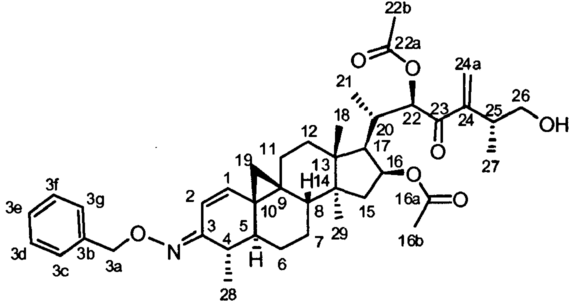 | 36 |
| | Formule Chimique : C₄₁H₅₅NO₇ |
| | Masse Exacte : 673,40 |
| | Poids moléculaire : 673,88 |
| 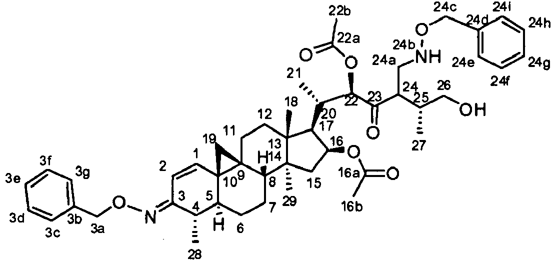 | 37 |
| | Formule Chimique : C₄₈H₆₄N₂O₇ |
| | Masse Exacte : 796.47 |
| | Poids moléculaire : 797.03 |

Protocole : 81.64 mg (0.6 mmol, 3 eq.) d'acétate de sodium, 35 µL (0.48 mmol, 5.5 eq.) d'acide acétique et 5 gouttes d'eau distillée sont ajoutés à 4 mL de dioxane. Le milieu réactionnel est refroidi à 0°C et 95.7 mg (0.6 mmol, 3 eq.) de chlorhydrate de O-benzylhydroxylamine sont ajoutés. A température ambiante, 114 mg de 2 (0.2 mmol) sont introduits et le milieu réactionnel est agité à température ambiante pendant 6 heures. Le milieu réactionnel est dilué dans l'acétate d'éthyle et la phase organique est lavée avec de l'eau distillée. La phase aqueuse est extraite 3 fois à l'acétate d'éthyle et les phases organiques rassemblées sont lavées avec une solution saturée de chlorure de sodium et séchées sur MgSO₄. Après concentration à l'évaporateur rotatif, le produit est purifié par chromatographie sur silice (éluant : Cyclohexane/AcOEt : 8/2 à 5/5) et par HPLC (éluant : Cyclohexane/AcOEt : 6/4). 36 est obtenu avec un rendement de 17%. Une fraction de la chromatographie sur gel de silice est repurifiée par chromatographie phase inverse (éluant : MeOH /H₂O : 100/5 à 100/0) puis par HPLC phase normale (éluant : Cyclohexane/Acétate d'éthyle : 6/4). Le composé 37 est isolé avec un rendement de 17% (26.6 mg).

### exemple 36 :

¹H RMN (500MHz, CD₃CN) δ = 7.20 - 7.43 (5H, m, H-3c, 3d, 3e, 3f, 3g), 6.67 (1H, d, J = 10.4 Hz, H-2), 6.25 (1H, d, J = 10.4 Hz, H-1), 6.04 (1H, s, H-24a'), 5.89 (1H, d, J = 0.9 Hz, H-24a"), 5.52 (1H, d, J = 2.4 Hz, H-22), 5.05 - 5.11 (1H, m, H-16), 4.99 - 5.11 (2H, m, H-3a', 3a"), 3.53 (1H, dt, J = 10.4 Hz, J = 5.2 Hz, H-26'), 3.39 (1H, dt, J = 10.5 Hz, J = 5.4 Hz, H-26"), 2.76 (1H, sxt, J = 6.7 Hz, H-25), 2.67 (1H, t, J = 5.0 Hz, OH-26), 2.53 - 2.64 (1H, m, H-20), 2.20 - 2.32 (2H, m, H-4, 17), 2.12 - 2.17 (1H, m, H-15'), 2.08 (3H, s, H-22b), 2.02 (3H, s, H-16b), 1.96 - 2.04 (2H, m, H-11', 8), 1.59 - 1.76 (4H, m, H-6', 5, 12', 12"), 1.50 - 1.59 (1H, m, H-11"), 1.40 - 1.49 (1H, m, H-7'), 1.35 (1H, dd, J = 14.0 Hz, J = 4.3 Hz, H-15"), 1.17 - 1.23 (1H, m, H-7"), 1.16 (3H, s, H-18), 1.08 (3H, d, J = 6.7 Hz, H-28), 1.01 (1H, d, J = 4.6 Hz, H-19'), 1.03 (3H, d, J = 7.0 Hz, H-27), 0.92 (3H, s, H-29), 0.79 - 0.88 (1H, m, H-6"), 0.83 (3H, d, J = 6.7 Hz, H-21), 0.42 (1H, d, J = 4.3 Hz, H-19")
¹³C RMN (126MHz, CD₃CN) δ = 199.6 (C-23), 171.7 (C-22a), 171.3 (C-16a), 157.9 (C-3), 150.3 (C-24), 144.7 (C-1), 139.7 (C-3b), 129.3 (C-3c, 3g), 129.2 (C-3d, 3f), 128.7 (C-3e), 124.6 (C-24a), 116.9 (C-2), 78.5 (C-22), 76.6 (C-16), 76.3 (C-3a), 66.4 (C-26), 51.2 (C-17), 48.4 (C-14), 46.8 (C-13), 46.6 (C-15), 44.7 (C-8), 43.4 (C-5), 39.8 (C-4), 37.9 (C-25), 33.2 (C-20), 33.0 (C-12), 32.8 (C-10), 28.1 (C-11), 27.5 (C-19), 26.8 (C-9), 24.2 (C-7), 23.7 (C-6), 22.1 (C-16b), 20.9 (C-22b), 19.9 (C-29), 18.1 (C-18), 17.2 (C-27), 13.3 (C-21), 13.1 (C-28)

### exemple 37:

¹H NMR (500MHz, CD₃CN) δ = 7.22 - 7.42 (10H, m, H-3c, 3d, 3e, 3f, 3g, 24^{e}, 24f, 24g, 24h, 24i), 6.67 (1H, d, J = 10.4 Hz, H-2), 6.26 (1H, d, J = 10.1 Hz, H-1), 5.13 (1H, td, J = 7.7 Hz, J = 4.4 Hz, H-16), 5.06 (1H, d, J = 12.2 Hz, H-3a'), 5.02 (1H, s, H-22), 5.02 (1H, d, J = 12.4 Hz, H-3a"), 4.57 (1H, d, J = 12.2 Hz, H-24c'), 4.60 (1H, d, J = 12.2 Hz, H-24c"), 3.42 - 3.51 (1H, m, H-26'), 3.34 - 3.42 (1H, m, H-26"), 3.24 (1H, td, J = 7.7 Hz, J = 4.4 Hz, H-24), 2.98 - 3.15 (2H, m, H-24a', 24a"), 2.77 - 2.83 (1H, m, OH-26), 2.69 - 2.78 (1H, m, H-20), 2.20 - 2.31 (1H, m, H-4), 2.12 - 2.20 (2H, m, H-15', 17), 2.09 (3H, s, H-22b), 2.05 (3H, s, H-16b), 2.00 - 2.07 (1H, m, H-8), 1.83 - 1.92 (2H, m, H-25, 11'), 1.52 - 1.76 (4H, m, H-6', 5, 12', 12"), 1.41 - 1.50 (1H, m, H-7'), 1.36 (1H, dd, J = 13.7 Hz, J = 4.3 Hz, H-15"), 1.18 (3H, s, H-18), 1.11 - 1.23 (1H, m, H-7"), 1.08 (3H, d, J = 6.7 Hz, H-28), 1.02 (1H, d, J = 4.3 Hz, H-19'), 0.90 (3H, s, H-29), 0.88 (3H, d, J = 7.0 Hz, H-27), 0.86 (3H, d, J = 6.7 Hz, H-21), 0.78 - 0.93 (1H, m, H-6"), 0.40 (1H, d, J = 4.3 Hz, H-19")
¹³C NMR (126MHz, CD₃CN) δ = 210.7 (C-23), 172.3 (C-22a), 171.5 (C-16a), 157.9 (C-3), 144.6 (C-1), 139.7 (C-3b), 139.7 (C-24d), 129.2 - 129-3 (C-3c, 3d, 3f, 3g, 24e, 24f, 24h, 24i), 128.5 - 128.7 (C-3e, 24g), 116.9 (C-2), 82.1 (C-22), 76.3 (C-3a), 76.2 (C-16), 76.1 (C-24c), 64.9 (C-26), 52.8 (C-24a), 51.2 (C-17), 48.5 (C-14), 48.4 (C-24), 46.7 (C-13), 46.2 (C-15), 44.2 (C-8), 43.2 (C-5), 39.7 (C-4), 36.4 (C-25), 32.8 (C-10), 32.7 (C-12), 30.8 (C-20), 28.0 (C-11), 27.1 (C-19), 26.9 (C-9), 24.0 (C-7), 23.6 (C-6), 22.0 (C-16b), 21.0 (C-22b), 19.7 (C-29), 17.7 (C-18), 16.2 (C-27), 13.4 (C-21), 13.1 (C-28)

### ◆ exemple 38 : 24a-O-méthyl neoboutomellerone

| | |
|---|---|
| 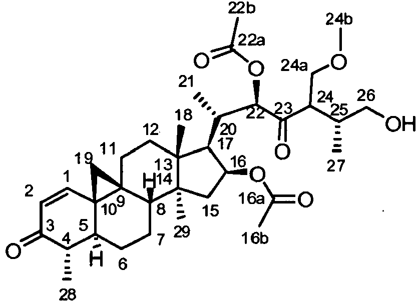 | Formule Chimique : C₃₅H₅₂O₈ |
| | Masse Exacte : 600,37 |
| | Poids moléculaire : 600,78 |

Protocole : A 10 mg de composé 2 en solution dans 200 µL de méthanol est ajouté 36 µL d'une solution d'hydroxyde de soude dans le méthanol (c = 0.5 N). Le milieu réactionnel est agité à température ambiante pendant 13 heures. Le milieu réactionnel est dilué à l'eau et filtré sur célite. Le filtrat est concentré à l'évaporateur rotatif et purifié par chromatographie sur gel de silice (éluant : gradient Cyclohexane/ AcOEt : 7/3 à 0/10) puis par HPLC phase normale (éluant : Cyclohexane/AcOEt : 65/35 + 5% THF) et par HPLC phase inverse (éluant : ACN/H₂O: 70/30). Les composés 1 (0.75 mg, 8%) et 38 (0.49 mg, 5%) sont ainsi isolés.
¹H NMR (500MHz, CD₃CN) δ = 6.94 (1H, d, J = 9.8 Hz, H-1), 5.90 (1H, d, J = 10.1 Hz, H-2), 5.21 (1H, td, J = 7.9 Hz, J = 4.7 Hz, H-16), 4.05 (1H, s, H-22), 3.54 (1H, dd, J = 8.9 Hz, J = 4.9 Hz, H-24a'), 3.40 - 3.50 (2H, m, H-26', 26"), 3.40 (1H, t, J = 8.9 Hz, H-24a"), 3.22 - 3.27 (1H, m, H-24), 3.18 (3H, s, H-24b), 2.50 - 2.61 (1H, m, H-20), 2.34 (1H, dd, J = 11.0 Hz, J = 7.9 Hz, H-17), 2.14 - 2.21 (2H, m, H-4, 15'), 2.02 - 2.09 (1H, m, H-8), 2.02 (3H, s, H-16b), 1.93 - 2.01 (3H, m, H-25, 11', 5), 1.63 - 1.76 (3H, m, H-6', 12', 12"), 1.54 - 1.63 (1H, m, H-11"), 1.42 - 1.51 (1H, m, H-7'), 1.38 (1H, dd, J = 13.7 Hz, J = 4.6 Hz, H-15"), 1.26 (1H, d, J = 4.6 Hz, H-19'), 1.20 (3H, s, H-18), 1.15 - 1.24 (1H, m, H-7"), 1.02 (3H, d, J = 6.7 Hz, H-28), 0.96 (3H, s, H-29), 0.92 - 0.98 (1H, m, H-6"), 0.89 (3H, d, J = 7.0 Hz, H-27), 0.71 (3H, d, J = 6.7 Hz, H-21), 0.56 (1H, d, J = 4.6 Hz, H-19")
¹³C NMR (126MHz, CD₃CN) δ = 216.4 (C-23), 202.4 (C-3), 171.4 (C-16a), 155.6 (C-1), 128.4 (C-2), 80.7 (C-22), 76.1 (C-16), 74.1 (C-24a), 65.1 (C-26), 59.2 (C-24b), 51.6 (C-17), 50.0 (C-24), 48.5 (C-14), 47.6 (C-4), 46.6 (C-13), 46.2 (C-15), 44.8 (C-8), 43.5 (C-5), 35.6 (C-25), 32.8 (C-12), 32.6 (C-20), 28.1 (C-11), 27.3 (C-19), 24.2 (C-7), 24.1, 21.7 (C-16b), 19.9 (C-29), 18.1 (C-18), 16.2 (C-27), 12.8 (C-21), 11.3 (C-28)

### ◆ exemple 39 : 26-carboxaldéhyde- neoboutomellerone

| | |
|---|---|
| 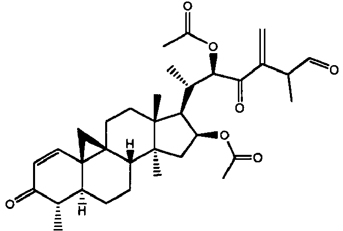 | Formule Chimique : C₃₅H₄₈O₇ |
| | Masse Exacte : 580,34 |
| | Poids moléculaire : 580,75 |

Protocole : 100 mg (0.18 mmol) de 2 est solubilisé dans 3.5 mL de dichlorométhane. 350 µL de pyridine (2mL/mmol) est ajouté et le mélange réactionnel est refroidi à 0°C. Ensuite, 1.76 mL (0.528 mmol, 3 eq., c=3 mol/L) du réactif de Dess Martin en solution dans le dichlorométhane est additionné et on laisse la température du milieu réactionnel remonter à température ambiante. Après 3h, le milieu réactionnel est dilué dans l'acétate d'éthyle et une solution de thiosulfate de sodium saturée est ajoutée. La phase aqueuse est extraite 3 fois à l'acétate d'éthyle et les phases organiques rassemblées sont lavées successivement avec une solution saturée d'hydrogénocarbonate de sodium et de chlorure de sodium. La phase organique est séchée sur MgSO₄, filtrée et concentrée à l'évaporateur rotatif. Le produit est purifié par chromatographie sur silice (éluant : Cyclohexane/AcOEt :7/3 à 6/4). L'aldéhyde est obtenu avec un rendement de 66% (66 mg).
¹H RMN (500MHz, CD₃CN) δ = 9.51 (1H, d, J = 0.6 Hz, H-26), 6.94 (1H, d, J = 10.1 Hz, H-1), 6.32 (1H, s, H-24a'), 6.06 (1H, s, H-24a"), 5.90 (1H, d, J = 10.1 Hz, H-2), 5.58 (1H, d, J = 2.1 Hz, H-22), 5.08 (1H, td, J = 7.7 Hz, J = 4.4 Hz, H-16), 3.45 (1H, q, J = 7.2 Hz, H-25), 2.58 - 2.69 (1H, m, H-20), 2.31 (1H, dd, J = 11.0 Hz, J = 7.6 Hz, H-17), 2.16 - 2.23 (2H, m, H-4, 15'), 2.09 (3H, s, H-22b), 2.04 (3H, s, H-16b), 1.99 - 2.07 (2H, m, H-8, 11'), 1.92 - 1.96 (1H, m, H-5), 1.63 - 1.76 (3H, m, H-6', 12', 12"), 1.52 - 1.62 (1H, m, H-11"), 1.42 - 1.49 (1H, m, H-7'), 1.38 (1H, dd, J = 13.7 Hz, J = 4.0 Hz, H-15"), 1.25 (1H, d, J = 4.3 Hz, H-19'), 1.18 - 1.22 (7H, m, H-7", 27, 18), 1.03 (3H, d, J = 6.7 Hz, H-28), 0.96 (3H, s, H-29), 0.94 (1H, qd, J = 12.8 Hz, J = 3.7 Hz, H-6"), 0.85 (3H, d, J = 7.0 Hz, H-21), 0.58 (1H, d, J = 4.6 Hz, H-19")
¹³C RMN (126MHz, CD₃CN) δ = 202.4 (C-3), 201.7 (C-26), 198.3 (C-23), 171.6 (C-22a), 171.2 (C-16a), 155.5 (C-1), 145.8 (C-24), 128.6 (C-24a), 128.4 (C-2), 78.2 (C-22), 76.8 (C-16), 51.2 (C-17), 48.9 (C-25), 48.3 (C-14), 47.6 (C-4), 46.9 (C-13), 46.7 (C-15), 45.2 (C-8), 43.6 (C-5), 33.8 (C-20), 33.0 (C-12), 28.1 (C-11), 27.6 (C-19), 27.2 (C-9), 24.3 (C-7, 6), 22.1 (C-16b), 20.9 (C-22b), 20.0 (C-29), 18.3 (C-18), 13.8 (C-27), 13.4 (C-21 ), 11.3 (C-28)

### ◆ exemple 40 (comparatif): 1,1-diméthyl (26)-neoboutomelleronylidène)-hydrazone

| | |
|---|---|
| 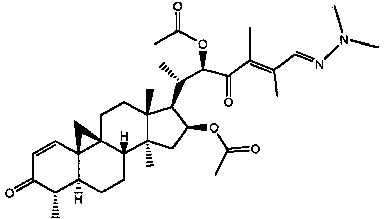 | Formule Chimique : C₃₇H₅₄N₂O₆ |
| | Masse Exacte : 622,40 |
| | Poids moléculaire : 622,83 |

Protocole : Le composé 39 (200 mg, 0,353 mmol) est mis en solution dans 15 ml d'éthanol et 85 mg d'hydrazine (0.107 ml, 1.412 mmol, 4 éq.) dilués dans l'éthanol sont ajoutés au goutte à goutte. La réaction est laissée sous agitation 3 heures à température ambiante. Le produit est alors adsorbé sur alumine et le solvant est évaporé sous pression réduite. Le produit est purifié sur colonne d'alumine avec un gradient heptane / acétate d'éthyle (de 100 /0 à 40/60%) pour conduire à 40 mg (20%) de composé 40.
¹H NMR (500MHz, ACETONITRILE-d₃) δ = 7.37 (1H, s, H-26), 6.94 (1H, d, J = 10.1 Hz, H-1), 5.83 - 5.95 (1H, m, H-2), 5.34 (1H, d, J = 1.5 Hz, H-22), 5.06 (1H, td, J = 7.5 Hz, J = 4.3 Hz, H-16), 2.84 (6H, s, H-26a, 26b), 2.51 - 2.60 (1H, m, H-20), 2.14 - 2.27 (2H, m, H-15, 4, 17), 2.11 (4H, s, H-22b), 1.96 - 2.05 (3H, m, H-5a, 8a, 11), 1.93 (8H, br. s., H-27, 24a), 1.88 (3H, s, H-16b), 1.63 - 1.73 (3H, m, H-12, 6<'>), 1.53 - 1.62 (1H, m, H-11<">), 1.40 - 1.48 (1H, m, H-7<'>), 1.32 (1H, dd, J = 14.2 Hz, J = 3.1 Hz, H-15<">), 1.24 (1H, d, J = 4.3 Hz, H-19<'>), 1.20 - 1.22(1H, m, H-7<">), 1.19 (3H, s, H-18), 1.03 (3H, d, J = 6.7 Hz, H-28), 0.96 - 0.98 (1H, m, M21), 0.94 (3H, s, H-29), 0.90 (3H, d, J = 7.0 Hz, H-21), 0.57 (1H, d, J = 4.6 Hz, H-19<">)
¹³C NMR (126MHz, ACETONITRILE-d₃) δ = 205.8 (C-23), 202.4 (C-3), 172.0 (C-22a, 16a), 171.4, 155.5 (C-1), 137.5 (C-24), 132.3 (C-26), 132.3 (C-25), 128.5 (C-2), 82.3 (C-22), 75.9 (C-16), 51.2 (C-17), 48.5 (C-14), 47.7 (C-4), 46.9 (C-13), 46.5 (C-15), 45.1 (C-8), 43.6, 43.1 (C-26b, 26a, 5), 33.0 (C-10), 32.9 (C-12), 31.8 (C-20), 28.1 (C-11), 27.6 (C-19), 27.3 (C-9), 24.3 (C-6), 24.3 (C-7), 21.9 (C-16b), 21.0 (C-22b), 20.0 (C-29), 18.2 (C-18), 17.2 (C-24a), 13.8 (C-21, 27), 11.3 (C-28)

### ◆ exemple 41 : Acide neoboutomellerone 26-carboxylique

| | |
|---|---|
| 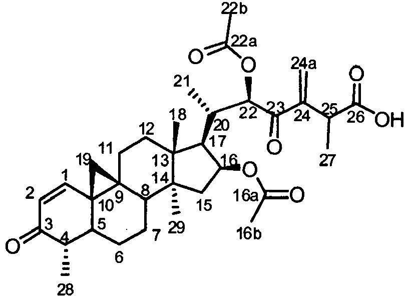 | Formule Chimique : C₃₄H₄₆O₈ |
| | Masse Exacte : 582,32 |
| | Poids moléculaire : 582,72 |

Protocole : L'aldéhyde intermédiaire 39 (45 mg, 0.0795) est solubilisé dans un mélange acétone/eau (1/1, 2 mL) et 84 µL de 2-méthyl-2-butène est ajouté. Ensuite une solution de 55 mg de dihydrogénophosphate de sodium (0.33 mmol, 5 eq.) dans 100 µL d'H₂O et une solution de 22 mg de chlorite de sodium (0.24 mmol, 3 eq.) dans 100 µL d'H₂O sont ajoutés. Après 24h, le milieu réactionnel est dilué avec une solution saturée de chlorure de sodium et extrait 3 fois à l'acétate d'éthyle. Les phases organiques rassemblées sont séchées sur MgSO₄, filtrées et concentrées à l'évaporateur rotatif. Le produit est purifié par chromatographie sur gel de silice (éluant : DCM/MeOH : 100/0 à 95/5) afin d'obtenir un solide blanc (28 mg, 61%), le produit étant obtenu sous forme d'un mélange d'épimères au niveau de C-25.
¹H RMN (500MHz, CD3CN) δ = 6.94 (1H, d, J = 10.1 Hz, H-1), 6.20 (1H, br. s., H-24a'), 6.04/6.08 (1H, br. s., H-24a"), 5.89 (1H, d, J = 9.8 Hz, H-2), 5.55 (1H, br. s., H-22), 5.02 - 5.14 (1H, m, H-16), 3.40 - 3.49 /3.51 - 3.62 (1H, m, H-25), 2.55 - 2.69 (1H, m, H-20), 2.24 - 2.33 (1H, m, H-17), 2.12 - 2.22 (2H, m, H-4, 15'), 2.08/2.08 (3H, s, H-22b), 2.03/2.03 (3H, s, H-16b), 1.98 - 2.05 (3H, m, H-5, 8, 11'), 1.62 - 1.75 (3H, m, H-6', 12', 12"), 1.51 - 1.60 (1H, m, H-11"), 1.40 - 1.49 (1H, m, H-7'), 1.36 (1H, dd, J = 14.0 Hz, J = 4.0 Hz, H-15"), 1.22 - 1.31 (4H, m, H-19', 27), 1.16 - 1.22 (1H, m, H-7"), 1.18 (3H, s, H-18), 1.02 (3H, d, J = 7.0 Hz, H-28), 0.95 (3H, s, H-29), 0.90 - 0.99 (1H, m, H-6"), 0.84 (3H, d, J = 7.0 Hz, H-21), 0.57 (1H, d, J = 4.6 Hz, H-19")
¹³C RMN (126MHz, CD3CN) δ = 202.4 (C-3), 197.7 (C-23), 175.5 (C-26), 171.6/171.7 (C-22a), 171.3/171.3 (C-16a), 155.6 (C-1), 147.3 (C-24), 128.4 (C-2), 126.3/127.2 (C-24a), 78.0/78.1 (C-22), 76.7/76.8 (C-16), 51.3/51.3 (C-17), 48.3/48.4 (C-14), 47.6 (C-4), 46.9/46.9 (C-13), 46.7 (C-15), 45.2 (C-8), 43.6 (C-5), 41.5/42.2 (C-25), 33.4/33.7 (C-20), 33.0 (C-12), 32.8 (C-10), 28.1/28.1 (C-11), 27.6 (C-19), 27.2/27.2 (C-9), 24.3 (C-6, 7), 22.1/22.1 (C-16b), 20.9/20.9 (C-22b), 20.0 (C-29), 18.3 (C-18), 15.9/17.2 (C-27), 13.2/13.4 (C-21), 11.3 (C-28)

### ◆ exemple 42 (comparatif): 22-déacétyl, 23, 23a dihydro, hémiacétal 16,22-neoboutomellerone

| | |
|---|---|
| 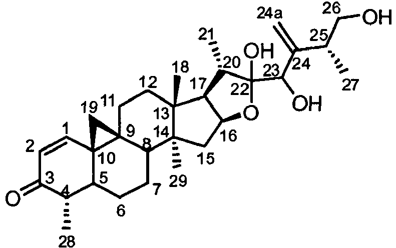 | Formule Chimique : C₃₀H₄₄O₅ |
| | Masse Exacte : 484,32 |
| | Poids moléculaire : 484,67 |

Protocole : 20 mg de 1 sont solubilisés dans 300 µL d'acétonitrile et 570 µL de NaOH 1M est ajouté. Le milieu réactionnel est agité à température ambiante pendant 22 heures. Le milieu réactionnel est dilué dans l'acétate d'éthyle et filtré sur célite. Après concentration du filtrat à l'évaporateur rotatif, le produit est purifié par chromatographie sur gel de silice (éluant : Cyclohexane/ AcOEt : 5/5) afin d'obtenir 42 (3 mg, 17%).
¹H RMN (500MHz, CD₃CN) δ = 6.97 (1H, d, J = 10.1 Hz, H-1), 5.91 (1H, d, J = 10.1 Hz, H-2), 5.22 (1H, s, H-24a'), 5.12 (1H, s, H-24a"), 4.64 (1H, q, J = 6.7 Hz, H-16), 3.98 (1H, d, J = 5.5 Hz, H-23), 3.79 (1H, d, J = 5.8 Hz, OH-23), 3.49 (1H, s, OH-22), 3.44 - 3.51 (1H, m, H-26'), 3.40 (1H, ddd, J = 9.6 Hz, J = 8.7 Hz, J = 4.6 Hz, H-26"), 3.24 (1H, t, J = 4.7 Hz, OH-26), 2.64 (1H, sxt, J = 7.0 Hz, H-25), 2.48 (1H, quin, J = 6.8 Hz, H-20), 2.11 - 2.25 (3H, m, H-4, 8, 17), 1.89 - 1.99 (2H, m, H5, 11'), 1.60 - 1.76 (5H, m, H-6', 11", 12', 12", 15alpha), 1.40 - 1.56 (2H, m, H-7', 15beta), 1.31 (1H, d, J = 4.6 Hz, H-19'), 1.22 - 1.30 (1H, m, H-7"), 1.14 (3H, s, H-18), 1.02 (3H, d, J = 6.7 Hz, H-28), 1.01 (3H, d, J = 7.0 Hz, H-27), 0.94 - 0.99 (1H, m, H-6"), 0.96 (3H, d, J = 6.7 Hz, H-21), 0.92 (3H, s, H-29), 0.50 (1H, d, J = 4.6 Hz, H-19")
¹³C RMN (126MHz, CD₃CN) δ = 202.3 (C-3), 155.3 (C-1), 153.3 (C-24), 128.5 (C-2), 114.2 (C-24a), 111.4 (C-22), 82.5 (C-16), 77.8 (C-23), 69.0 (C-26), 60.4 (C-17), 52.4 (C-14), 47.5 (C-4), 44.8 (C-13), 43.6 (C-8), 43.1 (C-5), 41.7 (C-15), 38.3 (C-25), 38.2 (C-20), 33.3 (C-10), 32.8 (C-12), 28.4 (C-11), 27.4 (C-9), 26.5 (C-19), 23.9 (C-6), 23.8 (C-7), 21.3 (C-18), 19.3 (C-29), 17.8 (C-27), 15.8 (C-21), 11.3 (C-28)

### ◆ exemple 43 : 26-sulfate- neoboutomellerone

| | |
|---|---|
| 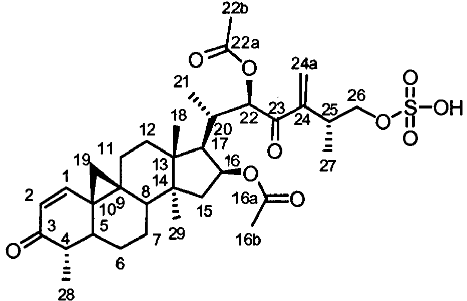 | Formule Chimique : C₃₄H₄₈O₁₀S |
| | Masse Exacte : 648,30 |
| | Poids moléculaire : 648,80 |

Protocole : 60 mg (0.088 mmol) de 2 est solubilisé dans 3 mL de tétrahydrofurane anhydre sous azote et 280 mg (1.76 mmol, 20 eq.) du complexe SO₃.pyridine est ajouté. Le milieu réactionnel est agité à température ambiante pendant 2 heures et concentré à l'évaporateur rotatif. Le produit est purifié par chromatographie sur gel de silice (éluant : DCM/MeOH :10/0 à 8/2) afin d'obtenir un solide blanc. Le solide blanc est repris dans une solution saturée d'hydrogénocarbonate de sodium et la phase aqueuse est extraite 3 fois à l'acétate d'éthyle. Les phases organiques rassemblées sont séchées sur MgSO₄, filtrées et concentrées sur l'évaporateur rotatif. Un solide blanc est obtenu avec un rendement de 63% (37 mg).
¹H RMN (500MHz, CD₃CN) δ = 6.94 (1H, d, J = 10.1 Hz, H-1), 6.10 (1H, s, H-24a'), 6.02 (1H, s, H-24a"), 5.89 (1H, d, J = 9.8 Hz, H-2), 5.54 (1H, d, J = 2.1 Hz, H-22), 5.08 (1H, td, J = 7.6 Hz, J = 4.6 Hz, H-16), 3.94 (1H, dd, J = 9.8 Hz, J = 6.4 Hz, H-26'), 3.81 (1H, dd, J = 9.8 Hz, J = 6.7 Hz, H-26"), 2.99 (1H, sxt,, J = 6.7 Hz, H-25), 2.60 (1H, dqd, J = 11.0 Hz, J = 7.0 Hz, J = 2.1 Hz, H-20), 2.29 (1H, dd, J = 11.0 Hz, J = 7.6 Hz, H-17), 2.13 - 2.22 (2H, m, H-4, 15'), 2.10 (3H, s, H-22b), 2.04 (3H, s, H-16b), 1.99 - 2.07 (2H, m, H-8, 11'), 1.96 - 1.99 (1H, m, H-5), 1.62 - 1.76 (3H, m, H-6', 12', 12"), 1.51 - 1.61 (1H, m, H-11"), 1.41 - 1.49 (1H, m, H-7'), 1.36 (1H, dd, J = 14.0 Hz, J = 4.0 Hz, H-15"), 1.24 (1H, d, J = 4.6 Hz, H-19'), 1.19 (3H, s, H-18), 1.16 - 1.21 (1H, m, H-7"), 1.06 (3H, d, J = 7.0 Hz, H-27), 1.02 (3H, d, J = 6.7 Hz, H-28), 0.95 (3H, s, H-29), 0.90 - 0.98 (1H, m, H-6"), 0.86 (3H, d, J = 7.0 Hz, H-21), 0.58 (1H, d, J = 4.6 Hz, H-19")
¹³C RMN (126MHz, CD₃CN) δ = 202.4 (C-3), 199.1 (C-23), 171.9 (C-22a), 171.4 (C-16a), 155.6 (C-1), 149.5 (C-24), 128.4 (C-2), 125.4 (C-24a), 78.5 (C-22), 76.7 (C-16), 70.6 (C-26), 51.3 (C-17), 48.3 (C-14), 47.6 (C-4), 46.9 (C-13), 46.7 (C-15), 45.2 (C-8), 43.6 (C-5), 35.0 (C-25), 33.3 (C-20), 33.0 (C-12), 32.9 (C-10), 28.1 (C-11), 27.7 (C-19), 27.2 (C-9), 24.3 (C-7, 6), 22.2 (C-16b), 21.0 (C-22b), 20.0 (C-29), 18.3 (C-18), 17.4 (C-27), 13.4 (C-21), 11.3 (C-28)

### ◆ exemple 44 : 26-sulfate, 22-déacétyl-neoboutomellerone

| | |
|---|---|
| 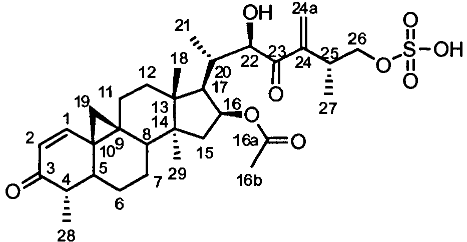 | Formule Chimique : C₃₂H₄₆O₉S |
| | Masse Exacte : 606,29 |
| | Poids moléculaire : 606,77 |

Protocole : 100 mg (0.19 mmol) de composé 1 sont dissous dans 0.2 ml de THF, puis une solution de réactif de Burgess (54 mg, 1.2 éq., 0.23 mmol) dans 0.4 ml de THF est ajoutée. La réaction est portée à reflux pendant 2 h. Revenu à température ambiante la réaction est hydrolysé à l'eau et extraite 2 fois par l'éther éthylique. La phase aqueuse est réextraite plusieurs fois par l'acétate d'éthyle. Ces phases sont réunies, séchées sur sulfate de sodium puis évaporée, pour permettre l'isolation du composé 44 (50 mg, 43%) qui ne nécessite pas de purifications supplémentaires.
¹H NMR (500MHz, ACETONITRILE-d₃) δ = 6.94 (1H, d, J = 10.1 Hz, H-1), 6.14 (1H, s, H-24aa), 6.05 - 6.11 (1H, m, H-24ab), 5.89 (1H, d, J = 9.8 Hz, H-2), 5.20 (1H, td, J = 7.5 Hz, J = 4.6 Hz, H-16), 4.67 - 4.78 (1H, m, H-22), 3.93 (1H, dd, J = 10.0 Hz, J = 3.5 Hz, H-26<'>), 3.78 (1H, dd, J = 10.0 Hz, J = 3.5 Hz, H-26<">), 3.58 (1H, d, J = 6.0 Hz, H-30), 3.56 (1H, br. s, H-42), 3.03 (1H, sxt, J = 6.5 Hz, H-25), 2.39 - 2.52 (1H, m, H-17, 20), 2.23 (1H, dd, J = 13.5 Hz, J = 7.5 Hz, H-15<'>), 2.17 (1H, dq, J = 12.5 Hz, J = 6.5 Hz, H-4), 2.04 - 2.06 (1H, m, H-8a), 2.04 (1H, s, H-16b), 1.98 - 2.02 (1H, m, H-5a, 11<'>), 1.62 - 1.74 (1H, m, H-12<">, 12<'>, 6<'>), 1.55 (1H, ddd, J = 15.0 Hz, J = 8.9 Hz, J = 6.1 Hz, H-11<">), 1.42 - 1.50 (1H, m, H-7<'>), 1.38 (1H, dd, J = 13.7 Hz, J = 4.2 Hz, H-15<">), 1.24 (1H, d, J = 4.5 Hz, H-19<'>), 1.18 (1H, s, H-18), 1.13 - 1.17 (1H, m, H-7<">), 1.07 (1H, d, J = 7.0 Hz, H-27), 1.03 (1H, d, J = 6.7 Hz, H-28), 0.97 (1H, s, H-29), 0.92 - 0.94 (1H, m, H-6<">), 0.66 (1H, d, J = 6.1 Hz, H-21), 0.57 (1H, d, J = 4.5 Hz, H-19<">)
¹³C NMR (126MHz, ACETONITRILE-d₃) δ = 205.4 (C-23), 202.4 (C-3), 171.4 (C-16b), 155.6 (C-1), 148.9 (C-24), 128.4 (C-2), 126.7 (C-24a), 77.3 (C-16), 75.8 (C-22), 70.4 (C-26), 51.5 (C-17), 48.4 (C-14), 47.7 (C-4), 47.0 (C-15), 46.7 (C-13), 45.4 (C-8), 43.7 (C-5), 36.3 (C-25), 35.1 (C-20), 33.2 (C-12), 33.0 (C-10), 28.2 (C-11), 27.8 (C-19), 27.3 (C-9), 24.4 (C-7), 24.4 (C-6), 22.2 (C-16b), 20.2 (C-29), 18.5 (C-18), 17.6 (C-27), 12.4 (C-21), 11.3 (C-28)

### ◆ exemple 45 : 22,26-disulfate,22-déacétyl-neoboutomellerone

| | |
|---|---|
| 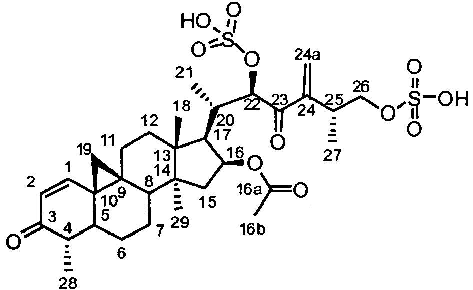 | Formule Chimique : C₃₂H₄₆O₁₂S₂ |
| | Masse Exacte : 686,24 |
| | Poids moléculaire : 686,83 |

Protocole : La même réaction que précédemment réalisées avec 2.5 éq. de réactif de Burgess conduit à la formation du composé 45 (21 mg, 16%) en plus du composé 44 (61 mg, 53%)
¹H NMR (500MHz, ACETONITRILE-d₃) δ = 6.94 (1H, d, J = 10.1 Hz, H-1), 6.22 (1H, s, H-24ab), 6.00 (1H, s, H-24aa), 5.89 (1H, d, J = 9.8 Hz, H-2), 5.49 (1H, td, J = 7.7 Hz, J = 4.3 Hz, H-16), 5.22 - 5.28 (1H, m, M06), 3.87 (1H, dd, J = 10.0 Hz, J = 5.5 Hz, H-26<'>), 3.80 (1H, dd, J = 10.0 Hz, J = 6.7 Hz, H-26<">), 3.12 (1H, sxt, J = 7.0 Hz, H-25), 2.44 - 2.50 (2H, m, H-17, 20), 2.18 - 2.23 (1H, m, H-15<'>), 2.15 - 2.18 (1H, m, H-4), 2.04 (3H, s, H-16b), 1.99 - 2.03 (2H, m, H-8a, 5a), 1.97 - 1.99 (1H, m, H-11<'>), 1.63 - 1.75 (3H, m, H-12<">, 12<'>, 6<'>), 1.57 (1H, ddd, J = 15.0 Hz, J = 9.0 Hz, J = 6.0 Hz, H-11<">), 1.42 - 1.49 (1H, m, H-7<'>), 1.35 (2H, dd, J = 7.0 Hz, J = 3.5 Hz, H-15<">), 1.24 (1H, d, J = 4.0 Hz, H-19<'>), 1.19 - 1.22 (1H, m, H-7<">), 1.17 (3H, s, H-18), 1.07 (3H, d, J = 7.1 Hz, H-27), 1.03 (3H, d, J = 7.2 Hz, H-28), 0.96 (3H, s, H-29), 0.91 - 0.95 (1H, m, H-6<">), 0.82 (3H, d, J = 7.0 Hz, H-21), 0.57 (1H, d, J = 4.0 Hz, H-19<">)
¹³C NMR (126MHz, ACETONITRILE-d₃) δ = 202.4 (C-23), 202.2 (C-3), 171.8 (C-16a), 155.6 (C-1), 150.1 (C-24), 128.4 (C-2), 126.0 (C-24a), 80.8 (C-16), 77.2 (C-22), 71.7 (C-26), 51.1 (C-17), 48.5 (C-14), 47.7 (C-4), 46.7 (C-13), 46.7 (C-15), 45.2 (C-8), 43.6 (C-5), 34.7 (C-25), 34.3 (C-20), 33.1 (C-12), 33.0 (C-10), 28.2 (C-11), 27.7 (C-19), 27.3 (C-9), 24.4 (C-6), 24.3 (C-7),

### ◆ exemple 46 : tosylate- neoboutomellerone

| | |
|---|---|
| 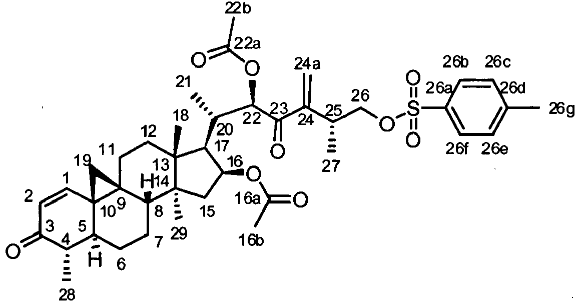 | Formule Chimique : C₄₁H₅₄O₉S |
| | Masse Exacte : 722,35 |
| | Poids moléculaire : 722,93 |

Protocole : 100 mg (0.18 mmol) de 2 est solubilisé dans 1 mL de dichlorométhane anhydre sous azote. 49 µL (0.36 mmol, 2 eq.) de triéthylamine et 46 mg (0.21 mmol, 1.2 eq.) de chlorure de tosyle sont ajoutés. Le milieu réactionnel est agité pendant 24 heures et dilué dans l'acétate d'éthyle. La phase organique est lavée avec une solution saturée de chlorure d'ammonium et séchée sur Na₂SO₄. Après concentration à l'évaporateur rotatif, le produit brut est purifié par chromatographie sur gel de silice (éluant : Cyclohexane/AcOEt : 7/3 à 6/4). Le tosylate est obtenu avec un rendement de 65% (82 mg).
¹H RMN (500MHz, ACETONITRILE-d₃) δ = 7.75 (2H, d, J = 8.2 Hz, H-26f, 26b), 7.43 (2H, d, J = 7.9 Hz, H-26e, 26c), 6.94 (1H, d, J = 9.8 Hz, H-1), 6.07 (1H, s, H-24a'), 5.89 (1H, d, J = 10.1 Hz, H-2), 5.85 (1H, s, H-24a"), 5.48 (1H, d, J = 1.8 Hz, H-22), 5.06 (1H, td, J = 7.6 Hz, J = 4.4 Hz, H-16), 4.05 (1H, dd, J = 10.4 Hz, J = 6.1 Hz, H-26'), 3.95 (1H, dd, J = 9.8 Hz, J = 6.1 Hz, H-26"), 2.97 (1H, sxt, J = 6.7 Hz, H-25), 2.49 - 2.55 (1H, m, H-20), 2.44 (3H, s, H-26g), 2.27 (1H, dd, J = 11.0 Hz, J = 7.6 Hz, H-17), 2.12 - 2.21 (2H, m, H-4, 15'), 2.08 (3H, s, H-22b), 1.99 (3H, s, H-16b), 1.96 - 2.05 (3H, m, H-5, 8, 11'), 1.61 - 1.76 (3H, m, H-6', 12', 12"), 1.52 - 1.60 (1H, m, H-11"), 1.40 - 1.49 (1H, m, H-7'), 1.36 (1H, dd, J = 13.9 Hz, J = 3.8 Hz, H-15"), 1.24 (1H, d, J = 4.6 Hz, H-19'), 1.16 (3H, s, H-18), 1.13 - 1.22 (1H, m, H-7"), 1.02 (3H, d, J = 6.7 Hz, H-27), 1.01 (3H, d, J = 7.0 Hz, H-28), 0.94 (3H, s, H-29), 0.90 - 0.98 (1H, m, H-6"), 0.80 (3H, d, J = 7.0 Hz, H-21), 0.57 (1H, d, J = 4.3 Hz, H-19")
¹³C RMN (126MHz, ACETONITRILE-d₃) δ = 202.4 (C-3), 198.6 (C-23), 171.6 (C-22a), 171.2 (C-16a), 155.5 (C-1), 147.6 (C-24), 146.5 (C-26d), 133.7 (C-26a), 131.1 (C-26e, 26c), 128.9 (C-26f, 26b), 128.4 (C-2), 126.5 (C-24a), 78.2 (C-22), 76.7 (C-16), 73.9 (C-26), 51.2 (C-17), 48.3 (C-14), 47.6 (C-4), 46.8 (C-13), 46.7 (C-15), 45.2 (C-8), 43.6 (C-5), 35.2 (C-25), 33.3 (C-20), 32.9 (C-12), 28.1 (C-11), 27.7 (C-19), 27.2 (C-9), 24.3 (C-7, 6), 22.1 (C-16b), 21.7 (C-26g), 20.9 (C-22b), 20.0 (C-29), 18.3 (C-18), 16.8 (C-27), 13.3 (C-21), 11.3 (C-28)

### ◆ exemple 47 (comparatif) : Dérivé azido méthyl dihydro furane neoboutomellerone

| | |
|---|---|
| 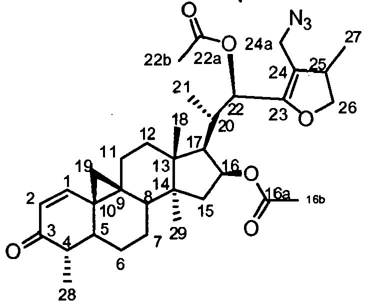 | Formule Chimique : C₃₄H₄₇N₃O₆ |
| | Masse Exacte : 593,35 |
| | Poids moléculaire : 593,75 |

Protocole : 20 mg (0.027 mmol) de 2 est solubilisé dans 200 µL de DMF anhydre sous azote. 4 mg (0.054 mmol, 2 eq.) d'azidure de sodium est ajouté. Le milieu réactionnel est agité et chauffé à 50°C pendant 24 heures. Le milieu réactionnel est dilué avec de l'eau distillée et est extrait avec de l'acétate d'éthyle (3 fois). La phase organique est séchée sur MgSO₄, filtrée et concentrée à l'évaporateur rotatif. Le produit brut est purifié par chromatographie sur gel de silice (éluant : Cyclohexane/AcOEt : 8/2 à 4/6). Le produit 47 est obtenu avec un rendement de 18% (3 mg).
¹H RMN (500MHz, CD3CN) δ = 6.94 (1H, d, J = 10.1 Hz, H-1), 5.90 (1H, d, J = 10.1 Hz, H-2), 5.33 (1H, s, H-22), 5.18 (1H, td, J = 7.9 Hz, J = 4.6 Hz, H-16), 4.42 (1H, t, J = 9.3 Hz, H-26'), 4.21 (1H, d, J = 13.7 Hz, H-24a'), 3.82 (1H, dd, J = 8.9 Hz, J = 7.3 Hz, H-26"), 3.76 (1H, d, J = 13.7 Hz, H-24a"), 3.08 (1H, sxt, J = 7.1 Hz, H-25), 2.31 (1H, dqd, J = 11.0 Hz, J = 6.7 Hz, J = 1.5 Hz, H-20), 2.14 - 2.21 (1H, m, H-4), 2.10 (3H, s, H-22b), 1.96 - 2.00 (3H, m, H-16b), 1.96 - 2.11 (5H, m, H-5, 8, 11', 15', 17), 1.62 - 1.73 (3H, m, H-6', 12', H2"), 1.54 - 1.62 (1H, m, H-11"), 1.40 - 1.51 (1H, m, H-7'), 1.34 (1H, dd, J = 13.9 Hz, J = 4.4 Hz, H-15"), 1.25 (1H, d, J = 4.3 Hz, H-19'), 1.17 - 1.23 (1H, m, H-7"), 1.16 (3H, s, H-18), 1.09 (3H, d, J = 6.7 Hz, H-27), 1.02 (3H, d, J = 7.0 Hz, H-21), 1.01 (3H, d, J = 7.0 Hz, H-28), 0.92 - 1.00 (1H, m, H-6"), 0.90 (3H, s, H-29), 0.56 (1H, d, J = 4.3 Hz, H-19")
¹³C RMN (126MHz, CD3CN) δ = 202.3 (C-3), 171.3 (C-16a), 171.0 (C-22a), 155.5 (C-1), 153.7 (C-23), 128.4 (C-2), 110.5 (C-24), 77.1 (C-26), 75.7 (C-16), 72.5 (C-22), 51.0 (C-17), 48.5 (C-14), 47.6 (C-4), 46.7 (C-13), 45.9 (C-24a), 45.8 (C-15), 44.7 (C-8), 43.4 (C-5), 40.0 (C-25), 34.6 (C-20), 32.9 (C-10), 32.7 (C-12), 28.0 (C-11), 27.3 (C-19), 27.0 (C-9), 24.2 (C-6), 24.1 (C-7), 21.5 (C-16b), 21.2 (C-22b), 19.8 (C-29), 18.2 (C-27), 17.9 (C-18), 12.9 (C-21), 11.3 (C-28)

### ◆ exemple 48 (comparatif): Dérivé méthyl propanone diméthyl dihydro furane-neoboutomellerone

| | |
|---|---|
| 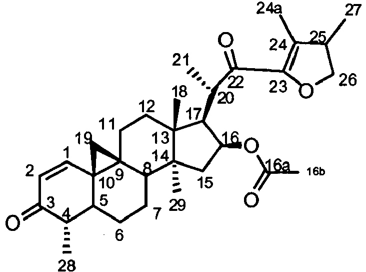 | Formule Chimique : C₃₂H₄₄O₅ |
| | Masse Exacte : 508,32 |
| | Poids moléculaire : 508,69 |

Protocole : 4.8 mg (0.008 mmol) de 2 sont dissous dans 2 ml de benzène deutérié, et est mis en présence d'acide *p*-toluène sulfonique à température ambiante. Après 5 heures, le produit est filtré sur silice, le solvant est évaporé pour conduire à l'isolation de 3.8 mg (89 %) de composé 48.
La même réaction réalisée à partir de 1 conduit à ce même produit 48.
¹H RMN (500MHz, CD3CN) δ = 6.95 (1H, d, J = 10.1 Hz, H-1), 5.91 (1H, d, J = 10.1 Hz, H-2), 5.05 (1H, td, J = 8.3 Hz, J = 6.0 Hz, H-16), 4.41 (1H, dd, J = 9.6 Hz, J = 9.0 Hz, H-26'), 3.82 (1H, t, J = 8.7 Hz, H-26"), 3.39 (1H, dq, J = 11.0 Hz, J = 7.1 Hz, H-20), 2.91 - 3.01 (1H, m, H-25), 2.55 (1H, dd, J = 11.0 Hz, J = 8.5 Hz, H-17), 2.14 - 2.21 (1H, m, H-4), 2.04 - 2.10 (1H, m, H-8), 1.96 - 2.03 (3H, m, H-5, 11', 15'), 1.91 (3H, d, J = 1.5 Hz, H-24a), 1.80 (3H, s, H-16b), 1.71 - 1.83 (1H, m, H-12'), 1.57 - 1.70 (3H, m, H-6', 11", 12"), 1.42 - 1.51 (1H, m, H-7'), 1.27 (1H, d, J = 4.3 Hz, H-19'), 1.17 - 1.30 (2H, m, H-7", 15"), 1.15 (3H, s, H-18), 1.10 (3H, d, J = 7.0 Hz, H-27), 1.04 (3H, d, J = 7.0 Hz, H-21), 1.02 (3H, d, J = 6.7 Hz, H-28), 0.97 (3H, s, H-29), 0.89 - 1.00 (1H, m, H-6"), 0.55 (1H, d, J = 4.6 Hz, H-19")
¹³C RMN (126MHz, CD3CN) δ = 202.3 (C-3), 200.1 (C-22), 170.7 (C-16a), 155.5 (C-1), 147.1 (C-23), 128.5 (C-24), 128.4 (C-2), 75.8 (C-26), 75.3 (C-16), 51.8 (C-17), 48.6 (C-14), 47.6 (C-4), 45.9 (C-13), 44.6 (C-15), 44.3 (C-8), 43.6 (C-25), 43.3 (C-5), 40.7 (C-20), 33.0 (C-10), 32.9 (C-12), 28.0 (C-11), 27.3 (C-9), 27.0 (C-19), 24.1 (C-7), 24.0 (C-6), 21.0 (C-16b), 19.4 (C-29), 18.7 (C-18), 17.5 (C-27), 16.4 (C-21), 11.3 (C-28), 11.1 (C-24a)

### ◆ exemples 49 : Dérivé 26-chloro-acétate- neoboutomellerone

| | |
|---|---|
| 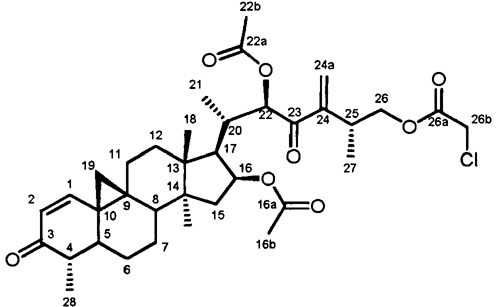 | Formule Chimique : C₃₆H₄₉ClO₈ |
| | Masse Exacte : 644,31 |
| | Poids moléculaire : 645,22 |

Protocole : 60 mg (0.1 mmol) de 2 est solubilisé dans 1 mL de tétrahydrofurane anhydre sous azote. 20 µL (0.11, 1.1 eq.) de triéthylamine est ajouté et le milieu réactionnel est refroidi à 0°C. 10 µL (0.11, 1.1 eq.) de chlorure de chloroacétyle est additionné goutte à goutte. Après 24 heures d'agitation à température ambiante, le milieu réactionnel est dilué dans l'acétate d'éthyle et lavé avec de l'eau distillée et avec une solution saturée de chlorure de sodium. La phase organique est séchée sur MgSO₄, filtrée et concentrée à l'évaporateur rotatif. Le produit brut est purifié par chromatographie sur gel de silice (éluant : Cyclohexane/AcOEt : 7/3 à 6/4). Le produit cyclique 49 et le produit chloroacétylé 49 sont obtenus respectivement avec un rendement de 7% (3 mg) et de 14% (10 mg).
¹H RMN (500MHz, CD3CN) δ = 6.94 (1H, d, J = 10.1 Hz, H-1), 6.15 (1H, s, H-24a'), 6.00 (1H, s, H-24a"), 5.90 (1H, d, J = 9.8 Hz, H-2), 5.53 (1H, d, J = 2.4 Hz, H-22), 5.09 (1H, td, J = 7.6 Hz, J = 4.6 Hz, H-16), 4.16 (1H, dd, J = 11.0 Hz, J = 7.0 Hz, H-26<'>), 4.13 (2H, s, H-26b), 4.12 (1H, dd, J = 10.7 Hz, J = 6.4 Hz, H-26<">), 3.05 (1H, sxt, J = 6.9 Hz, H-25), 2.58 (1H, dqd, J = 11.0 Hz, J = 7.0 Hz, J = 2.4 Hz, H-20), 2.30 (1H, dd, J = 11.0 Hz, J = 7.6 Hz, H-17), 2.14 - 2.21 (2H, m, H-4, 15<'>), 2.09 (3H, s, H-22b), 2.03 (3H, s, H-16b), 1.96 - 2.07 (3H, m, H-5, 8, 11<'>), 1.63 - 1.77 (3H, m, H-6<'>, 12<'>, 12<">), 1.52 - 1.61 (1H, m, H-11<">), 1.37 (1H, dd, J = 14.3 Hz, J = 4.0 Hz, H-15<">), 1.24 (1H, d, J = 4.3 Hz, H-19<'>), 1.19 (3H, s, H-18), 1.15 - 1.23 (1H, m, H-7<">), 1.09 (3H, d, J = 7.0 Hz, H-27), 1.03 (3H, d, J = 6.7 Hz, H-28), 0.95 (3H, s, M24), 0.88 - 1.00 (1H, m, H-6<">), 0.85 (3H, d, J = 7.0 Hz, H-21), 0.58 (1H, d, J = 4.3 Hz, H-19<">)
¹³C RMN (126MHz, CD3CN) δ = 202.3 (C-3), 198.7 (C-23), 171.6 (C-22a), 171.2 (C-16a), 168.3 (C-26a), 155.5 (C-1), 148.7 (C-24), 128.4 (C-2), 126.1 (C-24a), 78.3 (C-22), 76.7 (C-16), 69.5 (C-26), 51.3 (C-17), 48.4 (C-14), 47.6 (C-4), 46.9 (C-13), 46.7 (C-15), 45.2 (C-8), 43.6 (C-5), 42.1 (C-26b), 34.6 (C-25), 33.4 (C-20), 33.0 (C-12), 32.9 (C-10), 28.1 (C-11), 27.2 (C-9), 24.3 (C-7, 6), 22.1 (C-16b), 20.9 (C-22b), 20.0 (C-29), 18.3 (C-18), 17.0 (C-27), 13.3 (C-21), 11.3 (C-28)

### ◆ exemple 50 (comparatif) : 16-déacétyl, 22-déacétyl- neoboutomellerone 16,26-23acétal

| | |
|---|---|
| 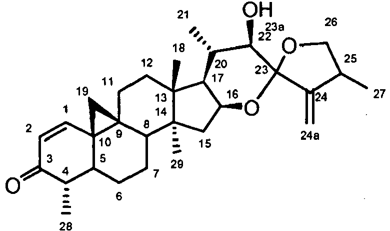 | Formule Chimique : C₃₀H₄₂O₄ |
| | Masse Exacte : 466,31 |
| | Poids moléculaire : 466,65 |

Protocole : Dans un tube scellé, 101 mg (0.178 mmol) de 2 est dissous dans 3.2 ml de tertiobutanol. Une solution de 122 mg (0.889 mmol) de carbonate de potassium dans 0.8 ml d'eau est ajoutée, puis le tube est scellé et la réaction est laissée 3 jours à 70°C. Le milieu réactionnel est extrait par l'acétate d'éthyle, séché sur sulfate de sodium et après évaporation des solvants, les différents produits sont purifiés par CCM préparative éluée 2 fois par un mélange de 20% d'acétate d'éthyle dans le cyclohexane, puis 5 fois par un mélange de 5% d'acétate d'éthyle dans le cyclohexane. Parmi les différents produits, on recueille 17 mg (20%) de composé 50 (Rf : 0.81 cyclohexane / acétate d'éthyle 50 / 50).
¹H RMN (500MHz, CD3CN) δ = 6.96 (1H, d, J = 9.8 Hz, H-1), 5.91 (1H, d, J = 9.8 Hz, H-2), 5.26 (1H, d, J = 2.7 Hz, H-24aa), 5.00 (1H, d, J = 2.7 Hz, H-24ab), 4.15 (1H, t, J = 7.9 Hz, H-26<'>), 4.06 (1H, td, J = 7.9 Hz, J = 6.4 Hz, H-16), 3.56 (1H, t, J = 10.8 Hz, H-22), 3.29 (1H, t, J = 8.1 Hz, H-26<">), 2.78 - 2.89 (1H, m, H-25), 2.34 (1H, d, J = 10.7 Hz, OH-24b), 2.10 - 2.20 (2H, m, H-4, 8), 1.89 - 2.00 (3H, m, H-5, 11<'>, 20), 1.53 - 1.77 (5H, m, H-15<'>, 6<'>, 12<">, 12<'>, 11<">), 1.45 - 1.53 (1H, m, H-7<'>), 1.24 - 1.34 (2H, m, H-15<">, 19<'>), 1.16 - 1.24 (1H, m, H-7<">), 1.15 (3H, s, H-18), 1.07 (3H, d, J = 6.7 Hz, H-27), 1.02 (3H, d, J = 7.0 Hz, H-28), 1.00 (3H, d, J = 6.4 Hz, H-21), 0.94 - 1.02 (1H, m, H-6<">), 0.85 (3H, s, H-29), 0.52 (1H, d, J = 4.6 Hz, H-19<">)
¹³C RMN (126MHz, CD3CN) δ = 202.3 (C-3), 155.4 (C-1), 154.4 (C-24), 128.4 (C-2), 107.1 (C-23), 105.3 (C-24a), 74.1 (C-26), 73.0 (C-22), 71.8 (C-16), 56.2 (C-17), 48.0 (C-14), 47.5 (C-4), 45.7 (C-13), 44.0 (C-15), 43.6 (C-8), 43.2 (C-5), 36.5 (C-25), 33.4 (C-12), 33.1 (C-10), 32.3 (C-20), 28.1 (C-11), 27.5 (C-9), 26.6 (C-19), 24.0 (C-6), 23.9 (C-7), 20.0 (C-18), 19.1 (C-29), 16.9 (C-21), 15.4 (C-27), 11.3 (C-28)

### ◆ exemple 51 (comparatif) : 16-decacétyl, 26-méthoxy- neoboutomellerone hémiacétal

| | |
|---|---|
| 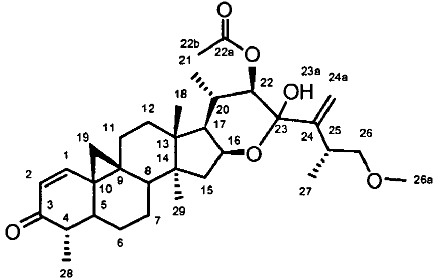 | Formule Chimique : C₃₃H₄₈O₆ |
| | Masse Exacte : 540,35 |
| | Poids moléculaire : 540,73 |

Protocole : 568 mg (1.0 mmol) de 2 sont dissous dans 1 ml de THF qui a ajouté à 0°C sur une suspension de 50.4 mg (2.1 éq, 2.1 mmol) de NaH dans 1 ml de THF. Après 15 minutes d'agitation à température ambiante, le milieu réactionnel est refroidi à 0°C, puis l'iodométhane (312 µl, 5 éq , 5 mmol) et la réaction est laissée sous agitation une nuit. Après disparition complète du produit de départ, contrôlé par CCM (éluant AcOEt / cyclohexane : 1 / 1), la réaction est hydrolysée par 4 ml d'acide chlorhydrique 2N et extraite avec de l'acétate d'éthyle. La phase organique est lavée successivement par de l'eau, une solution de thiosulfate, de l'eau puis de la saumure. On recueille 635 mg de brut réactionnel qui est purifié sur gel de silice et élué par un gradient de cyclohexane / acétate d'éthyle de 100 / 0 à 40 / 60. On recueille, entre autre produit, 80 mg (15%) du composé 51 (Rf : 0.76 - cyclohexane / acétate d'éthyle 50 / 50)
¹H RMN (500MHz, CD3CN) δ = 6.96 (1H, d, J = 10.1 Hz, H-1), 5.91 (1H, d, J = 10.1 Hz, H-2), 5.52 (1H, s, H-24aa), 5.16 (1H, s, H-24ab), 4.99 (1H, s, OH-23a), 4.88 (1H, d, J = 11.3 Hz, H-22), 4.49 (1H, td, J = 7.9 Hz, J = 6.4 Hz, H-16), 3.46 (1H, dd, J = 7.9 Hz, J = 4.6 Hz, H-26<'>), 3.28 (3H, s, H-26a), 3.03 (1H, dd, J = 10.7 Hz, J = 7.9 Hz, H-26<">), 2.51 - 2.63 (1H, m, H-25), 2.09 - 2.27 (2H, m, H-4, 8), 2.02 (3H, s, H-22b), 1.97 (2H, s, H-5, 11<'>), 1.79 - 1.88 (3H, m, H-17, 20, 15<'>), 1.55 - 1.75 (4H, m, H-6<'>, 11<">, 12<">, 12<'>), 1.48 - 1.55 (1H, m, H-7<'>), 1.41 1.48 (1H, m, H-15<">), 1.29 (1H, d, J = 4.6 Hz, H-19<'>), 1.19 (3H, s, H-18), 1.19 - 1.27 (1H, m, H-7<">), 1.07 (3H, d, J = 7.3 Hz, H-27), 1.03 (3H, d, J = 7.0 Hz, H-28), 0.94 - 1.05 (1H, m, H-6<">), 0.91 (3H, s, H-29), 0.78 (3H, d, J = 6.1 Hz, H-21), 0.54 (1H, d, J = 4.3 Hz, H-19<">)
¹³C RMN (126MHz, CD3CN) δ = 202.3 (C-3), 171.2 (C-22a), 155.4 (C-1), 154.7 (C-24), 128.5 (C-2), 113.0 (C-24a), 99.9 (C-23), 81.8 (C-26), 81.4 (C-22), 71.3 (C-16), 59.4 (C-26a), 56.8 (C-17), 48.4 (C-14), 47.6 (C-4), 45.8 (C-13), 44.1 (C-8), 43.7 (C-15), 43.3 (C-5), 35.7 (C-25), 33.3 (C-12), 33.1 (C-10), 30.8 (C-20), 28.1 (C-11), 27.4 (C-9), 26.9 (C-19), 24.1 (C-7, 6), 21.1 (C-22b), 19.9 (C-18), 19.1 (C-29), 17.4 (C-27), 15.7 (C-21), 11.3 (C-28)

### ◆ exemple 52 : 26-TBDMS - neoboutomellerone

| | |
|---|---|
| 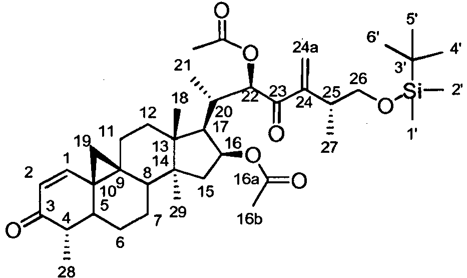 | Formule Chimique : C₄₀H₆₂O₇Si |
| | Masse Exacte : 682,43 |
| | Poids moléculaire : 683,00 |

Protocole : 200 mg (0.352 mmol) de composé 2 sont dissous dans 3 ml de DCM, puis 6.5 éq. (2.29 mmol, 157 mg) d'imidazole sont ajoutés. La réaction est refroidie à 0°C, puis à cette température une solution de TBDMSOTf (3 éq., 1.05 mmol, 0.241 ml) dans 6 ml de DCM. La réaction est laissée sous agitation à 0°C pendant 2h. La réaction est alors hydrolysée avec de l'hydrogénocarbonate de sodium, extraite 3 fois avec 10 ml de DCM, et les phases organiques réunies sont lavées à la saumure. Après séchage sur sulfate de sodium et évaporation du solvant, on recueille 207 mg de brut réactionnel. Le produit est purifié sur colonne de gel de silice et élué par un gradient cyclohexane / acétate d'éthyle de 100 / 0 à 80 / 20. On obtient 141 mg (59 %) de produit 52 (Rf : 0.32 - cyclohexane / acétate d'éthyle 80 / 20)
¹H NMR (500MHz, ACETONITRILE-d₃) δ = 6.94 (1H, d, J = 10.1 Hz, H-1), 6.05 (1H, s, H-24a'), 5.90 (1H, d, J = 0.9 Hz, H-24a"), 5.90 (1H, d, J = 9.8 Hz, H-2), 5.52 (1H, d, J = 2.4 Hz, H-22), 5.08 (1H, td, J = 7.6 Hz, J = 4.3 Hz, H-16), 3.68 (1H, dd, J = 9.9 Hz, J = 5.6 Hz, H-26<'>), 3.47 (1H, dd, J = 9.9 Hz, J = 6.6 Hz, H-26<">), 2.77 (1H, sxt, J = 6.6 Hz, H-25), 2.58 (1H, dqd, J = 10.7 Hz, J = 6.7 Hz, J = 2.1 Hz, H-20), 2.29 (1H, dd, J = 11.0 Hz, J = 7.6 Hz, H-17), 2.15 - 2.20 (2H, m, H-4, 15<'>), 2.08 (3H, s, H-22b), 2.02 (3H, s, H-16b), 1.96 - 2.06 (3H, m, 5, 8, H-11<'>), 1.62 - 1.76 (3H, m, H-6<'>, 12<'>, 12<">), 1.51 - 1.61 (1H, m, H-11<">), 1.41 - 1.48 (1H, m, H-7<'>), 1.36 (1H, dd, J = 14.0 Hz, J = 4.3 Hz, H-15<">), 1.24 (1H, d, J = 4.3 Hz, H-19<'>), 1.18 (3H, s, H-18), 1.14 - 1.22 (1H, m, H-7<">), 1.05 (3H, d, J = 7.0 Hz, H-27), 1.02 (3H, d, J = 6.7 Hz, H-28), 0.95 (3H, s, H-29), 0.91 - 0.99 (1H, m, H-6<">), 0.88 (9H, s, H-4', 5', 6'), 0.84 (3H, d, J = 7.0 Hz, H-21), 0.58 (1H, d, J = 4.3 Hz, H-19<">), 0.04 (3H, s, H-1'), 0.03 (3H, s, H-2')
¹³C NMR (126MHz, ACETONITRILE-d₃) δ = 202.4 (C-3), 199.3 (C-23), 171.6 (C-22a), 171.2 (C-16a), 155.5 (C-1), 150.0 (C-24), 128.4 (C-2), 125.0 (C-24a), 78.4 (C-22), 76.7 (C-16), 67.4 (C-26), 51.3 (C-17), 48.4 (C-14), 47.6 (C-4), 46.8 (C-13), 46.7 (C-15), 45.2 (C-8), 43.6 (C-5), 38.1 (C-25), 33.3 (C-20), 33.0 (C-12), 32.9 (C-10), 30.7, 28.1 (C-11), 27.7 (C-19), 27.2 (C-9), 26.3 (C-4', 5', 6'), 24.3 (C-6), 24.3 (C-7), 22.1 (C-16b), 20.9 (C-22b), 20.0 (C-29), 18.9 (C-3'), 18.3 (C-18), 17.1 (C-27), 13.4 (C-21), 11.3 (C-28), -5.1 (C-1'), -5.1 (C-2')

### ◆ exemple 53 : 26-TBDMS-22-déacetyl- neoboutomellerone

| | |
|---|---|
| 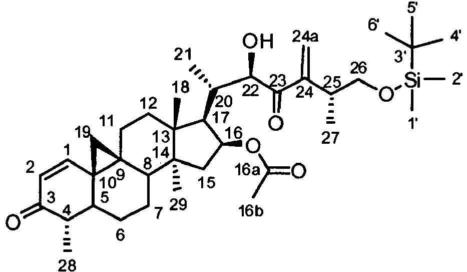 | Formule Chimique : C₃₈H₆₀O₆Si |
| | Masse Exacte : 640,42 |
| | Poids moléculaire : 640,96 |

Protocole : 400 mg (0.760 mmol) de 1 sont dissous dans 6 ml de DCM, puis 6.5 éq.(4.94 mmol, 336 mg) d'imidazole sont ajoutés. La réaction est refroidie à 0°C, puis à cette température une solution de TBDMSOTf (3 éq., 2.27 mmol, 0.520 ml) dans 12 ml de DCM. La réaction est laissée sous agitation à 0°C jusqu'à disparition du produit de départ (environ 1 h). La réaction est alors hydrolysée avec de l'hydrogénocarbonate de sodium, extraite 3 fois avec 10 ml de DCM, et les phases organiques réunies sont lavées à la saumure. Après séchage sur sulfate de sodium et évaporation du solvant, on recueille 750 mg de brut réactionnel. Le produit est purifié sur colonne de gel de silice et élué par un gradient cyclohexane / acétate d'éthyle de 100 / 0 à 80 / 20. On obtient 430 mg (88 %) de produit 53 (Rf: 0.32 - cyclohexane / acétate d'éthyle 80 / 20)
¹H RMN (500MHz, CD3CN) δ = 6.93 (1H, d, J = 10.1 Hz, H-1), 6.13 (1H, s, H-24a'), 5.99 (1H, s, H-24a"), 5.89 (1H, d, J = 10.1 Hz, H-2), 5.20 (1H, td, J = 7.3 Hz, J = 4.6 Hz, H-16), 4.70 (1H, dd, J = 6.1 Hz, J = 1.8 Hz, H-22), 3.70 (1H, dd, J = 9.8 Hz, J = 5.5 Hz, H-26<'>), 3.53 (1H, d, J = 6.1 Hz, OH-22), 3.49 (1H, dd, J = 9.8 Hz, J = 6.7 Hz, H-26<">), 2.84 (1H, sxt, J = 6.5 Hz, H-25), 2.37 - 2.51 (2H, m, H-17, 20), 2.22 (1H, dd, J = 14.0 Hz, J = 7.9 Hz, H-15<'>), 2.15 - 2.21 (1H, m, H-4), 2.02 (3H, s, H-16b), 1.95 - 2.08 (3H, m, H-5, 8, 11<'>), 1.59 - 1.75 (3H, m, H-6<'>, 12<'>, 12<">), 1.50 - 1.58 (1H, m, H-11<">), 1.41 - 1.49 (1H, m, H-7<'>), 1.38 (1H, dd, J = 14.0 Hz, J = 4.0 Hz, H-15<">), 1.23 (1H, d, J = 4.3 Hz, H-19<'>), 1.18 - 1.27 (1H, m, H-7<">), 1.17 (3H, s, H-18), 1.06 (3H, d, J = 7.0 Hz, H-27), 1.02 (3H, d, J = 6.7 Hz, H-28), 0.96 (3H, s, H-29), 0.91 - 0.99 (1H, m, H-6<">), 0.88 (9H, s, H-6', 5', 4'), 0.64 (3H, d, J = 6.4 Hz, H-21), 0.57 (1H, d, J = 4.6 Hz, H-19<">), 0.04 (3H, s, H-1'), 0.03 (3H, s, H-2')
¹³C RMN (126MHz, CD3CN) δ = 205.3 (C-3), 202.4, 171.2 (C-16a), 155.6 (C-1), 148.8 (C-24), 128.4 (C-2), 126.8 (C-24a), 77.2 (C-16), 75.8 (C-22), 67.6 (C-26), 51.4 (C-17), 48.3 (C-14), 47.6 (C-4), 46.9 (C-15), 46.7 (C-13), 45.3 (C-8), 43.6 (C-5), 37.8 (C-25), 36.4 (C-20), 33.1 (C-12), 32.9 (C-10), 28.1 (C-11), 27.7 (C-19), 27.2 (C-9), 26.3 (C-4', 5', 6'), 24.3 (C-6), 24.3 (C-7), 22.1 (C-16b), 20.1 (C-29), 18.9 (C-3'), 18.5 (C-18), 17.1 (C-27), 12.3 (C-21), 11.3 (C-28), -5.1 (C-1'), -5.1 (C-2')

### ◆ exemple 54 : 22-N-Boc-glycinate de 22-déacetyl- neoboutomellerone -(26-TBDMS)

| | |
|---|---|
| 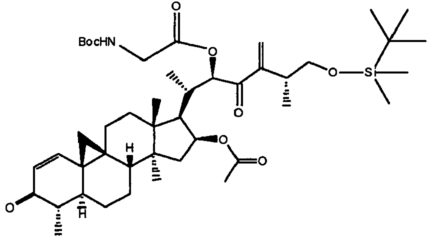 | Formule Chimique : C₃₂H₄₈O₆ |
| | Masse Exacte : 528,35 |
| | Poids moléculaire : 528,72 |

Protocole : 100 mg de composé 55 sont dissous dans 3 ml de DCM, puis 2.5 ml (1.6 éq.) d'une solution 0.1 M de DCC est ajouté, suivie d'une quantité catalytique de DMAP (2 mg, 0.1 éq.) en enfin de 41 mg (1.5 éq.) de N-Boc-Gly-OH. La réaction est laissée 4h sous agitation à température ambiante. Le milieu réactionnel est filtré sur célite^{®}, et le produit 54 (Rf : 0.43 - cyclohexane / acétate d'éthyle 70 / 30) est purifié sur gel de silice (éluant : gradient cyclohexane / acétate d'éthyle: de 100 / 0 à 70 / 30).
¹H NMR (500MHz, ACETONITRILE-d₃) δ = 6.94 (1H, d, J = 10.1 Hz, H-1), 6.06 (1H, s, H-24aa), 5.92 (1H, s, H-24ab), 5.90 (1H, d, J = 10.1 Hz, H-2), 5.58 (1H, d, J = 2.1 Hz, H-22), 5.06 (1H, td, J = 7.6 Hz, J = 4.6 Hz, H-16), 3.93 (1H, dd, J = 17.7 Hz, J = 6.4 Hz, H-22b<'>), 3.82 (1H, dd, J = 17.7 Hz, J = 6.1 Hz, H-22b<">), 3.68 (1H, dd, J = 10.1 Hz, J = 5.5 Hz, H-26<'>), 3.47 (1H, dd, J = 9.9 Hz, J = 6.6 Hz, H-26<">), 2.78 (1H, sxt, J = 6.6 Hz, H-25), 2.54 - 2.66 (1H, m, H-20), 2.30 (1H, dd, J = 10.8 Hz, J = 7.5 Hz, H-17), 2.15 - 2.23 (2H, m, H-4, 15<'>), 2.02 (3H, s, H-16b), 1.96 - 2.09 (3H, m, H-5, 8, 11<'>), 1.63 - 1.74 (3H, m, H-6<'>, 12<">, 12<'>), 1.53 - 1.63 (1H, m, H-11<">), 1.41 (9H, s, H-22f, 22g, 22h), 1.35 - 1.45 (1H, m, H-7<'>), 1.24 (1H, d, J = 4.3 Hz, H-19<'>), 1.18 (3H, s, H-18), 1.14 - 1.20 (1H, m, H-6<">), 1.05 (3H, d, J = 7.0 Hz, H-27), 1.02 (3H, d, J = 6.7 Hz, H-28), 0.97 (3H, s, H-29), 0.91 - 0.96 (1H, m, H-7<">), 0.88 (9H, s, H-26g, 26f, 26e), 0.84 (3H, d, J = 7.0 Hz, H-21), 0.57 (1H, d, J = 4.6 Hz, H-19<">), 0.04 (3H, s, H-26c), 0.03 (3H, s, H-26b)
¹³C NMR (126MHz, ACETONITRILE-d₃) δ = 202.3 (C-3), 198.7 (C-23), 171.3 (C-22a), 171.2 (C-16a), 156.9 (C-22d), 155.5 (C-1), 149.8 (C-24), 128.4 (C-2), 125.3 (C-24a), 80.0 (C-22e), 78.9 (C-22), 76.7 (C-16), 67.3 (C-26), 51.2 (C-17), 47.6 (C-4), 46.7 (C-15), 45.0 (C-8), 43.5 (C-5), 43.0 (C-22b), 38.1 (C-25), 33.6 (C-20), 33.0 (C-12), 28.6 (C-22h, 22g, 22f), 28.1 (C-11), 27.5 (C-19), 26.3 (C-26g, 26f, 26e), 24.2 (C-6), 24.2 (C-7), 22.1 (C-16b), 20.0 (C-29), 18.9 (C-26d), 18.2 (C-18), 17.1 (C-27), 13.4 (C-21), 11.3 (C-28), -5.1 (C-26c), -5.1 (C-26b)

### ◆ exemples 55 : 22-N-Boc-glycinate de 22-déacétyl- neoboutomellerone

| | |
|---|---|
| 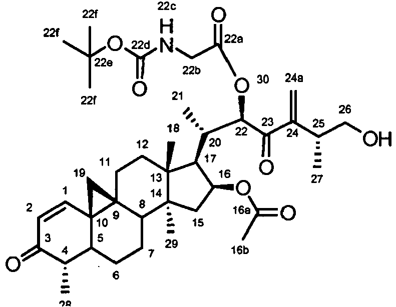 | Formule Chimique : C₃₉H₅₇NO₉ |
| | Masse Exacte : 683,40 |
| | Poids moléculaire : 683,87 |

Protocole : 118 mg de composé 54 (0.148 mmol) sont dissous dans 0.25 ml de THF. On introduit 0.32 ml de pyridine puis ensuite 3 fois 1.1 ml d'une solution d'HF dans la pyridine. La réaction est laissée 21h sous agitation à température ambiante. Le milieu réactionnel est dilué avec 2 ml d'acétate d'éthyle, puis le milieu est neutralisé jusqu'à pH = 7 avec une solution d'hydrogénocarbonate de sodium. Les différentes phases sont séparées, et la phase organique est lavée avec une solution de sulfate de cuivre, et rincée plusieurs fois avec de l'eau, puis de la saumure. Après séchage sur sulfate de sodium et évaporation du solvant, on recueille 84 mg de brut réactionnel.
Le produit est purifié sur colonne de gel de silice et élué par un gradient cyclohexane / acétate d'éthyle de 70 / 30 à 40 / 60. On obtient 44.5 mg (51 %) de produit (Rf: 0.24 - cyclohexane / acétate d'éthyle 60 / 40).
¹H NMR (500MHz, ACETONITRILE-d₃) δ = 6.93 (1H, d, J = 10.0 Hz, H-1), 6.05 (1H, s, H-24ab), 5.91 (2H, s, H-24aa), 5.90 (1H, d, J = 10.0 Hz, H-2), 5.59 (1H, d, J = 2.0 Hz, H-22), 5.07 (1H, td, J = 7.4 Hz, J = 4.4 Hz, H-16), 3.94 (1H, dd, J = 7.0 Hz, J = 2.6 Hz, H-22b<'>), 3.82 (1H, dd, J = 7.0 Hz, J = 2.5 Hz, H-22b<">), 3.53 (1H, ddd, J = 12.0 Hz, J = 6.0 Hz, H-26<'>), 3.39 (1H, ddd, J = 12.0 Hz, J = 6.0 Hz, H-26<">), 2.78 (1H, sxt, J = 7.0 Hz, H-25), 2.68 - 2.73 (1H, m, H-17), 2.59 - 2.67 (1H, m, H-20), 2.29 (1H, dd, J = 11.0 Hz, J = 7.0 Hz, H-15<'>), 2.12 - 2.21 (6H, m, H-11<'>, 22c, 4, 37), 2.03 (3H, s, H-16b), 1.98 - 2.02 (1H, m, H-5a), 1.62 - 1.76 (3H, m, H-12<">, 12<'>, 6<'>), 1.52 - 1.62 (1H, m, H-11<">), 1.42 - 1.50 (1H, m, H-7<'>), 1.41 (9H, s, H-22f, 22f, 22f), 1.33 - 1.36 (1H, m, H-15<">), 1.23 - 1.26 (1H, m, H-19<'>), 1.18 (3H, s, H-18), 1.03 (4H, d, J = 4.0 Hz, H-27), 1.02 (3H, d, J = 3.7 Hz, H-28), 0.97 (3H, s, H-29), 0.85 (3H, d, J = 7.0 Hz, H-21), 0.57 (1H, d, J = 4.5 Hz, H-19<">)
¹³C NMR (126MHz, ACETONITRILE-d₃) δ = 202.4 (C-3), 199.1 (C-23), 171.4 (C-22a), 171.3 (C-16a), 156.8 (C-22d), 155.5 (C-1), 150.2 (C-24), 128.4 (C-2), 124.9 (C-24a), 80.1 (C-22e), 79.0 (C-22), 76.7 (C-16), 66.4 (C-26), 51.2 (C-17), 48.4 (C-14), 47.6 (C-4), 46.8 (C-13), 46.7 (C-15), 45.0 (C-8), 43.5 (C-5), 43.0 (C-22b), 37.9 (C-25), 33.5 (C-20), 33.0 (C-12), 33.0 (C-10), 28.6 (C-40, 46, 22f), 28.1 (C-11), 27.5 (C-19), 27.2 (C-9), 24.2 (C-7), 24.2 (C-6), 22.1 (C-16b), 20.0 (C-29), 18.2 (C-18), 17.2 (C-27), 13.3 (C-21), 11.3 (C-28).

### ◆ exemple 56 : 26-N-Boc-glycinate de neoboutomellerone

| | |
|---|---|
| 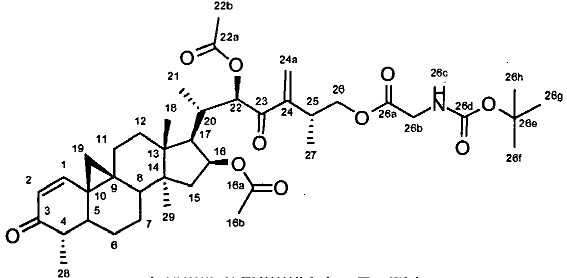 | Formule Chimique : C₄₁H₅₉NO₁₀ |
| | Masse Exacte : 725,41 |
| | Poids moléculaire : 725,91 |

Protocole : 100 mg (0.18 mmol) de 2 sont solubilisés dans 1 mL de dichlorométhane anhydre. 40 mg (0.21mmol, 1.2 eq.) d'EDC, 37 mg (0.21mmol, 1.2 eq.) de N-Boc-glycine et 2 mg (0.017 mmol, 0.1 eq.) de DMAP sont ajoutés successivement. Après 18h d'agitation à température ambiante, le milieu réactionnel est dilué dans l'acétate d'éthyle. La phase organique est lavée successivement avec une solution d'HCl 4%, une solution saturée d'hydrogénocarbonate de sodium et une solution saturée de chlorure de sodium. La phase organique est séchée sur MgSO₄, filtrée et concentrée à l'évaporateur rotatif. Le produit est purifié par chromatographie sur silice (éluant : Cyclohexane/AcOEt : 6/4). Un solide blanc est obtenu avec un rendement de 85% (80 mg).
¹H RMN (500MHz, CD3CN) δ = 6.94 (1H, d, J = 10.1 Hz, H-1), 6.13 (1H, s, H-24aa), 5.98 (1H, s, H-24ab), 5.90 (1H, d, J = 10.1 Hz, H-2), 5.57 (1H, t, J = 6.0 Hz, H-26c), 5.53 (1H, d, J = 2.1 Hz, H-22), 5.09 (1H, td, J = 7.7 Hz, J = 4.4 Hz, H-16), 4.11 (1H, dd, J = 10.7 Hz, J = 6.7 Hz, H-26<'>), 4.01 - 4.07 (1H, m, H-26<">), 3.72 (2H, d, J = 6.4 Hz, H-26b), 3.02 (1H, sxt, J = 6.8 Hz, H-25), 2.58 (1H, dqd, J = 11.0 Hz, J = 6.7 Hz, J = 2.1 Hz, H-20), 2.30 (1H, dd, J = 10.8 Hz, J = 7.5 Hz, H-17), 2.14 - 2.22 (2H, m, H-4, 15<'>), 2.09 (3H, s, H-22b), 2.04 (3H, s, H-16b), 1.97 (4H, s, H-5, 8, 11<'>), 1.62 - 1.76 (3H, m, H-6<'>, 12<">, 12<'>), 1.51 - 1.60 (1H, m, H-11<">), 1.41 - 1.48 (1H, m, H-7<'>), 1.41 (9H, s, H-26h, 26g, 26f), 1.37 (1H, dd, J = 14.3 Hz, J = 4.0 Hz, H-15<">), 1.24 (1H, d, J = 4.3 Hz, H-19<'>), 1.19 (3H, s, H-18), 1.16 - 1.23 (1H, m, H-7<">), 1.08 (3H, d, J = 7.0 Hz, H-27), 1.02 (3H, d, J = 6.7 Hz, H-28), 0.95 (3H, s, H-29), 0.93 (1H, qd, J = 12.8 Hz, J = 4.0 Hz, H-6<">), 0.85 (3H, d, J = 6.7 Hz, H-21), 0.57 (1H, d, J = 4.6 Hz, H-19<">)
\3C RMN (126MHz, CD3CN) δ = 202.3 (C-3), 198.8 (C-23), 171.7 (C-22a), 171.4 (C-26a), 171.2 (C-16a), 155.5 (C-1), 148.9 (C-24), 128.4 (C-2), 126.0 (C-24a), 78.3 (C-22), 76.7 (C-16), 68.5 (C-26), 51.3 (C-17), 48.4 (C-14), 47.6 (C-4), 46.9 (C-13), 46.7 (C-15), 45.2 (C-8), 43.6 (C-5), 43.0 (C-26b), 34.7 (C-25), 33.4 (C-20), 32.9 (C-10, 12), 28.6 (C-26h, 26g, 26f), 28.1 (C-11), 27.7 (C-19), 27.2 (C-9), 24.3 (C-7, 6), 22.1 (C-16b), 20.9 (C-22b), 20.0 (C-29), 18.3 (C-18), 17.1 (C-27), 13.3 (C-21), 11.3 (C-28)

### ◆ exemple 57 : 26-N-glycinate de neoboutomellerone

| | |
|---|---|
| 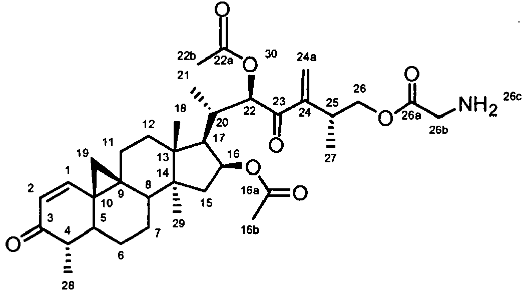 | Formule Chimique : C₃₄H₅₃NO₇ |
| | Masse Exacte : 623.38 |
| | Poids moléculaire : 623.82 |

Protocole : 29 mg (0.04 mmol, 1 eq.) de 56 sont solubilisés dans 600 µL de dichlorométhane anhydre à 0°C. 30 µL (0.4 mmol, 10 eq .) d'acide trifluoroacétique sont ajoutés et le milieu réactionnel est agité à 0°C. Au bout de 30 minutes, 30 µL (0.4 mmol, 10 eq .) d'acide trifluoroacétique sont ajoutés et le milieu réactionnel est agité à température ambiante pendant 20h. Le milieu réactionnel est alors concentré à l'évaporateur rotatif. Le résidu est repris dans l'eau. La phase aqueuse est alors basifiée avec une solution saturée de carbonate de sodium. La phase aqueuse est ensuite extraite cinq fois avec de l'acétate d'éthyle. La phase organique est lavée avec une solution saturée de chlorure de sodium. La phase organique est séchée sur Na₂SO₄, filtrée et concentrée à l'évaporateur rotatif. Le produit est purifié par chromatographie sur silice (éluant : DCM/MeOH : 95/5). Un solide blanc 57 est obtenu avec un rendement de 24% (6.6 mg).
¹H NMR (500MHz, ACETONITRILE-d₃) δ = 6.94 (1H, d, J = 10.1 Hz, H-1), 6.12 (1H, s, H-24aa), 5.97 (1H, s, H-24ab), 5.90 (1H, d, J = 9.8 Hz, H-2), 5.52 (1H, d, J = 2.1 Hz, H-22), 5.10 (1H, td, J = 7.7 Hz, J = 4.4 Hz, H-16), 3.99 - 4.16 (2H, m, H-26), 3.25-3.31 (2H, m, H-26b), 3.00 (1H, sxt, J = 7.0 Hz, H-25), 2.52 - 2.66 (1H, m, H-20), 2.29 (1H, dd, J = 10.9 Hz, J = 7.5 Hz, H-17), 2.12 - 2.22 (2H, m, H-15<'>, 4), 2.09 (3H, s, H-22b), 2.03 (4H, s, H-11<">, 16b), 1.95-1.98 (2H, m, H-8a, 5a), 1.63 - 1.74 (3H, m, H-12<">, 12<'>, 6<'>), 1.52 - 1.62 (1H, m, H-11<">), 1.40 - 1.49 (1H, m, H-7<">), 1.37 (1H, dd, J = 14.2 Hz, J = 4.1 Hz, H-15<">), 1.24 (1H, d, J = 4.3 Hz, H-19<'>), 1.21 - 1.23 (1H, m, H-7<'>), 1.18 (3H, s, H-18), 1.08 (3H, d, J = 7.0 Hz, H-27), 1.02 (3H, d, J = 6.7 Hz, H-28), 0.95 (4H, s, H-29), 0.90 - 0.93 (1H, m, H-6<">), 0.84 (3H, d, J = 7.0 Hz, H-21), 0.58 (1H, d, J = 4.6 Hz, H-19<">)
¹³C NMR (126MHz, ACETONITRILE-d₃) δ = 202.4 (C-3), 198.9 (C-23), 175.3 (C-26a), 171.7 (C-22a), 171.3 (C-16a), 155.5 (C-1), 149.1 (C-24), 128.4 (C-2), 125.8 (C-24a), 78.3 (C-22), 76.7 (C-16), 68.0 (C-26), 51.3 (C-17), 48.4 (C-14), 47.6 (C-4), 46.8 (C-13), 46.7 (C-15), 45.2 (C-8), 44.7 (C-26b), 43.6 (C-5), 34.9 (C-25), 33.3 (C-20), 32.9 (C-10, 12), 28.1 (C-11), 27.7 (C-19), 27.2 (C-9), 24.3 (C-6, 7), 22.1 (C-16b), 20.9 (C-22b), 20.0 (C-29), 18.3 (C-18), 17.2 (C-27), 13.3 (C-21), 11.3 (C-28)

### ◆ exemple 58 : 26-N-Boc-glycinate de 22-déacétyl- neoboutomellerone

| | |
|---|---|
| 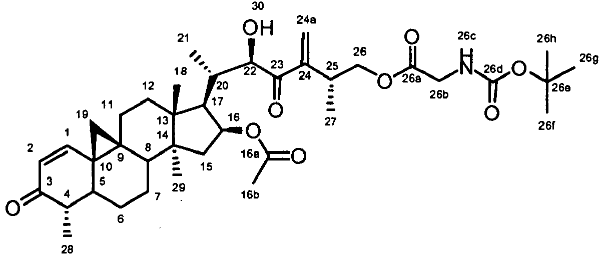 | Formule Chimique : C₃₉H₅₇NO₉ |
| | Masse Exacte : 683,40 |
| | Poids moléculaire : 683,87 |

Protocole 1 : La même réaction que pour l'exemple 56 a été effectuée sur 100 mg de composé 1 (0.19 mmol) avec 36 mg d'EDC (0.19 mmol, 1 eq.), 33 mg de N-Boc-glycine (0.19 mmol, 1 eq.) et 2 mg de DMAP (cat.) dans 1 mL de dichlorométhane anhydre. Le produit est obtenu avec un rendement de 51 % après purification sur gel de silice (éluant : Cyclohexane/AcOEt : 6/4 à 5/5).

Protocole 2 : Ce composé 58 peut également être obtenu à partir du composé 55. Une solution de TBAF dans le THF est ajoutée à une solution de 55 dans le THF et le milieu réactionnel est laissé sous agitation pendant 3 heures.

Après conversion complète du substrat (suivi par CCM : AcOEt / cyclohexane : 6 / 4), le milieu réactionnel est dilué avec de l'éther, lavé à l'eau, puis à la saumure. Après séchage sur sulfate de sodium et évaporation des solvants on recueille le composé 58 qui est purifié sur gel de silice par un gradient cyclohexane / acétate d'éthyle de 70 / 30 à 40 / 60.
¹H RMN (500MHz, CD3CN) δ = 6.94 (1H, d, J = 9.8 Hz, H-1), 6.19 (1H, s, H-24aa), 6.06 (1H, d, J = 0.6 Hz, H-24ab), 5.89 (1H, d, J = 10.1 Hz, H-2), 5.56 (1H, t, J = 5.2 Hz, NH-26c), 5.20 (1H, td, J = 7.4 Hz, J = 4.4 Hz, H-16), 4.72 (1H, dd, J = 6.1 Hz, J = 1.5 Hz, H-22), 4.07 - 4.19 (2H, m, H-26<">, 26<'>), 3.72 (2H, d, J = 6.4 Hz, H-26b), 3.53 (1H, d, J = 6.1 Hz, OH-30), 3.06 (1H, sxt, J = 6.9 Hz, H-25), 2.38 - 2.50 (2H, m, H-20, 17), 2.22 (1H, dd, J = 14.0 Hz, J = 7.9 Hz, H-15<'>), 2.15 - 2.21 (1H, m, H-4), 2.04 (3H, s, H-16b), 1.95 - 2.09 (3H, m, H-5, 8, 11<'>), 1.60 - 1.75 (3H, m, H-6<'>, 12<">, 12<'>), 1.50 - 1.59 (1H, m, H-11<">), 1.42 - 1.49 (1H, m, H-7<'>), 1.40 (9H, s, H-26h, 26g, 26f), 1.34 - 1.41 (1H, m, H-15<">), 1.24 (1H, d, J = 4.3 Hz, H-19<'>), 1.18 (3H, s, H-18), 1.15 - 1.25 (1H, m, H-7<">), 1.10 (3H, d, J = 7.0 Hz, H-27), 1.03 (3H, d, J = 7.0 Hz, H-28), 0.96 (3H, s, H-29), 0.89 - 0.99 (1H, m, H-6<">), 0.64 (3H, d, J = 6.4 Hz, H-21), 0.57 (1H, d, J = 4.3 Hz, H-19<">)
¹³C RMN (126MHz, CD3CN) δ = 204.9 (C-23), 202.4 (C-3), 171.4 (C-26a), 171.3 (C-16a), 157.0 (C-26d), 155.6 (C-1), 147.7 (C-24), 128.4 (C-2), 127.7 (C-24a), 80.0 (C-26e), 77.3 (C-16), 75.8 (C-22), 68.5 (C-26), 51.4 (C-17), 48.3 (C-14), 47.6 (C-4), 46.9 (C-15), 46.7 (C-13), 45.3 (C-8), 43.6 (C-5), 43.0 (C-26b), 36.4 (C-20), 34.6 (C-25), 33.1 (C-12), 32.9 (C-10), 28.6 (C-26h, 26g, 26f), 28.1 (C-11), 27.7 (C-19), 27.2 (C-9), 24.3 (C-7), 24.3 (C-6), 22.1 (C-16b), 20.1 (C-29), 18.5 (C-18), 17.1 (C-27), 12.3 (C-21), 11.3 (C-28)

### ◆ exemple 59 : 22,26-bis(N-Boc-glycinate) de 22-déacétyl-neoboutomellerone

| | |
|---|---|
| 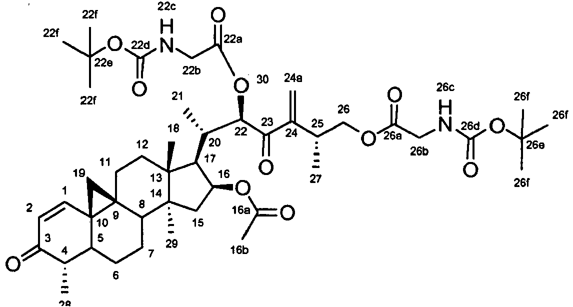 | Formule Chimique : C₄₆H₆₈N₂O₁₂ |
| | Masse Exacte : 840.48 |
| | Poids moléculaire : 841.04 |

Protocole : 200 mg (0.38 mmol) de 1 sont solubilisés dans 18 mL de DCM anhydre. Sont ensuite rajoutés 401 mg (2.01 mmol, 5.5 eq.) d'EDC, 333 mg (1.91 mmol, 5.0 eq.) de N-Boc-glycine et 23 mg (0.19 mmol, 0.5 eq.) de DMAP. Après 1 semaine à température ambiante, le milieu réactionnel est dilué dans l'acétate d'éthyle. La phase organique est lavée successivement avec une solution d'HCl 4%, une solution saturée d'hydrogénocarbonate de sodium et une solution saturée de chlorure de sodium. La phase organique est séchée sur MgSO₄, filtrée et concentrée à l'évaporateur rotatif. Le mélange est purifié par HPLC préparative (colonne sunfire, 250*30 ; 10µm ; débit : 25 ml/min) et élué par un mélange cyclohexane/AcOEt : 6/4. On obtient 153 mg (48%) de composé 59 ainsi que 94 mg (36%) de composé 58.
¹H NMR (500MHz, ACETONITRILE-d₃) δ = 6.94 (1H, d, J = 9.8 Hz, H-1), 6.15 (1H, s, H-24aa), 6.01 (1H, s, H-24ab), 5.90 (1H, d, J = 10.1 Hz, H-2), 5.59 (1H, d, J = 2.1 Hz, H-22), 5.07 (1H, td, J = 7.7 Hz, J = 4.4 Hz, H-16), 4.37 (1H, s, H-26c), 4.32 (1H, d, J = 5.8 Hz, H-22c), 4.07 - 4.12 (2H, m, H-26<">, 26<'>), 3.95 (1H, dd, J = 17.7 Hz, J = 6.4 Hz, H-22b<'>), 3.82 (1H, dd, J = 17.7 Hz, J = 6.1 Hz, H-22b<">), 3.70 - 3.75 (2H, m, H-26b<">, 26b<'>), 3.04 (1H, sxt, J = 6.8 Hz, H-25), 2.62 (1H, dtd, J = 13.8 Hz, J = 6.0 Hz, J = 2.1 Hz, H-20), 2.30 (1H, dd, J = 10.7 Hz, J = 7.6 Hz, H-17), 2.14 - 2.21 (5H, m, H-15<'>, 4), 2.04 (3H, s, H-16b), 2.01 (1H, dd, J = 9.6 Hz, J = 3.8 Hz, H-11<'>, 5a, 8a), 1.64 - 1.81 (3H, m, H-12<">, 12<'>, 6<'>), 1.51 - 1.62 (1H, m, H-11<">), 1.43 - 1.48 (1H, m, H-7<'>), 1.41 (18H, s, H-22f, 22f, 22f, 26f, 26f, 26f), 1.34 - 1.39 (2H, m, H-15<">), 1.26 - 1.29 (1H, m, H-7<">), 1.25 (1H, d, J = 4.3 Hz, H-19<'>), 1.19 (3H, d, J = 1.2 Hz, H-18), 1.08 (3H, d, J = 7.0 Hz, H-27), 1.02 (3H, d, J = 6.7 Hz, H-28), 0.97 (3H, s, H-29), 0.92 - 0.96 (1H, m, H-6<">), 0.84 (3H, d, J = 7.0 Hz, H-21), 0.57 (1H, d, J = 4.3 Hz, H-19<">).
¹³C NMR (126MHz, ACETONITRILE-d₃) δ = 202.4 (C-3), 198.2 (C-23), 171.5 (C-22a), 171.4 (C-26a), 171.2 (C-16a), 157.0 (C-26d), 156.9 (C-22d), 155.5 (C-1), 148.8 (C-24), 128.4 (C-2), 126.3 (C-24a), 80.0 (C-22e), 80.0 (C-26e), 78.9 (C-22), 76.8 (C-16), 68.6 (C-26), 51.1 (C-17), 48.4 (C-14), 47.6 (C-4), 46.9 (C-13), 46.7 (C-15), 45.0 (C-8), 43.5 (C-5), 43.0 (C-22b), 43.0 (C-26b), 34.5 (C-25), 33.7 (C-20), 33.0 (C-12), 32.9 (C-10), 28.6 (C-26f, 26f, 26f), 28.6 (C-22f, 22f, 22f), 28.1 (C-11), 27.5 (C-19), 27.2 (C-9), 24.2 (C-7), 24.2 (C-6), 22.1 (C-16b), 20.0 (C-29), 18.2 (C-18), 17.0 (C-27), 13.3 (C-21), 11.3 (C-28).

### ◆ exemple 60 : 22 (N-Boc-glycinate), 26 acétyl de 22-déacétyl-neoboutomellerone

| | |
|---|---|
| 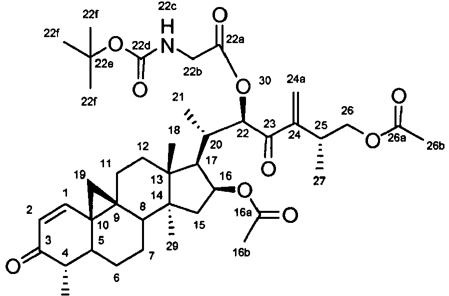 | Formule Chimique : C₄₆H₅₉NO₁₀ |
| | Masse Exacte : 725.41 |
| | Poids moléculaire : 725.91 |

Protocole : 66 mg (0.118 mmol) de composé 17 sont dissous dans 2 ml de DCM, puis 1.8 ml (1.6 éq.) d'une solution 0.1 M de DCC est ajouté, suivie d'une quantité catalytique de DMAP (2 mg, 0.1 éq.) et enfin de 31 mg (1.5 éq., 0.176 mmol) de *N-*Boc-Gly-OH. La réaction est laissée 3h sous agitation à température ambiante. Le milieu réactionnel est filtré sur célite^{®}, et le produit est purifié sur gel de silice (éluant : gradient cyclohexane/AcOEt: de 90 / 10 à 50 / 50). On obtient 44 mg (52%) de produit 60 (Rf : 0.64 - cyclohexane / acétate d'éthyle 50 / 50).
¹H NMR (500MHz, ACETONITRILE-d₃) δ = 6.94 (1H, d, J = 9.8 Hz, H-1), 6.12 (1H, s, H-24aa), 5.97 (1H, s, H-24ab), 5.90 (1H, d, J = 10.1 Hz, H-2), 5.62 (1H, br. s., H-22c), 5.59 (1H, d, J = 2.1 Hz, H-22), 5.08 (1H, td, J = 7.6 Hz, J = 4.6 Hz, H-16), 4.04 (1H, dd, J = 10.8 Hz, J = 6.4 Hz, H-26<'>), 4.00 (1H, dd, J = 10.8 Hz, J = 6.4 Hz, H-26<">), 3.94 (1H, dd, J = 17.7 Hz, J = 6.4 Hz, H-22b<'>), 3.82 (1H, dd, J = 17.7 Hz, J = 6.2 Hz, H-22b<">), 3.01 (1H, sxt, J = 6.9 Hz, H-25), 2.61 (1H, dtd, J = 13.9 Hz, J = 6.9 Hz, J = 2.2 Hz, H-20), 2.30 (1H, dd, J = 11.0 Hz, J = 7.6 Hz, H-17), 2.19 (1H, d, J = 6.7 Hz, H-4, 15<'>), 2.15 (3H, s, H-26b), 2.04 - 2.06 (1H, m, H-11<'>), 2.03 (3H, s, H-16b), 2.00 (2H, dd, J = 6.9 Hz, J = 4.1 Hz, H-8a, 5a), 1.64 - 1.75 (3H, m, H-12<">, 12<'>, 6<'>), 1.52 - 1.61 (1H, m, H-11<">), 1.43 - 1.47 (1H, m, H-7<'>), 1.41 (9H, s, H-22f, 22f, 22f), 1.39 - 1.39 (1H, m, M30), 1.37 (1H, dd, J = 13.9 Hz, J = 4.1 Hz, H-15<">), 1.27 (1H, d, J = 3.7 Hz, H-7<">), 1.25 (1H, d, J = 4.6 Hz, H-19<'>), 1.18 (3H, s, H-18), 1.07 (3H, d, J = 7.0 Hz, H-27), 1.02 (3H, d, J = 7.0 Hz, H-28), 0.97 (3H, s, H-29), 0.91 - 0.96 (1H, m, H-6<">), 0.85 (3H, d, J = 6.7 Hz, H-21), 0.57 (1H, d, J = 4.3 Hz, H-19<">)
¹³C NMR (126MHz, ACETONITRILE-d₃) δ = 202.4 (C-3), 198.4 (C-23), 171.6 (C-26a), 171.4 (C-22a), 171.2 (C-16a), 156.9 (C-22d), 155.5 (C-1), 149.0 (C-24), 128.4 (C-2), 126.0 (C-24a), 80.0 (C-22e), 78.8 (C-22), 76.7 (C-16), 67.8 (C-26), 51.2 (C-17), 48.4 (C-14), 47.6 (C-4), 46.9 (C-13), 46.7 (C-15), 45.0 (C-8), 43.5 (C-5), 43.0 (C-22b), 34.9 (C-25), 33.6 (C-20), 33.0 (C-12), 33.0 (C-10), 28.6 (C-22f, 22f, 22f), 28.1 (C-11), 27.5 (C-19), 27.2 (C-9), 24.2 (C-7), 24.2 (C-6), 22.1 (C-16b), 21.1 (C-26b), 20.0 (C-29), 18.2 (C-18), 17.3 (C-27), 13.3 (C-21), 11.3 (C-28)

### ◆ exemples 61 : 22-chloroacétyl-26-acétyl-neoboutomellerone

| | |
|---|---|
| 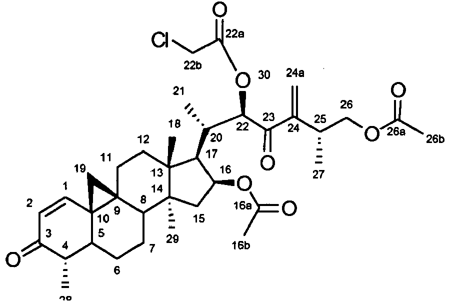 | Formule Chimique : C₃₆H₄₉ClO₈ |
| | Masse Exacte : 644,31 |
| | Poids moléculaire : 645,22 |

Protocole : 64 mg (0.113 mmol) de composé 17 sont dissous dans 2 ml de DCM, puis 1.8 ml (1.6 éq.) d'une solution 0.1 M de DCC est ajouté, suivie d'une quantité catalytique de DMAP (2 mg, 0.1 éq.) et enfin de 16 mg (1.5 éq., 0.169 mmol) d'acide chloroacétique. La réaction est laissée 1h sous agitation à température ambiante. Le milieu réactionnel est filtré sur célite^{®}, et le produit est purifié sur gel de silice (éluant : gradient cyclohexane/AcOEt: de 90 / 10 à 50 / 50). On obtient 70mg (97%) de produit 61 (Rf : 0.75 - cyclohexane / acétate d'éthyle 50 / 50).
¹H NMR (500MHz, ACETONITRILE-d₃) δ = 6.94 (1H, d, J = 10.0 Hz, H-1), 6.14 (1H, s, H-24aa), 6.01 (1H, s, H-24ab), 5.90 (1H, d, J = 10.0 Hz, H-2), 5.59 (1H, d, J = 2.1 Hz, H-22), 5.10 (1H, td, J = 7.7 Hz, J = 4.4 Hz, H-16), 4.33 (1H, d, J = 15.1 Hz, H-22b<'>), 4.25 (1H, d, J = 15.1 Hz, H-22b<">), 3.99 - 4.08 (3H, m, H-26<">, 26<'>), 3.02 (1H, sxt, J = 7.0 Hz, H-25), 2.63 (1H, dtd, J = 13.7 Hz, J = 7.1 Hz, J = 2.3 Hz, H-20), 2.32 (1H, dd, J = 11.1 Hz, J = 7.6 Hz, H-17), 2.17 - 2.21 (1H, m, H-4), 2.05 - 2.10 (1H, m, H-11<'>), 2.04 (3H, s, H-16b), 1.98 - 2.03 (2H, m, H-5a, 8a), 1.97 (4H, s, H-26b), 1.63 - 1.75 (4H, m, H-12<">, 12<'>, 6<'>), 1.53 - 1.61 (1H, m, H-11<">), 1.41 - 1.49 (1H, m, H-7<'>), 1.38 (1H, dd, J = 13.8 Hz, J = 4.2 Hz, H-15<">), 1.25 (1H, d, J = 4.4 Hz, H-19<'>), 1.19 - 1.21 (1H, m, H-7<">), 1.19 (3H, s, H-18), 1.12 - 1.17 (1H, m, M32), 1.08 (4H, d, J = 7.0 Hz, H-28), 1.02 (3H, d, J = 6.7 Hz, H-27), 0.95 (3H, s, H-29), 0.90 - 0.94 (1H, m, H-6<">), 0.86 (3H, d, J = 6.9 Hz, H-21), 0.57 (1H, d, J = 4.4 Hz, H-19<">).
¹³C NMR (126MHz, ACETONITRILE-d₃) δ = 202.4 (C-3), 197.7 (C-23), 171.8 (C-26a), 171.2 (C-16a), 168.2 (C-22a), 155.5 (C-1), 148.9 (C-24), 128.4 (C-2), 126.4 (C-24a), 80.0 (C-22), 76.7 (C-16), 67.8 (C-26), 51.1 (C-17), 48.4 (C-14), 47.6 (C-4), 46.9 (C-13), 46.7 (C-15), 45.1 (C-8), 43.5 (C-5), 42.1 (C-22b), 34.9 (C-25), 34.5 (C-10), 33.6 (C-20), 32.9 (C-12), 28.1 (C-11), 27.6 (C-19), 27.2 (C-9), 24.3 (C-6), 24.2 (C-7), 22.1 (C-16b), 21.1 (C-26b), 19.9 (C-29), 18.2 (C-18), 17.2 (C-27), 13.2 (C-21), 11.3 (C-28).

### ◆ exemples 62 & 63 : 26-N diméthyl glycinate de neoboutomellerone (62) et Chlorhydrate de 26-N diméthyl glycinate de neoboutomellerone (63)

| | |
|---|---|
| 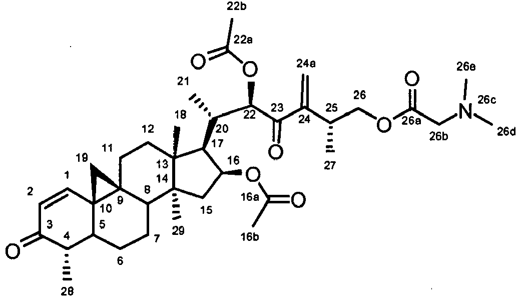 | 62 |
| | Formule Chimique : C₃₈H₅₅NO₈ |
| | Masse Exacte : 653,39 |
| | Poids moléculaire : 653,85 |
| 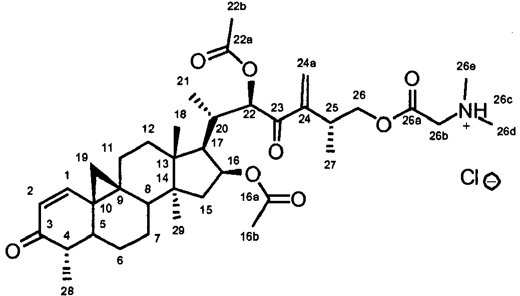 | 63 |
| | Formule Chimique : C₃₈H₅₆NO₈⁺ (contre-ion : Cl⁻) |
| | Masse Exacte : 654,40 |
| | Poids moléculaire : 654,85 |

Protocole : 3.5 mL (0.1 mol/L, 0.35 mmol, 2 eq.) d'une solution de DCC dans le dichlorométhane est mise dans un bicol sous azote. 36 mg (0.35 mmol, 2 eq.) de diméthylglycine et 2 mg (0.018 mmol, 0.1 eq.) de DMAP sont ajoutés. Finalement, 100 mg (0.18 mmol) de 2 est ajouté. Le milieu réactionnel est agité à température ambiante pendant 24 heures. Le milieu réactionnel est filtré et le filtrat est lavé avec de l'eau distillée et séché sur Na₂SO₄. Le produit est purifié par chromatographie sur gel de silice (éluant : Cyclohexane/AcOEt : 3/7) afin d'obtenir l'amine 62 sous la forme d'une huile translucide avec un rendement de 23%. 3 mL d'HCl 0.1 M sont ajoutés à 26 mg de diméthylamine 63 et le milieu réactionnel est agité à température ambiante pendant 1 heure. Le milieu réactionnel est ensuite lyophilisé afin d'obtenir le chlorhydrate 63 (100%).

### exemple 62 :

¹H RMN (500MHz, CD3CN) δ = 6.94 (1H, d, J = 10.1 Hz, H-1), 6.12 (1H, s, H-24aa), 5.97 (1H, s, H-24ab), 5.90 (1H, d, J = 9.8 Hz, H-2), 5.53 (1H, d, J = 2.1 Hz, H-22), 5.09 (1H, td, J = 7.6 Hz, J = 4.6 Hz, H-16), 4.00 - 4.15 (2H, m, H-26<">, 26<'>), 3.10 (2H, s, H-26b), 3.02 (1H, sxt, J = 6.9 Hz, H-25), 2.58 (1H, dqd, J = 11.0 Hz, J = 7.0 Hz, J = 2.1 Hz, H-20), 2.30 (1H, dd, J = 11.0 Hz, J = 7.3 Hz, H-17), 2.26 (6H, s, H-26e, 26d), 2.13-2.22 (2H, m, H-4, 15<'>), 2.09 (3H, s, H-22b), 2.03 (3H, s, H-16b), 1.95 - 2.06 (3H, m, H-5, 8, 11<'>), 1.63 - 1.77 (3H, m, H-6<'>, 12<">, 12<'>), 1.52 - 1.60 (1H, m, H-11<">), 1.41 - 1.50 (1H, m, H-7<'>), 1.37 (1H, dd, J = 14.2 Hz, J = 3.8 Hz, H-15<">), 1.24 (1H, d, J = 4.3 Hz, H-19<'>), 1.19 (3H, s, H-18), 1.15 - 1.22 (1H, m, H-7<">), 1.07 (3H, d, J = 7.3 Hz, H-27), 1.02 (3H, d, J = 6.7 Hz, H-28), 0.95 (3H, s, H-29), 0.89 - 0.99 (1H, m, H-6<">), 0.85 (3H, d, J = 7.0 Hz, H-21), 0.58 (1H, d, J = 4.6 Hz, H-19<">)
¹³C RMN (126MHz, CD3CN) δ = 202.4 (C-3), 198.8 (C-23), 171.6 (C-22a), 171.5 (C-26a), 171.2 (C-16a), 155.5 (C-1), 149.1 (C-24), 128.4 (C-2), 125.8 (C-24a), 78.3 (C-22), 76.7 (C-16), 67.7 (C-26), 60.7 (C-26b), 51.3 (C-17), 48.4 (C-14), 47.6 (C-4), 46.9 (C-13), 46.7 (C-15), 45.3 (C-26d, 26e), 45.2 (C-8), 43.6 (C-5), 34.9 (C-25), 33.3 (C-20), 33.0 (C-12), 32.9 (C-10), 28.1 (C-11), 27.7 (C-19), 27.6, 27.2 (C-9), 24.3 (C-7, 6), 22.1 (C-16b), 20.9 (C-22b), 20.0 (C-29), 18.3 (C-18), 17.3 (C-27), 13.3 (C-21), 11.3 (C-28)

### exemple 63 :

¹H RMN (500MHz, D2O) δ = 7.19 (1H, d, J = 9.8 Hz, H-1), 6.40 (1H, br. s., H-24aa), 6.28 (1H, s, H-24ab), 5.94 - 6.06 (1H, m, J = 5.5 Hz, H-2), 5.67 (1H, br. s., H-22), 5.08 (1H, br. s., H-16), 4.36 (1H, dd, J = 10.8 Hz, J = 5.6 Hz, H-26<'>), 4.23 (1H, dd, J = 11.0 Hz, J = 7.9 Hz, H-26<">), 4.11 (1H, d, J = 17.1 Hz, H-26b<'>), 4.05 (1H, d, J = 17.4 Hz, H-26b<">), 3.09 - 3.21 (1H, m, H-25), 2.67 (1H, br. s., H-20), 2.29 - 2.42 (2H, m, H-4, 17), 2.21 (3H, s, H-22b), 2.18 - 2.28 (1H, m, H-15<'>), 2.15 (3H, s, H-16b), 1.95 - 2.12 (3H, m, H-5, 8, 11<'>), 1.71 (3H, br. s., H-6<'>, 12<">, 12<'>), 1.64 (1H, br. s., H-11<">), 1.40 - 1.54 (2H, m, H-7<'>, 15<">), 1.37 (1H, br. s., H-19<'>), 1.19 (3H, br. s., H-18), 1.15 - 1.27 (1H, m, H-7<">), 1.12 (3H, d, J = 7.0 Hz, H-27), 1.04 (3H, d, J = 6.7 Hz, H-28), 0.91 - 1.00 (1H, m, H-6<">), 0.95 (3H, br. s., H-29), 0.88 (3H, d, J = 6.4 Hz, H-21), 0.64 (1H, br. s., H-19<">)
¹³C RMN (126MHz, D2O) δ = 174.8 (C-16a, 22a), 167.3 (C-26a), 160.6 (C-1), 147.9 (C-24), 129.2 (C-24a), 127.4 (C-2), 79.4 (C-22), 78.2 (C-16), 70.8 (C-26), 58.0 (C-26b), 51.1 (C-17), 48.2 (C-14), 47.4 (C-4), 46.7 (C-13), 46.1 (C-15), 44.7 (C-26e, 26d), 44.2 (C-8), 42.8 (C-5), 33.7 (C-20), 33.4 (C-10), 33.3 (C-25), 32.6 (C-12), 27.8 (C-11), 27.8 (C-9), 27.2 (C-19), 23.8 (C-6), 23.6 (C-7), 22.3 (C-16b), 21.0 (C-22b), 19.8 (C-29), 17.9 (C-18), 16.7 (C-27), 13.3 (C-21), 11.1 (C-28)

### ◆ exemples 64 & 65: 26-N diméthyl glycinate de (22-déacétyl-neoboutomellerone) (64) et Chlorhydrate de 26-N diméthyl glycinate de (22-déacétyl-neoboutomellerone) (65)

| | |
|---|---|
| 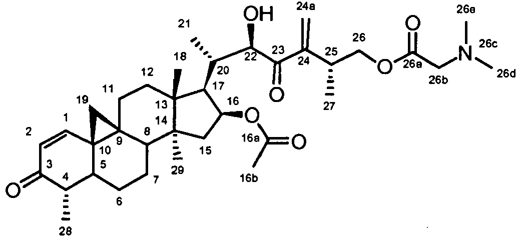 | 64 |
| | Formule Chimique : C₃₆H₅₃NO₇ |
| | Masse Exacte : 611.38 |
| | Poids moléculaire : 611.81 |
| 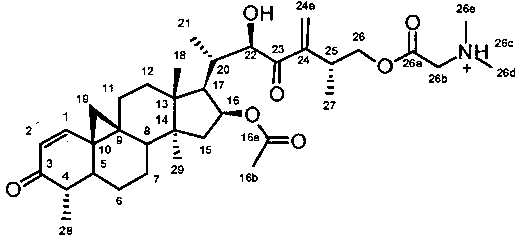 | 64 |
| | Formule Chimique : C₃₆H₅₄NO₇ (contre-ion : Cl⁻) |
| | Masse Exacte : 612.38 |
| | Poids moléculaire : 612.82 |

Protocole : 20 mg (0.19 mmol, 1 eq.) de diméthylglycine est solubilisé dans 1 mL de dichlorométhane anhydre. 80 µL (0.58, 3 eq.) de triéthylamine et 69 mg (0.21 mmol, 1.1 eq) de TBTU sont ajoutés et le milieu réactionnel est agité pendant 5 minutes. Puis, 100 mg (0.19 mmol, 1 eq.) de 1 est ajouté. Après 16h d'agitation à température ambiante, le milieu réactionnel est dilué dans l'acétate d'éthyle et la phase organique est lavée successivement avec une solution d'HCl 4%, une solution saturée d'hydrogénocarbonate de sodium et de chlorure de sodium. La phase organique est séchée sur MgSO₄, filtrée et concentrée à l'évaporateur rotatif. Le produit est purifié par chromatographie sur gel de silice (éluant : AcOEt/MeOH :100/0 à 95/5). Un solide blanc 64 est obtenu avec un rendement de 57% (112 mg). 42 mg (0.068 mmol, 1 eq.) de 64 sont solubilisés dans 2 mL d'éthanol et 700 µL d'HCl 0.1 M (1 eq.) sont ajoutés. Le milieu réactionnel est agité à température ambiante pendant 30 minutes et concentré à l'évaporateur rotatif. La solution aqueuse est ensuite lyophilisée afin d'obtenir le chlorhydrate 65 (90%).

### exemple 64 :

¹H RMN (500MHz, CD3CN) δ = 6.94 (1H, d, J = 10.1 Hz, H-1), 6.18 (1H, s, H-24aa), 6.05 (1H, d, J = 0.9 Hz, H-24ab), 5.89 (1H, d, J = 9.8 Hz, H-2), 5.20 (1H, td, J = 7.4 Hz, J = 4.4 Hz, H-16), 4.72 (1H, d, J = 4.6 Hz, H-22), 4.04 - 4.15 (2H, m, H-26<'>, 26<">), 3.54 (1H, d, J = 5.8 Hz, OH-30), 3.10 (2H, s, H-26b), 3.07 (1H, sxt, J = 7.0 Hz, H-25), 2.37 - 2.49 (2H, m, H-17, 20), 2.25 (6H, s, H-26d, 26e), 2.16 - 2.24 (2H, m, H-4, 15<'>), 2.02 (3H, s, H-16b), 2.02 (3H, s, H-5, 8, 11<'>), 1.60 - 1.74 (3H, m, H-6<'>, 12<">, 12<'>), 1.50 - 1.59 (1H, m, H-11<">), 1.41 - 1.49 (1H, m, H-7<'>), 1.38 (1H, dd, J = 14.0 Hz, J = 3.7 Hz, H-15<">), 1.24 (1H, d, J = 4.3 Hz, H-19<'>), 1.18 (3H, s, H-18), 1.14 - 1.26 (1H, m, H-7<">), 1.10 (3H, d, J = 7.0 Hz, H-27), 1.03 (3H, d, J = 6.7 Hz, H-28), 0.96 (3H, s, H-29), 0.88 - 0.99 (1H, m, H-6<">), 0.64 (3H, d, J = 6.1 Hz, H-21), 0.57 (1H, d, J = 4.3 Hz, H-19<">)
¹³C RMN (126MHz, CD3CN) δ = 205.0 (C-23), 202.4 (C-3), 171.5 (C-26a), 171.2 (C-16a), 155.6 (C-1), 148.0 (C-24), 128.4 (C-2), 127.5 (C-24a), 77.3 (C-16), 75.8 (C-22), 67.8 (C-26), 60.7 (C-26b), 51.4 (C-17), 48.3 (C-14), 47.6, 46.9 (C-15), 46.7 (C-13), 45.3 (C-8), 45.3 (C-26d, 26e), 43.6 (C-5), 36.4 (C-20), 34.8 (C-25), 33.1 (C-12), 32.9 (C-10), 28.1 (C-11), 27.7 (C-19), 27.2 (C-9), 24.3 (C-6), 24.3 (C-7), 22.1 (C-16b), 20.1 (C-29), 18.5 (C-18), 17.3 (C-27), 12.3 (C-21), 11.3 (C-28)

### exemple 65 :

¹H NMR (500MHz, DMSO-d₆) δ = 10.15 (1H, br. s, H-26c), 6.96 (1H, d, J = 10.0 Hz, H-1), 6.12 (1H, s, H-24ab), 6.07 (1H, s, H-24aa), 5.90 (1H, d, J = 10.1 Hz, H-2), 5.12 - 5.22 (1H, m, H-16), 4.73 - 4.86 (1H, m, H-30), 4.55 - 4.66 (1H, m, H-22), 4.19 (2H, m, H-26), 4.07 - 4.16 (2H, m, H-26b), 3.04 (1H, sxt, J = 6.9 Hz, H-25), 2.81 (6H, s, H-26d, 26e), 2.30 - 2.41 (2H, m, H-20, 17), 2.08 - 2.18 (2H, m, H-4, 15<'>), 2.02 (3H, s, H-16b), 1.92 - 2.01 (2H, m, H-11<'>, 8a), 1.86 - 1.94 (1H, td, J = 12.3 Hz, J = 4.2 Hz, H-5a), 1.57-1.64 (3H, m, H-6<'>, 12), 1.51 - 1.56 (1H, m, H-11<">), 1.38 - 1.44 (1H, m, H-7<'>), 1.31 (1H, dd, J = 14.0 Hz, J = 4.0 Hz, H-15<">), 1.25 (1H, d, J = 4.3 Hz, H-19<'>), 1.12 (4H, s, H-7<">, 18), 1.06 (3H, d, J = 7.0 Hz, H-27), 0.98 (4H, d, J = 6.7 Hz, H-28), 0.92 - 0.95 (1H, m, H-6<">), 0.90 (3H, s, H-29), 0.65 (3H, d, J = 6.1 Hz, H-21), 0.57 (1H, d, J = 4.3 Hz, H-19<">)
¹³C NMR (126MHz, DMSO-d₆) δ = 203.4 (C-23), 200.6 (C-3), 169.9 (C-16a), 166.0 (C-26a), 154.7 (C-1), 146.9 (C-24), 127.3 (C-2), 125.2 (C-24a), 75.3 (C-16), 74.2 (C-22), 68.5 (C-26), 56.1 (C-26b), 49.5 (C-17), 47.0 (C-14), 46.1 (C-4), 45.3 (C-15), 45.2 (C-13), 43.3 (C-26e, 26d), 43.3 (C-8), 42.0 (C-5), 34.3 (C-20), 32.6 (C-25), 31.8 (C-12), 31.6 (C-10), 26.7 (C-11), 26.2 (C-19), 25.9 (C-9), 22.8 (C-6, 7), 21.4 (C-16b), 19.1 (C-29), 17.6 (C-18), 16.5 (C-27), 11.7 (C-21), 10.8 (C-28)

### ◆ exemples 66 & 67 : 26-N diéthyl β-alaninate de (26-déacétyl-neoboutomellerone) (66) et 26-acrylate de (26-déacétyl-neoboutomellerone) (67)

| | |
|---|---|
| 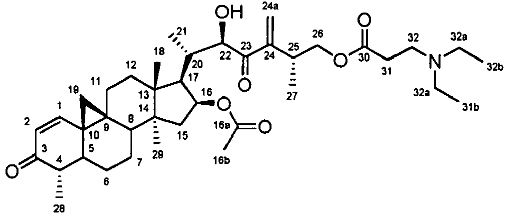 | 66 |
| | Formule Chimique : C₃₉H₅₉NO₇ |
| | Masse Exacte : 653.43 |
| | Poids moléculaire : 653.89 |
| 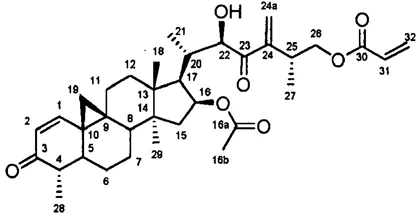 | 67 |
| | Formule Chimique : C₃₅H₄₈O₇ |
| | Masse Exacte : 580.34 |
| | Poids moléculaire : 580.75 |

Protocole : Le composé 1 (200 mg, 0.38 mmol) est dissous dans 2 ml de DCM, puis on y rajoute une solution dans le DCM de chlorhydrate de diéthyl β-alanine (138 mg, 2 éq., 0.76 mmol), de TBTU (254 mg, 2.1 éq., 0.82 mmol) et de triéthylamine (0.21 ml, 4 éq., 1.52 mmol). La réaction est laissée sous agitation à température ambiante une nuit. La réaction est hydrolysée à l'eau et la phase organique est lavée 3 fois par une solution de carbonate de potassium à 3%, puis 3 fois à l'eau. On lave ensuite cette même phase organique 4 fois avec une solution d'acide citrique à 5 %, et ces dernières phases aqueuses réunies sont rebasifiées par une solution saturée de carbonate de sodium. On réextrait le produit de cette phase aqueuse par l'acétate d'éthyle, et cette phase organique est lavée à l'eau puis à la saumure. On recueille ainsi 215 mg de brut réactionnel qui est filtré sur gel de silice. Le résidu obtenu après évaporation des solvants est repurifié par chromatographie préparative (éluant : AcOEt/NEt₃ : 90/10), pour obtenir 90 mg de produit 66, et 36 mg de composé 67.

### exemple 66

¹H NMR (500MHz, ACETONITRILE-d₃) δ = 6.93 (1H, d, J = 10.0 Hz, H-1), 6.10 (1H, s, H-24aa), 5.99 (1H, d, J = 0.9 Hz, H-24ab), 5.90 (1H, s, H-31, 2), 5.24 (1H, ddd, J = 7.3 Hz, J = 1.8 Hz, J = 1.5 Hz, H-32aa), 5.20 (1H, td, J = 7.4 Hz, J = 4.3 Hz, H-16), 5.12 (1H, qd, J = 15.5 Hz, J = 1.5 Hz, H-32ab), 4.71 (1H, dd, J = 5.5 Hz, J = 1.5 Hz, H-22), 3.93 (1H, dd, J = 5.4 Hz, J = 1.4 Hz, H-30), 3.51 - 3.52 (1H, m, H-38), 3.51 (1H, dd, J = 9.4 Hz, J = 6.4 Hz, H-26<'>), 3.33 (1H, dd, J = 9.3 Hz, J = 6.6 Hz, H-26<">), 2.97 (1H, sxt, J = 6.7 Hz, H-25), 2.38 - 2.50 (1H, m, H-17, 20), 2.23 (1H, dd, J = 13.9 Hz, J = 7.8 Hz, H-15<'>), 2.15 - 2.20 (1H, m, H-4), 2.03 - 2.09 (1H, m, H-8a, 11 <'>), 2.03 (2H, s, H-16b), 1.96 - 2.02 (2H, m, H-5a), 1.61 - 1.74 (2H, m, H-12<">, 12<'>, 6<'>), 1.55 (1H, ddd, J = 14.9 Hz, J = 8.6 Hz, J = 6.2 Hz, H-11<">), 1.42 - 1.50 (1H, m, H-7<'>), 1.38 (1H, dd, J = 14.2 Hz, J = 4.2 Hz, H-15<">), 1.24 (1H, d, J = 4.5 Hz, H-19<'>), 1.19 - 1.23 (1H, m, H-7<">), 1.18 (2H, s, H-18), 1.08 (1H, d, J = 7.0 Hz, H-27), 1.03 (2H, d, J = 6.7 Hz, H-28), 0.97 (2H, s, H-29), 0.91 - 0.96 (1H, m, H-6<">), 0.65 (1H, d, J = 6.1 Hz, H-21), 0.57 (1H, d, J = 4.5 Hz, H-19<">).
¹³C NMR (126MHz, ACETONITRILE-d₃) δ = 205.4 (C-23), 202.4 (C-3), 171.3 (C-16a), 155.6 (C-1), 149.2 (C-24), 136.4 (C-31), 128.4 (C-2), 126.5 (C-24a), 116.7 (C-32), 77.3 (C-16), 75.9 (C-22), 74.7 (C-26), 72.4 (C-30), 51.5 (C-17), 48.3 (C-14), 47.8 (C-4), 47.0 (C-15), 46.7 (C-13), 45.4 (C-8), 43.7 (C-5), 36.2 (C-25), 35.8 (C-20), 33.2 (C-12), 33.0 (C-10), 28.2 (C-11), 27.8 (C-19), 27.3 (C-9), 24.4 (C-6), 24.4 (C-7), 22.1 (C-16b), 20.1 (C-29), 18.6 (C-18), 17.7 (C-27), 12.3 (C-21), 11.3 (C-28).

### exemple 67

¹H NMR (500MHz, ACETONITRILE-d₃) δ = 6.93 (1H, d, J = 10.0 Hz, H-1), 6.10 (1H, s, H-24aa), 5.99 (1H, d, J = 0.9 Hz, H-24ab), 5.90 (1H, s, H-31, 2), 5.24 (1H, ddd, J = 7.3 Hz, J = 1.8 Hz, J = 1.5 Hz, H-32aa), 5.20 (1H, td, J = 7.4 Hz, J = 4.3 Hz, H-16), 5.12 (1H, qd, J = 15.5 Hz, J = 1.5 Hz, H-32ab), 4.71 (1H, dd, J = 5.5 Hz, J = 1.5 Hz, H-22), 3.93 (1H, dd, J = 5.4 Hz, J = 1.4 Hz, H-30), 3.51 - 3.52 (1H, m, H-38), 3.51 (1H, dd, J = 9.4 Hz, J = 6.4 Hz, H-26<'>), 3.33 (1H, dd, J = 93 Hz, J = 6.6 Hz, H-26<">), 2.97 (1H, sxt, J = 6.7 Hz, H-25), 2.38 - 2.50 (1H, m, H-17, 20), 2.23 (1H, dd, J = 13.9 Hz, J = 7.8 Hz, H-15<'>), 2.15 - 2.20 (1H, m, H-4), 2.03 - 2.09 (1H, m, H-8a, 11<'>), 2.03 (2H, s, H-16b), 1.96 - 2.02 (2H, m, H-5a), 1.61 - 1.74 (2H, m, H-12<">, 12<'>, 6<'>), 1.55 (1H, ddd, J = 14.9 Hz, J = 8.6 Hz, J = 6.2 Hz, H-11<">), 1.42 - 1.50 (1H, m, H-7<'>), 1.38 (1H, dd, J = 14.2 Hz, J = 4.2 Hz, H-15<">), 1.24 (1H, d, J = 4.5 Hz, H-19<'>), 1.19 - 1.23 (1H, m, H-7<">), 1.18 (2H, s, H-18), 1.08 (1H, d, J = 7.0 Hz, H-27), 1.03 (2H, d, J = 6.7 Hz, H-28), 0.97 (2H, s, H-29), 0.91 - 0.96 (1H, m, H-6<">), 0.65 (1H, d, J = 6.1 Hz, H-21), 0.57 (1H, d, J = 4.5 Hz, H-19<">).
¹³C NMR (126MHz, ACETONITRILE-d₃) δ = 205.4 (C-23), 202.4 (C-3), 171.3 (C-16a), 155.6 (C-1), 149.2 (C-24), 136.4 (C-31), 128.4 (C-2), 126.5 (C-24a), 116.7 (C-32), 77.3 (C-16), 75.9 (C-22), 74.7 (C-26), 72.4 (C-30), 51.5 (C-17), 48.3 (C-14), 47.8 (C-4), 47.0 (C-15), 46.7 (C-13), 45.4 (C-8), 43.7 (C-5), 36.2 (C-25), 35.8 (C-20), 33.2 (C-12), 33.0 (C-10), 28.2 (C-11), 27.8 (C-19), 27.3 (C-9), 24.4 (C-6), 24.4 (C-7), 22.1 (C-16b), 20.1 (C-29), 18.6 (C-18), 17.7 (C-27), 12.3 (C-21), 11.3 (C-28).

### ◆ exemples 68 et 69 : 26-succinate- neoboutomellerone (68) et Sel de N-méthyl glutamine de 26-succinate- neoboutomellerone (69)

| | |
|---|---|
| 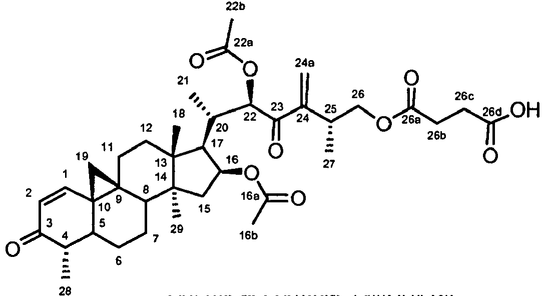 | 68 |
| | Formule Chimique : C₃₈H₅₂O₁₀ |
| | Masse Exacte : 668,36 |
| | Poids moléculaire : 668,81 |
| 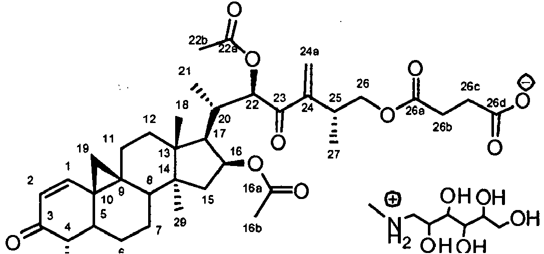 | 69 |
| | Formule Chimique : C₃₈H₅₂O₁₀ |
| | Masse Exacte : 667,36 |
| | Poids moléculaire : 667,81 |

Protocole : 100 mg (0.18 mmol) de 2 est solubilisé dans 3.5 mL de dichlorométhane anhydre. 75 mg d'anhydride succinique (0.70 mmol, 4 eq.) et 86 mg de DMAP (0.70 mmol, 4 eq.) sont ajoutés. Après 1h d'agitation à température ambiante, la réaction est terminée. Après dilution dans l'acétate d'éthyle, la phase organique est lavée successivement avec une solution d'HCl 4%, une solution saturée d'hydrogénocarbonate de sodium et une solution saturée de chlorure de sodium. La phase organique est séchée sur MgSO₄, filtrée et concentrée à l'évaporateur rotatif. Le produit est purifié par chromatographie sur silice (éluant : DCM/MeOH :100/0 à 95/5) puis par HPLC RP-18 (gradient : H₂O/CH₃CN : 90/10 à 0/100). Un solide blanc 68 est obtenu avec un rendement de 95% (112 mg). 94 mg (0.14 mmol, 1 eq.) de 68 est dissout dans 5 mL d'éthanol. 27 mg (0.14 mmol, 1 eq.) de N-methyl-D-glucamine sont dissous dans 2 mL d'eau et ajoutés à la solution précédemment préparée. Le milieu réactionnel est agité pendant 10 minutes et concentrée à l'évaporateur rotatif. Le résidu est repris dans 5 mL d'eau, filtré sur un filtre 45 m et lyophilisé afin de donner 100 mg de 69 (83%).

### exemple 68 :

¹H RMN (500MHz, CD3CN) δ = 6.94 (1H, d, J = 10.1 Hz, H-1), 6.13 (1H, s, H-24aa), 5.97 (1H, s, H-24ab), 5.90 (1H, d, J = 10.1 Hz, H-2), 5.53 (1H, d, J = 2.1 Hz, H-22), 5.09 (1H, td, J = 7.6 Hz, J = 4.6 Hz, H-16), 3.98 - 4.12 (2H, m, H-26<">, 26<'>), 3.00 (1H, sxt, J = 6.9 Hz, H-25), 2.59 (1H, dqd, J = 11.0 Hz, J = 6.7 Hz, J = 2.1 Hz, H-20), 2.48 - 2.55 (4H, m, H-26c<">, 26c<'>, 26b<">, 26b<'>), 2.30 (1H, dd, J = 11.0 Hz, J = 7.6 Hz, H-17), 2.13 - 2.23 (2H, m, H-4, 15<'>), 2.09 (3H, s, H-22b), 2.04 (3H, s, H-16b), 1.96 - 2.07 (3H, m, H-5, 8, 11<'>), 1.62 - 1.76 (3H, m, H-6<'>, 12<">, 12<'>), 1.52 - 1.61 (1H, m, H-11<">), 1.41 - 1.49 (1H, m, H-7<'>), 1.37 (1H, dd, J = 14.0 Hz, J = 4.0 Hz, H-15<">), 1.24 (1H, d, J = 4.3 Hz, H-19<'>), 1.19 (3H, s, H-18), 1.14 - 1.24 (1H, m, H-7<">), 1.07 (3H, d, J = 7.3 Hz, H-27), 1.03 (3H, d, J = 7.0 Hz, H-28), 0.95 (3H, s, H-29), 0.94 (1H, qd, J = 12.8 Hz, J = 4.0 Hz, H-6<">), 0.85 (3H, d, J = 7.0 Hz, H-21), 0.5 8 (1H, d, J = 4.6 Hz, H-19<">)
¹³C RMN (126MHz, CD3CN) δ = 202.4 (C-3), 198.8 (C-23), 174.0 (C-26d), 173.1 (C-26a), 171.7 (C-22a), 171.3 (C-16a), 155.5 (C-1), 148.9 (C-24), 128.4 (C-2), 125.9 (C-24a), 78.4 (C-22), 76.7 (C-16), 67.9 (C-26), 51.3 (C-17), 48.4 (C-14), 47.6 (C-4), 46.8 (C-13), 46.7 (C-15), 45.2 (C-8), 43.6 (C-5), 34.8 (C-25), 33.4 (C-20), 33.0 (C-12), 32.9 (C-10), 29.8 (C-26b), 29.2 (C-26c), 28.1 (C-11), 27.6 (C-19), 27.2 (C-9), 24.3 (C-7, 6), 22.1 (C-16b), 20.9 (C-22b), 20.0 (C-29), 18.3 (C-18), 17.2 (C-27), 13.3 (C-21), 11.3 (C-28)

### exemple 69 :

¹H NMR (500MHz, DEUTERIUM OXIDE) δ = 6.99 (1H, d, J = 9.5 Hz, H-1), 6.26 (1H, br. s., H-24aa), 6.18 (1H, br. s., H-24ab), 5.94 (1H, d, J = 9.5 Hz, H-2), 5.63 (1H, br. s., H-22), 5.08 (1H, d, J = 4.0 Hz, H-16), 4.03 - 4.17 (3H, m, H-26, 5'), 3.79 - 3.84 (2H, m, H-1'<'>, 4'), 3.73 - 3.78 (1H, m, H-3'), 3.62 - 3.67 (2H, m, H-1'<">, 2'), 3.18 (1H, dd, J = 12.8 Hz, J = 3.4 Hz, H-6'<">), 3.12 (1H, dd, J = 12.8 Hz, J = 9.5 Hz, H-6'<'>), 3.06 (1H, q, J = 6.6 Hz, H-25), 2.72 (3H, s, H-8'), 2.56 - 2.65 (1H, m, H-20), 2.53 (2H, t, J = 6.9 Hz, H-26c), 2.42 (2H, t, J = 6.7 Hz, H-26b), 2.25 - 2.34 (1H, m, H-17), 2.19 - 2.25 (1H, m, H-15<">, 4), 2.17 (3H, s, H-22b), 2.13 (3H, s, H-16b), 1.89 - 2.06 (3H, m, H-8a, 5a, 11<'>), 1.61 - 1.76 (3H, m, H-12, 6<'>), 1.52 - 1.60 (1H, m, H-11<">), 1.41 - 1.49 (1H, m, H-7<'>), 1.33 - 1.41 (1H, m, H-15<'>), 1.23 - 1.30 (1H, m, H-19<'>), 1.19 - 1.23 (1H, m, H-7<">), 1.16 (3H, br. s., H-18), 1.10 (3H, d, J = 7.0 Hz, H-27), 1.01 (3H, d, J = 5.8 Hz, H-28), 0.92 (3H, br. s., H-29), 0.86 (3H, d, J = 5.2 Hz, H-21), 0.56 (1H, br. s., H-19<"»)
¹³C NMR (126MHz, DEUTERIUM OXIDE) δ = 203.9 (C-3), 199.3 (C-23), 180.5 (C-26d), 175.5 (C-26a), 172.8 (C-22a), 172.6 (C-16a), 156.5 (C-1), 147.5 (C-24), 127.3 (C-2), 126.9 (C-24a), 77.8 (C-22), 76.3 (C-16), 70.8 (C-3'), 70.6 (C-2'), 70.5 (C-4'), 68.3 (C-5'), 67.7 (C-26), 62.6 (C-1'), 51.1 (C-6'), 50.1 (C-17), 47.2 (C-14), 46.6 (C-4), 45.7 (C-15, 13), 43.6 (C-8), 42.1 (C-5), 33.2 (C-25), 33.0 (C-8'), 32.4 (C-20), 32.1 (C-12), 31.9 (C-26b), 30.5 (C-26c), 27.1 (C-11), 26.4 (C-19), 23.0 (C-6, 7), 21.3 (C-16b), 20.1 (C-22b), 19.1 (C-29), 17.3 (C-18), 16.2 (C-27), 12.5 (C-21), 10.5 (C-28)

### ◆ exemples 70, 71 & 72: 22,26-bis-succinate, 22 déacétyl-neoboutomellerone (70), 26-succinate, 22 déacétyl-neoboutomellerone (71) et sel de N-méthylglucamine de 26-succinate, 22 déacétyl-neoboutomellerone (72)

| | |
|---|---|
| 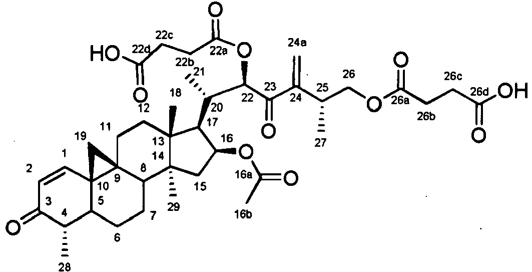 | 70 |
| | Formule Chimique : C₄₀H₅₄O₁₂ |
| | Masse Exacte : 726,36 |
| | Poids moléculaire : 726,85 |
| 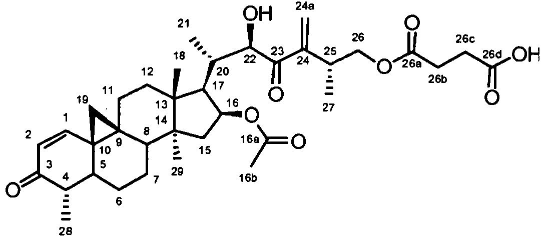 | 71 |
| | Formule Chimique : C₃₆H₅₀O₉ |
| | Masse Exacte : 626,35 |
| | Poids moléculaire : 626,78 |
| 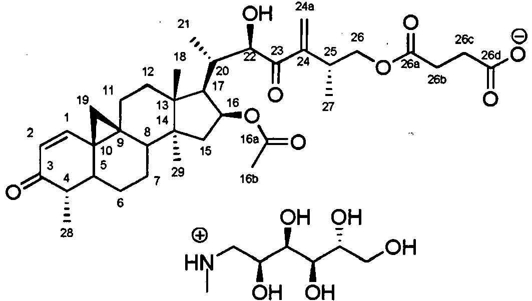 | 72 |
| | Formule Chimique : C₄₃H₆₆NO₁₄ |
| | Masse Exacte : 820.45 |
| | Poids moléculaire : 820.98 |

Protocole : 100 mg (0.19 mmol) de 1 sont solubilisés dans 3.5 mL de dichlorométhane anhydre. 21 mg d'anhydride succinique (0.21 mmol, 1.1 eq.) et 35 mg de DMAP (0.29 mmol, 1.5 eq.) sont ajoutés. Après 20h d'agitation à température ambiante, la réaction est terminée. La phase organique est lavée avec une solution d'HCl 4% puis extraite 3 fois à l'acétate d'éthyle. Les phases organiques rassemblées sont ensuite lavées avec une solution saturée d'hydrogénocarbonate de sodium et une solution saturée de chlorure de sodium. La phase organique est séchée sur Na₂SO₄, filtrée et concentrée à l'évaporateur rotatif. Le résidu est purifié par HPLC RP-18 (gradient : H₂O/CH₃CN : 90/10 à 30/70). 71 et 70 sont obtenus respectivement avec un rendement de 66% (78 mg) et 11% (14 mg) sous forme d'un film translucide et d'un solide blanc. 76 mg (0.12 mmol, 1 eq.) de 71 sont dissous dans 5 mL d'éthanol absolue. 24 mg (0.12 mmol, 1 eq.) de N-méthyl-D-glucamine sont dissous dans 2 mL d'eau et ajoutés à la solution précédemment préparée. Le milieu réactionnel est agité pendant 10 minutes et concentrée à l'évaporateur rotatif. Le résidu est repris dans 5 mL d'eau, filtré sur un filtre 45 m et lyophilisé afin de donner 100 mg de 72 (99%).

### exemple 70 :

¹H NMR (500MHz, ACETONITRILE-d₃) δ = 6.95 (1H, d, J = 10.1 Hz, H-1), 6.13 (1H, s, H-24aa), 5.97 (1H, s, H-24ab), 5.90 (1H, d, J = 10.1 Hz, H-2), 5.57 (1H, d, J = 2.1 Hz, H-22), 5.05 (1H, td, J = 7.6 Hz, J = 4.4 Hz, H-16), 4.07 (1H, dd, J = 10.8 Hz, J = 6.8 Hz, H-26<'>), 4.03 (1H, dd, J = 10.8 Hz, J = 6.3 Hz, H-26<">), 3.00 (1H, sxt, J = 6.9 Hz, H-25), 2.66 (1H, s, H-20), 2.55 - 2.64 (4H, m, H-22c, 22b), 2.48 - 2.55 (4H, m, H-26b, 26c), 2.29 (1H, dd, J = 11.0 Hz, J = 7.5 Hz, H-17), 2.12 - 2.22 (2H, m, H-4, 15<'>), 2.03 (3H, s, H-16b), 1.97 - 2.03 (2H, m, H-11<'>, 5a, 8a), 1.63 - 1.76 (3H, m, H-6<'>, 12), 1.56 (1H, qd, J = 8.6 Hz, J = 6.2 Hz, H-11<">), 1.41 - 1.48 (1H, m, H-7<'>), 1.36 (1H, dd, J = 13.9 Hz, J = 4.1 Hz, H-15<">), 1.24 (1H, d, J = 4.3 Hz, H-19<'>), 1.20 - 1.22 (1H, m, H-7<">), 1.18 (3H, s, H-18), 1.06 (3H, d, J = 7.0 Hz, H-27), 1.02 (3H, d, J = 7.0 Hz, H-28), 0.95 (3H, s, H-29), 0.89 - 0.93 (1H, m, H-6<">), 0.85 (3H, d, J = 7.0 Hz, H-21 ), 0.5 8 (1H, d, J = 4.6 Hz, H-19<">)
¹³C NMR (126MHz, ACETONITRILE-d₃) δ = 202.5 (C-3), 198.7 (C-23), 174.2 (C-26d), 174.1 (C-22d), 173.2 (C-26a), 173.0 (C-22a), 171.3 (C-16a), 155.6 (C-1), 148.9 (C-24), 128.4 (C-2), 126.0 (C-24a), 78.5 (C-22), 76.8 (C-16), 68.0 (C-26), 51.2 (C-17), 48.3 (C-14), 47.6(C-4), 46.8 (C-13), 46.7 (C-15), 45.2 (C-8), 43.6 (C-5), 34.8 (C-25), 33.5 (C-20), 32.9 (C-10, 12), 32.9, 29.9 (C-26b), 29.8 (C-22b), 29.3 (C-22c), 29.3 (C-26c), 28.1 (C-11), 27.6 (C-19), 27.2 (C-9), 24.3 (C-6, 7), 22.1 (C-16b), 20.0 (C-29), 18.3 (C-18), 17.2 (C-27), 13.3 (C-21), 11.3 (C-28)

### exemple 71 :

¹H NMR (500MHz, ACETONITRILE-d₃) δ = 6.94 (1H, d, J = 10.1 Hz, H-1), 6.19 (1H, s, H-24aa), 6.05 (1H, s, H-24ab), 5.89 (1H, d, J = 10.1 Hz, H-2), 5.20 (1H, td, J = 7.6 Hz, J = 4.4 Hz, H-16), 4.72 (1H, d, J = 1.5 Hz, H-22), 4.10 (1H, dd, J = 10.8 Hz, J = 6.5 Hz, H-26<'>), 4.07 (1H, dd, J = 10.8 Hz, J = 6.4 Hz, H-26<">), 3.06 (1H, sxt, J = 6.9 Hz, H-25), 2.48 - 2.56 (4H, m, H-26c, 26b), 2.39 - 2.47 (3H, m, H-20, 17), 2.13 - 2.26 (7H, m, H-15<'>, 4), 2.04 (4H, s, H-16b), 1.96 - 2.03 (3H, m, H-11<'>, 8a, 5a), 1.63 - 1.71 (3H, m, H-6<'>, 12), 1.54 (1H, qd, J = 8.3 Hz, J = 6.6 Hz, H-11<">), 1.42 - 1.49 (1H, m, H-7<'>), 1.38 (1H, dd, J = 14.0 Hz, J = 4.3 Hz, H-15 <">), 1.24 (1H, d, J = 4.6 Hz, H-19<'>), 1.19 - 1.22 (1H, m, H-7<">), 1.18 (3H, s, H-18), 1.09 (3H, d, J = 7.0 Hz, H-27), 1.03 (3H, d, J = 7.0 Hz, H-28), 0.96 (3H, s, H-29), 0.89 - 0.95 (1H, m, H-6<">), 0.64 (3H, d, J = 6.1 Hz, H-21), 0.57 (1H, d, J = 4.6 Hz, H-19<">)
¹³C NMR (126MHz, ACETONITRILE-d₃) δ = 204.8 (C-23), 202.3 (C-3), 173.9 (C-26d), 173.1 (C-26a), 171.3 (C-16a), 155.5 (C-1), 147.7 (C-24), 128.3 (C-2), 127.5 (C-24a), 77.2 (C-16), 75.7 (C-22), 68.0 (C-26), 51.3 (C-17), 48.2 (C-14), 47.6 (C-4), 46.8 (C-15), 46.6 (C-13), 45.2 (C-8), 43.5 (C-5), 36.4 (C-20), 34.6 (C-25), 33.0 (C-12), 32.9 (C-10), 29.7 (C-26b), 29.2 (C-26c), 28.0 (C-11), 27.7 (C-19), 27.2 (C-9), 24.2 (C-6, 7), 22.0 (C-16b), 20.0, 18.4 (C-18), 17.1 (C-27), 12.2 (C-21), 11.2 (C-28)

### exemple 72 :

¹H NMR (500MHz, DMSO-d₆) δ = 6.97 (1H, d, J = 10.1 Hz, H-1), 6.07 (1H, s, H-24aa), 6.00 (1H, s, H-24ab), 5.89 (1H, d, J = 10.1 Hz, H-2), 5.09 - 5.19 (1H, m, H-16), 4.61 (1H, s, H-22), 4.02 (1H, dd, J = 10.7 Hz, J = 6.5 Hz, H-26<'>), 3.98 (1H, dd, J = 10.7 Hz, J = 6.5 Hz, H-26<">), 3.68 - 3.73 (1H, m, H-5'), 3.63 (1H, dd, J = 5.2 Hz, J = 1.5 Hz, H-4'), 3.58 (1H, dd, J = 11.0 Hz, J = 3.4 Hz, H-1'<'>), 3.45 - 3.50 (1H, m, H-2'), 3.34 - 3.41 (1H, m, H-1'<">), 2.95 (1H, sxt, J = 6.8 Hz, H-25), 2.62 - 2.71 (2H, m, H-6'), 2.41 - 2.48 (2H, m, H-26c), 2.34 - 2.40 (3H, m, H-17, 26b), 2.33 (3H, s, H-8'), 2.07 - 2.16 (2H, m, H-4, 15<'>), 2.02 (3H, s, H-16b), 1.94 - 2.00 (2H, m, H-8a, 11<'>), 1.89 (2H, s, H-5a), 1.49 - 1.64 (4H, m, H-12, 6<'>, 11<">), 1.36 - 1.45 (1H, m, H-7<'>), 1.29 (1H, dd, J = 14.2 Hz, J = 4.5 Hz, H-15<">), 1.24 (1H, d, J = 4.3 Hz, H-19<'>), 1.14 - 1.19 (1H, m, H-7<">), 1.11 (3H, s, H-18), 1.03 (2H, d, J = 7.3 Hz, H-27), 0.98 (3H, d, J = 7.0 Hz, H-28), 0.91 - 0.95 (1H, m, H-6<">), 0.89 (3H, s, H-29), 0.64 (2H, d, J = 6.1 Hz, H-21), 0.57 (1H, d, J = 4.3 Hz, H-19<">)
¹³C NMR (126MHz, DMSO-d₆) δ = 203.5 (C-23), 200.7 (C-3), 173.9 (C-26d), 172.3 (C-26a), 170.0 (C-16a), 154.9 (C-1), 147.3 (C-24), 127.3 (C-2), 125.1 (C-24a), 75.3 (C-16), 74.2 (C-22), 71.2 (C-2'), 70.4 (C-3'), 70.3 (C-4'), 70.2 (C-5'), 66.7 (C-26), 63.7 (C-1'), 52.2 (C-6'), 49.6 (C-17), 47.0 (C-14), 46.2 (C-4), 45.5 (C-13), 45.2 (C-15), 43.4 (C-8), 42.1 (C-5), 35.1 (C-8'), 34.3 (C-20), 33.3 (C-25), 31.8 (C-12), 31.6 (C-10), 29.6 (C-26b), 29.3 (C-26c), 26.7 (C-11), 26.3 (C-19), 26.0 (C-9), 22.9 (C-6), 22.8 (C-7), 21.5 (C-16b), 19.2 (C-29), 17.7 (C-18), 16.7 (C-27), 11.8 (C-21), 10.8 (C-28)

### ◆ exemples 73 & 74: 22-déacétyl,22-succinate, 26 acétyl-neoboutomellerone (73) et sel de N-méthylglucamine de 22-déacétyl,22-succinate, 26 acétyl-neoboutomellerone (74)

| | |
|---|---|
| 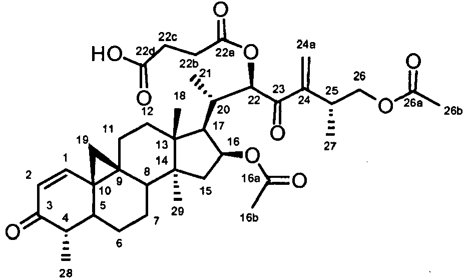 | 73 |
| | Formule Chimique : C₃₈H₅₂O₁₀ |
| | Masse Exacte : 668,36 |
| | Poids moléculaire : 668,81 |
| 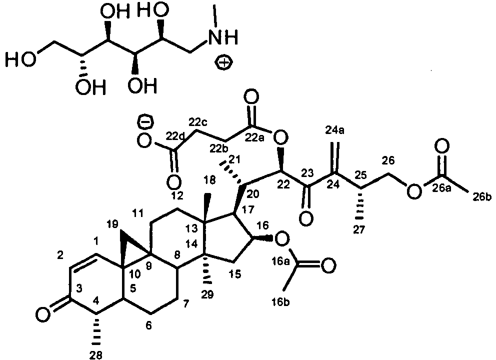 | 74 |
| | Formule Chimique : C₄₅H₆₈NO₁₅ |
| | Masse Exacte : 862.46 |
| | Poids moléculaire : 863.02 |

Protocole : 59 mg (0.104 mmol) de 17 sont solubilisés dans 2 mL de dichlorométhane anhydre. 42 mg d'anhydride succinique (0.418 mmol, 4 eq.) et 50 mg de DMAP (0.418 mmol, 4 eq.) sont ajoutés. Après 90h d'agitation à température ambiante, la réaction est terminée. La phase organique est lavée avec une solution d'HCl 4% puis extraite 3 fois à l'acétate d'éthyle. Les phases organiques rassemblées sont ensuite lavées avec une solution saturée d'hydrogénocarbonate de sodium et une solution saturée de chlorure de sodium. La phase organique est séchée sur Na₂SO₄, filtrée et concentrée à l'évaporateur rotatif. Le résidu est purifié par chromatographie sur gel de silice (éluant : DCM/MeOH : 95/5) et par HPLC RP-18 (gradient : H₂O/CH₃CN : 90/10 à 0/100 afin d'obtenir 54 mg d'acide succinique 73 (78%). 35 mg (0.12 mmol, 1 eq.) de 73 sont dissout dans 5 mL d'éthanol absolue. 10 mg (0.05 mmol, 1 eq.) de N-méthyl-D-glucamine sont dissous dans 2 mL d'eau et ajoutés à la solution précédemment préparée. Le milieu réactionnel est agité pendant 10 minutes et concentré à l'évaporateur rotatif. Le résidu est repris dans 5 mL d'eau, filtré sur un filtre 45 m et lyophilisé afin de donner 42 mg de 74 (95%).

### exemple 73:

¹H NMR (500MHz, ACETONITRILE-d₃) δ = 6.94 (1H, d, J = 10.1 Hz, H-1), 6.11 (1H, s, H-24ab), 5.95 (1H, s, 24aa), 5.90 (1H, d, J = 10.1 Hz, H-2), 5.56 (1H, d, J = 2.1 Hz, H-22), 5.06 (1H, td, J = 7.6 Hz, J = 4.3 Hz, H-16), 4.03 (1H, dd, J = 10.8 Hz, J = 7.0 Hz, H-26<'>), 4.01 (1H, dd, J = 10.8 Hz, J = 6.4 Hz, H-26<">), 3.00 (1H, sxt, J = 6.9 Hz, H-25), 2.64 - 2.71 (1H, m, 20), 2.54 - 2.64 (4H, m, H-22b, H-22c), 2.28 (1H, dd, J = 11.0 Hz, J = 7.6 Hz, H-17), 2.14 - 2.21 (2H, m, H-4, 15<'>), 2.03 (3H, s, H-16b), 1.97 - 2.02 (3H, m, H-11<'>, 5a, 8a), 1.97 (3H, s, H-26b), 1.63 - 1.76 (3H, m, H-6<'>, 12), 1.56 (1H, qd, J = 8.7 Hz, J = 6.2 Hz, H-11<">), 1.44 (1H, s, H-7<'>), 1.36 (1H, dd, J = 13.9 Hz, J = 4.1 Hz, H-15<">), 1.24 (1H, d, J = 4.3 Hz, H-19<'>), 1.19 - 1.22 (1H, m, H-7<">), 1.18 (3H, s, H-18), 1.07 (3H, d, J = 7.0 Hz, H-27), 1.02 (3H, d, J = 6.7 Hz, H-28), 0.95 (3H, s, H-29), 0.88 - 0.94 (1H, m, H-6<">), 0.85 (3H, d, J = 7.0 Hz, M25), 0.57 (1H, d, J = 4.3 Hz, H-19<">)
¹³C NMR (126MHz, ACETONITRILE-d₃) δ = 202.3 (C-3), 198.9 (C-23), 174.2 (C-22d), 173.0 (C-22a), 171.6 (C-26a), 171.3 (C-16a), 155.6 (C-1), 149.0 (C-24), 128.4 (C-2), 125.7 (C-24a), 78.4 (C-22), 76.7 (C-16), 67.8 (C-26), 51.2 (C-17), 48.3 (C-14), 47.6 (C-4), 46.8 (C-13), 46.7 (C-15), 45.2 (C-8), 43.6 (C-5), 34.9 (C-25), 33.4 (C-20), 33.0 (C-10), 32.9 (C-12), 29.9 (C-22b), 29.5 (C-22c), 28.1 (C-11), 27.6 (C-19), 27.2 (C-9), 24.3 (C-6, 7), 22.1 (C-16b), 21.1 (C-26b), 20.0 (C-29), 18.3 (C-18), 17.3 (C-27), 13.3 (C-21), 11.3 (C-28)

### exemple 74:

¹H NMR (500MHz, DMSO-d₆) δ = 6.97 (1H, d, J = 10.1 Hz, H-1), 6.05 (2H, s, H-24ab, 24aa), 5.90 (1H, d, J = 10.1 Hz, H-2), 5.48 (1H, d, J = 1.5 Hz, H-22), 4.97 - 5.07 (1H, m, H-16), 3.94 - 4.04 (2H, m, H-26), 3.66 - 3.71 (1H, m, H-5'), 3.64 (1H, d, J = 4.9 Hz, H-4'), 3.59 (1H, dd, J = 10.8 Hz, J = 3.5 Hz, H-1'<'>), 3.45 - 3.50 (1H, m, H-2'), 3.33 - 3.43 (2H, m, H-3', 1'<">), 2.94 (1H, sxt, J = 7.0 Hz, H-25), 2.56 - 2.68 (3H, m, H-22c, 6'), 2.42 - 2.48 (3H, m, H-20, 22b), 2.31 (3H, s, H-8'), 2.24 (1H, dd, J = 11.0 Hz, J = 7.6 Hz, H-17), 2.07 - 2.18 (2H, m, H-15<'>, 4), 2.04 (3H, s, H-16b), 1.98 (3H, s, H-26b), 1.94 - 1.97 (2H, m, H-11<'>, 8a), 1.90 (2H, td, J = 12.4 Hz, J = 4.3 Hz, H-5a), 1.50 - 1.71 (4H, m, H-12, 6<'>, 11<">), 1.35 - 1.45 (1H, m, H-7<'>), 1.26 - 1.33 (1H, dd, J = 13.8 Hz, 4.2 Hz, H-15<">), 1.23 (1H, d, J = 4.3 Hz, H-19<'>), 1.14 - 1.17 (1H, m, H-7<">), 1.13 (3H, s, H-18), 1.03 (3H, d, J = 7.0 Hz, H-27), 0.98 (3H, d, J = 6.7 Hz, H-28), 0.90 (3H, s, H-29), 0.83 - 0.89 (1H, m, M30), 0.81 (3H, d, J = 6.7 Hz, H-21), 0.58 (1H, d, J = 4.3 Hz, H-19<">)
¹³C NMR (126MHz, DMSO-d₆) δ = 200.6 (C-3), 197.2 (C-23), 173.4 (C-22d), 171.9 (C-22a), 170.1 (C-26a), 169.7 (C-16a), 154.7 (C-1), 147.4 (C-24), 127.3 (C-2), 124.9 (C-24a), 76.5 (C-22), 75.2 (C-16), 71.2 (C-2'), 70.5 (C-4', 3'), 70.4 (C-5'), 66.4 (C-26), 63.7 (C-1'), 52.4 (C-6'), 49.6 (C-17), 46.9 (C-14), 46.1 (C-4), 45.4 (C-13, 15), 43.3 (C-8), 42.0 (C-5), 35.4 (C-8'), 33.5 (C-25), 31.9 (C-20), 31.6 (C-12), 31.6 (C-10), 29.2 (C-22b, 22c), 26.6 (C-9), 26.3 (C-11), 25.9 (C-19), 22.9 (C-6), 22.8 (C-7), 21.4 (C-16b), 20.6 (C-26b), 19.0 (C-29), 17.4 (C-18), 16.6 (C-27), 12.3 (C-21), 10.8 (C-28)

### ◆ exemple 75: 22,26-bis(triméthylsilyléthoxymethyl)-(26-déacetyl-neoboutomellerone)

| | |
|---|---|
| 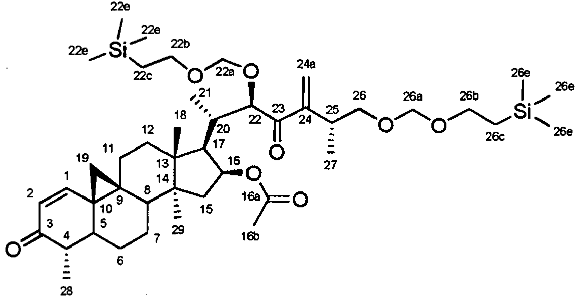 | Formule Chimique : C₄₄H₇₄O₈Si₂ |
| | Masse Exacte : 786.49 |
| | Poids moléculaire : 787.22 |

Protocole : Le composé 1 (50 mg, 0.095 mmol) est dissous dans 0.5 ml de DCM, puis on y rajoute 0.37 ml de base d'Hünig (270 mg, 22 éq., 2.09 mmol) et le milieu réactionnel est refroidi par un bain de glace à 0°C. On ajoute ensuite 0.25 ml de chlorure de triméthylsilyléthoxyméthane (237 mg, 15 éq., 1.42 mmol), et on laisse la réaction revenir à température ambiante. Après 8 h de réaction on observe un mélange de composés monoprotégés (Rf: 0.80 - cyclohexane / acétate d'éthyle 50 / 50) et du 75 bisprotégé (Rf: 0.88 - cyclohexane / acétate d'éthyle 50 / 50), après une nuit seul le composé 75 est présent dans le milieu réactionnel. La réaction est hydrolysée avec une solution de chlorure d'ammonium, et extraite à l'acétate d'éthyle. La phase organique est lavée à l'eau puis à la saumure, séchée sur sulfate de sodium et les solvants sont évaporés. On recueille 147 mg de brut réactionnel qui est purifié sur colonne de gel de silice (éluant : gradient cyclohexane/AcOEt: de 100 / 0 à 90 / 10). On obtient 71mg (94%) de produit 75.
¹H NMR (500MHz, ACETONITRILE-d₃) δ = 6.93 (1H, d, J = 10.0 Hz, H-1), 5.95 (1H, s, H-24ab), 5.89 (1H, d, J = 10.0 Hz, H-2), 5.82 (1H, d, J = 0.5 Hz, H-24aa), 5.23 (1H, td, J = 7.2 Hz, J = 4.5 Hz, H-16), 4.67 (1H, d, J = 7.2 Hz, H-22a<'>), 4.59 (1H, d, J = 6.5 Hz, H-26a<'>), 4.58 (1H, d, J = 6.5 Hz, H-26a<">), 4.42 (1H, d, J = 7.2 Hz, H-22a<">), 3.64 - 3.73 (1H, m, H-22b<'>), 3.51 - 3.61 (4H, m, H-26<'>, 26b<">, 26b<'>, 22b<">), 3.39 - 3.45 (1H, m, H-26<">), 2.93 (1H, sxt, J = 6.6 Hz, H-25), 2.38 - 2.47 (2H, m, H-20), 2.17 - 2.22 (2H, m, H-15<'>), 2.13 - 2.16 (1H, m, H-4), 2.02 - 2.05 (1H, m, H-8a, 11<'>), 2.01 (4H, s, H-16b), 1.96 - 1.99 (1H, m, H-5a), 1.62 - 1.73 (3H, m, H-12<">, 12<'>, 6<'>), 1.50 - 1.59 (1H, m, H-11<">), 1.44 - 1.48 (1H, m, H-7<'>), 1.33 - 1.40 (1H, m, H-15<">), 1.21 - 1.25 (1H, m, H-19<'>), 1.18 - 1.21 (1H, m, H-7<">), 1.17 (3H, s, H-18), 1.04 (3H, d, J = 7.0 Hz, H-27), 1.02 (3H, d, J = 6.7 Hz, M27), 0.96 (3H, s, H-29), 0.87 - 0.93 (5H, m, H-22c<">, 22c<'>, 26c<">, 26c<'>, 6<">), 0.77 - 0.81 (3H, m, H-21), 0.57 (1H, d, J = 4.5 Hz, H-19<">), 0.01 (19H, s, H-22e, 22e, 22e, 26e, 26e, 26e).
¹³C NMR (126MHz, ACETONITRILE-d₃) δ = 202.9 (C-23), 202.4 (C-3), 171.3 (C-16a), 155.6 (C-1), 151.5 (C-24), 128.4 (C-2), 123.8 (C-24a), 95.6 (C-26a), 95.2 (C-22a), 80.7 (C-22), 76.5 (C-16), 72.0 (C-26), 66.8 (C-26b), 65.7 (C-22b), 51.2 (C-17), 48.4 (C-14), 47.6 (C-4), 46.8 (C-15), 46.8 (C-13), 45.3 (C-8), 43.6 (C-5), 35.8 (C-25), 34.2 (C-20), 33.0 (C-12), 32.9 (C-10), 28.1 (C-11), 27.8 (C-19), 27.2 (C-9), 24.3 (C-6), 24.3 (C-7), 22.2 (C-16b), 20.5 (C-29), 18.8 (C-26c), 18.7 (C-22c), 18.3 (C-18), 17.8 (C-27), 12.9 (C-21), 11.3 (C-28), -1.2 (C-22e, 22e, 22e), -1.3 (C-26e, 26e, 26e).

### ◆ exemple 76 : 26-triméthylsilyléthoxyméthyl-neoboutomellerone

| | |
|---|---|
| 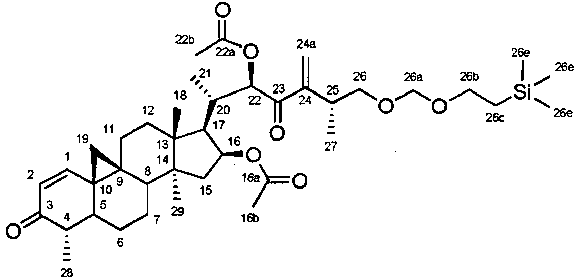 | Formule Chimique : C₃₈H₆₀O₇Si |
| | Masse Exacte : 656.41 |
| | Poids moléculaire : 656.96 |

### Protocole : Le même protocole que précédemment effectué à partir du composé 2 permet l'obtention du composé 76.

¹H NMR (500MHz, ACETONITRILE-d₃) δ = 6.94 (1H, d, J = 10.1 Hz, H-1), 6.04 (1H, s, H-24aa), 5.88 - 5.92 (2H, m, H-2, 24ab), 5.53 (1H, d, J = 2.1 Hz, H-22), 5.09 (1H, td, J = 7.7 Hz, J = 4.4 Hz, H-16), 4.57 - 4.61 (2H, m, H-26a<">, 26a<'>), 3.53 - 3.61 (4H, m, H-26b<">, 26b<'>, 26<'>), 3.42 (1H, dd, J = 9.8 Hz, J = 6.7 Hz, H-26<">), 2.92 (1H, sxt, J = 6.9 Hz, H-25), 2.60 (1H, dtd, J = 13.8 Hz, J = 6.9 Hz, J = 2.2 Hz, H-20), 2.29 (1H, dd, J = 11.0 Hz, J = 7.3 Hz, H-17), 2.18 (2H, dd, J = 13.0 Hz, J = 6.6 Hz, H-4, 15<'>), 2.09 (3H, s, H-22b), 2.02 - 2.03 (3H, m, H-16b), 1.96 - 2.02 (3H, m, H-11<'>, 8a, 5a), 1.64 - 1.73 (3H, m, H-6<'>, 12<">, 12<'>), 1.53 - 1.60 (1H, m, H-11<">), 1.40 - 1.50 (1H, m, H-7<'>), 1.36 (1H, dd, J = 14.3 Hz, J = 4.0 Hz, H-15<">), 1.23 - 1.26 (1H, m, H-19<'>), 1.19 (3H, s, H-18), 1.04 - 1.07 (3H, m, H-27), 1.03 (3H, d, J = 6.7 Hz, H-28), 0.96 (3H, s, H-29), 0.93 - 0.94 (1H, m, H-6<">), 0.90 (2H, dd, J = 8.9 Hz, J = 7.6 Hz, H-26c<">, 26c<'>), 0.86 (3H, d, J = 7.0 Hz, H-21), 0.58 (1H, d, J₁₉<">_{,16} = 4.6 Hz, H-19<">), 0.01 (9H, s, H-26e, 26e, 26e).
¹³C NMR (126MHz, ACETONITRILE-d₃) δ = 202.4 (C-3), 199.2 (C-23), 171.6 (C-22a), 171.2 (C-16a), 155.5 (C-1), 150.4 (C-24), 128.5 (C-2), 124.6 (C-24a), 95.7 (C-26a), 78.4 (C-22), 76.7 (C-16), 71.9 (C-26), 65.8 (C-26b), 51.4 (C-17), 48.4 (C-14), 47.7 (C-4), 46.9 (C-13), 46.7 (C-15), 45.2 (C-8), 43.6 (C-5), 35.9 (C-25), 33.1 (C-20), 33.0 (C-12), 33.0 (C-10), 28.1 (C-11), 27.7 (C-19), 27.3 (C-9), 24.3 (C-6, 7), 22.1 (C-16b), 21.0 (C-22b), 20.0 (C-29), 18.7 (C-26c), 18.3 (C-18), 17.8 (C-27), 13.3 (C-21), 11.3 (C-28), -1.2 (C-26e, 26e, 26e)

### ◆ exemple 77 (comparatif) :

| | |
|---|---|
| 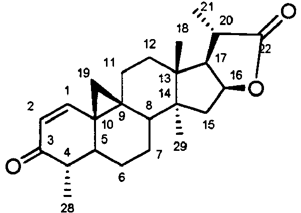 | Formule Chimique : C₂₄H₃₂O₃ |
| | Masse Exacte : 368.24 |
| | Poids moléculaire : 368.51 |

Protocole : Le composé 2 (60 mg, 0.106 mmol) est dissous dans 2 ml de DCM, puis on y rajoute 1 ml d'une solution 1M d'hydrure de Di-*iso*butyl-Aluminium. Après conversion complète, la réaction est neutralisée au méthanol et les solvants sont évaporés. Le résidu est repris dans l'acétate d'éthyle, et lavé à l'eau puis à la saumure, séché sur sulfate de sodium et concentré. Après purification grossière sur silice, on recueille 22 mg d'un mélange de 4 diastéréoisomères correspondant aux produits déacétylés en position 16 et 22 et réduit en position 3 et 23 (m/z = 488, Rf = 0.36, 0.25, 0.19, 0.11 - cyclohexane / acétate d'éthyle 20 / 80). Ce mélange est entièrement repris dans 6 ml de DCM à température ambiante sur lequel on rajoute 65 mg d'oxyde de manganèse. La réaction est laissée à température ambiante et après disparition des produits initiaux, l'oxyde de manganèse est adsorbé sur carbonate de sodium et filtré sur celite^{®}. Après chromatographie sur gel de silice on recueille 9 mg de composé 77 (23 % pour 2 étapes).
¹H NMR (500MHz, ACETONITRILE-d₃) δ = 6.96 (1H, d, J = 10.0 Hz, H-1), 5.92 (1H, d, J = 10.0 Hz, H-2), 5.05 (1H, td, J = 8.0 Hz, J = 5.0 Hz, H-16), 2.63 (1H, qd, J = 7.5 Hz, J = 1.0 Hz, H-20), 2.45 - 2.48 (1H, m, H-17), 2.18 - 2.22 (1H, m, H-4), 2.01 - 2.06 (1H, m, H-11<'>, 15<'>), 1.96 - 2.01 (1H, m, H-8a), 1.90 - 1.92 (1H, m, H-5a), 1.58 - 1.76 (5H, m, H-11<">, 12<">, 12<'>, 6<'>), 1.45 - 1.57 (2H, m, H-7<'>, 15<">), 1.30 - 1.32 (1H, m, H-19<'>), 1.25 - 1.28 (4H, m, H-21), 1.18 - 1.24 (1H, m, H-7<">, 1.00 - 1.04 (6H, m, H-29, 28), 0.88 - 0.91 (3H, m, H-18), 0.49 - 0.53 (1H, m, H-19<">).
¹³C NMR (126MHz, ACETONITRILE-d₃) δ = 202.2 (C-3), 182.2 (C-22), 155.1 (C-1), 128.6 (C-2), 83.9 (C-16), 66.3, 56.2 (C-17), 50.7 (C-14), 47.4 (C-4), 45.8 (C-13), 43.2 (C-15), 42.9 (C-5, 8), 37.5 (C-20), 33.2 (C-10), 31.0 (C-12), 27.8 (C-11), 27.4 (C-9), 26.0 (C-19), 23.8 (C-6), 23.6 (C-7), 19.0 (C-29), 18.7 (C-18), 18.4 (C-21), 11.3 (C-28).

### ◆ exemple 78 : 26-allyl-neoboutomellerone éther

| | |
|---|---|
| 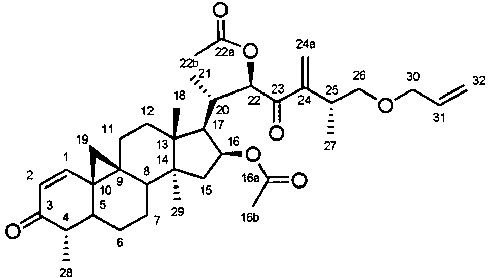 | Formule Chimique : C₃₇H₅₂O₇₂ |
| | Masse Exacte : 608.37 |
| | Poids moléculaire : 608.80 |

Protocole : Le composé 2 (284 mg, 0.5 mmol) est dissous, à 0°C, dans 2 ml de DCM, puis on y rajoute 170 µl de 2,6 di-*tertio*butylpyridine (143 mg, 1.5 éq., 0.75 mmol). On protège la réaction de la lumière par du papier d'aluminium avant d'ajouter 141 mg de triflate d'argent (1.21 éq., 0.55 mmol), et ensuite le bromure d'allyle (52µl, 73 mg, 1.2 éq., 0.6 mmol). On laisse la réaction revenir à température ambiante, et on laisse sous agitation 4 heures. La réaction est hydrolysée par une solution d'acide chlorhydrique 1N, et dilué par l'acétate d'éthyle. La phase organique est séparée, lavée avec de l'eau, de la saumure, puis séchée sur sulfate de sodium et concentrée. Le mélange est purifié sur gel de silice et conduit à 107 mg de composé 78 (35 % - Rf: 0.87 - cyclohexane / acétate d'éthyle 50 / 50), ainsi que 116 mg de composé 2 n'ayant pas réagit.
¹H NMR (500MHz, ACETONITRILE-d₃) δ = 6.93 (1H, d, J = 9.8 Hz, H-1), 6.02 (1H, s, H-24ab), 5.85 - 5.93 (3H, m, H-2, 31, 24aa), 5.53 (1H, d, J = 2.1 Hz, H-22), 5.24 (1H, ddd, J = 17.4 Hz, J = 1.8 Hz, J = 1.5 Hz, H-32aa), 5.13 (1H, ddd, J = 15.0 Hz, J = 2.0 Hz, J = 1.5 Hz, H-32ab), 5.10 (1H, td, J = 7.7 Hz, J = 4.3 Hz, H-16), 3.93 (2H, dq, J = 5.5 Hz, J = 1.3 Hz, H-30<">, 30<'>), 3.45 - 3.47 (1H, m, M26), 3.48 (1H, dd, J = 9.5 Hz, J = 6.5 Hz, H-26<'>), 3.32 (1H, dd, J = 9.3 Hz, J = 6.6 Hz, H-26<">), 2.92 (1H, sxt, J = 6.7 Hz, H-25), 2.59 (1H, qdd, J = 11.0 Hz, J = 6.9 Hz, J = 2.3 Hz, H-20), 2.30 (1H, dd, J = 11.0 Hz, J = 7.6 Hz, H-17), 2.17 (2H, dd, J = 13.0 Hz, J = 6.9 Hz, H-15<'>), 2.09 (3H, s, H-22b), 2.03 - 2.06 (2H, m, H-11<'>, 8a), 2.00 (1H, d, J = 4.6 Hz, H-5a), 2.02 (5H, s, H-16b), 1.63 - 1.76 (4H, m, H-12<">, 12<'>, 6<'>), 1.57 (1H, ddd, J = 15.0 Hz, J = 8.9 Hz, J = 6.1 Hz, H-11<">), 1.44 - 1.50 (1H, m, H-7<'>), 1.36 (1H, dd, J = 14.0 Hz, J = 3.5 Hz, H-15<">), 1.24 (1H, d, J = 4.3 Hz, H-19<'>), 1.20 - 1.23 (1H, m, H-7<">), 1.18 (3H, s, H-18), 1.06 (3H, d, J = 7.0 Hz, H-27), 1.03 (3H, d, J = 7.0 Hz, H-28), 0.95 (4H, s, H-29), 0.91 - 0.93 (1H, m, H-6<">), 0.85 (4H, d, J = 7.0 Hz, H-21), 0.57 (1H, d, J = 4.6 Hz, H-19<">).
¹³C NMR (126MHz, ACETONITRILE-d₃) δ = 202.3 (C-3), 199.3 (C-23), 171.6 (C-22a), 171.2 (C-16a), 155.4 (C-1), 150.3 (C-24), 136.4 (C-31), 128.5 (C-2), 124.8 (C-24a), 116.8 (C-32), 78.4 (C-22), 76.6 (C-16), 74.3 (C-26), 72.4 (C-30), 51.4 (C-17), 48.4 (C-14), 47.7 (C-4), 46.9 (C-13), 46.7 (C-15), 45.2 (C-8), 43.6 (C-5), 36.3 (C-25), 33.1 (C-20), 33.0 (C-12), 33.0 (C-10), 28.1 (C-11), 27.7 (C-19), 27.2 (C-9), 24.3 (C-7, 6), 22.1 (C-16b), 21.0 (C-22b), 20.1 (C-29), 18.3 (C-18), 17.7 (C-27), 13.3 (C-21), 11.3 (C-28).

### ◆ exemple 79 : 26-allyl, 22-déacétyl-neoboutomellerone

| | |
|---|---|
| 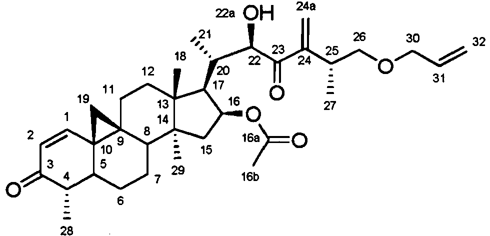 | Formule Chimique : C₃₅H₅₀O₆ |
| | Masse Exacte : 566.36 |
| | Poids moléculaire : 566.77 |

Protocole : Le composé 1 (263 mg, 0.5 mmol) est dissous, dans 2.5 ml de DCM, puis on y rajoute 135 mg de sulfate de magnésium (1.1 éq., 0.55 mmol). On protège la réaction de la lumière par du papier d'aluminium avant d'ajouter 340 mg d'oxyde d'argent (3 éq., 1.5 mmol), et après 1 heure d'agitation à température ambiante le bromure d'allyle (650µl, 907 mg, 15 éq., 7.5 mmol). On laisse la réaction sous agitation jusqu'à conversion complète. La réaction est hydrolysée par une solution d'acide chlorhydrique 1N, et dilué par l'acétate d'éthyle. La phase organique est séparée, lavée avec de l'eau, de la saumure, puis séchée sur sulfate de sodium et concentrée. Le mélange est purifié sur gel de silice et conduit à 220 mg de composé 79 (78 % - Rf: 0.78 - cyclohexane / acétate d'éthyle 50 / 50).
¹H NMR (500MHz, ACETONITRILE-d₃) δ = 6.93 (1H, d, J = 10.0 Hz, H-1), 6.10 (1H, s, H-24aa), 5.99 (1H, d, J = 0.9 Hz, H-24ab), 5.90 (1H, s, H-31, 2), 5.24 (1H, ddd, J = 7.3 Hz, J = 1.8 Hz, J = 1.5 Hz, H-32aa), 5.20 (1H, td, J = 7.4 Hz, J = 4.3 Hz, H-16), 5.12 (1H, qd, J = 15.5 Hz, J = 1.5 Hz, H-32ab), 4.71 (1H, dd, J = 5.5 Hz, J = 1.5 Hz, H-22), 3.93 (1H, dd, J = 5.4 Hz, J = 1.4 Hz, H-30), 3.51 - 3.52 (1H, m, H-38), 3.51 (1H, dd, J = 9.4 Hz, J = 6.4 Hz, H-26<'>), 3.33 (1H, dd, J = 9.3 Hz, J = 6.6 Hz, H-26<">), 2.97 (1H, sxt, J = 6.7 Hz, H-25), 2.38 - 2.50 (1H, m, H-17, 20), 2.23 (1H, dd, J = 13.9 Hz, J = 7.8 Hz, H-15<'>), 2.15 - 2.20 (1H, m, H-4), 2.03 - 2.09 (1H, m, H-8a, 11<'>), 2.03 (2H, s, H-16b), 1.96 - 2.02 (2H, m, H-5a), 1.61 - 1.74 (2H, m, H-12<">, 12<'>, 6<'>), 1.55 (1H, ddd, J = 14.9 Hz, J = 8.6 Hz, J = 6.2 Hz, H-11 <">), 1.42 - 1.50 (1H, m, H-7<'>), 1.38 (1H, dd, J = 14.2 Hz, J = 4.2 Hz, H-15 <">), 1.24 (1H, d, J = 4.5 Hz, H-19<'>), 1.19 - 1.23 (1H, m, H-7<">), 1.18 (2H, s, H-18), 1.08 (1H, d, J = 7.0 Hz, H-27), 1.03 (2H, d, J = 6.7 Hz, H-28), 0.97 (2H, s, H-29), 0.91 - 0.96 (1H, m, H-6<">), 0.65 (1H, d, J = 6.1 Hz, H-21), 0.57 (1H, d, J = 4.5 Hz, H-19<">).
¹³C NMR (126MHz, ACETONITRILE-d₃) δ = 205.4 (C-23), 202.4 (C-3), 171.3 (C-16a), 155.6 (C-1), 149.2 (C-24), 136.4 (C-31), 128.4 (C-2), 126.5 (C-24a), 116.7 (C-32), 77.3 (C-16), 75.9 (C-22), 74.7 (C-26), 72.4 (C-30), 51.5 (C-17), 48.3 (C-14), 47.8 (C-4), 47.0 (C-15), 46.7 (C-13), 45.4 (C-8), 43.7 (C-5), 36.2 (C-25), 35.8 (C-20), 33.2 (C-12), 33.0 (C-10), 28.2 (C-11), 27.8 (C-19), 27.3 (C-9), 24.4 (C-6), 24.4 (C-7), 22.1 (C-16b), 20.1 (C-29), 18.6 (C-18), 17.7 (C-27), 12.3 (C-21), 11.3 (C-28).

### ◆ exemple 80 : 1,2-dihydroxy-26-sn1 glycéryléther-neoboutomellerone

| | |
|---|---|
| 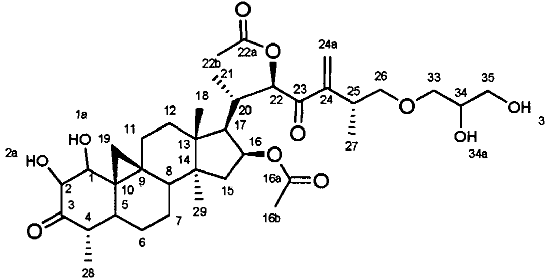 | Formule Chimique : C₃₇H₅₈O₁₁ |
| | Masse Exacte : 676.38 |
| | Poids moléculaire : 676.83 |

Protocole : Le composé 78 (38 mg, 0.063 mmol) est dissous dans 1.5 ml d'un mélange *tert*-butanol / eau (9/1) dans lequel on ajoute 17.5 mg (1.1 éq., 0.069 mmol) d'oxyde de N-méthylmorpholine, puis 1 µl (0.05 éq., 0.003 mmol) d'une solution de tétroxyde d'osmium dans le *tert*-butanol. La réaction est quenchée par une solution d'hydrogénosulfite de sodium, et extraite par l'acétate d'éthyle. La phase organique est lavée par une solution d'hydrogénocarbonate de sodium, de l'eau puis de la saumure, séchée sur sulfate de sodium et concentrée. Le brut réactionnel (42 mg) est purifié sur gel de silice (éluant : gradient AcOEt/cyclohexane: de 70 / 30 à 90 / 10) pour fournir le composé 80 (38%) ainsi que d'autres composés polyhydroxylés.
¹H NMR (500MHz, ACETONITRILE-d₃) δ = 6.05 (1H, d, J = 1.2 Hz, H-24ab), 5.91 (1H, s, H-24aa), 5.53 (1H, d, J = 2.1 Hz, H-22), 5.09 (1H, td, J = 7.6 Hz, J = 4.3 Hz, H-16), 4.20 - 4.27 (1H, m, H-1), 3.74 - 3.79 (1H, m, H-2), 3.70 (1H, d, J = 4.9 Hz, H-1a), 3.66 (1H, dt, J = 6.0 Hz, J = 4.7 Hz, H-34), 3.46 - 3.52 (1H, m, H-33<'>, 26<'>), 3.39 - 3.44 (2H, m, H-35<">, 35<'>, 26<">), 3.32 - 3.39 (1H, m, H-33<">), 3.02 (1H, d, J = 2.1 Hz, H-2a), 2.92 (1H, d, J = 5.5 Hz, H-34a), 2.69 (1H, t, J = 6.0 Hz, H-35a), 2.59 (1H, dtd, J = 13.9 Hz, J = 6.9 Hz, J = 2.2 Hz, H-20), 2.32 - 2.38 (1H, m, H-25), 2.28 - 2.34 (1H, m, H-4, 8a), 2.29 (1H, dd, J = 10.8 Hz, J = 7.5 Hz, H-17), 2.23 - 2.26 (1H, m, H-11<'>), 2.23 - 2.28 (1H, m, M30), 2.20 (1H, dd, J = 13.9 Hz, J = 7.8 Hz, H-15<'>), 2.09 (2H, s, H-22b), 2.03 - 2.09 (1H, m, H-5a), 2.03 (1H, d, J = 0.6 Hz, H-16b), 1.66 - 1.81 (2H, m, H-6<'>, 12<">, 12<'>), 1.40 - 1.46 (2H, m, H-11<">), 1.36 (2H, dd, J = 13.8 Hz, J = 4.3 Hz, H-15<">), 1.30 - 1.34 (1H, m, H-7<'>), 1.27 (1H, s, M34), 1.22 (2H, s, H-18), 1.08 - 1.19 (3H, m, H-7<">), 1.05 (5H, d, J = 7.0 Hz, H-27), 0.99 - 1.02 (1H, m, H-29), 0.98 (5H, d, J = 6.4 Hz, H-28), 0.88 - 0.94 (1H, m, H-6<">), 0.86 (2H, d, J = 6.7 Hz, H-21), 0.81 (2H, d, J = 4.6 Hz, H-19<'>), 0.57 (1H, d, J = 4.6 Hz, H-19<">).
¹³C NMR (126MHz, ACETONITRILE-d₃) δ = 212.6 (C-3), 199.3 (C-23), 171.7 (C-22a), 171.3 (C-16a), 150.3 (C-24), 124.9 (C-24a), 78.5 (C-22), 78.0 (C-1), 77.9 (C-2), 76.9 (C-16), 75.6 (C-35), 75.5 (C-35), 73.4 (C-33), 73.4 (C-33), 71.7 (C-34), 71.7 (C-34), 64.5 (C-26), 51.5 (C-17), 48.1 (C-14), 48.0 (C-4), 48.0 (C-4), 47.6 (C-15), 46.9 (C-13), 40.3 (C-5), 35.8 (C-25), 35.8 (C-8), 33.2 (C-20), 33.2 (C-12), 32.1 (C-10), 27.4 (C-19), 26.8 (C-11), 26.1 (C-7), 25.7 (C-6), 25.0 (C-9), 22.1 (C-16b), 21.0 (C-22b), 20.6 (C-29), 19.2 (C-18), 17.6 (C-27), 13.3 (C-21), 10.7 (C-28).

### ◆ exemple 81 : 26-acétoxyméthyl-(22-déacétyl-neoboutomellerone) éther

| | |
|---|---|
| 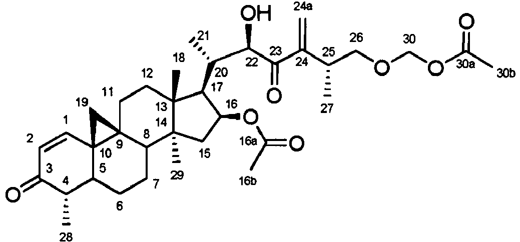 | Formule Chimique : C₃₈H₅₂O₈ |
| | Masse Exacte : 598.35 |
| | Poids moléculaire : 598.77 |

Protocole : 50 mg (0.095 mmol) de composé 1 sont dissous dans 0.5 ml de DCM avec 0.33 ml (20 éq., 1.9 mmol) de base d'Hünig. Le milieu réactionnel est refroidi à 0°C, et à cette température est introduit, 0.095 ml (10 éq., 0.95 mmol) d'acétate de bromométhyle. Après 1 h, le bain de glace est retiré et l'agitation est maintenue une nuit. Le milieu réactionnel est hydrolysé avec de l'eau, et extrait par l'acétate d'éthyle. Les phases organiques sont réunies, lavées à l'eau, à la saumure et séchées sur sulfate de sodium. Le brut réactionnel (66 mg) est purifié sur gel de silice (éluant : gradient cyclohexane/AcOEt: de 80 / 20 à 30 / 70). On obtient 33mg (59%) de produit 81 (Rf: 0.75 - cyclohexane / acétate d'éthyle 50 / 50), ainsi que 21 mg (42 %) de composé 1.
¹H NMR (500MHz, ACETONITRILE-d₃) δ = 6.93 (1H, d, J = 9.8 Hz, H-1), 6.14 (1H, s, H-24ab), 6.00 (1H, d, J = 0.9 Hz, H-24aa), 5.89 (1H, d, J = 10.1 Hz, H-2), 5.20 - 5.23 (1H, m, H-16), 5.19 - 5.21 (1H, m, H-30<'>), 5.18 (1H, s, H-30<">), 4.72 (2H, dd, J = 5.8 Hz, J = 1.5 Hz, H-22), 3.71 (1H, dd, J = 9.8 Hz, J = 6.4 Hz, H-26<'>), 3.55 (1H, dd, J = 9.6 Hz, J = 6.6 Hz, H-26<">), 3.51 (1H, dd, J = 6.1 Hz, J = 0.6 Hz, H-38), 2.98 (1H, sxt, J = 6.6 Hz, H-25), 2.43 (2H, d, J = 6.4 Hz, H-17, 20), 2.22 (1H, dd, J = 13.7 Hz, J = 7.9 Hz, H-15<'>), 2.18 (1H, dd, J = 12.8 Hz, J = 6.7 Hz, H-4), 2.03 (4H, s, H-30b), 2.02 (2H, s, H-16b), 2.01 (1H, br. s., H-11<'>), 1.96 - 1.98 (16H, m, H-8a, 5a), 1.63 - 1.72 (27H, m, H-6<'>, 12<">, 12<'>), 1.55 (1H, ddd, J = 13.9 Hz, J = 8.9 Hz, J = 6.0 Hz, H-11<">), 1.46 (1H, dtd, J = 10.1 Hz, J = 6.7 Hz, J = 4.0 Hz, H-7<'>), 1.38 (1H, dd, J = 14.0 Hz, J = 4.3 Hz, H-15<">), 1.24 (1H, d, J = 4.3 Hz, H-19<'>), 1.19 - 1.22 (1H, m, H-7<">), 1.19 - 1.19 (1H, m, M08), 1.18 (3H, s, H-18), 1.06 (2H, d, J = 7.0 Hz, H-27), 1.03 (3H, d, J = 7.0 Hz, H-28), 0.97 (3H, s, H-29), 0.91 - 0.95 (1H, m, H-6<">), 0.65 (3H, d, J = 6.1 Hz, H-21), 0.57 (1H, d, J = 4.3 Hz, H-19<">).
¹³C NMR (126MHz, ACETONITRILE-d₃) δ = 205.2 (C-3), 202.4 (C-23), 171.4 (C-30a), 171.3 (C-16a), 155.6 (C-1), 148.6 (C-24), 128.4 (C-2), 127.0 (C-24a), 89.9 (C-30), 77.3 (C-16), 75.9 (C-22), 74.4 (C-26), 51.5 (C-17), 48.4 (C-14), 47.7 (C-4), 46.9 (C-15), 46.7 (C-13), 45.3 (C-8), 43.7 (C-5), 36.4 (C-25), 35.5 (C-20), 33.2 (C-12), 33.0 (C-10), 28.2 (C-11), 27.8 (C-19), 27.3 (C-9), 24.4 (C-6), 24.4 (C-7), 22.1 (C-16b), 21.3 (C-30b), 20.2 (C-29), 18.5 (C-18), 17.5 (C-27), 12.3 (C-21), 11.3 (C-28).

### ◆ exemple 82 : 26-(2,3,4,6 tétraacétyl-glucosylate) de neoboutomellerone

| | |
|---|---|
| 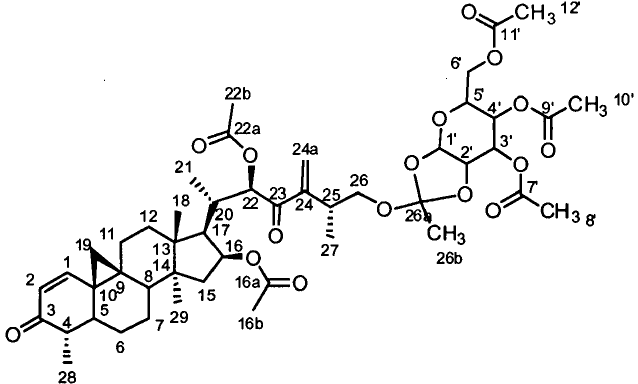 | Formule Chimique : C₄₈H₆₆O₁₆ |
| | Masse Exacte : 898,44 |
| | Poids moléculaire : 899,03 |

### Etape 1 : Synthèse du peracétyl-glucose

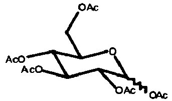

5g (25.2 mmol) de glucose (mélange α / β : 3 / 1) sont dissout dans l'anhydride acétique en présence d'acétate de sodium. Le mélange réactionnel est laissé sous agitation pendant 2h à 100°C. Le milieu réactionnel est dilué avec de l'acétate d'éthyle puis filtré. Le filtrat est ensuite lavé avec de l'eau, avec une solution d'hydrogénocarbonate de sodium puis avec de la saumure. Après séchage sur sulfate de sodium et évaporation du solvant, on recueille 240 mg de brut réactionnel. Le produit est purifié sur colonne de gel de silice et élué par un mélange cyclohexane / acétate d'éthyle de 80 / 20. On recueille 7.27 g de produit soit 74% de rendement (Rf: 0.60 - cyclohexane / acétate d'éthyle 50 / 50).

### Etape 2 : Synthèse du 2,3,4,6-tétraacétyl-glucose

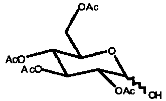

3.5 g (8.97 mmol) de *per*acétyl-glucose sont dissous dans 10 ml de DMF, puis 1.07 g (1.61 mmol,0.18 éq) d'acétate d'hydrazine sont ajoutés. Le mélange est chauffé 20 minutes à 50 °C, puis, après retour à température ambiante, est dilué par de l'acétate d'éthyle et filtré. Le filtrat est successivement lavé avec de l'eau, une solution d'hydrogénocarbonate de sodium, de l'eau, une solution aqueuse de chlorure de lithium à 15%, de l'eau et enfin de la saumure. Après séchage sur sulfate de sodium et évaporation du solvant, on recueille 240 mg de brut réactionnel. Le produit est purifié sur colonne de gel de silice et élué par un mélange cyclohexane / acétate d'éthyle de 50 / 50. On recueille 2.57 g de produit soit 82 % (Rf : 0.20 - cyclohexane / acétate d'éthyle 60 / 40).

### Etape 3 : Synthèse du (1-trichloroacétimidate)-(2,3,4,6 tétraacétyl)-glucose

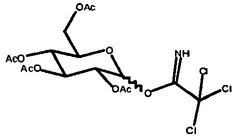

2.57 g (7.39 mmol) de *2,3,4,6*-tétraacétyl-glucose sont dissous dans 10 ml de DCM, puis sont successivement ajouté 7.5 ml (75 mmol) de trichloroacétonitrile et 107 µl (10%) de DBU. Le mélange réactionnel est laissé sous agitation à 0°C pendant 2 h. Après évaporation des solvants, le mélange est purifié colonne de gel de silice par un gradient cyclohexane / acétate d'éthyle de 60 / 40 à 40 / 60. On obtient 2.36 g (65 %) de (*1*-trichloroacétimidate)-(*2,3,4,6* tétraacétyl)-glucose (Rf: 0.50 - cyclohexane / acétate d'éthyle 60 / 40)

### Etape 4 : Synthèse de 26-(2,3,4,6 tétraacétyl-glucosylate) de neoboutomellerone

500 mg (0.88 mmol) de 2 et 650 mg de (*1*-trichloroacétimidate)-(*2,3,4,6* tétraacétyl)-glucose (1.5 éq, 1.32 mmol) sont dissous dans 10 ml d'acétonitrile. On rajoute alors une quantité catalytique de triflate de triméthylsilyle (20%, 0.088 mmol, 32 µl), et on laisse la réaction sous agitation pendant une nuit. On dilue le milieu réactionnel avec de l'acétate d'éthyle puis on filtre sur fritté les sels. Le filtrat est lavé avec de l'eau puis de la saumure. Après séchage sur sulfate de sodium et évaporation du solvant, on recueille 1.174 g de brut réactionnel. Le produit est purifié sur colonne de gel de silice et élué par un gradient de cyclohexane / acétate d'éthyle de 60 / 40 à 50 / 50. On recueille entre autres produits 65.7 mg du composé 26-(*2,3,4,6* tétraacétyl-glucosylate) de neoboutomellerone.
¹H NMR (500MHz, ACETONITRILE-d₃) δ = 6.94 (1H, d, J = 10.1 Hz, H-1), 6.14 (1H, s, H-24aa), 5.95 (1H, s, H-24ab), 5.90 (1H, d, J = 10.1 Hz, H-2), 5.60 (1H, d, J = 5.2 Hz, H-1') 5.52 (1H, d, J = 2.1 Hz, H-22), 5.06 (1H, td, J = 7.6 Hz, J = 4.4 Hz, H-16), 5.02 (1H, t, J = 2.1 Hz, H-3'), 4.83 (1H, ddt, J = 9.5 Hz, J = 2.1 Hz, J = 1.1 Hz, H-4'), 4.29 (1H, ddd, J = 5.1 Hz, J = 2.8 Hz, J = 0.9 Hz, H-2'), 4.13 (1H, dd, J = 12.5 Hz, J = 3.1 Hz, H-6'<'>), 4.09 (1H, dd, J = 12.5 Hz, J = 5.5 Hz, H-6'<">), 3.88 (1H, ddd, J = 8.9 Hz, J = 4.9 Hz, J = 2.7 Hz, H-5'), 3.38 - 3.51 (2H, m, H-26<">, 26<'>), 2.91 (1H, sxt, J = 6.4 Hz, H-25), 2.51 - 2.63 (1H, m, H-20), 2.30 (1H, dd, J = 10.8 Hz, J = 7.5 Hz, H-17), 2.15 - 2.23 (2H, m, H-4, 15<'>), 1.95 - 2.11 (18H, m, H-5, 8, 8', 10', 12', 16b, 22b, 11<'>), 1.64 (3H, s, H-26b), 1.61 - 1.75 (3H, m, H-6<'>, 12<">, 12<'>), 1.51 - 1.60 (1H, m, H-11<">), 1.40 - 1.49 (1H, m, H-7<'>), 1.37 (1H, dd, J = 14.3 Hz, J = 4.9 Hz, H-15<">), 1.24 (1H, d, J = 4.6 Hz, H-19<'>), 1.18 (3H, s, H-18), 1.16 - 1.21 (1H, m, H-7<">), 1.03 (3H, d, J = 6.7 Hz, H-28), 1.02 (3H, d, J = 7.0 Hz, H-27), 0.96 (3H, s, H-29), 0.88 - 0.97 (1H, m, H-6<">), 0.83 (3H, d, J = 7.0 Hz, H-21), 0.58 (1H, d, J = 4.6 Hz, H-19<">)
¹³C NMR (126MHz, ACETONITRILE-d₃) δ = 202.4 (C-3), 198.6 (C-23), 171.7 (C-22a), 171.5 (C-7'), 171.3 (C-16a), 170.8 (C-9'), 170.4 (C-11'), 155.5 (C-1), 150.0 (C-24), 128.4 (C-2), 125.6 (C-24a), 122.2 (C-26a), 97.7 (C-1'), 78.3 (C-22), 76.7 (C-16), 73.4 (C-2'), 70.7 (C-3'), 69.0 (C-4'), 68.0 (C-26), 67.8 (C-5'), 64.2 (C-6'), 51.2 (C-17), 48.3 (C-14), 47.6 (C-4), 46.9 (C-13), 46.8 (C-15), 45.2 (C-8), 43.6 (C-5), 34.9 (C-25), 33.3 (C-20), 33.0 (C-12), 32.9 (C-10), 28.1 (C-11), 27.7 (C-19), 27.2 (C-9), 24.3 (C-7, 6), 22.2 (C-16b), 21.2 (C-12'), 21.1 (C-10'), 21.1 (C-8'), 21.0, 21.0 (C-26b), 20.9 (C-22b), 20.0 (C-29), 18.3 (C-18), 17.6 (C-27), 13.3 (C-21), 11.3 (C-28)

### ◆exemples 83 : TétraBoc spermine acétate de neoboutomellerone

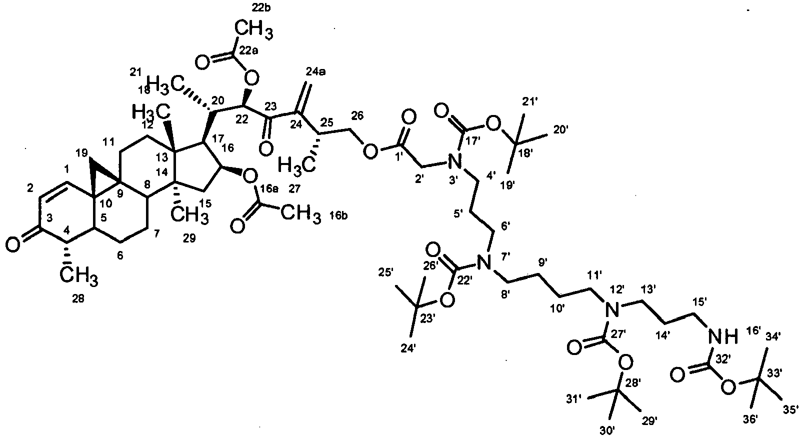

Formule Chimique : C₆₆H₁₀₆N₄O₁₆
Masse Exacte : 1210,76 ; Poids moléculaire : 1211,57

### Etape 1 : Synthèse du triBoc spermine acétate d'éthyle

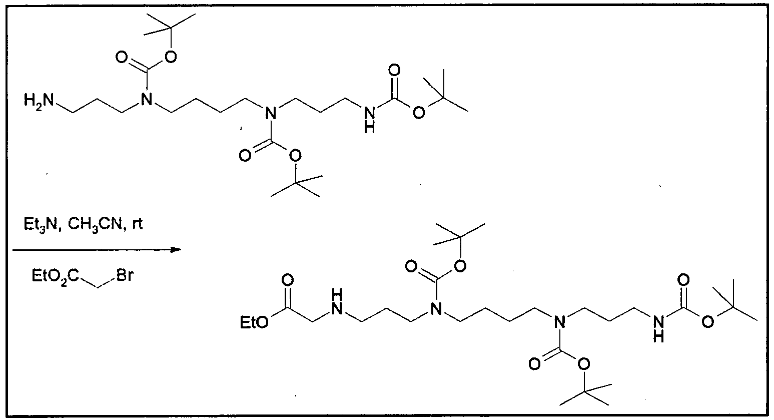

A une solution de 3,96 g triBoc spermine (7,3 mmol, 1 eq) dans 100 mL d'acétonitrile, sont ajoutés 2,6 mL de triéthylamine (18,4 mmol, 2,5 eq). On introduit alors, rapidement sous agitation 0,81 mL de bromoacétate d'éthyle (7,3 mmol, 1 eq) à cette solution. Après 5 h d'agitation à température ordinaire, on jette le milieu réactionnel dans l'eau saturée de NaCl (100 mL) et on extrait par de l'acétate d'éthyle (2x100mL). Après séchage des phases organiques, filtration et évaporation, on purifie le résidu obtenu par flash chromatographie sur SiO₂ (élution avec gradient à partir de l'heptane pur au CH₂Cl₂ pur, puis avec CH₂Cl₂/ MeOH - 90/10). On obtient 2,77 g d'une huile incolore (Rdt =64%), CCM Rf = 0.57 (CH₂Cl₂/MeOH - 90/10), récupération de 1,05 g de triBoc spermine de départ, rendement corrigé : 87%.

### Etape 2 : Synthèse du tétraBoc spermine acétate d'éthyle

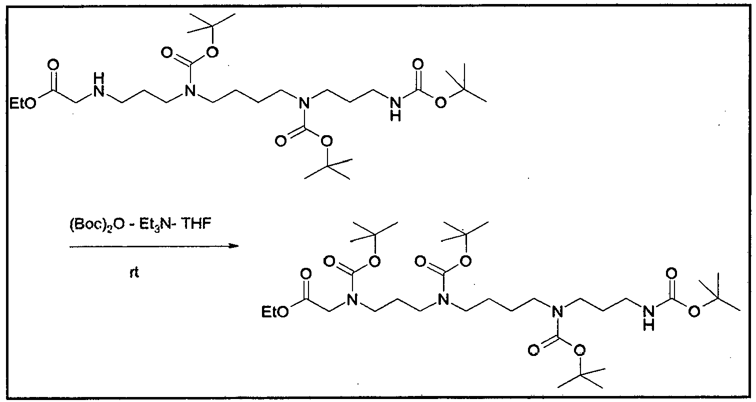

A une solution à température ambiante de 3.35 g de l'intermédiaire obtenu à l'étape 1 (5.7 mmol, 1 eq) dans 60 mL de THF, on ajoute 1 mL de triéthylamine (6.8 mmol, 1.2 eq), puis sous agitation, une solution de 1.36 g de BoC₂O (6.2 mmol, 1.1 eq), et on laisse agiter pendant 2h. Le milieu réactionnel est alors jeté dans l'eau (300 mL), et extrait par l'acétate d'éthyle (3 x 200mL). Les phases organiques une fois séchées sur sulfate de sodium, et filtrées, sont évaporées puis flash chromatographiées sur SiO₂ (élution par gradient de l'heptane pur à l'acétate d'éthyle pur), pour fournir 2.52 g d'une huile incolore. Rdt = 64%, CCM Rf = 0.43 (SiO₂ Heptane/AcOEt - 50/50).

### Etape 3 : Synthèse de l'acide tétraBoc spermine acétique

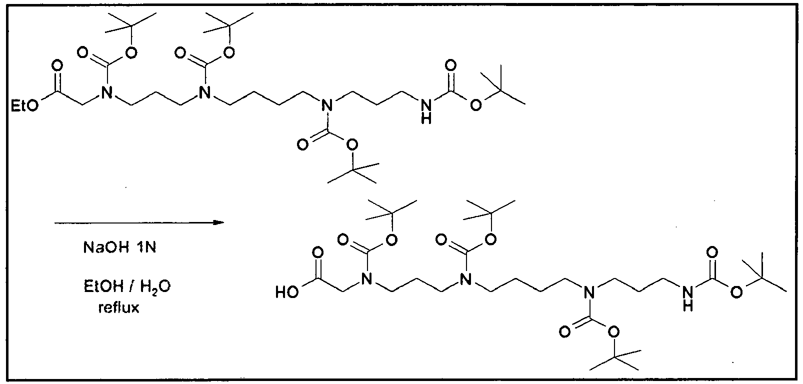

L'ester intermédiaire obtenu à l'étape 2 (2.52 g, 3.6 mmol, 1 eq) est chauffé à reflux sous agitation dans 50 mL d'un mélange EtOH/H₂O et 5.5 mL d'une solution de soude 1N, pendant 3h. On jette dans l'eau glacée (300mL) et on acidifie avec HCl 1N (5.6 mL). Après extraction par du CH₂Cl₂, séchage sur Na₂SO₄, filtration et évaporation, on purifie sur flash chromatographie de SiO₂ (élution gradient d'heptane pur à CH₂Cl₂ pur puis à CH₂Cl₂/MeOH - 90/10). On obtient alors 2.27 g de l'acide tétraBOC spermine acétique sous forme d'une huile incolore. Rdt = 94%, CCM Rf 0.4 (SiO2 CH2Cl2/MeOH - 90/10).

### Etape 4 : Synthèse du tétraBoc spermine acétate de neoboutomellerone

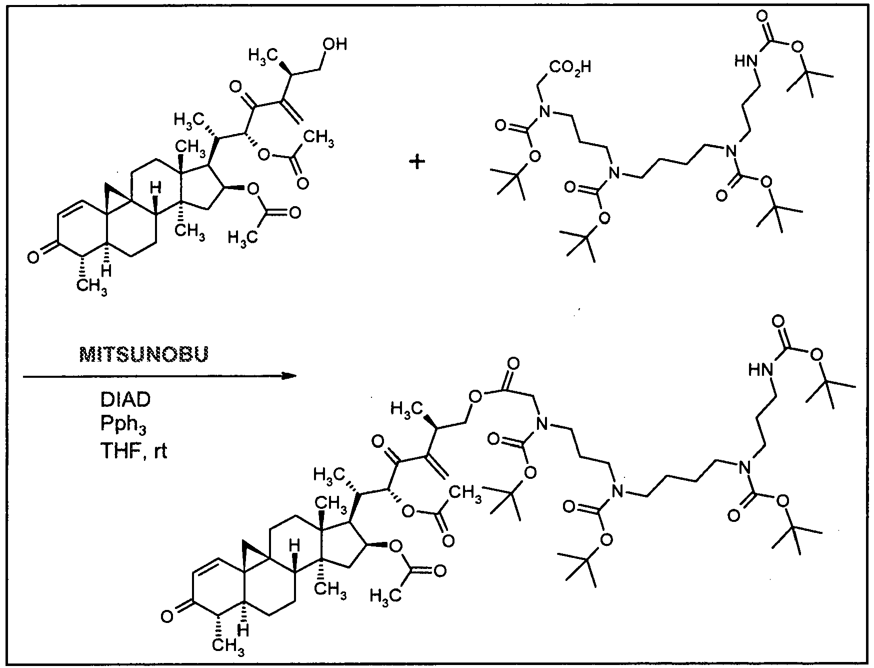

A la solution mélange de 2, du composé obtenu à l'étape 3, triphénylphosphine dans le THF est ajouté goutte à goutte de DIAD à température ambiante. Après 30 min. la réaction est complète (suivi CCM SiO₂ : Heptane 30 / AcOEt 70). Le milieu réactionnel est fixé sur silice puis flash chromatographié (élution gradient Heptane 100% à Heptane 60 / AcOEt 40) pour obtenir 520 mg de produit impur. La purification est complétée par une HPLC préparative sur Phase inverse C8-Xbridge de Waters 5 microns- 1: 220 nm. gradient CH₃CN 50 : H₂O 50 (injection du produit dans le DMSO), Les fractions pures concernées sont évaporées pour éliminer l'acétonitrile puis extraite à l'acétate d'éthyle. Après séchage sur sulfate de sodium anhydre et filtration, la phase organique est évaporée sous pression réduite pour donner 250 mg d'huile limpide et incolore, Rdt : 47%

¹H RMN (500MHz, DMSO-d₆) δ = 6.97 (1H, d, J = 10.1 Hz, H-1), 6.74 (1H, br. s., NH-16'), 6.09 (2H, s, H-24a), 5.89 (1H, d, J = 9.8 Hz, H-2), 5.45 (1H, s, H-22), 5.02 (1H, td, J = 7.5 Hz, J = 4.6 Hz, H-16), 4.05 - 4.17 (1H, m, H-26<'>), 3.97 - 4.04 (1H, m, H-26<">), 3.81 - 3.96 (2H, m, H-2'<">, 2'<'>), 3.02 - 3.22 (10H, m, H-4', 6', 8', 11', 13'), 2.95 (1H, dt, J = 14.5 Hz, J = 7.1 Hz, H-25), 2.87 (2H, q, J = 6.5 Hz, H-15'), 2.22 (1H, dd, J = 10.8 Hz, J = 7.5 Hz, H-17), 2.09 (3H, s, H-22b), 2.07 - 2.16 (2H, m, H-4, 15<'>), 2.05 (3H, s, H-16b), 1.93 - 2.01 (2H, m, H-8, 11<'>), 1.89 (1H, td, J = 12.4 Hz, J = 4.1 Hz, H-5), 1.47 - 1.71 (8H, m, H-5', 14', 6<'>, 11<">, 12<">, 12<'>), 1.26 - 1.44 (42H, m, H-7<'>, 9', 10', 15<">, 19', 20', 21', 24', 25', 26', 29', 30', 31', 34', 35', 36'), 1.24 (1H, d, J = 4.3 Hz, H-19<'>), 1.13 (4H, s, H-7<">, 18), 1.03 (3H, d, J = 7.0 Hz, H-27), 0.98 (3H, d, J = 6.7 Hz, H-28), 0.89 (4H, s, H-6<">, 29), 0.79 (3H, d, J = 6.7 Hz, H-21), 0.57 (1H, br. s., H-19<">)
¹³C RMN (126MHz, DMSO-d₆) δ = 200.7 (C-3), 197.1, 197.0, 170.2 (C-22a), 170.2 (C-22a), 170.0 (C-1'), 169.9 (C-16a), 169.7 (C-1') 155.6 (C-32'), 154.8 (C-1), 154.6 (C-17', 22', 27'), 154.5 (C-17', 22', 27'), 147.1 (C-24), 127.3 (C-2), 125.4 (C-24a), 125.3 (C-24a), 79.2 (C-18', 23', 28', 33'), 79.1 (C-18', 23', 28', 33'), 78.3 (C-18', 28', 23', 33'), 77.5 (C-18', 23', 28', 33'), 76.7 (C-22), 75.2 (C-16), 67.2 (C-26), 67.1 (C-26), 49.7 (C-17), 48.9 (C-2'), 48.6 (C-6', 8', 11', 13'), 47.0 (C-14), 46.5 (C-6', 8', 11', 13'), 46.2 (C-4), 46.0 (C-6', 8', 11', 13'), 45.5 (C-15), 45.4 (C-4'), 44.4 (C-6', 8', 11', 13'), 43.5 (C-8), 42.1 (C-5), 37.6 (C-15'), 33.3 (C-25), 33.1 (C-25), 32.0 (C-20), 31.7 (C-12), 31.6, 28.9 (C-5', 9', 10', 14'), 28.3, 28.1, 28.0, 27.8, 26.7 (C-11), 26.5 (C-5', 9', 10', 14'), 26.0 (C-19), 25.7 (C-9', 10', 14'), 25.1 (C-5'), 23.0 (C-6), 22.9 (C-7), 21.5 (C-16b), 20.5 (C-22b), 19.2 (C-29), 17.6 (C-18), 16.6 (C-27), 12.5 (C-21), 10.8 (C-28)

### ◆ exemple 84: Sel tétrachlorhydrate de spermine acétate de neoboutomellerone

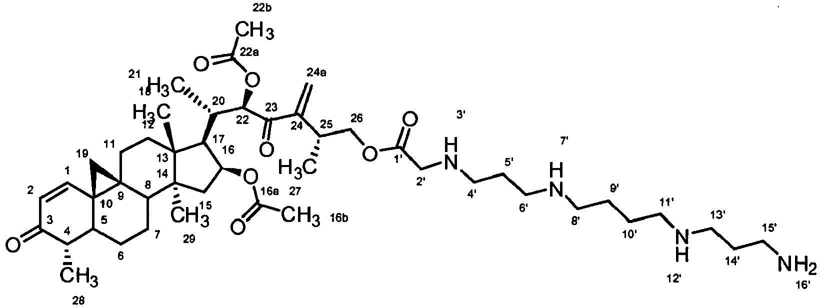

Formule Chimique : C₄₆H₇₄N₄O₈
Masse exacte : 810,55 ; Poids moléculaire : 811,10

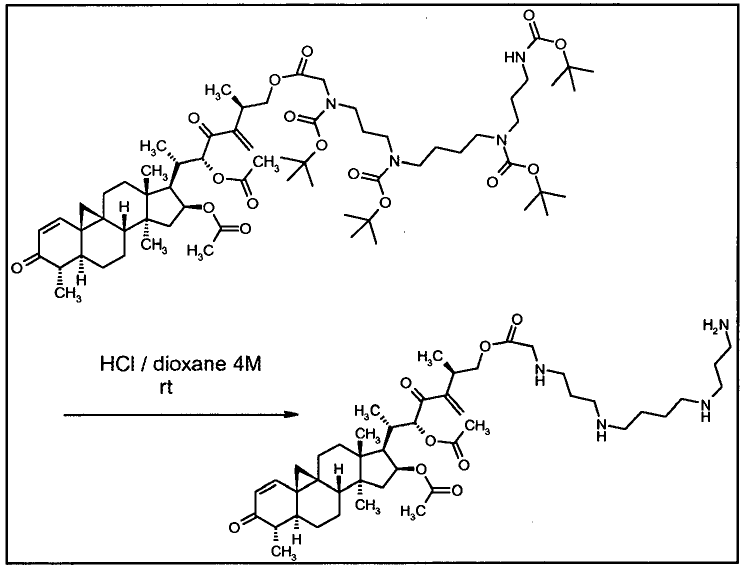

Protocole : L'intermédiaire 83 est laissé sous agitation 3 heures à température ambiante en solution dans une solution 4M de HCl dans le dioxane. Le précipité blanc obtenu est filtré puis rincer à l'éther isopropylique, P : 160 mg.

Le produit est purifié par HPLC préparative sur Phase inverse C8-Xbridge de Waters 5 microns- 1: 220 nm. gradient HCl 5 mM 80% / CH₃CN 20 % à HCl 5 mM 65% / CH₃CN 35%. Après suivi HPLC analytique, les fractions concernées sont évaporées sous pression réduite pour éliminer l'acétonitrile puis lyophilisées pour donner 20 mg du produit attendu sous sa forme tétrachlorhydrate (poudre blanche).
¹H RMN (500MHz, DMSO-d₆) δ = 9.53 (1H, br. s., NH-3'), 9.21 (2H, br. s., NH-12', 7'), 8.11 (2H, br. s., H-16'), 6.97 (1H, d, J = 10.1 Hz, H-1), 6.18 (1H, s, H-24aa), 6.14 (1H, s, H-24ab), 5.90 (1H, d, J = 9.8 Hz, H-2), 5.46 (1H, d, J = 1.8 Hz, H-22), 5.03 (1H, td, J = 7.4 Hz, J = 4.7 Hz, H-16), 4.19 (1H, dd, J = 10.7 Hz, J = 6.4 Hz, H-26<'>), 4.13 (1H, dd, J = 10.7 Hz, J = 7.0 Hz, H-26<">), 3.96 (2H, br. s., H-2'<">, 2'<'>), 3.00 - 3.14 (3H, m, H-4', 25), 2.98 (4H, br. s., H-6', 13'), 2.89 (6H, br. s., H-8', 11', 15'), 2.47 - 2.52 (1H, m, H-20), 2.23 (1H, dd, J = 11.0 Hz, J = 7.3 Hz, H-17), 2.11 (3H, s, H-22b), 2.04 - 2.16 (4H, m, H-15<'>, 4, 5'), 2.07 (3H, s, H-16b), 1.98 (4H, m, H-11<'>, 8, 14'), 1.89 (1H, td, J = 12.4 Hz, J = 4.4 Hz, H-5), 1.67 - 1.78 (4H, m, H-10', 9'), 1.50 - 1.67 (4H, m, H-6<'>, 11<">, 12<">, 12<'>), 1.34 - 1.45 (1H, m, H-7<'>), 1.31 (1H, dd, J= 13.9 Hz, J = 4.1 Hz, H-15<">), 1.24 (1H, d, J = 4.3 Hz, H-19<'>), 1.13 (3H, s, H-18), 1.17 (1H, d, J = 3.1 Hz, H-7<">), 1.06 (3H, d, J = 7.0 Hz, H-27), 0.98 (3H, d, J = 6.7 Hz, H-28), 0.90 (3H, s, H-29), 0.86 - 0.95 (1H, m, H-6<">), 0.80 (3H, d, J = 6.7 Hz, H-21), 0.58 (1H, d, J = 4.0 Hz, H-19<">)
¹³C RMN (126MHz, DMSO-d₆) δ = 200.7 (C-3), 197.1 (C-23), 170.4 (C-22a), 170.0 (C-16a), 154.9 (C-1), 146.8 (C-24), 127.3 (C-2), 125.6 (C-24a), 76.7 (C-22), 75.2 (C-16), 68.2 (C-26), 49.7 (C-17), 47.0 (C-14), 46.6 (C-2'), 46.2 (C-4), 46.0 (C-11'), 45.9 (C-8'), 45.5 (C-13), 45.4 (C-15), 44.0 (C-4'), 43.8 (C-6', 13'), 43.4 (C-8), 42.1 (C-5), 36.2 (C-15'), 32.6 (C-25), 32.1 (C-20), 31.7 (C-12), 31.6 (C-10), 26.7 (C-11), 26.4 (C-19), 25.9 (C-9), 23.6 (C-14'), 23.0 (C-7), 22.8 (C-6), 22.6 (C-10'), 22.6 (C-9'), 22.1 (C-5'), 21.6 (C-16b), 20.6 (C-22b), 19.2 (C-29), 17.6 (C-18), 16.5 (C-27), 12.5 (C-21), 10.8 (C-28)

### ◆ exemple 85 (comparatif): Dérivé trichloroacétamide dihydro furane neoboutomellerone

| | |
|---|---|
| 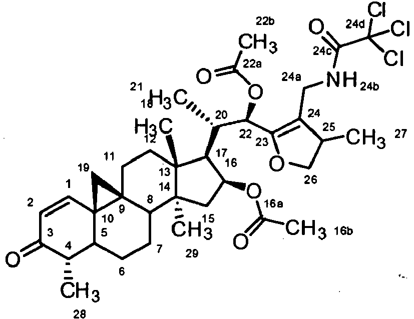 | Formule Chimique : C₃₆H₄₈NO₇ |
| | Masse Exacte : 711,25 |
| | Poids moléculaire : 713,13 |

### Etape 1 :

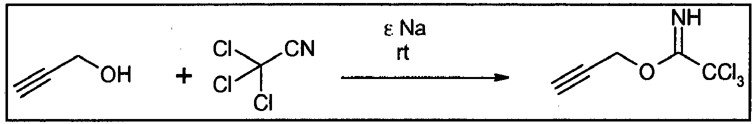

Au mélange des réactifs, ajouter le sodium et laisser en contact à température ambiante 4 heures. Reprendre le milieu réactionnel avec de l'éther isopropylique, laver avec une solution saturée en NaCl, décanter et sécher sur sulfate de sodium anhydre, Filtrer et évaporer sous pression réduite le solvant. Distiller au « Kügelrohr » (T_{Eb}: 100°C à 5mm Hg). On obtient un liquide incolore et limpide.

### Etape 2: Synthèse du dérivé trichloroacétamide dihydro furane neoboutomellerone

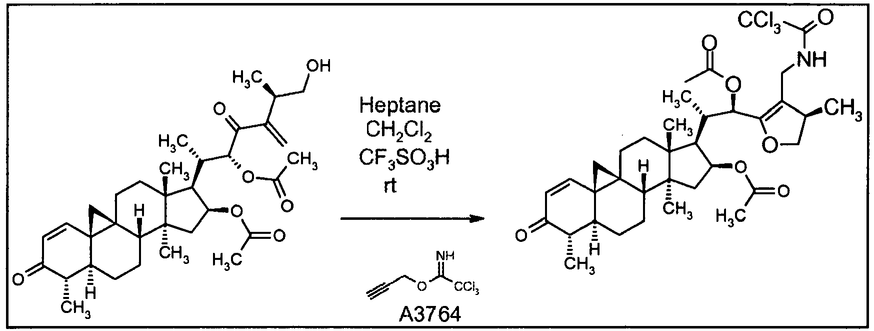

A la solution de 2 et du composé obtenu à l'étape 1, dans le mélange Heptane / CH₂Cl₂, ajouter la goutte d'acide triflique à température ambiante. Laisser 10 minutes en contact sous agitation, puis jeter le milieu réactionnel sur une solution NaHCO₃, Décanter, sécher sur sulfate de sodium anhydre, filtrer et évaporer sous pression réduite, Flash chromatographie gradient sur Silice : Heptane 100 % à éther isopropylique 100% sur 15 minutes. Evaporer les fractions concernées pour obtenir une mousse blanche.
¹H RMN (500MHz, ACETONITRILE-d₃) δ = 7.37 (1H, br. s., NH-24b), 6.94 (1H, d, J = 9.8 Hz, H-1), 5.90 (1H, d, H-2), 5.37 (1H, s, H-22), 5.24 (1H, td, J = 7.9 Hz, J = 4.6 Hz, H-16), 4.34 (1H, dd, J = 9.5 Hz, J = 8.9 Hz, H-26<'>), 4.24 (1H, dd, J = 14.8 Hz, J = 4.7 Hz, H-24a<'>), 3.94 (1H, dd, J = 14.6 Hz, J = 6.1 Hz, H-24a<">), 3.82 (1H, dd, J = 8.9 Hz, J = 6.4 Hz, H-26<">), 3.00 (1H, sxt, J = 7.2 Hz, H-25), 2.29 - 2.40 (1H, m, H-20), 2.15 - 2.21 (2H, m, H-15<'>, 4), 2.08 (3H, s, H-22b), 1.97 (3H, s, H-16b), 1.95 - 2.12 (4H, m, H-11<'>, 5, 17, 8), 1.62 - 1.73 (3H, m, H-6<'>, 12<">, 12<'>), 1.54 - 1.62 (1H, m, H-11<">), 1.41 - 1.50 (1H, m, H-7<'>), 1.34 (1H, dd, J = 13.7 Hz, J = 4.0 Hz, H-15<">), 1.25 (1H, d, J = 4.6 Hz, H-19<'>), 1.16 (3H, s, H-18), 1.13 - 1.23 (1H, m, H-7<">), 1.07 (3H, d, J = 7.0 Hz, H-27), 1.02 (3H, d, J = 6.7 Hz, H-28), 1.02 (3H, d, J = 7.0 Hz, H-21), 0.91 - 0.99 (1H, m, H-6<">), 0.90 (3H, s, H-29), 0.56 (1H, d, J = 4.6 Hz, H-19<">)
¹³C RMN (126MHz, ACETONITRILE-d₃) δ = 202.3 (C-3), 171.4 (C-22a), 171.4 (C-16a), 162.6 (C-24c), 155.5 (C-1), 151.7 (C-23), 128.4 (C-2), 111.8 (C-24), 76.9 (C-26), 75.7 (C-16), 72.4 (C-22), 51.1 (C-17), 48.5 (C-14), 47.6 (C-4), 46.7 (C-13), 45.8 (C-15), 44.7 (C-8), 43.4 (C-5), 39.7 (C-25), 36.2 (C-24a), 34.5 (C-20), 32.9 (C-10), 32.7 (C-12), 28.0 (C-11), 27.3 (C-19), 24.2 (C-6), 24.1 (C-7), 21.6 (C-16b), 21.3 (C-22b), 19.8 (C-29), 18.5 (C-27), 17.9 (C-18), 13.1 (C-21), 11.3 (C-28)

### ◆ exemple 86 (comparatif) : O-benzoyl N-méthyl hydroxylamine méthyl dihydro furane neoboutomellerone

| | |
|---|---|
| 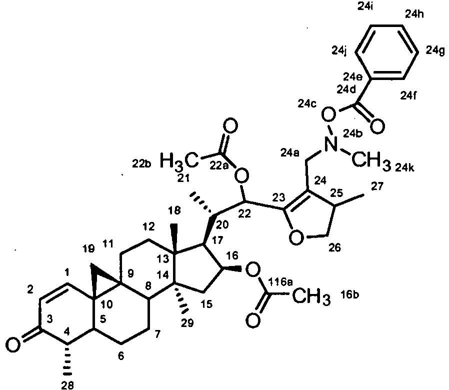 | Formule Chimique : C₄₂H₅O₈ |
| | Masse Exacte : 701,39 |
| | Poids moléculaire : 701,89 |

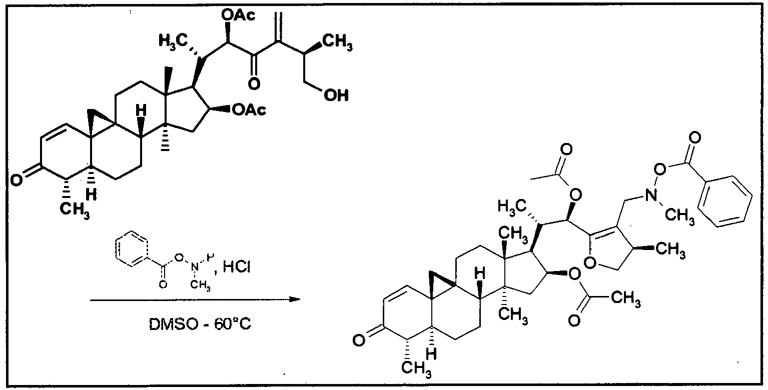

Protocole : Le mélange réactionnel est laissé sous agitation à 60°C pendant 1 heure puis hydrolysé avec de l'eau et extrait à l'acétate d'éthyle. Après décantation, séchage sur sulfate de sodium anhydre et filtration, la phase organique est évaporée sous pression réduite. Le produit est purifié dans un premier temps par flash chromatographie gradient sur silice 35-70 microns : heptane 100% à éther isopropylique 100%. Les fractions concernées sont évaporées pour donner 45 mg de produit sous forme de poudre blanche.

Le produit est dans un deuxième temps re-purifié par flash chromatographie gradient sur silice 15-40 microns isocratique cyclohexane/ acétate d'éthyle 80/20. Les fractions concernées sont évaporées pour donner 28 mg.
¹H RMN (500MHz, CD3CN) δ = 7.90 (2H, d, J = 7.3 Hz, H-24j, 24f), 7.61 (1H, t, J = 7.6 Hz, H-24h), 7.48 (2H, dd, J = 8.2 Hz, J = 7.3 Hz, H-24g, 24i), 6.94 (1H, d, J = 9.8 Hz, H-1), 5.90 (1H, d, J = 10.1 Hz, H-2), 5.31 (1H, s, H-22), 5.14 (1H, td, J = 7.8 Hz, J = 4.6 Hz, H-16), 4.23 (1H, t, J = 9.2 Hz, H-26<'>), 3.89 (1H, d, J = 13.4 Hz, H-24a<'>), 3.75 (1H, dd, J = 8.7 Hz, J = 6.6 Hz, H-26<">), 3.60 (1H, d, J = 13.4 Hz, H-24a<">), 3.15 (1H, sxt, J = 7.0 Hz, H-25), 2.81 (3H, s, H-24k), 2.27 - 2.38 (1H, m, H-20), 2.17 (1H, dq, J = 13.0 Hz, J = 6.7 Hz, H-4), 2.12 (3H, s, H-22b), 1.99 (3H, s, H-16b), 1.96 - 2.11 (5H, m, H-5, 8, 11<'>, 15<'>, 17), 1.62 - 1.70 (3H, m, H-6<'>, 12<">, 12<'>), 1.52 - 1.61 (1H, m, H-11<">), 1.41 - 1.49 (1H, m, H-7<'>), 1.29 - 1.36 (1H, m, H-15<">), 1.25 (1H, d, J = 4.3 Hz, H-19<'>), 1.15 - 1.21 (1H, m, H-7<">), 1.14 (3H, s, H-18), 1.11 (3H, d, J = 6.7 Hz, H-27), 1.02 (3H, d, J = 6.7 Hz, H-28), 0.96 (3H, d, J = 6.7 Hz, H-21), 0.90 - 0.95 (1H, m, H-6<">), 0.90 (3H, s, H-29), 0.56 (1H, d, J = 4.3 Hz, H-19<">)
¹³C RMN (126MHz, CD3CN) δ = 202.3 (C-3), 171.3 (C-16a), 171.1 (C-22a), 165.7 (C-24d), 155.5 (C-1), 134.1 (C-24h), 130.7 (C-24e), 130.1 (C-24f, 24j), 129.6 (C-24g, 24i), 128.4 (C-2), 76.7 (C-26), 75.8 (C-16), 72.5 (C-22), 55.1 (C-24a), 50.8 (C-17), 48.3 (C-14), 47.6 (C-4), 46.7 (C-24k), 46.7 (C-13), 46.0 (C-15), 44.8 (C-8), 43.5 (C-5), 40.2 (C-25), 34.8 (C-20), 32.9 (C-10), 32.7 (C-12), 28.0 (C-11), 27.3 (C-19), 27.3 (C-9), 24.2 (C-6), 24.1 (C-7), 21.8 (C-16b), 21.4 (C-22b), 19.9 (C-29), 18.3 (C-27), 18.0 (C-18), 13.1 (C-21), 11.3 (C-28)

### ◆ exemple 87 (comparatif) : Dérivé méthyl propanone méthyl chlorométhyl tetrahydrofurane- neoboutomellerone

| | |
|---|---|
| 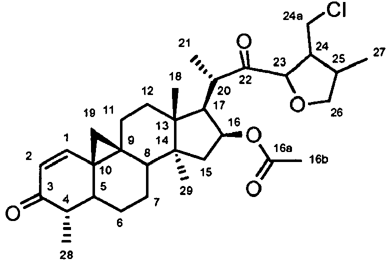 | Formule Chimique : C₃₂H₄₅ClO₅ |
| | Masse Exacte : 544,30 |
| | Poids moléculaire : 545,15 |

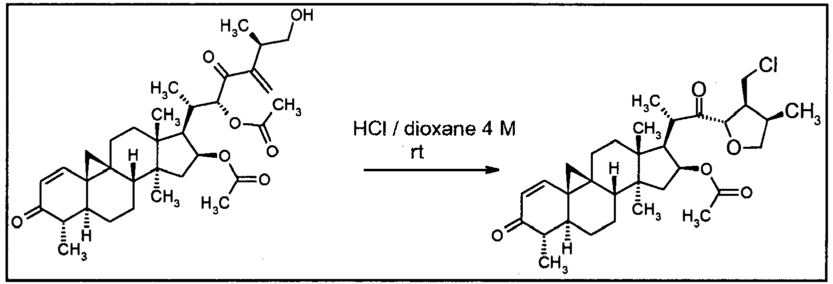

Protocole : Laisser le mélange des réactifs en contact 1 heure sous agitation à température ambiante. Jeter le milieu réactionnel sur une solution NaHCO₃, extraire à l'acétate d'éthyle, décanter, sécher la phase organique sur du sulfate de sodium anhydre, filtrer et évaporer sous pression réduite. Prépurification par Flash chromatographie Silice: gradient Heptane 100% à éther isopropylique 100% pour obtenir 5,5 mg sous forme de mousse.

Purification du produit par HPLC préparative : Colonne Lichrospher 100RP 18, 5 µm, 10x250 mm, λ : 270 nm, débit 6 mL/min. Injection dans Acétonitrile 100%, phase mobile : H20/CH3CN 20/80. Après suivi HPLC analytique, les fractions pures concernées par le produit sont évaporées sous pression réduite (1.6 mg).
¹H RMN (500MHz, CD3CN) δ = 6.95 (1H, d, J = 10.1 Hz, H-1), 5.90 (1H, d, J = 10.1 Hz, H-2), 5.04 (1H, td, J = 8.1 Hz, J = 5.5 Hz, H-16), 4.29 (1H, d, J = 7.0 Hz, H-23), 3.99 (1H, dd, J = 8.4 Hz, J = 5.6 Hz, H-26<'>), 3.64 - 3.74 (2H, m, H-24a<'>, 24a<">), 3.62 (1H, dd, J = 8.2 Hz, J = 4.3 Hz, H-26<">), 3.35 (1H, dq, J = 10.9 Hz, J = 7.2 Hz, H-20), 2.36 - 2.64 (3H, m, H-17, 25, 24), 2.14 - 2.21 (1H, m, H-4), 1.95 - 2.13 (4H, m, H-5, 8, 11<'>, 15<'>), 1.91 (3H, s, H-16b), 1.71 - 1.82 (1H, m, H-12<'>), 1.56 - 1.70 (3H, m, H-6<'>, 11<">, 12<">), 1.41 - 1.50 (1H, m, H-7<'>), 1.26 (2H, d, J = 4.6 Hz, H-7<">, 19<'>), 1.18 (3H, s, H-18), 1.15 (3H, d, J = 7.0 Hz, H-21), 1.02 (3H, d, J = 6.7 Hz, H-27), 1.01 (3H, d, J = 6.7 Hz, H-28), 0.98 - 1.05 (1H, m, H-6<">), 0.97 (3H, s, H-29), 0.56 (1H, d, J = 4.3 Hz, H-19<">)
¹³C RMN (126MHz, CD3CN) δ = 215.1 (C-22), 202.3 (C-3), 170.9 (C-16a), 155.5 (C-1), 128.4 (C-2), 84.3 (C-23), 76.1 (C-26), 76.1 (C-16), 51.0 (C-17), 49.2 (C-24), 47.6 (C-4), 45.7 (C-15), 44.5 (C-8), 44.2 (C-24a), 43.4 (C-5), 41.1 (C-20), 36.6 (C-25), 33.0 (C-12), 28.0 (C-11), 27.1 (C-19), 24.2 (C-6), 24.1 (C-7), 21.7 (C-16b), 19.6 (C-29), 18.5 (C-18), 16.6 (C-21), 12.4 (C-27), 11.3 (C-28)

### ◆ exemple 88 (comparatif) : Dérivé résorcinolé de neoboutomellerone

| | |
|---|---|
| 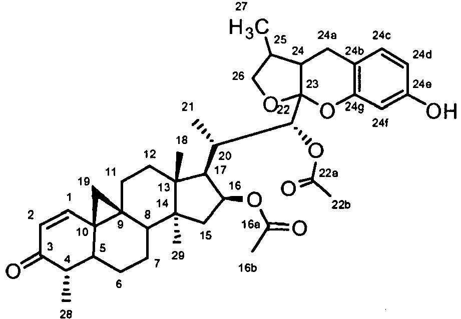 | Formule Chimique : C₄₀H₅₂O₈ |
| | Masse Exacte : 660,37 |
| | Poids moléculaire : 660,84 |

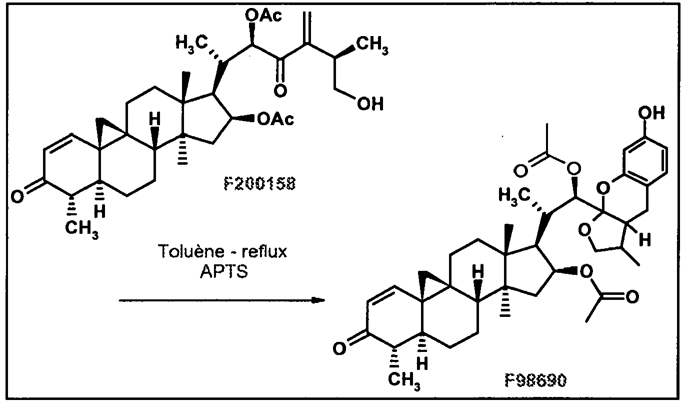

Protocole : Le mélange réactionnel des matières premières dans le toluène est porté au reflux 15 minutes.

Après évaporation du toluène, le résidu obtenu est pré-purifié par flash chromatographie sur silice 35-75 microns ; élution éther isopropylique 100 % pour donner 16 mg de solide blanc.

Purification du produit par HPLC préparative : Colonne Lichrospher 100RP 18, 5 µm, 25x250 mm, λ : 200 nm, débit 30 mL/min. Injection dans Acétonitrile 100%, phase mobile : gradient H20/CH3CN 35/65 à 10/90 en 40min.. Après suivi HPLC analytique, les fractions pures concernées par le produit sont évaporées sous pression réduite (4.9 mg).
¹H RMN (500MHz, CD3CN) δ = 6.94 (1H, d, J = 9.8 Hz, H-1), 6.90 (1H, d, J = 7.9 Hz, H-24c), 6.84 (1H, br. s., OH-24e), 6.34 (1H, dd, J = 7.9 Hz, J = 2.4 Hz, H-24d), 6.21 (1H, d, J = 2.4 Hz, H-24f), 5.89 (1H, d, J = 9.8 Hz, H-2), 5.26 (1H, td, J = 7.9 Hz, J = 4.7 Hz, H-16), 5.03 (1H, s, H-22), 3.99 (1H, t, J = 8.1 Hz, H-26<'>), 3.35 (1H, dd, J = 9.2 Hz, J = 8.2 Hz, H-26<">), 2.68 - 2.82 (2H, m, H-20, 24a<'>), 2.59 (1H, dd, J = 16.3 Hz, J = 1.7 Hz, H-24a<">), 2.11 - 2.22 (1H, m, H-4), 2.00 (3H, s, H-22b), 1.95 - 2.07 (4H, m, H-5, 8, 11<'>, 15<'>), 1.90 - 1.96 (1H, m, H-24), 1.89 (1H, dd, J = 11.4 Hz, J = 7.8 Hz, H-17), 1.77 - 1.85 (1H, m, H-25), 1.68 - 1.74 (2H, m, H-12<">, 12<'>), 1.61-1.68 (1H, m, H-6<'>), 1.54 - 1.62 (1H, m, H-11<">), 1.37 - 1.47 (1H, m, H-7<'>), 1.40 (3H, s, H-16b), 1.24 (1H, d, J = 4.6 Hz, H-19<'>), 1.22 - 1.29 (1H, m, H-15<">), 1.18 (3H, s, H-18), 1.18 (3H, d, J = 7.6 Hz, H-21), 1.11 - 1.21 (1H, m, H-7<">), 1.01 (3H, d, J = 6.7 Hz, H-28), 0.96 (3H, d, J = 6.4 Hz, H-27), 0.93 (1H, qd, J = 13.0 Hz, J = 4.1 Hz, H-6<">), 0.86 (3H, s, H-29), 0.56 (1H, d, J = 4.6 Hz, H-19<'>)
¹³C RMN (126MHz, CD3CN) δ = 202.4 (C-3), 172.2 (C-22a), 171.3 (C-16a), 157.7 (C-24e), 155.6 (C-1), 154.8 (C-24g), 130.9 (C-24c), 128.4 (C-2), 113.1 (C-24b), 110.0 (C-23), 109.5 (C-24d), 104.3 (C-24f), 75.7 (C-16), 75.4 (C-26), 74.7 (C-22), 52.3 (C-17), 48.4 (C-14), 47.6 (C-4), 46.9 (C-24), 46.7 (C-13), 45.9 (C-15), 44.7 (C-8), 43.4 (C-5), 34.9 (C-25), 33.0 (C-12), 32.9 (C-10), 31.2 (C-20), 28.1 (C-11), 27.3 (C-9), 27.2 (C-19), 24.2 (C-6), 24.1 (C-7), 21.0 (C-22b), 20.7 (C-16b), 19.9 (C-29), 17.9 (C-18), 15.3 (C-27), 14.1 (C-21), 11.2 (C-28)

### ◆ exemple 89 : 26-phosphate- neoboutomellerone

| | |
|---|---|
| 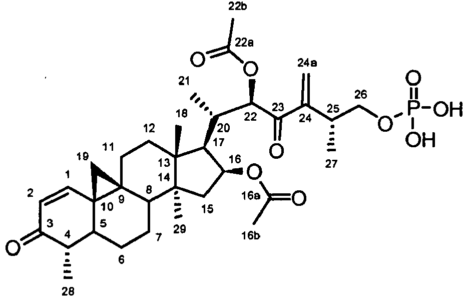 | Formule Chimique : C₃₄H₄₉O₁₀P |
| | Masse Exacte : 648,31 |
| | Poids moléculaire : 648,72 |

Protocole : A la solution 2, Et₃N, THF à 0°C; ajouter goutte à goutte à 0°C POCl₃, Laisser 2 heures en contact à cette température. Ajouter HCl 1N et laisser sous agitation pendant 1 heure. Extraire 2 fois à l'AcOEt, décanter et sécher la phase organique sur sulfate de sodium anhydre. Filtrer et évaporer sous pression réduite le solvant: obtention de 150mg de mousse blanche. Purification du produit par HPLC préparative : Colonne Waters X-bridge C8 5 m, 30x250 mm, λ : 220 nm, débit 40 mL/min. Injection dans DMSO 100%, phase mobile : gradient HCl 5 mM 80%-CH3CN 20% à HCl 5 mM 50-CH₃CN 50%. Les fractions pures concernées par le produit sont évaporée sous pression réduite pour éliminer l'acétonitrile, puis extraite à l'acétate d'éthyle, sécher sur sulfate de sodium anhydre, filtrer et évaporer sous pression réduite pour conduire 85 mg de produit cristallisé blanc. Pureté 98,6%.
¹H RMN (500MHz, CD3CN) δ = 6.94 (1H, d, J = 10.1 Hz, H-1), 6.12 (1H, s, H-24aa), 5.99 (1H, s, H-24ab), 5.89 (1H, d, J = 9.8 Hz, H-2), 5.53 (1H, d, J = 1.8 Hz, H-22), 5.09 (1H, td, J = 7.6 Hz, J = 4.4 Hz, H-16), 3.97 - 4.04 (1H, m, H-26<'>), 3.80 - 3.94 (1H, m, H-26<">), 3.00 (1H, sxt, J = 6.8 Hz, H-25), 2.54 - 2.65 (1H, m, H-20), 2.29 (1H, dd, J = 11.0 Hz, J = 7.6 Hz, H-17), 2.12 - 2.22 (2H, m, H-4, 15<'>), 2.10 (3H, s, H-22b), 2.03 (3H, s, H-16b), 1.98 - 2.07 (1H, m, H-8, 11<'>), 1.94 - 1.97 (1H, m, H-5), 1.63 - 1.76 (3H, m, H-7<'>, 12<">, 12<'>), 1.51 - 1.61 (1H, m, H-11<">), 1.40 - 1.49 (1H, m, H-6<'>), 1.36 (1H, dd, J = 14.0 Hz, J = 4.0 Hz, H-15<">), 1.24 (1H, d, J = 4.3 Hz, H-19<'>), 1.19 (3H, s, H-18), 1.15 - 1.23 (1H, m, H-6<">), 1.08 (3H, d, J = 7.0 Hz, H-27), 1.02 (3H, d, J = 6.7 Hz, H-28), 0.95 (3H, s, H-29), 0.93 (1H, qd, J = 12.5 Hz, J = 3.7 Hz, H-7<">), 0.86 (3H, d, J = 6.7 Hz, H-21), 0.58 (1H, d, J = 4.3 Hz, H-19<">)
¹³C RMN (126MHz, CD3CN) δ = 202.4 (C-3), 199.0 (C-23), 171.8 (C-22a), 171.3 (C-16a), 155.5 (C-1), 148.6 (C-24), 128.4 (C-2), 125.9 (C-24a), 78.4 (C-22), 76.7 (C-16), 70.1 (C-26), 51.3 (C-17), 48.4 (C-14), 47.6 (C-4), 46.9 (C-13), 46.7 (C-15), 45.2 (C-8), 43.6 (C-5), 36.3 (C-25), 33.3 (C-20), 33.0 (C-12), 32.9 (C-10), 28.1 (C-11), 27.7 (C-19), 27.2 (C-9), 24.3 (C-7, 6), 22.2 (C-16b), 21.0 (C-22b), 20.0 (C-29), 18.3 (C-18), 17.0 (C-27), 13.4 (C-21), 11.3 (C-28)

### ◆ exemple 90 : Sel de N-méthyl glutamine de 26-phosphate-neoboutomellerone

| | |
|---|---|
| 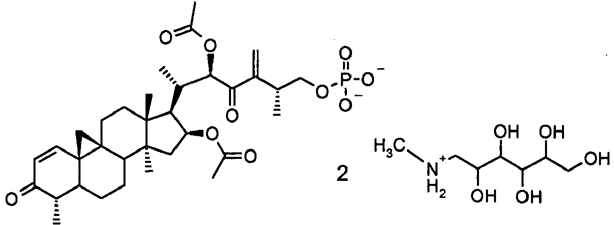 | Formule Chimique : C₄₁H₆₇NO₁₅P⁺ |
| | Masse Exacte : 844,42 |
| | Poids moléculaire : 844,94 |

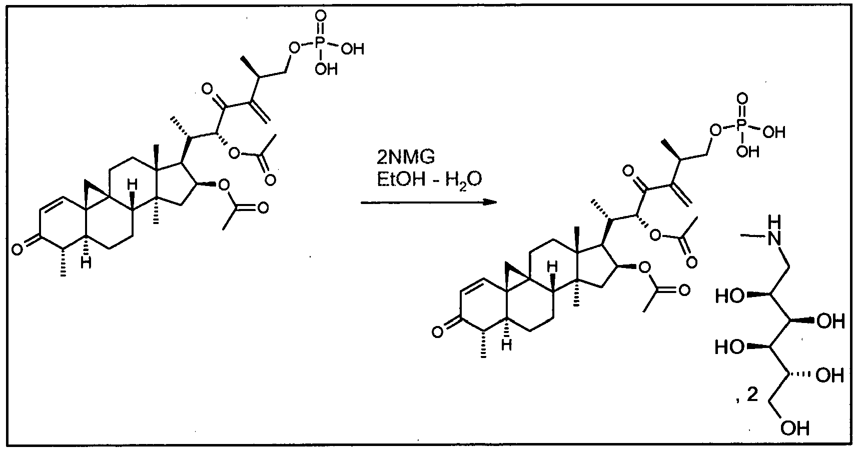

Protocole : Dissoudre le composé 89 dans l'éthanol, puis ajouter les 2 éq, de NMG en solution dans l'eau. La solution obtenue est évaporée sous pression réduite, reprise dans 5 mL H₂O, filtrer sur filtre 0.45 m. La solution obtenue est congelée et lyophilisée pour donner 55 mg de solide blanc, Pureté HPLC : 98,6% L'analyse RMN permet d'évaluer approximativement la présence de 2,5 éq de NMG.
¹H RMN (500MHz, D2O) δ = 7.22 (1H, d, J = 9.8 Hz, H-1), 6.29 (1H, s, H-24aa), 6.22 (1H, s, H-24ab), 6.02 (1H, d, J = 9.8 Hz, H-2), 5.74 (1H, d, J = 2.1 Hz, H-22), 5.02 - 5.12 (1H, m, H-16), 4.06 (2H, ddd, J = 9.2 Hz, J = 5.3 Hz, J = 3.5 Hz, H-5'), 3.78 - 3.88 (5H, m, H-2', 26<'>, 1'<'>), 3.73 - 3.78 (2H, m, H-3'), 3.67 - 3.71 (1H, m, H-26<">), 3.61 - 3.68 (4H, m, H-4', 1'<">), 3.12 (2H, dd, J = 12.8 Hz, J = 3.4 Hz, H-6'<'>), 3.06 (2H, dd, J = 12.8 Hz, J = 9.5 Hz, H-6'<">), 2.93 (1H, sxt, J = 6.9 Hz, H-25), 2.67 - 2.74 (1H, m, H-20), 2.68 (6H, s, H-8'), 2.26 - 2.41 (2H, m, H-4, 17), 2.21 (3H, s, H-22b), 2.19 - 2.26 (1H, m, H-15<'>), 2.17 (3H, s, H-16b), 1.97 - 2.13 (3H, m, H-5, 8, 11<'>), 1.59 - 1.78 (4H, m, H-7<'>, 11<">, 12<">, 12<'>), 1.41 - 1.54 (2H, m, H-6<'>, 15<">), 1.39 (1H, d, J = 4.3 Hz, H-19<'>), 1.19 (3H, s, H-18), 1.15 - 1.27 (1H, m, J = 6.4 Hz, H-6<">), 1.07 (3H, d, J = 7.0 Hz, H-27), 1.04 (3H, d, J = 6.7 Hz, H-28), 0.95 (3H, s, H-29), 0.92 - 1.00 (1H, m, H-7<">), 0.90 (3H, d, J = 6.7 Hz, H-21), 0.65 (1H, d, J = 4.6 Hz, H-19<">)
¹³C RMN (126MHz, D2O) δ = 208.1 (C-3), 201.7 (C-23), 174.2 (C-16a), 173.8 (C-22a), 160.0 (C-1), 148.0 (C-24), 127.2 (C-24a), 126.2 (C-2), 78.4 (C-22), 77.2 (C-16), 70.8 (C-3'), 70.6 (C-4'), 70.6 (C-2'), 68.6 (C-5'), 67.0 (C-26), 62.6 (C-1'), 51.3 (C-6'), 50.1 (C-17), 47.2 (C-14), 46.3 (C-4), 45.6 (C-13), 45.1 (C-15), 43.0 (C-8), 41.8 (C-5), 34.4 (C-25), 33.2 (C-8'), 32.5 (C-20), 32.4 (C-10), 31.5 (C-12), 26.8 (C-9), 26.8 (C-11), 26.1 (C-19), 22.7 (C-7), 22.5 (C-6), 21.3 (C-16b), 19.9 (C-22b), 18.7 (C-29), 16.7 (C-18), 16.5 (C-27), 12.2 (C-21), 10.0 (C-28)

### ◆ exemple 91: 26-phosphonoacétate de neoboutomellerone

| | |
|---|---|
| 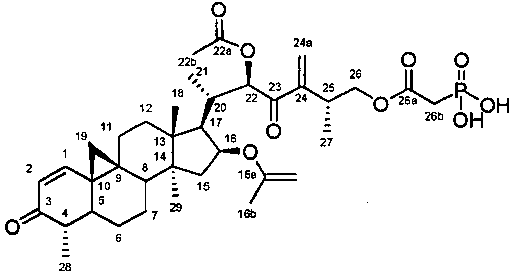 | Formule Chimique : C₃₇H₅₃O₁₀P |
| | Masse Exacte : 688.34 |
| | Poids moléculaire : 688.78 |

Protocole : Le composé 2 (250 mg , 0.44 mmol) est solubilisé dans 40 ml d'acétonitrile, en présence de l'acide phosphonoacétique (185 mg, 1.32 mmol, 3 éq.). La DCC (272 mg, 1.32 mmol, 3 éq.) est rajoutée et le milieu réactionnel est laissé sous agitation à température ambiante. Après conversion du produit de départ et filtration du précipité blanc, l'acétonitrile est évaporé. Le produit est purifié par HPLC préparative sur colonne X-bridge C8, 10 µm, 30 x250 mm, 1 : 220 nm, débit : 40mL/ minutes. Phase mobile : gradient HCl 5mmol, 100% à HCl 5mmol 50% / acétonitrile 50%. Evaporer l'acétonitrile sous pression réduite des fractions concernées et extraire le produit à l'acétate d'éthyle pour obtenir 76 mg (25 %) du produit attendu sous forme de mousse blanche,
¹H NMR (500MHz, DMSO-d₆) δ = 6.97 (1H, d, J = 10.1 Hz, H-1), 6.12 (1H, s, H-24aa), 6.07 (1H, s, H-24ab), 5.89 (1H, d, J = 10.1 Hz, H-2), 5.46 (1H, d, J = 1.5 Hz, H-22), 5.02 (1H, td, J = 7.6 Hz, J = 4.3 Hz, H-16), 4.06 (1H, dd, J = 10.4 Hz, J = 5.8 Hz, H-26<'>), 3.91 (1H, dd, J = 10.7 Hz, J = 7.0 Hz, H-26<">), 2.94 (1H, dq, J = 13.2 Hz, J = 6.7 Hz, H-25), 2.70 (2H, d, J = 21.1 Hz, H-26b), 2.46 - 2.51 (1H, m, H-20), 2.22 (1H, dd, J = 10.8 Hz, J = 7.5 Hz, H-17), 2.10 (3H, s, H-22b), 2.07 - 2.16 (2H, m, H-4, 15<'>), 2.05 (3H, s, H-16b), 1.93 - 2.01 (2H, m, H-11<'>, 8), 1.89 (1H, td, J = 12.5 Hz, J = 3.7 Hz, H-5), 1.48 - 1.69 (4H, m, H-6<'>, 11<">, 12<">, 12<'>), 1.34 - 1.44 (1H, m, H-7<'>), 1.30 (1H, dd, J = 14.3 Hz, J = 3.7 Hz, H-15<">), 1.24 (1H, d, J = 4.6 Hz, H-19<'>), 1.13 (3H, s, H-18), 1.08 - 1.19 (1H, m, H-7<">), 1.04 (3H, d, J = 7.0 Hz, H-27), 0.98 (3H, d, J = 6.7 Hz, H-28), 0.90 (3H, s, H-29), 0.84 - 0.94 (1H, m, H-6<">), 0.79 (3H, d, J = 6.7 Hz, H-21), 0.58 (1H, d, J = 4.3 Hz, H-19<">)
¹³C NMR (126MHz, DMSO-d₆) δ = 200.7 (C-3), 197.2 (C-23), 170.3 (C-22a), 170.0 (C-16a), 154.9 (C-1), 147.2 (C-24), 127.3 (C-2), 125.4 (C-24a), 76.7 (C-22), 75.1 (C-16), 66.8 (C-26), 49.7 (C-17), 48.6, 47.0 (C-14), 46.2 (C-4), 45.4 (C-15, 13), 43.5 (C-8), 42.1, 36.7 (C-26b), 33.2 (C-25), 31.9 (C-20), 31.7 (C-12), 31.6 (C-10), 26.7 (C-11), 26.5 (C-19), 26.0 (C-9), 23.0 (C-7), 22.9 (C-6), 21.5 (C-16b), 20.5 (C-22b), 19.2 (C-29), 17.6 (C-18), 16.6 (C-27), 12.5 (C-21), 10.8 (C-28)

### ◆exemple 92 : 26-(diéthylphosphate)- neoboutomellerone

| | |
|---|---|
| 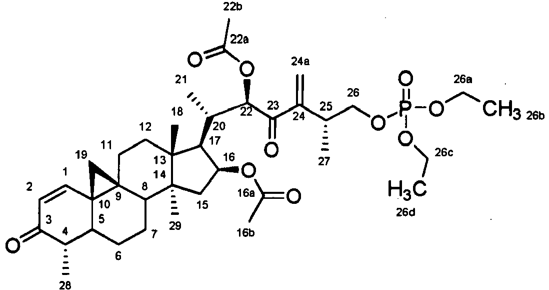 | Formule Chimique : C₃₈H₅₇O₁₀P |
| | Masse Exacte : 704,37 |
| | Poids moléculaire : 704,83 |

Protocole : 300 mg (0.528 mmol) de 2 sont dissous dans 5 ml de DCM, puis 2.3 ml (4 éq, 2.11 mmol) de lutidine sont ajoutés. La réaction est refroidie à 0°C, et le chlorure de phosphoryle (0.89 ml, 2 éq, 1.06 mmol) est ajouté, on laisse la température remonter pendant une nuit. La réaction est hydrolysée avec de l'eau. La phase organique est lavée successivement avec de l'eau, une solution de sulfate de cuivre, de l'eau et de la saumure. Le produit est purifié par CCM préparative en faisant 3 fois par un mélange de 30 % d'acétate d'éthyle dans le cyclohexane puis 3 fois un mélange de 50 % d'acétate d'éthyle dans le cyclohexane. On recueille 43 mg (11 %) du composé 92 (Rf : 0.14 - cyclohexane / acétate d'éthyle 60 / 40).
¹H RMN (500MHz, CD3CN) δ = 6.94 (1H, d, J = 10.1 Hz, H-1), 6.15 (1H, s, H-24a'), 6.00 (1H, s, H-24a"), 5.90 (1H, d, J = 10.1 Hz, H-2), 5.53 (1H, d, J = 2.1 Hz, H-22), 5.08 (1H, dt, J = 7.7 Hz, J = 3.9 Hz, H-16), 3.96 - 4.12 (5H, m, H-26a, 26c, 26<'>), 3.89 (1H, dt, J = 10.1 Hz, J = 6.3 Hz, H-26<">), 3.01 (1H, sxt, J = 6.7 Hz, H-25), 2.54 - 2.63 (1H, m, H-20), 2.30 (1H, dd, J = 11.1 Hz, J = 7.5 Hz, H-17), 2.14 - 2.22 (2H, m, H-4, 15<'>), 2.09 (3H, s, H-22b), 2.03 (3H, s, H-16b), 1.96 - 2.09 (3H, m, H-5, 8, 11<'>), 1.63 - 1.76 (3H, m, H-6<'>, 12<'>, 12<">), 1.52 - 1.62 (1H, m, H-11<">), 1.41 - 1.50 (1H, m, H-7<'>), 1.37 (1H, dd, J = 13.7 Hz, J = 4.6 Hz, H-15<">), 1.27 (6H, td, J = 7.0 Hz, J = 0.9 Hz, H-26b, 26d), 1.24 (1H, d, J = 4.9 Hz, H-19<'>), 1.19 (3H, s, H-18), 1.20 (1H, s, H-7<">), 1.09 (3H, d, J = 7.0 Hz, H-27), 1.03 (3H, d, J = 6.7 Hz, H-28), 0.95 (3H, s, H-29), 0.88 - 0.94 (1H, m, H-6<">), 0.85 (3H, d, J = 7.0 Hz, H-21), 0.58 (1H, d, J = 4.6 Hz, H-19<">)
¹³C RMN (126MHz, CD3CN) δ = 202.4 (C-3), 198.8 (C-23), 171.6 (C-22a), 171.2 (C-16a), 155.5 (C-1), 148.4 (C-24), 128.4 (C-2), 126.1 (C-24a), 78.3 (C-22), 76.7 (C-16), 70.6 (C-26), 64.6 (C-26c, 26a), 51.3 (C-17), 48.4 (C-14), 47.6 (C-4), 46.9 (C-13), 46.7 (C-15), 45.2 (C-8), 43.6 (C-5), 36.2 (C-25), 33.3 (C-20), 33.0 (C-12), 32.7 (C-10), 28.1 (C-11), 27.7 (C-19), 24.3 (C-7, 6), 22.1 (C-16b), 20.9 (C-22b), 20.0 (C-29), 18.3 (C-18), 16.9 (C-27), 16.5 (C-26b, 26d), 13.4 (C-21), 11.3 (C-28)

### 2. Résultats biologiques

Les tests biologiques ont été réalisés selon des protocoles décrits dans les articles suivants : Ausseil F. et al. J. Biomol Screen. 2007, 12, 106-116 et Vandenberghe I. et al. Biochemical Pharmacology 2008, 76, 453-462.

Afin de sélectionner de nouveaux composés inhibiteurs du protéasome, il est nécessaire de mesurer indirectement l'activité de ce protéasome au sein des cellules. Dans ce but, nous avons construit une lignée cellulaire stable, DLD-1 4Ub-Luc, issue d'une lignée de tumeur du colon humaine, produisant une protéine rapportrice chimérique appelée 4Ub-luc. Cette protéine est une fusion entre une étiquette « 4 ubiquitines » et la luciférase Luc. Cette étiquette est capable d'adresser toute protéine qui la comporte vers le protéasome. La protéine 4Ub-Luc est donc efficacement dégradée par le protéasome contrairement à la protéine Luc non fusionnée (« sauvage »). Lorsque la lignée DLD-1 4Ub-Luc est traitée avec un inhibiteur du protéasome, la protéine de fusion est beaucoup moins efficacement dégradée et s'accumule dans la cellule. Cette accumulation est détectée par une augmentation de l'activité luciférase dans l'extrait traité par rapport à l'extrait de cellules non traitées. En guise de contrôle nous vérifions que l'inhibiteur n'a pas d'effet sur l'accumulation de la protéine Luc sauvage produite par la lignée stable DLD-1 RF. Pour les deux lignées et chaque produit nous déterminons un facteur d'induction pour le produit testé (ou la référence) correspondant au rapport entre les activités luciférase mesurées dans les cellules traitées par ce produit (ou la référence) et les cellules traitées avec le solvant seul.

On définit ensuite l'activité du produit par son facteur d'induction relatif (c'est-à-dire le rapport de son facteur d'induction (FI % de luminescence) sur celui de la molécule de référence qui est l'époxomycine (FI = 100%)) (voir les données présentées dans le tableau ci-dessous).

Ainsi, le test mis en place par le groupe Pierre-Fabre est un test cellulaire qui présente l'avantage, par rapport à un essai purement enzymatique, de ne détecter que des produits capables de pénétrer dans la cellule.

### Mode opératoire :

Deux lignées cellulaires sont utilisées pour ce test : DLD-1 RF et DLD-1 4Ub-Luc.

Le milieu de culture utilisé est le suivant :
- MEM
- 5% de sérum foetal de veau
- 5 ml de peni-streptomycine
- 2,5 ml de fungizone
- 10 ml d'α-glutamine

Jour 1 : ensemencer des plaques blanches 96 puits traitées pour la culture, (white view plate réf: 6005181, Perkin Elmer) avec 100 µl de suspension cellulaire à 1.10⁵ c/ml par puits. Incuber 24 heures pour que les cellules adhèrent. Sur chaque plaque 5 colonnes seront ensemencées par les DLD-1 4Ub-Luc et 5 autres par les DLD-1 RF selon le schéma 1 ci-dessous.
Jour 2 : enlever le milieu en retournant les plaques sur de la ouate, puis réaliser le traitement avec les composés de l'invention, le solvant seul ou un composé de référence tel que l'époxomycine. Chaque composé de référence sera utilisé à la concentration de 10⁻⁷ M, alors que les composés de l'invention seront testés aux concentrations de 10⁻⁶, 7,5. 10⁻⁷, 5.10⁻⁷ et 2,5.10⁻⁷ M pour une période de 8 heures.

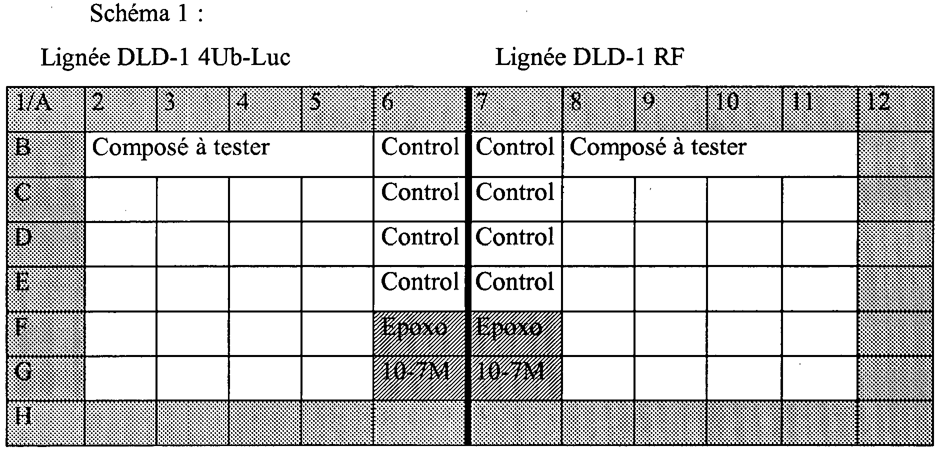

Les colonnes sont associées 2 à 2 (exemple 2 et 8, 3 et 9, 4 et 10 et 5 et 11), et sont traitées avec le même composé. Les colonnes 6 et 7 reçoivent les témoins de l'expérience à savoir le DMSO à 0.1% dans le milieu de culture pour les puits B, C, D, et E des colonnes 6 et 7, et le témoin positif (l'époxomycine à 10⁻⁷ M) dans les puits F et G.

Après incubation, retourner la plaque sur de la ouate pour bien vider les puits, rincer avec 50 µL de PBS pour enlever toute trace du cytotoxique testé puis ajouter 50µl de tampon de lyse passive (Passive Lysis Buffer, 5X, Promega réf :E1941) dilué au 1/5 dans de l'eau. Mettre à agiter de façon énergique 5 à 10 minutes à température ambiante sur l'agitateur de plaque avant de congeler à -20°C.

### Mesure de l'activité luciférase :

La mesure est réalisée à l'aide du kit Luciférase Assay System 10-Pack (Promega réf :E1501).

Le jour de la mesure de l'activité de la luciférase, mettre à décongeler les plaques et attendre qu'elles soient revenues à température ambiante. La température optimale de l'activité de la luciférase se situe entre 20-25°C.
Reconstituer le substrat de la luciférase Firefly en décongelant le tampon et mettre 10 ml du tampon dans le flacon contenant le substrat lyophilisé. Attendre que la solution soit à température ambiante avant de commencer le dosage, l'émission de lumière est quantifiée sur le luminomètre (mettre un papier adhésif blanc sous la plaque avant la lecture au luminomètre).

### Luminomètre (Luminoskan, modèle RT) :

volume injecté : 100µl
temps d'intégration (total time) : 15 secondes

Les résultats obtenus sont les suivants :

| **Composé testé** | **Dose testée (M)** | | | | **Référence : Epoxomicine** |
|---|---|---|---|---|---|
| | 10⁻⁵ | 5.10⁻⁶ | 10⁻⁶ | 5.10⁻⁷ | |
| 1 | 3 | 28 | 45 | 1 | 100% |
| 2 | 4 | 37 | 48 | 2 | 100% |
| 4 | 24 | 84 | 2 | - | 100% |
| 6 | - | 31 | 5 | - | 100% |
| 7 | 21 | 65 | 2 | - | 100% |
| 8 | 19 | 74 | 7 | - | 100% |
| 9 | 44 | 34 | 1 | - | 100% |
| 10 | 53 | 5 | 1 | - | 100% |
| 11 | 48 | 45 | 1 | - | 100% |
| 12 | 15 | 72 | 3 | - | 100% |
| 13 | 60 | 71 | 2 | - | 100% |
| 14 | 77 | 10 | 1 | - | 100% |
| 15 | 74 | 11 | 2 | - | 100% |
| 16 | - | 35 | 1 | - | 100% |
| 17 | 6 | 29 | 26 | 2 | 100% |
| 18 | - | 48 | 12 | 1 | 100% |
| 19 | - | 45 | 40 | 1 | 100% |
| 20 | - | 60 | 42 | 6 | 100% |
| 22 | 1 | 4 | 68 | 8 | 100% |
| 23 | 2 | 7 | 69 | 15 | 100% |
| 24 | 1 | 5 | 70 | 12 | 100% |
| 25 | 60 | 51 | 49 | 19 | 100% |
| 26 | 37 | 54 | 53 | 11 | 100% |
| 27 | - | 103 | 1 | 1 | 100% |
| 35 | 30 | 99 | 1 | - | 100% |
| 36 | 6 | 12 | 1 | - | 100% |
| 47 | 36 | 78 | 7 | - | 100% |
| 49 | 15 | 44 | 19 | - | 100% |
| 51 | 60 | 31 | 1 | - | 100% |
| 56 | 7 | 34 | 21 | - | 100% |
| 58 | 10 | 46 | 8 | - | 100% |
| 60 | 2 | 4 | 71 | 76 | 100% |
| 61 | 6 | 21 | 64 | 23 | 100% |
| 62 | 7 | 49 | 11 | - | 100% |
| 63 | 3 | 11 | 50 | 66 | 100% |
| 64 | 7 | 35 | 22 | - | 100% |
| 65 | 4 | 34 | 17 | 1 | 100% |
| 66 | 2 | 9 | 13 | - | 100% |
| 68 | 4 | 18 | 52 | 1 | 100% |
| 71 | 4 | 11 | 62 | 6 | 100% |
| 74 | 2 | 14 | 60 | - | 100% |
| 82 | 48 | 59 | 2 | - | 100% |
| 88 | 78 | 47 | 1 | - | 100% |
| 89 | 40 | 3 | 1 | - | 100% |
| 90 | 51 | 4 | 1 | - | 100% |
| 92 | 2 | 16 | 43 | 3 | 100% |

| | | | | | |
|---|---|---|---|---|---|
| - signifie que le composé n'a pas été testé pour la dose en question | | | | | |

## Revendications

1. Composé de formule (I) suivante : 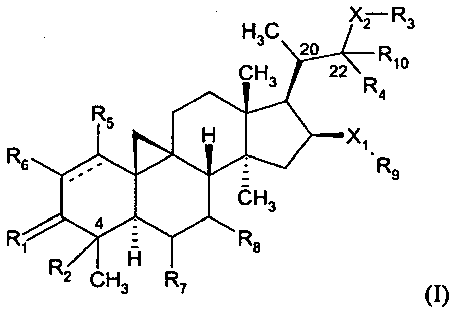 ou un sel pharmaceutiquement acceptable de celui-ci, pour lequel : 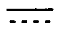 désigne une liaison simple ou une liaison double,
- X₁ et X₂ représentent, indépendamment l'un de l'autre, un atome d'oxygène ou de soufre, et notamment un atome d'oxygène,
- R₁ représente un atome d'oxygène, un atome de soufre ou un groupe N-OR₁₁ ou N-NHCO-NH₂,
- R₂ représente un atome d'hydrogène ou un groupe OR₁₂ ou SR₁₂,
- R₃ représente un atome d'hydrogène, -SO₂R₅₅, -CH₂OCH₂CH₂SiR₆₁R₆₂R₆₃ ou un groupe -CO-(C₁-C₆)alkyle, tel que -COCH₃, ou -CO-(C₂-C₆)alcényle, ledit groupe étant éventuellement substitué par un atome d'halogène ou un groupe COOH ou NR₅₆R₅₇,
- R₄ représente une chaîne : 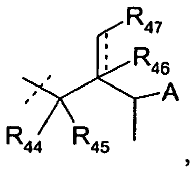 où :
■ 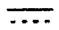 représente une liaison simple ou une liaison double,
■ R₄₄ et R₄₅ forment ensemble un groupe =O,
■ R₄₆ représente un atome d'hydrogène et R₄₇ représente un atome d'hydrogène, un groupe (C₁-C₆)alcoxy, -NH-OR₄₉ ou un hétérocycle à 5 ou 6 chaînons lié au reste de la molécule par l'intermédiaire d'un atome d'azote, tel qu'un imidazolyle, lorsque 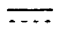 représente une liaison simple, ou R₄₆ est absent et R₄₇ représente un atome d'hydrogène lorsque 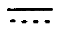 représente une liaison double, et
■ A représente un groupe -CHO, -COOH, -CH₂A₁ ou -CH₂OCH₂A₁ avec :
∘ A₁ représentant un atome d'hydrogène, un atome d'halogène, -OH, -OSO₂R₁₃, -N₃, (C₁-C₆)alcoxy éventuellement substitué par un ou plusieurs groupes -OH ; (C₂-C₆)alcénoxy, -OCH₂OR₆₆, -Z₁C(X)R₁₄, -Z₃C(X)Z₄R₁₆, -NH-OR₁₇, -OSiR₁₉R₂₀R₂₁, -OP(O)(OR₂₂)(OR₂₃), -NR₂₄R₂₅, un hétérocycle à 5 ou 6 chaînons ou un résidu de sucre, un ou plusieurs groupes hydroxy dudit résidu de sucre étant éventuellement substitués par un groupe acétyle ou -P(O)(OH)₂,
∘ R₄₈ et R₄₉ représentant, indépendamment l'un de l'autre, un atome d'hydrogène, un groupe (C₁-C₆)alkyle, aryle ou aryl-(C₁-C₆)alkyle,
∘ R₆₆ représentant un groupe -CO-((C₁-C₆)alkyle) ou (C₁-C₆)alkyle éventuellement substitué par un groupe SiR₆₇R₆₈R₆₉, et
∘ R₆₇, R₆₈ et R₆₉ représentant, indépendamment les uns des autres, un groupe (C₁-C₆)alkyle,
- R₅ et R₆ représentent chacun un atome d'hydrogène lorsque 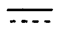 représente une liaison double, ou
R₅ et R₆ représentent chacun, indépendamment l'un de l'autre, un atome d'hydrogène ou un groupe OR₄₈, tel que OH, ou R₅ et R₆ forment ensemble, avec les atomes de carbone qui les portent, un cycle époxyde, lorsque 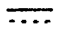
représente une liaison simple,
- R₇ représente un atome d'hydrogène ou un groupe OR₄₉, tel que OH,
- R₈ représente un atome d'hydrogène, ou
R₇ et R₈ forment ensemble, avec les atomes de carbone qui les portent, un cycle époxyde,
- R₉ représente un groupe -CO-(C₁-C₆)alkyle,
- R₁₀ représente un atome d'hydrogène, ou
R₁₀ et R₃ forment ensemble une liaison,
avec :
■ R₁₁, R₂₆, R₂₈, R₃₀, R₃₁, R₃₆, R₃₇, R₄₁, R₄₂, R₄₃, R₄₈, R₄₉ et R₅₀ représentant, indépendamment les uns des autres, un atome d'hydrogène ou un groupe (C₁-C₆)alkyle, (C₂-C₆)alcényle, aryle ou aryl-(C₁-C₆)alkyle,
■ R₁₂ représentant un atome d'hydrogène ou un groupe (C₁-C₆)alkyle ou (C₂-C₆)alcényle, et notamment un atome d'hydrogène,
■ R₁₃ et R₅₅ représentent, indépendamment l'un de l'autre, un groupe -OH, (C₁-C₆)alcoxy, aryle, -NR₃₀R₃₁, (C₁-C₆)alkyl-aryle, ou un groupe (C₁-C₆)alkyle éventuellement substitué par un groupe -NR₃₀R₃₁,
■ R₁₄ représentant un groupe (C₁-C₆)alkyle, (C₂-C₆)alcényle, aryle, (C₁-C₆)alkyl-aryle ou aryl-(C₁-C₆)alkyle, ledit groupe étant éventuellement substitué par un groupe choisi parmi un atome d'halogène, un groupe-NR₃₂-[(CH₂)ₐ-NR₃₃]_{d}-[(CH₂)_{b}-NR₃₄-(CH₂)_{c}-N-R₃₅]ₑ-R₅₂, -P(O)(OH)₂ ou -COOH, avec a, b et c représentant un nombre entier compris entre 1 et 5 et d et e représentant chacun 0 ou 1,
■ R₁₅ et R₁₆ représentant, indépendamment l'un de l'autre, un atome d'hydrogène ou un groupe (C₁-C₆)alkyle, (C₂-C₆)alcényle, aryle, (C₁-C₆)alkyl-aryle ou aryl-(C₁-C₆)alkyle, ledit groupe étant éventuellement substitué par un groupe choisi parmi un atome d'halogène, un groupe -NR₃₂-[(CH₂)ₐ-NR₃₃]_{d}-[(CH₂)_{b}-NR₃₄-(CH₂)_{c}-N-R₃₅]ₑ-R₅₂ ou -COOH, avec a, b, c, d et e tels que définis ci-dessus,
■ R₁₇ et R₁₈ représentant, indépendamment l'un de l'autre, un atome d'hydrogène ou un groupe (C₁-C₆)alkyle, (C₂-C₆)alcényle, aryle ou aryl-(C₁-C₆)alkyle,
■ R₁₉, R₂₀, R₂₁, R₅₈, R₅₉, R₆₀, R₆₁, R₆₂ et R₆₃ représentant, indépendamment les uns des autres, un groupe (C₁-C₆)alkyle, (C₂-C₆)alcényle ou aryle,
■ R₂₂ et R₂₃, identiques ou différents, et notamment identiques, représentant un atome d'hydrogène ou un groupe (C₁-C₆)alkyle ou (C₂-C₆)alcényle, ledit groupe étant éventuellement substitué par un groupe -OC(O)-(C₁-C₆)alkyle, NR₃₆R₃₇ et -N⁺R₃₈R₃₉R₄₀,
ou R₂₂ et R₂₃ formant ensemble, avec les atomes d'oxygène qui les portent et l'atome de phosphore, un cycle, notamment à 5 ou 6 chaînons,
■ R₂₄ et R₂₅, représentant, indépendamment l'un de l'autre, un atome d'hydrogène ou un groupe -CO-(C₁-C₆)alkyle, -CO-(C₂-C₆)alcényle, (C₂-C₆)alcényle, (C₃-C₇)cycloalkyle ou (C₁-C₆)alkyle éventuellement substitué par un groupe NR₄₁R₄₂, ou
R₂₄ et R₂₅ formant ensemble, avec l'atome d'azote qui les porte, un hétérocycle à 5 ou 6 chaînons, ledit hétérocycle pouvant comporter, en plus de l'atome d'azote portant R₂₄ et R₂₅, un ou plusieurs hétéroatomes choisis parmi l'atome d'azote, d'oxygène et de soufre, et étant éventuellement substitué par un groupe (C₁-C₆)alkyle,
■ R₂₇ représentant un groupe aryle, (C₁-C₆)alkyle ou (C₂-C₆)alcényle, ledit groupe étant éventuellement substitué par un ou plusieurs atomes d'halogène,
■ R₂₉ représentant un groupe (C₁-C₆)alkyle, (C₂-C₆)alcényle, aryle ou aryl-(C₁-C₆)alkyle,
■ R₃₂, R₃₃, R₃₄, R₃₅, R₅₂, R₅₃, R₅₄, R₅₆ et R₅₇ représentant, indépendamment les uns des autres, un atome d'hydrogène ou un groupe (C₁-C₆)alkyle, (C₂-C₆)alcényle, -CO-(C₁-C₆)alkyle, -CO-(C₂-C₆)alcényle, -CO₂-(C₁-C₆)alkyle ou -CO₂-(C₂-C₆)alcényle,
■ R₃₈, R₃₉ et R₄₀ représentant, indépendamment les uns des autres, un groupe (C₁-C₆)alkyle ou (C₂-C₆)alcényle,
■ X représentant O, S ou NR₅₀, et notamment O,
■ Z₁, Z₂, Z₃ et Z₄ représentant, indépendamment les uns des autres, O ou NR₄₃, ou Z₂R₁₅ et/ou Z₄R₁₆ représentant, indépendamment l'un de l'autre, un hétérocycle à 5 ou 6 chaînons éventuellement substitué par un groupe (C₁-C₆)alkyle, l'hétérocycle comportant au moins un atome d'azote par lequel il est lié au reste de la molécule.

2. Composé selon la revendication 1, **caractérisé en ce que** R₁ est choisi parmi un atome d'oxygène, un groupe -N-OH, -N-OMe, -N-OBn et -N-NHCO-NH₂, et notamment est un atome d'oxygène.

3. Composé selon l'une quelconque des revendications 1 et 2, **caractérisé en ce que** R₂ représente un atome d'hydrogène.

4. Composé selon l'une quelconque des revendications 1 à 3, **caractérisé en ce que** R₅ et R₆ représentent chacun un atome d'hydrogène et 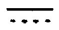 représente une liaison double.

5. Composé selon l'une quelconque des revendications 1 à 4, **caractérisé en ce que** R₇ et R₈ représentent chacun un atome d'hydrogène.

6. Composé selon l'une quelconque des revendications 1 à 5, **caractérisé en ce que** R₉ représente un groupe -COCH₃.

7. Composé selon l'une quelconque des revendications 1 à 6, **caractérisé en ce que** X₂-R₃ représente un groupe OH ou OC(O)CH₃ et R₁₀ représente un atome d'hydrogène ou R₃ et R₁₀ forment ensemble une liaison.

8. Composé selon l'une quelconque des revendications 1 à 7, **caractérisé en ce qu'**il répond à la formule (Ia) ou (Ib) suivante : 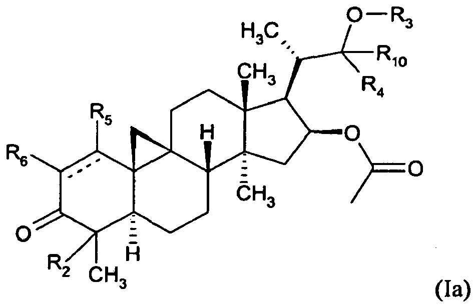 ou 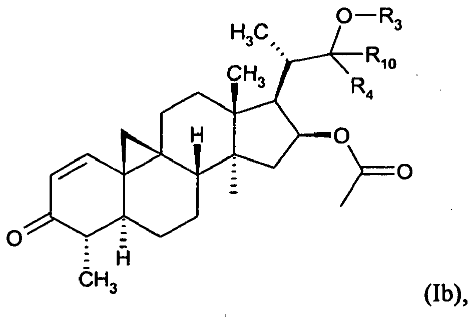 avec R₂, R₃, R₄, R₅, R₆ et R₁₀ tels que définis à la revendication 1.

9. Composé selon l'une quelconque des revendications 1 à 8, **caractérisé en ce que** R₄ représente une chaîne : 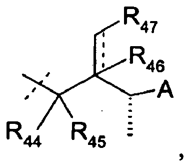 avec R₄₄ à R₄₇ et A tels que définis à la revendication 1.

10. Composé selon l'une quelconque des revendications 1 à 9, **caractérisé en ce qu'**il est choisi parmi :

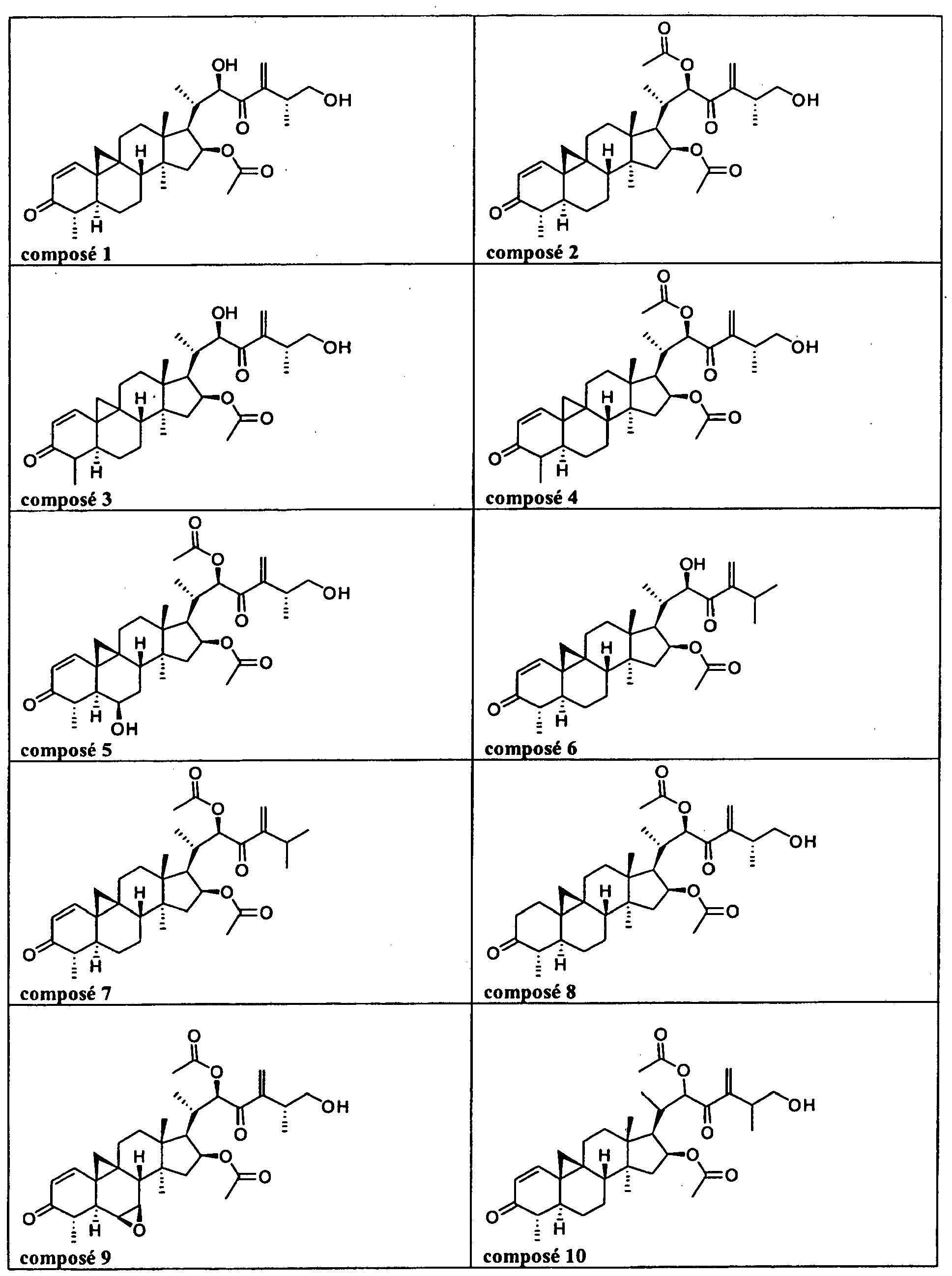

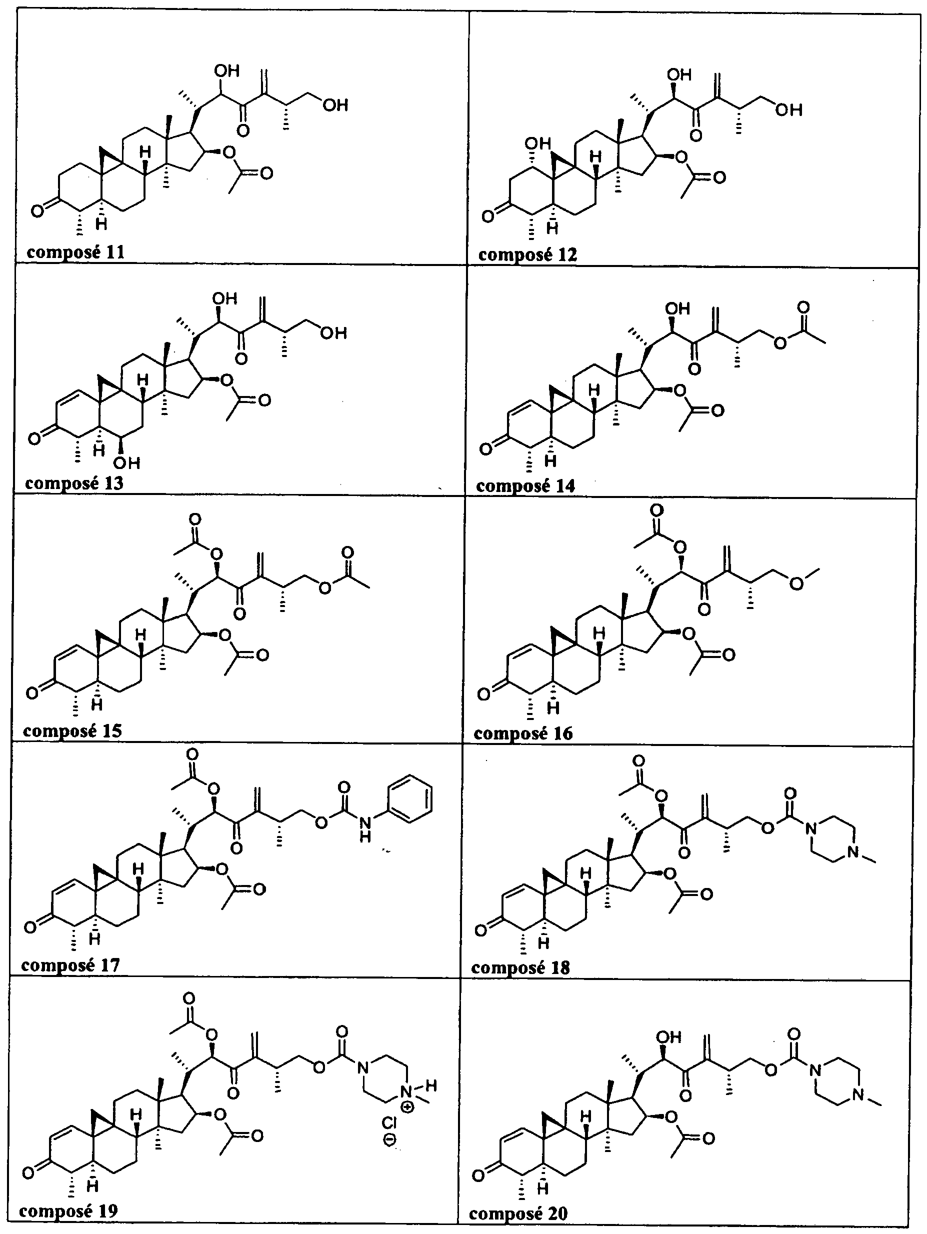

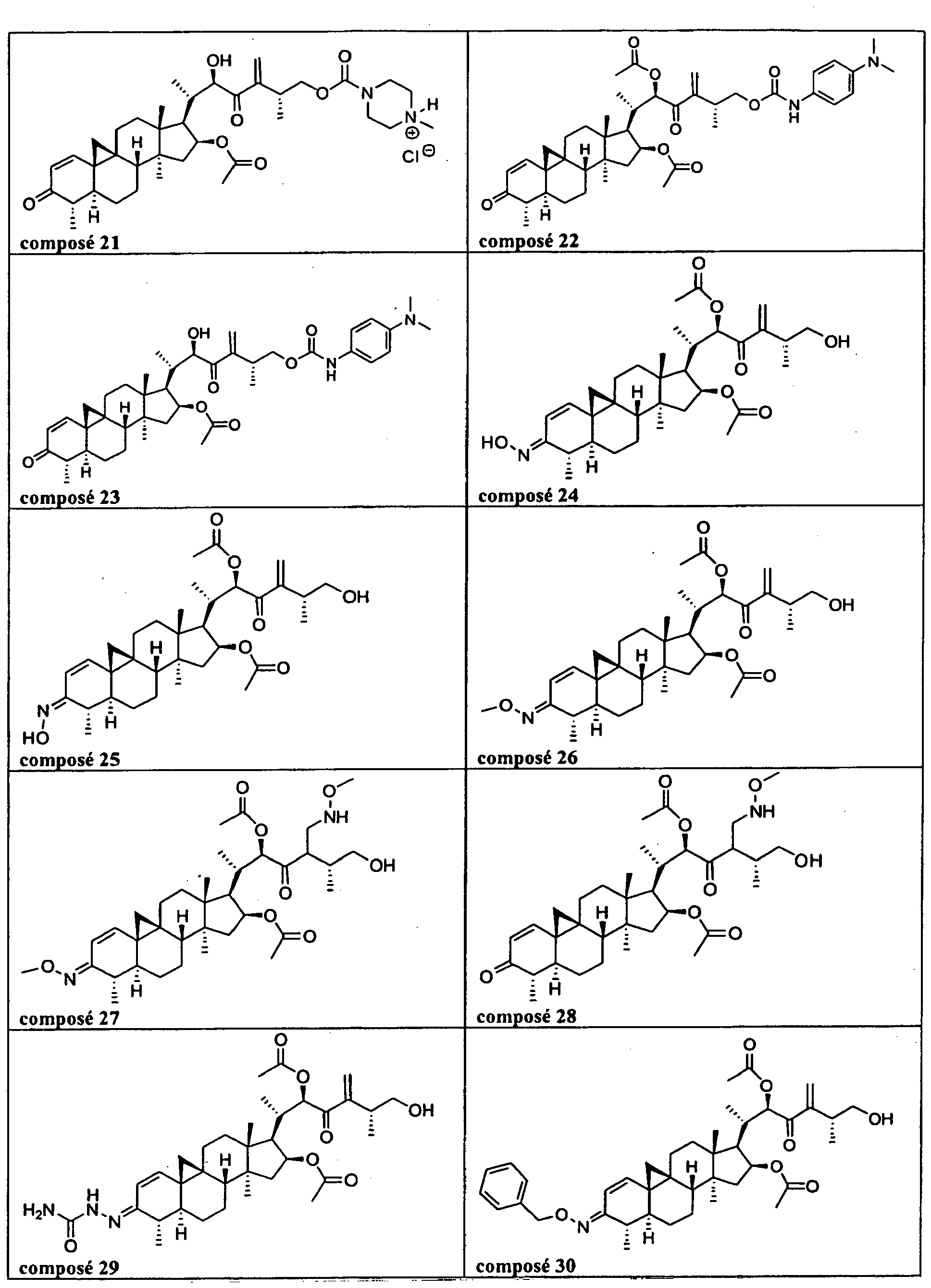

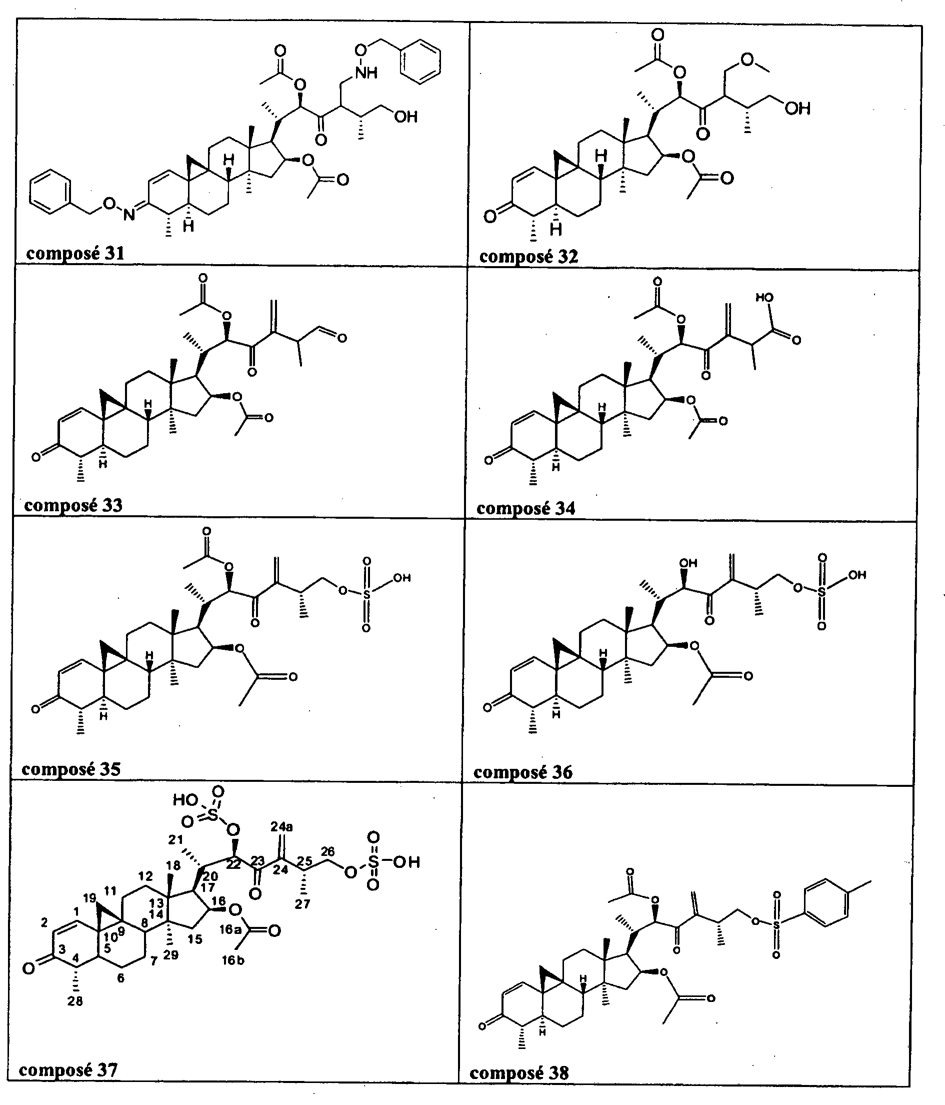

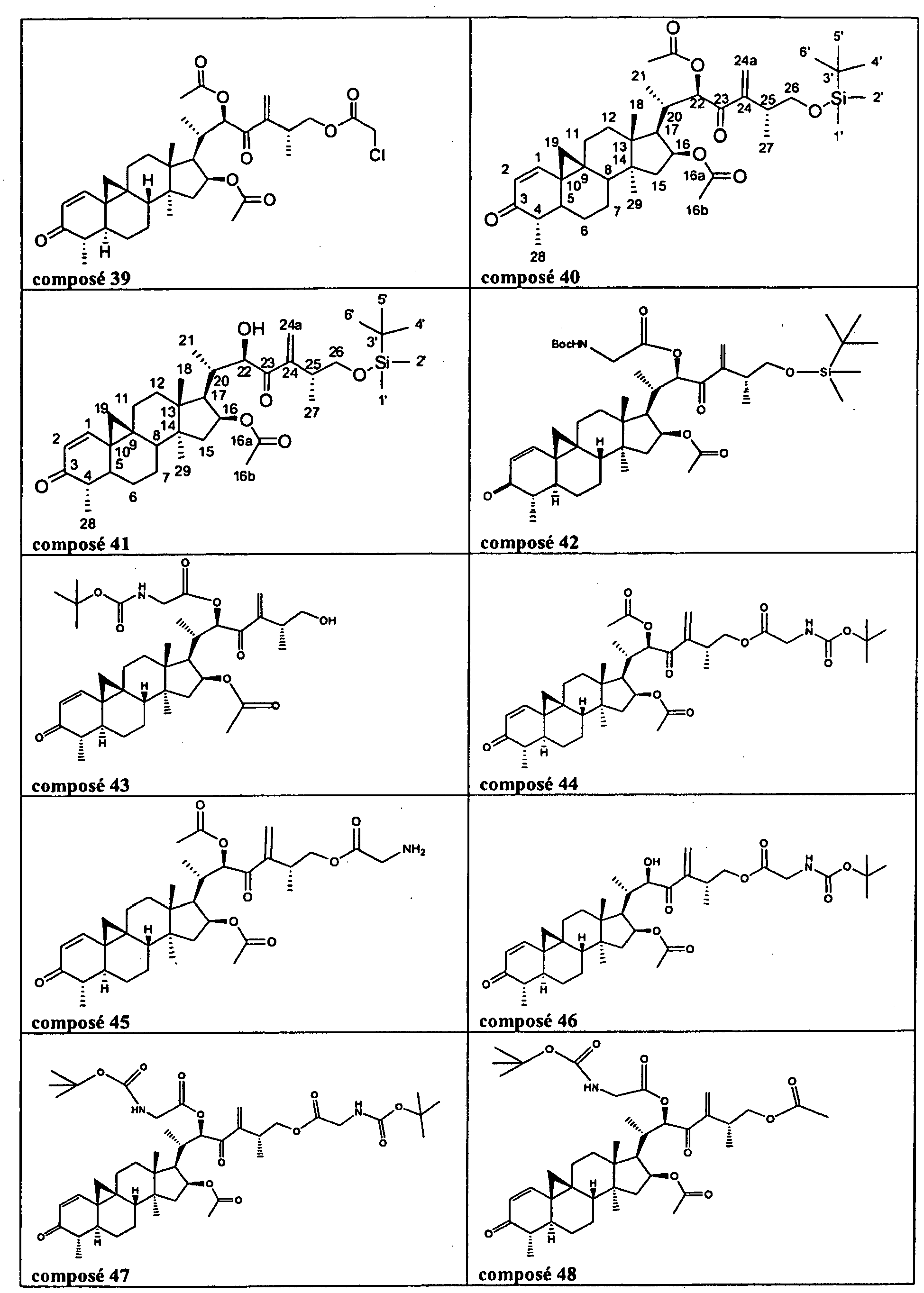

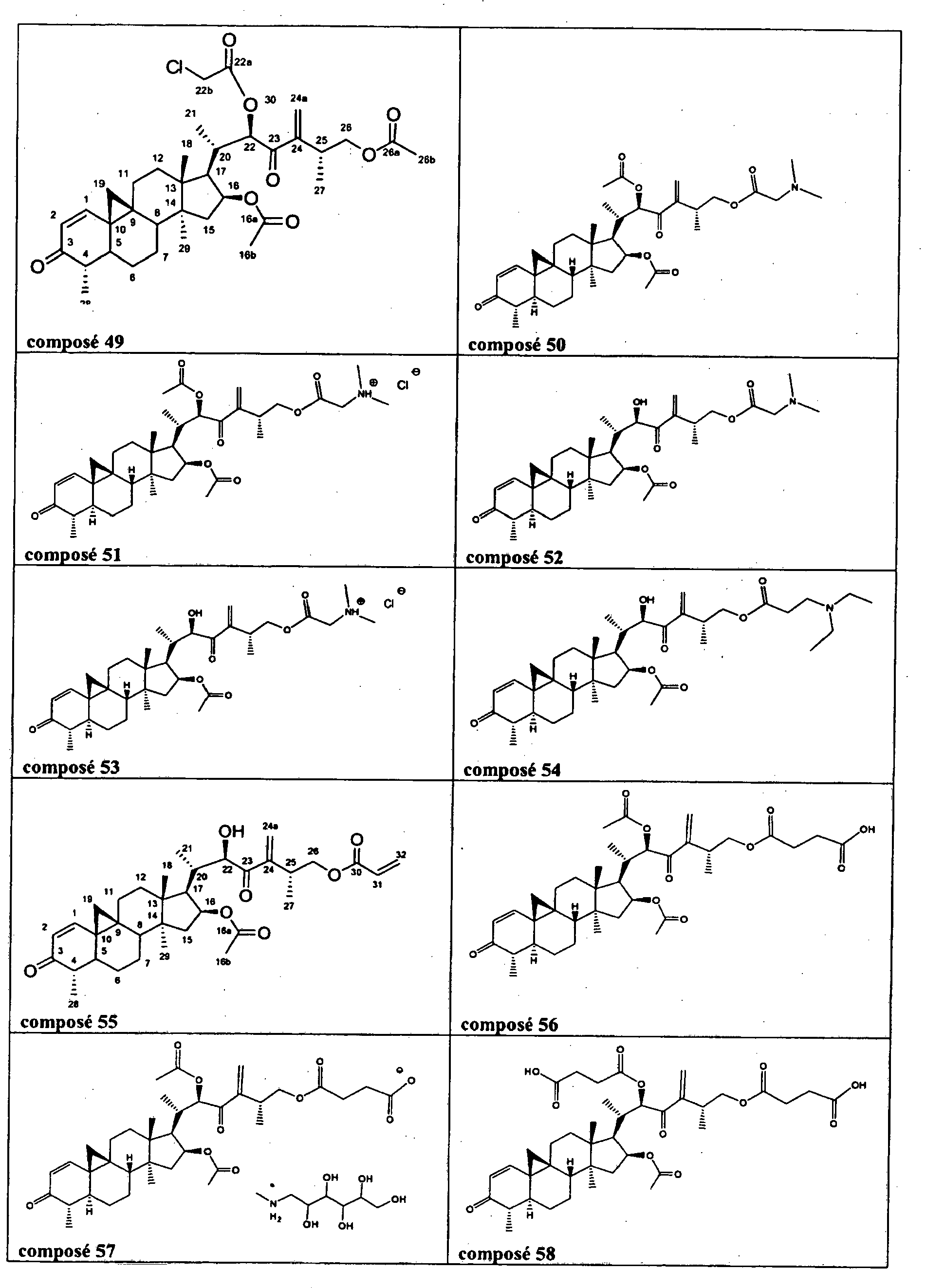

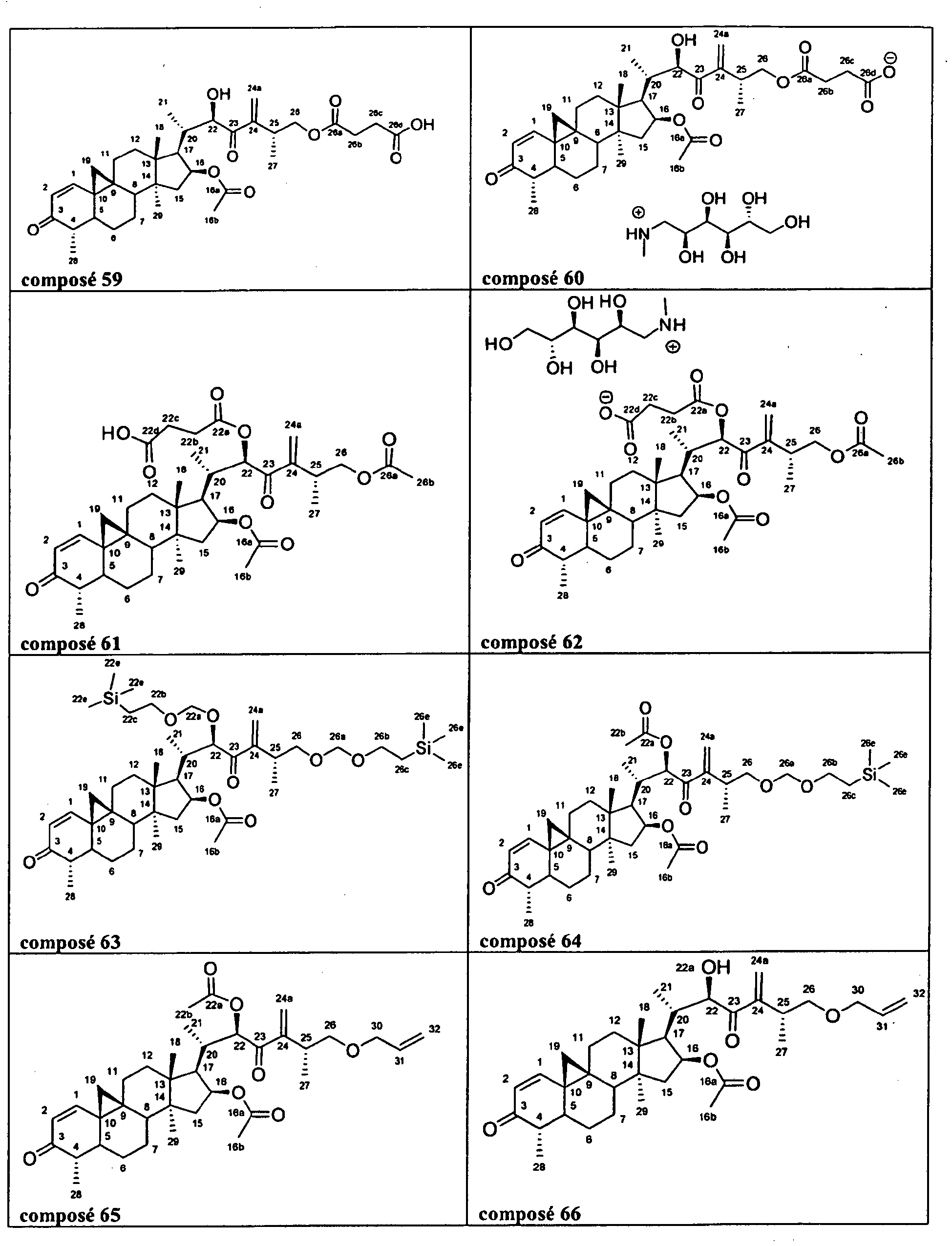

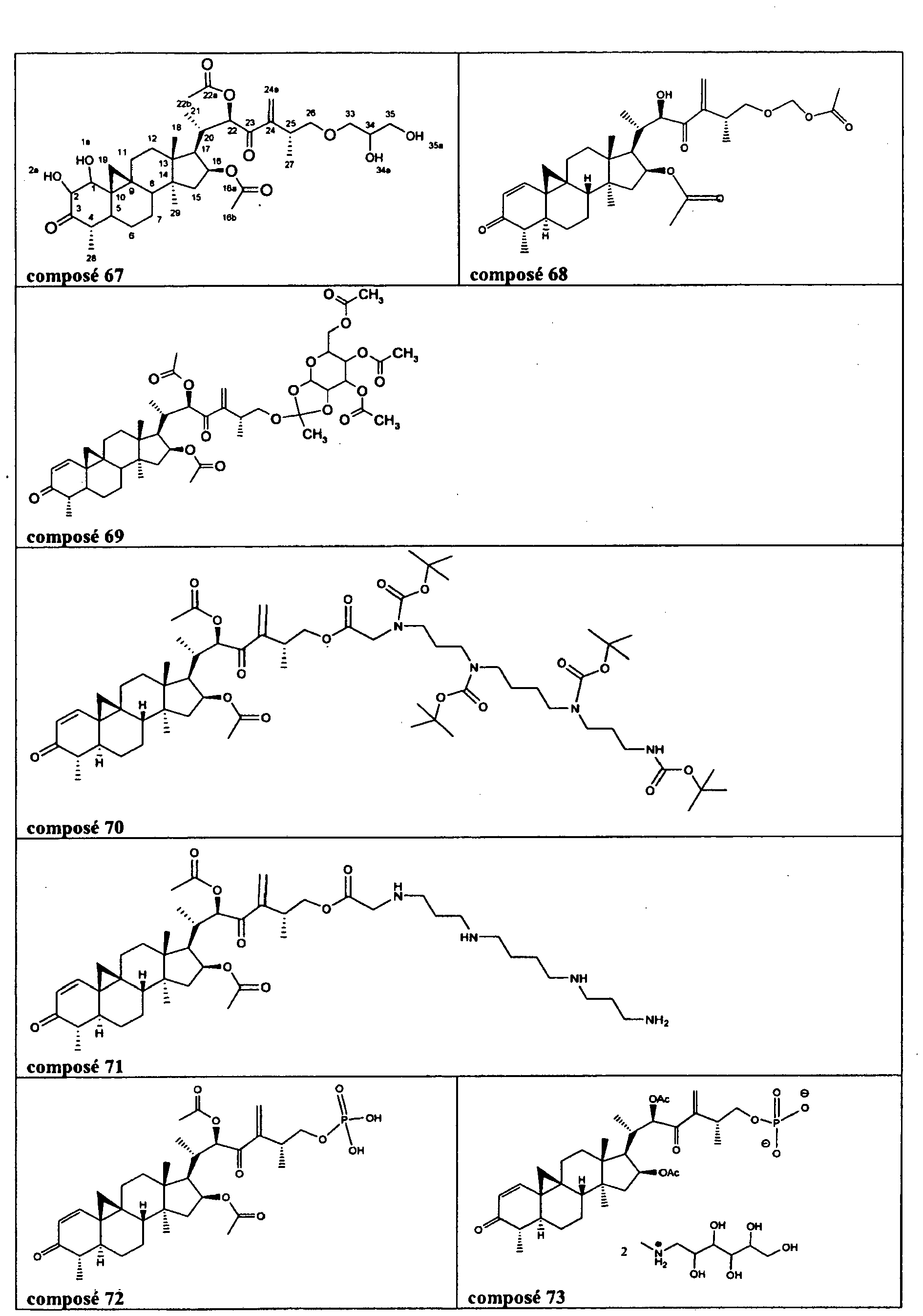

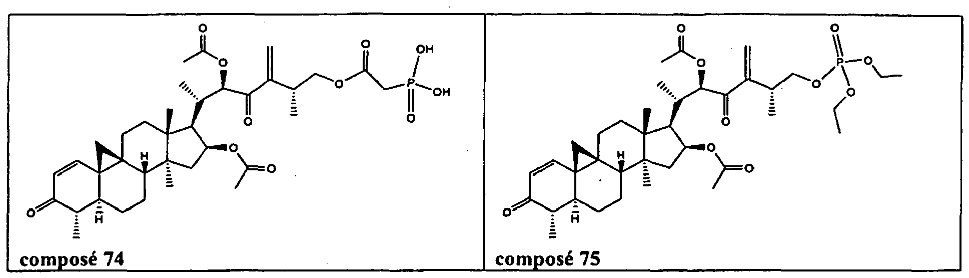

11. Composé selon l'une quelconque des revendications 1 à 10 pour son utilisation en tant que médicament, destiné notamment au traitement d'une maladie proliférative telle qu'un cancer.

12. Composition pharmaceutique comprenant au moins un composé selon l'une quelconque des revendications 1 à 10 et au moins un véhicule pharmaceutiquement acceptable.

13. Composition pharmaceutique selon la revendication 12, **caractérisée en ce qu'**elle comprend en outre au moins un autre principe actif, tel qu'un un agent anticancéreux.

14. Composition pharmaceutique comprenant :
(i) au moins un composé selon l'une quelconque des revendications 1 à 10, et
(ii) au moins un autre principe actif, tel qu'un agent anticancéreux,
en tant que produits de combinaison pour une utilisation simultanée, séparée ou étalée dans le temps.

15. Composition pharmaceutique selon l'une quelconque des revendications 12 à 14 pour son utilisation en tant que médicament, destiné notamment au traitement d'une maladie proliférative telle qu'un cancer.

## Patentansprüche

1. Verbindung der folgenden Formel (I): 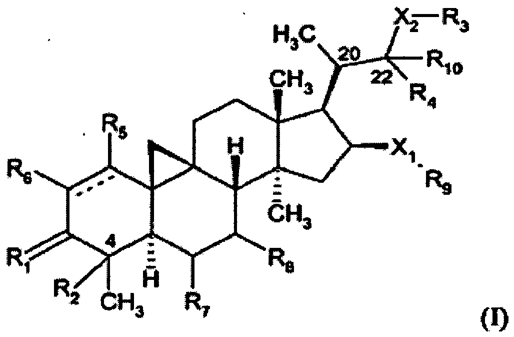 oder ein pharmazeutisch annehmbares Salz von dieser, für welche: 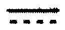 eine Einfachbindung oder eine Doppelbindung bezeichnet,
- X₁ und X₂ unabhängig voneinander für ein Sauerstoff- oder Schwefelatom und insbesondere für ein Sauerstoffatom stehen;
- R₁ für ein Sauerstoffatom, ein Schwefelatom oder eine Gruppe N-OR₁₁ oder N-NHCO-NH₂ steht,
- R₂ für ein Wasserstoffatom oder eine Gruppe OR₁₂ oder SR₁₂ steht,
- R₃ für ein Wasserstoffatom, -SO₂R₅₅, -CH₂OCH₂CH₂SiR₆,R₆₂R₆₃ oder eine Gruppe -CO-(C₁-C₆)-Alkyl, wie -COCH₃, oder -CO-(C₂-C₆)-Alkenyl steht, wobei die Gruppe gegebenenfalls durch ein Halogenatom oder eine Gruppe COOH oder NR₅₆R₅₇ substituiert ist,
- R₄ für eine Kette: 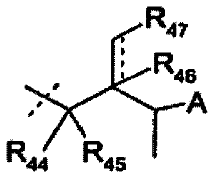 steht, worin:
■ 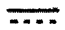 für eine Einfachbindung oder eine Doppelbindung steht,
■ R₄₄ und R₄₅ zusammen eine Gruppe =O bilden,
■ R₄₆ für ein Wasserstoffatom steht und R₄₇ für ein Wasserstoffatom, eine Gruppe (C₁-C₆)-Alkoxy, -NH-OR₄₉ oder einen Heterocyclus mit 5 oder 6 Ringatomen, der mit dem Rest des Moleküls durch das Zwischenglied eines Stickstoffatoms verbunden ist, wie ein Imidazolyl, steht, wenn 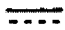 für eine Einfachbindung steht, oder R₄₆ nicht vorhanden ist und R₄₇ für ein Wasserstoffatom steht, wenn 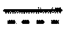 für eine Doppelbindung steht, und
■ A für eine Gruppe -CHO, -COOH, -CH₂A₁ oder -CH₂OCH₂A₁ steht, worin:
∘ A₁ für ein Wasserstoffatom, ein Halogenatom, -OH, -OSO₂R₁₃, -N₃, (C₁-C₆)-Alkoxy, das gegebenenfalls durch eine oder mehrere Gruppen OH substituiert ist; (C₂-C₆)-Alkenoxy, -OCH₂OR₆₆, -Z₁C(X)R₁₄, -Z₃C(X)Z₄R₁₆, -NH-OR₁₇, -OSiR₁₉R₂₀R₂₁, -OP(O)(OR₂₂)(OR₂₃), -NR₂₄R₂₅, einen Heterocyclus mit 5 oder 6 Ringatomen oder einen Zuckerrest, wobei eine oder mehrere Hydroxygruppen des Zuckerrests gegebenenfalls durch eine Acetylgruppe oder -P(O)(OH)₂ substituiert sind, steht,
∘ R₄₈ und R₄₉ unabhängig voneinander für ein Wasserstoffatom, eine (C₁-C₆)-Alkyl-, Aryl- oder Aryl-(C₁-C₆)-alkylgruppe stehen,
∘ R₆₆ für eine -CO-((C₁-C₆)-Alkyl)- oder (C₁-C₆)-Alkylgruppe, die gegebenenfalls durch eine Gruppe SiR₆₇R₆₈R₆₉ substituiert ist, steht und
∘ R₆₇, R₆₈ und. R₆₉ unabhängig voneinander für eine (C₁-C₆)-Alkylgruppe stehen,
- R₅ und R₆ jeweils für ein Wasserstoffatom stehen, wenn 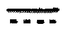 für eine Doppelbindung steht, oder R₅ und R₆ jeweils unabhängig voneinander für ein Wasserstoffatom oder eine Gruppe OR₄₈, wie OH, stehen oder R₅ und R₆ zusammen mit den Kohlenstoffatomen, die diese tragen, einen Epoxyd-Cyclus bilden, wenn 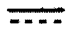 für eine Einfachbindung steht,
- R₇ für ein Wasserstoffatom oder eine Gruppe OR₄₉, wie OH, steht,
- R₈ für ein Wasserstoffatom steht oder
R₇ und R₈ zusammen mit den Kohlenstoffatomen, die diese tragen, einen Epoxyd-Cyclus bilden,
- R₉ für eine Gruppe -CO-(C₁-C₆)-Alkyl steht,
- R₁₀ für ein Wasserstoffatom steht oder
R₁₀ und R₃ zusammen eine Bindung bilden,
wobei:
■ R₁₁, R₂₆, R₂₈, R₃₀, R₃₁, R₃₆, R₃₇, R₄₁, R₄₂, R₄₃, R₄₈. R₄₉ und R₅₀ unabhängig voneinander für ein Wasserstoffatom oder eine (C₁-C₆)-Alkyl-, (C₂-C₆)-Alkenyl-, Aryl- oder Aryl-(C₁-C₆)-alkylgruppe stehen,
■ R₁₂ für ein Wasserstoffatom oder eine (C₁-C₆)-Alkyl- oder (C₂-C₆)-Alkenylgruppe und insbesondere für ein Wasserstoffatom steht,
■ R₁₃ und R₅₅ unabhängig voneinander für eine Gruppe -OH, (C₁-C₆)-Alkoxy, Aryl, -NR₃₀R₃₁, (C₁-C₆)-Alkylaryl oder eine (C₁-C₆)-Alkylgruppe, die gegebenenfalls durch eine Gruppe -NR₃₀R₃₁ substituiert ist, stehen,
■ R₁₄ für eine (C₁-C₆)-Alkyl-, (C₂-C₆)-Alkenyl-, Aryl-, (C₁-C₆)-Alkylaryl- oder Aryl-(C₁-C₆)-alkylgruppe steht, wobei die Gruppe gegebenenfalls substituiert ist durch eine Gruppe, ausgewählt aus einem Halogenatom, einer Gruppe -NR₃₂-[(CH₂)ₐ-NR₃₃]_{d}-[(CH₂)_{b}-NR₃₄-(CH₂)_{c}-N-R₃₅]ₑ-R₅₂, -P(O)(OH)₂ oder -COOH, wobei a, b und c für eine ganze Zahl zwischen 1 und 5 stehen und d und e jeweils für 0 oder 1 stehen,
■ R₁₅ und R₁₆ unabhängig voneinander für ein Wasserstoffatom oder eine (C₁-C₆)-Alkyl-, (C₂-C₆)-Alkenyl-, Aryl-, (C₁-C₆)-Alkylaryl- oder Aryl-(C₁-C₆)-alkylgruppe stehen, wobei die Gruppe gegebenenfalls substituiert ist durch eine Gruppe, ausgewählt aus einem Halogenatom, einer Gruppe -NR₃₂-[(CH₂)ₐ-NR₃₃]_{d}-[(CH₂)_{b}-NR₃₄-(CH₂)_{c}-N-R₃₅]ₑ-R₅₂ oder -COOH, wobei a, b, c, d und e wie oben definiert sind,
■ R₁₇ und R₁₈ unabhängig voneinander für ein Wasserstoffatom oder eine (C₁-C₆)-Alkyl-, (C₂-C₆)-Alkenyl-, Aryl- oder Aryl-(C₁-C₆)-alkylgruppe stehen,
■ R₁₉, R₂₀, R₂₁, R₅₈, R₅₉, R₆₀, R₆₁, R₆₂ und R₆₃ unabhängig voneinander für eine (C₁-C₆)-Alkyl-, (C₂-C₆)-Alkenyl- oder Arylgruppe stehen,
■ R₂₂ und R₂₃, die gleich oder verschieden und insbesondere gleich sind, für ein Wasserstoffatom oder eine (C₁-C₆)-Alkyl- oder (C₂-C₆)-Alkenylgruppe stehen, wobei die Gruppe gegebenenfalls durch eine Gruppe -OC(O)-(C₁-C₆)-Alkyl, NR₃₆R₃₇ und -N⁺R₃₈R₃₉R₄₀ substituiert ist,
oder R₂₂ und R₂₃ zusammen mit den Sauerstoffatomen, die sie tragen, und dem Phosphoratom einen Cyclus, insbesondere mit 5 oder 6 Ringatomen, bilden,
■ R₂₄ und R₂₅ unabhängig voneinander für ein Wasserstoffatom oder eine Gruppe -CO-(C₁-C₆)-Alkyl, -CO-(C₂-C₆)-Alkenyl, (C₂-C₆)-Alkenyl, (C₃-C₇)-Cycloalkyl oder (C₁-C₆)-Alkyl, die gegebenenfalls durch eine Gruppe NR₄₁R₄₂ substituiert ist, stehen oder
R₂₄ und R₂₅ zusammen mit dem Stickstoffatom, das sie trägt, einen Heterocyclus mit 5 oder 6 Ringatomen bilden, wobei der Heterocyclus außer dem Stickstoffatom, das R₂₄ und R₂₅ trägt, ein oder mehrere Heteroatome, ausgewählt unter einem Stickstoff-, Sauerstoff und Schwefelatom, umfassen kann und gegebenenfalls durch eine (C₁-C₆)-Alkylgruppe substituiert ist,
■ R₂₇ für eine Aryl-, (C₁-C₆-)Alkyl- oder (C₂-C₆)-Alkenylgruppe steht, wobei die Gruppe gegebenenfalls durch ein oder mehrere Halogenatome substituiert ist,
■ R₂₉ für eine (C₁-C₆)-Alkyl-, (C₂-C₆)-Alkenyl-, Aryl- oder Aryl-(C₁-C₆)-alkylgruppe steht,
■ R₃₂, R₃₃, R₃₄, R₃₅, R₅₂, R₅₃, R₅₄, R₅₆ und R₅₇ unabhängig voneinander für ein Wasserstoffatom oder eine (C₁-C₆)-Alkyl-, (C₂-C₆)-Alkenyl-, -CO-(C₁-C₆)-Alkyl-, -CO-(C₂-C₆)-Alkenyl-, -CO₂-(C₁-C₆)-Alkyl- oder -CO₂-(C₂-C₆)-Alkenylgruppe stehen,
■ R₃₈, R₃₉ und R₄₀ unabhängig voneinander für eine (C₁-C₆)-Alkyl- oder (C₂-C₆)-Alkenylgruppe stehen,
■ X für O, S oder NR₅₀, und insbesondere für O, steht,
■ Z₁, Z₂, Z₃ und Z₄ unabhängig voneinander für O oder NR₄₃ stehen oder Z₂R₁₅ und/oder Z₄R₁₆ unabhängig voneinander für einen Heterocyclus mit 5 oder 6 Ringatomen, der gegebenenfalls durch eine (C₁-C₆)-Alkylgruppe substituiert ist, stehen, wobei der Heterocyclus mindestens ein Stickstoffatom umfasst, durch welches er an den Rest des Moleküls gebunden ist.

2. Verbindung nach Anspruch 1, **dadurch gekennzeichnet, dass** R₁ unter einem Sauerstoffatom, einer Gruppe -N-OH, -N-OMe, -N-OBn und -N-NHCO-NH₂ ausgewählt ist und insbesondere ein Sauerstoffatom ist.

3. Verbindung nach einem der Ansprüche 1 und 2, **dadurch gekennzeichnet, dass** R₂ für ein Wasserstoffatom steht.

4. Verbindung nach einem der Ansprüche 1 bis 3, **dadurch gekennzeichnet, dass** R₅ und R₆ jeweils für ein Wasserstoffatom stehen und 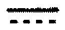 für eine Doppelbindung steht.

5. Verbindung nach einem der Ansprüche 1 bis 4, **dadurch gekennzeichnet, dass** R₇ und R₈ jeweils für ein Wasserstoffatom stehen.

6. Verbindung nach einem der Ansprüche 1 bis 5, **dadurch gekennzeichnet, dass** R₉ für eine Gruppe -COCH₃ steht.

7. Verbindung nach einem der Ansprüche 1 bis 6, **dadurch gekennzeichnet, dass** X₂-R₃ für eine Gruppe OH oder OC(O)CH₃ steht und R₁₀ für ein Wasserstoffatom steht oder R₃ und R₁₀ zusammen eine Bindung bilden.

8. Verbindung nach einem der Ansprüche 1 bis 7, **dadurch gekennzeichnet, dass** sie der folgenden Formel (la) oder (Ib) entspricht: 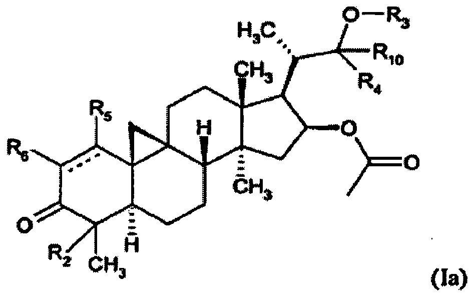 oder 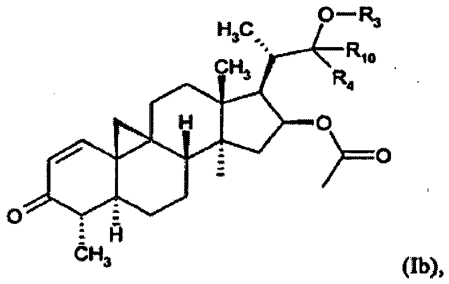 wobei R₂, R₃, R₄, R₅, R₆ und R₁₀ wie in Anspruch 1 definiert sind.

9. Verbindung nach einem der Ansprüche 1 bis 8, **dadurch gekennzeichnet, dass** R₄ für eine Kette: 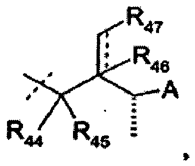 wobei R₄₄ bis R₄₇ und A wie in Anspruch 1 definiert sind, steht.

10. Verbindung nach einem der Ansprüche 1 bis 9, **dadurch gekennzeichnet, dass** sie ausgewählt ist unter:

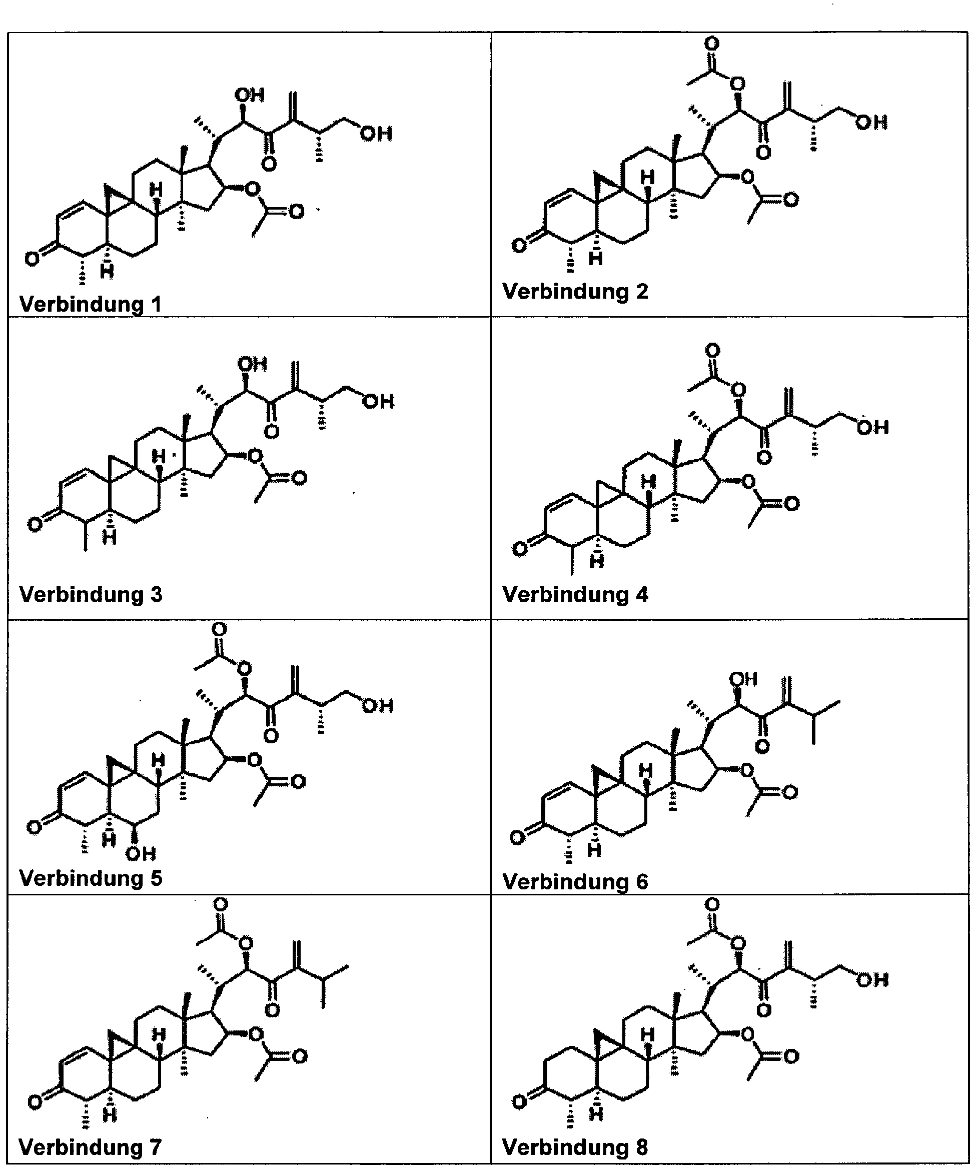

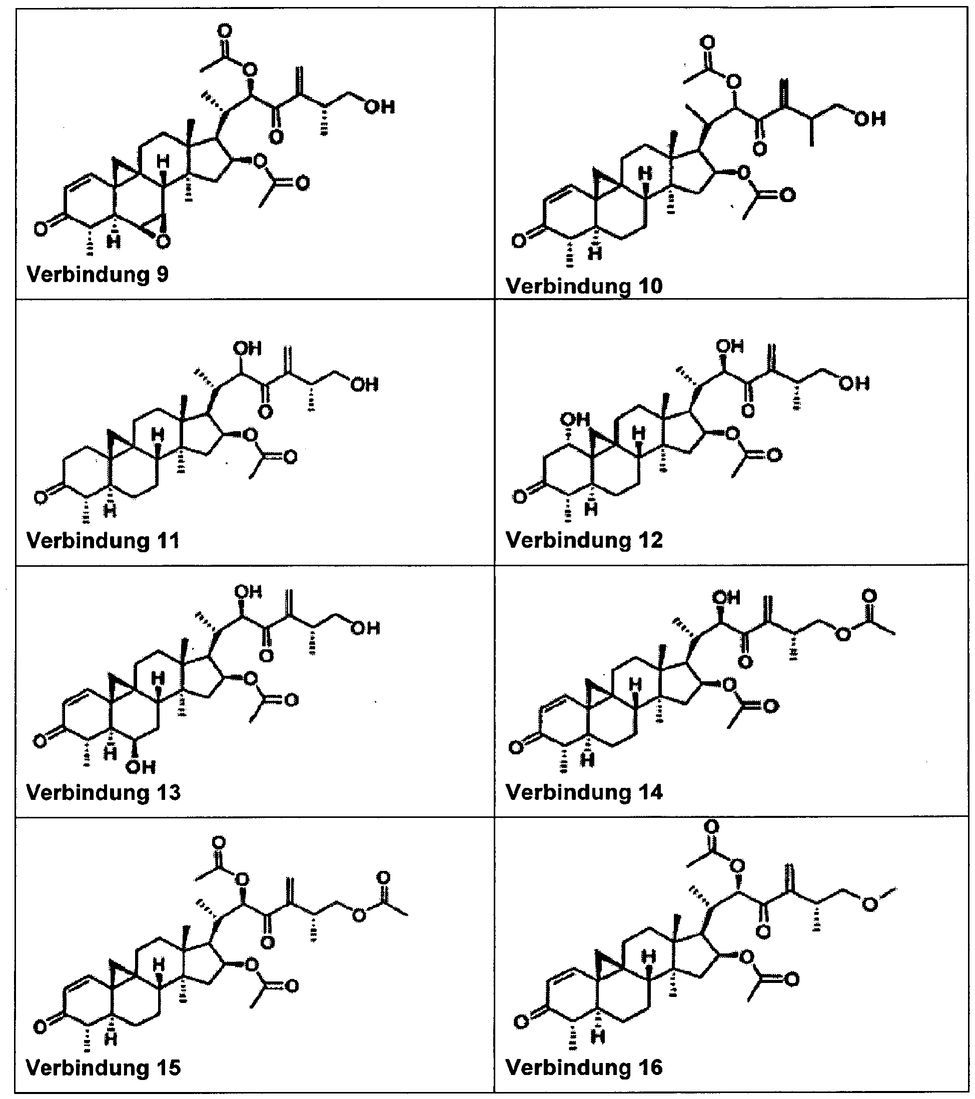

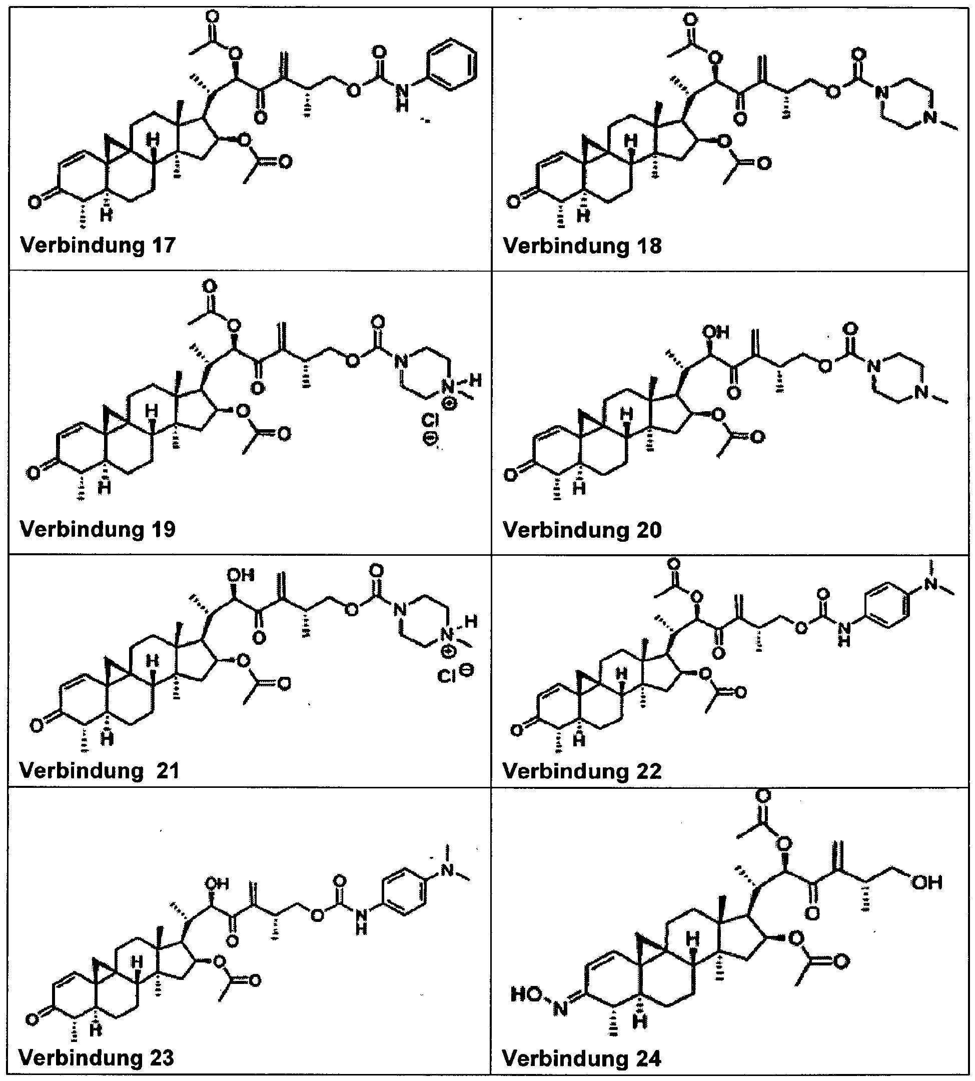

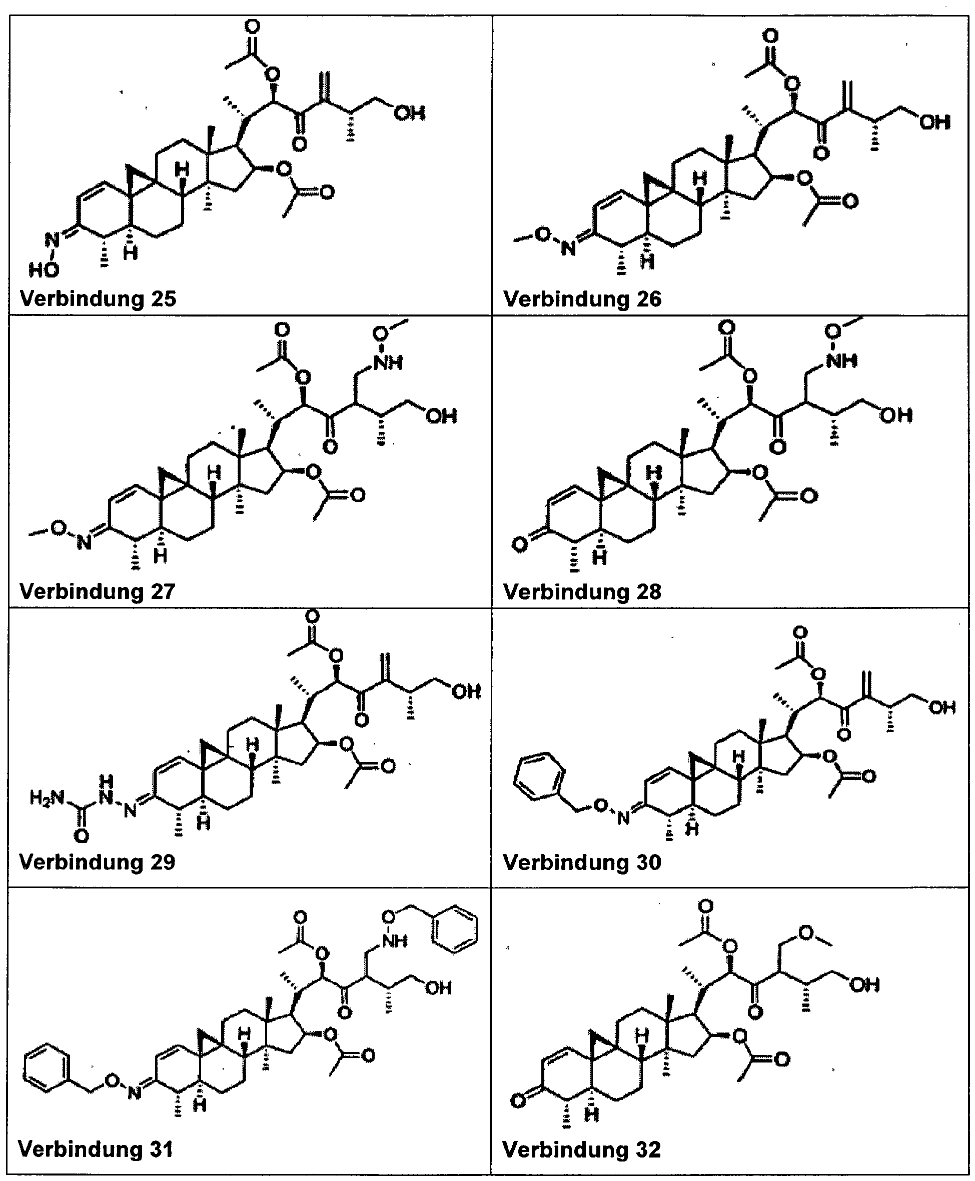

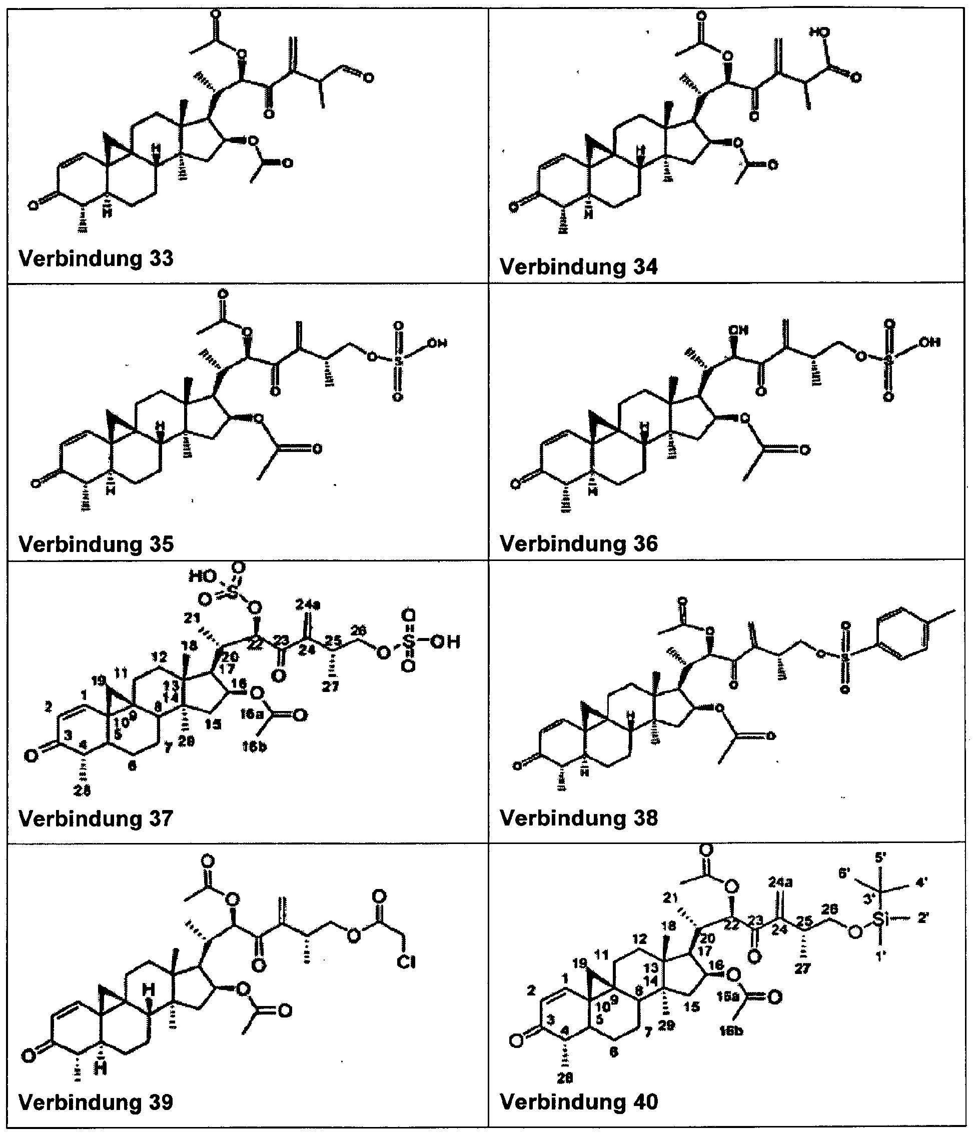

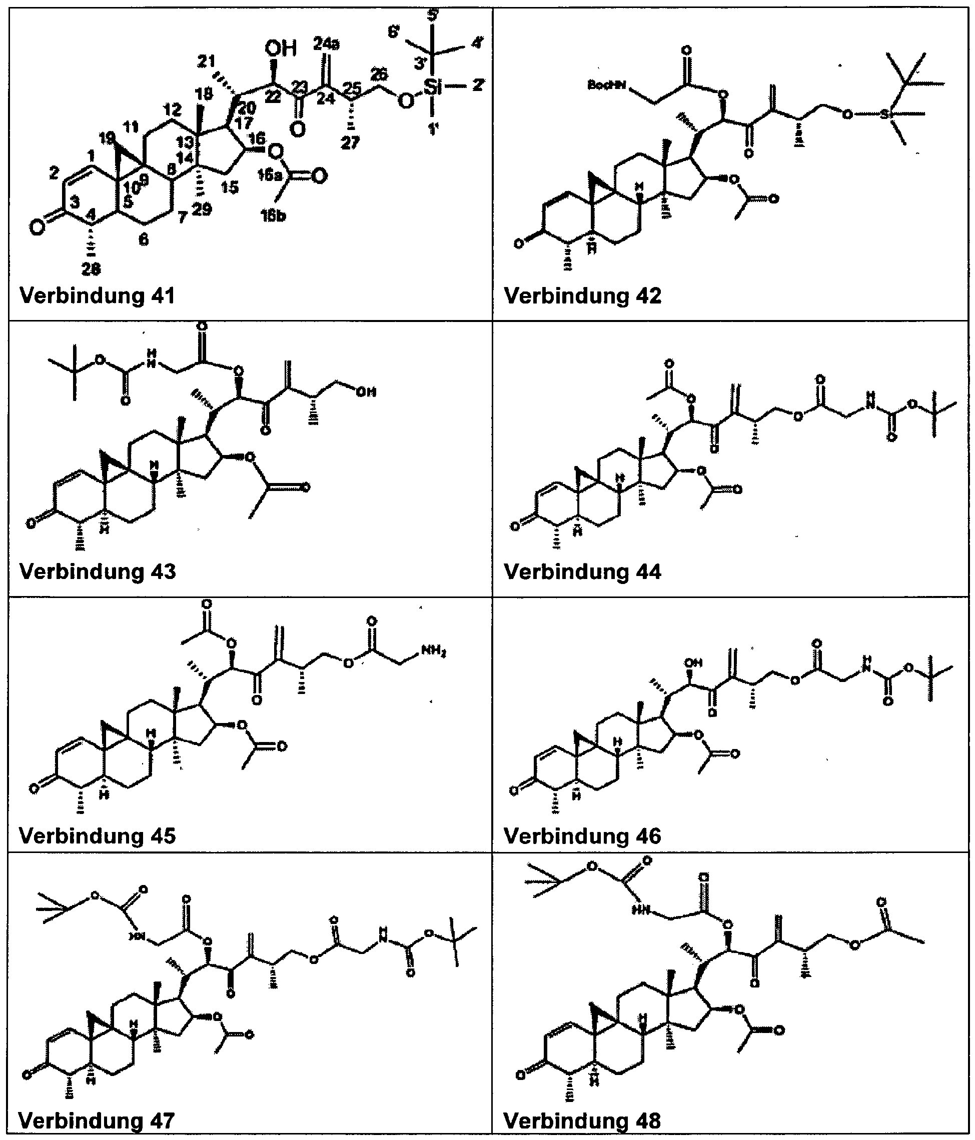

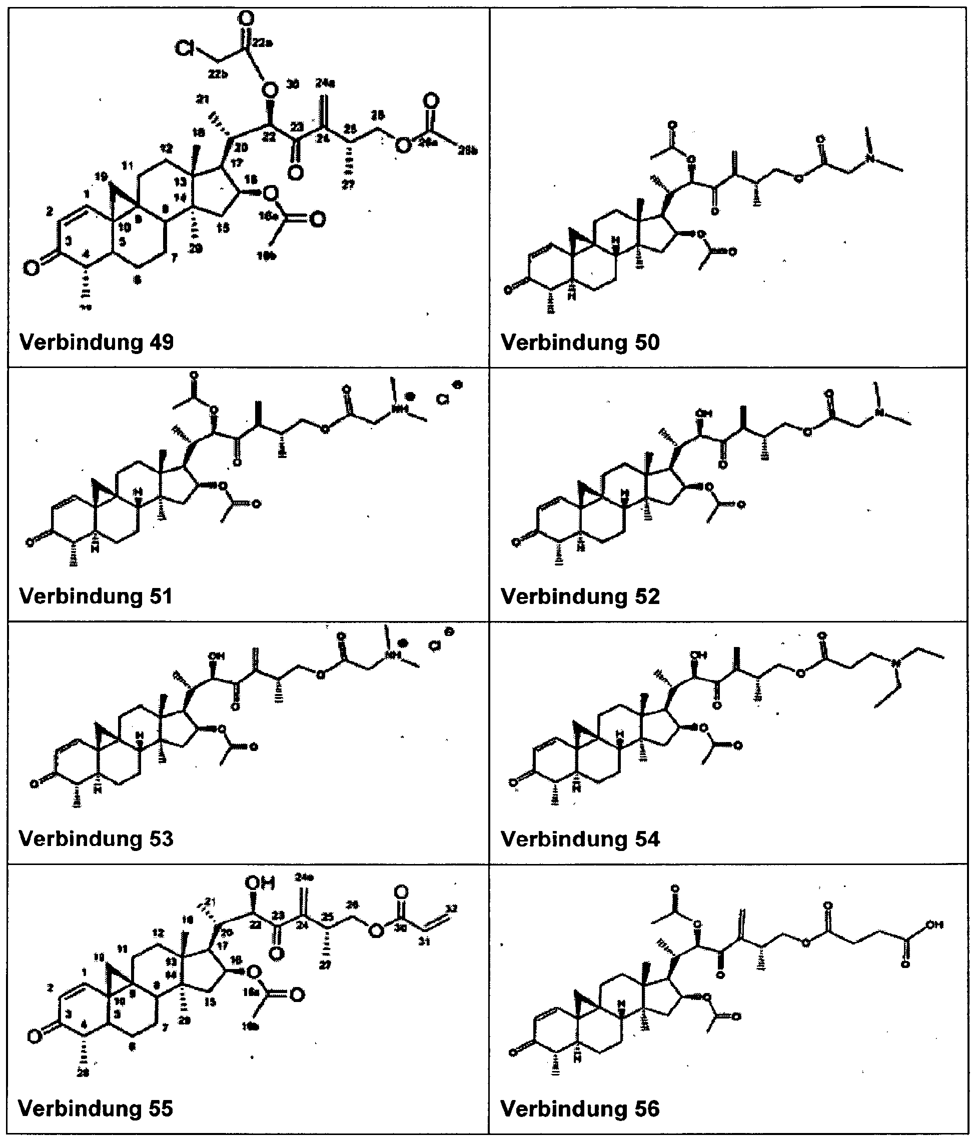

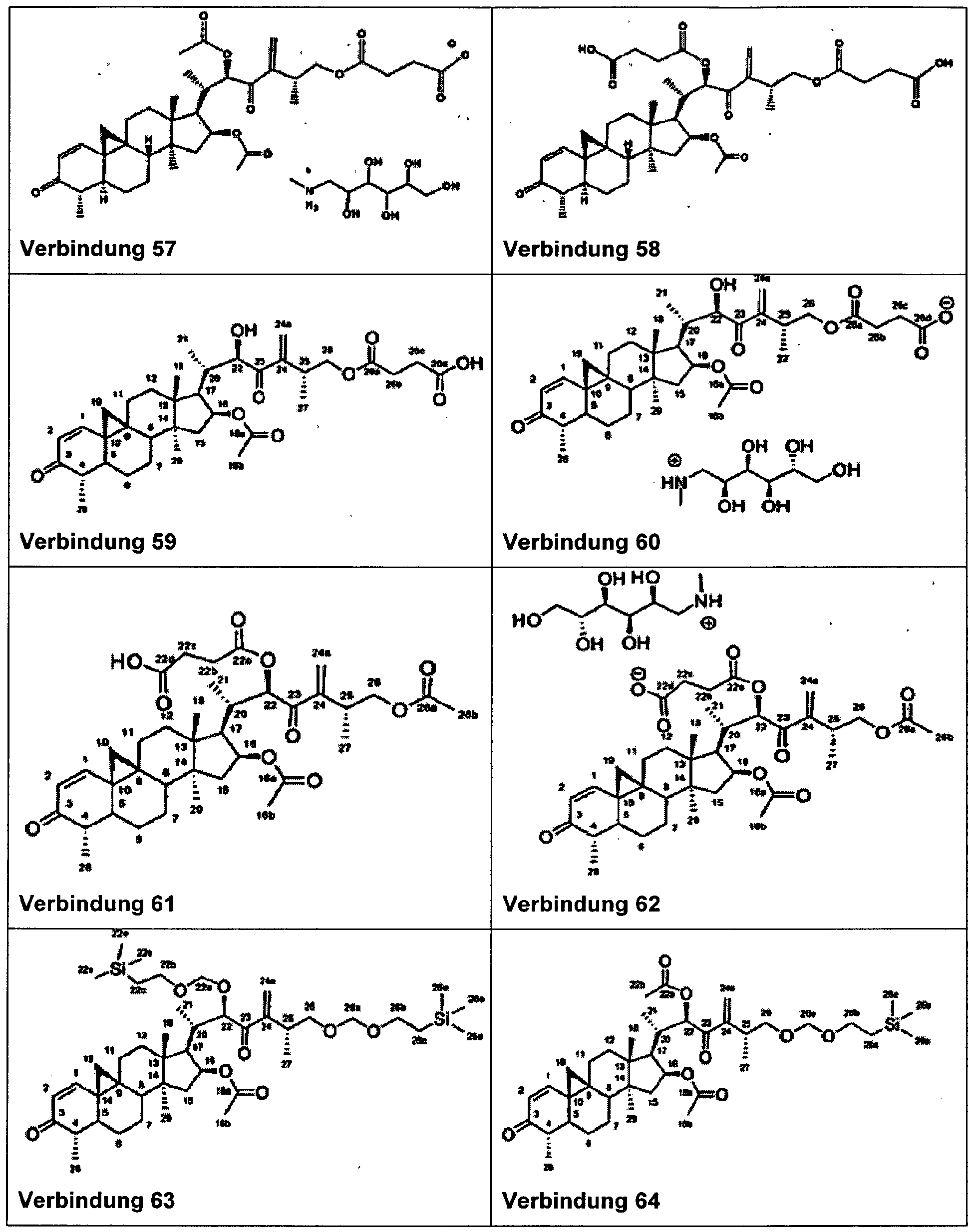

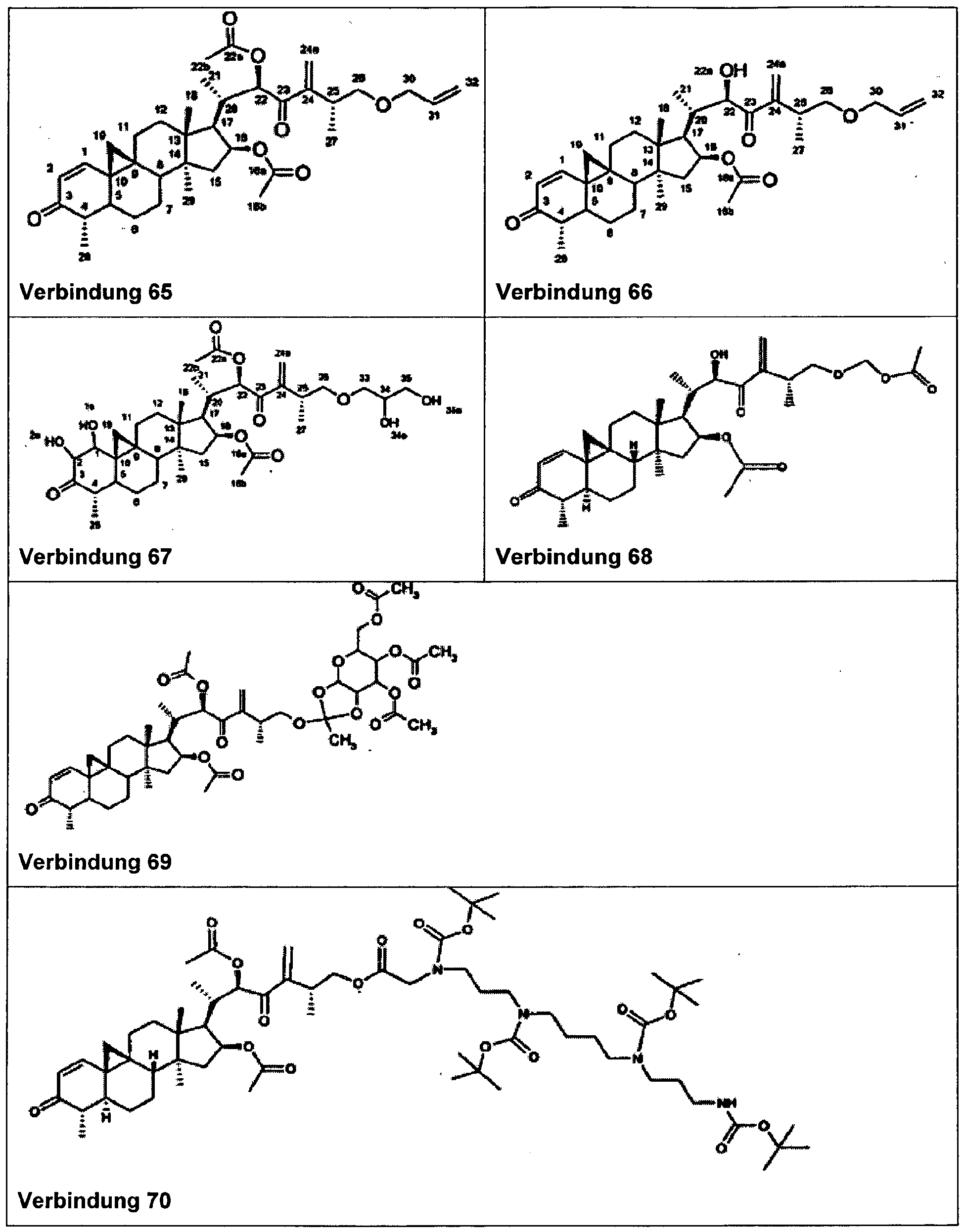

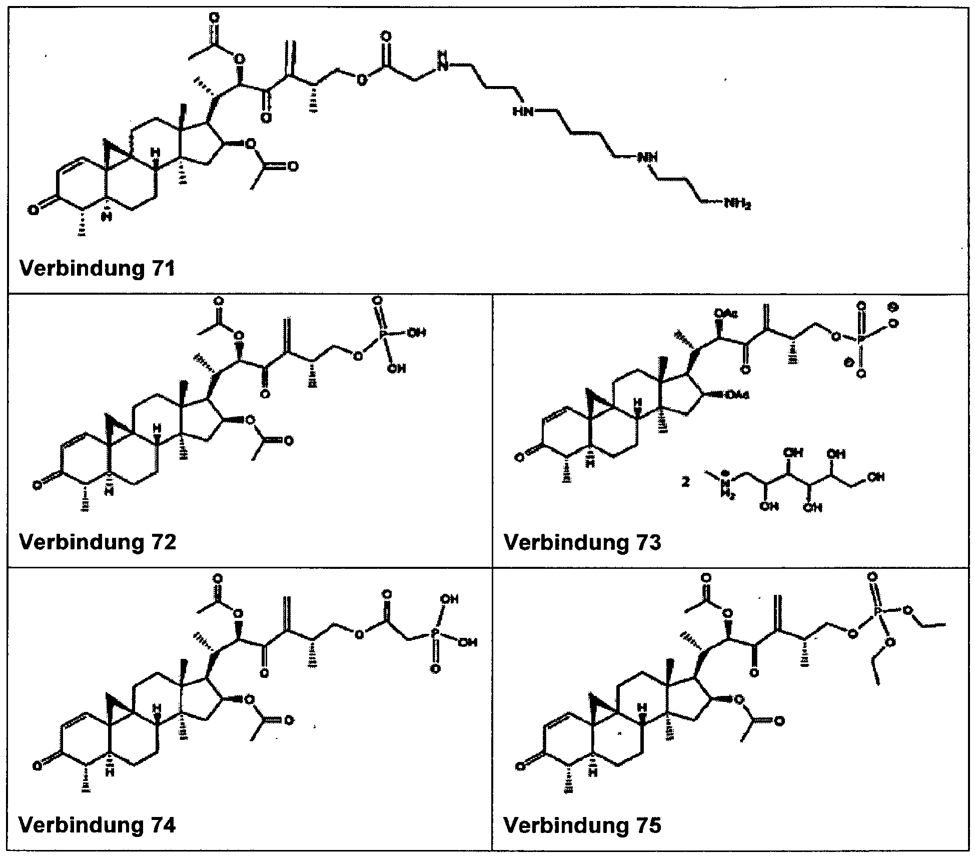

11. Verbindung nach einem der Ansprüche 1 bis 10 für deren Verwendung als Arzneimittel, das insbesondere für die Behandlung einer proliferativen Erkrankung, wie einer Krebserkrankung, bestimmt ist.

12. Pharmazeutische Zusammensetzung, welche mindestens eine Verbindung nach einem der Ansprüche 1 bis 10 und mindestens einen pharmazeutisch annehmbaren Träger umfasst.

13. Pharmazeutische Zusammensetzung nach Anspruch 12, **dadurch gekennzeichnet, dass** sie außerdem mindestens einen anderen Wirkstoff, wie ein Antikrebsmittel, umfasst.

14. Pharmazeutische Zusammensetzung, umfassend:
(i) mindestens eine Verbindung nach einem der Ansprüche 1 bis 10 und
(ii) mindestens einen anderen Wirkstoff, wie ein Antikrebsmittel,
als Kombinationsprodukte für eine gleichzeitige, getrennte oder zeitlich gestaffelte Verwendung.

15. Pharmazeutische Zusammensetzung nach einem der Ansprüche 12 bis 14 für deren Verwendung als Arzneimittel, das insbesondere für die Behandlung einer proliferativen Erkrankung, wie einer Krebserkrankung, bestimmt ist.

## Claims

1. A compound of following formula (I): 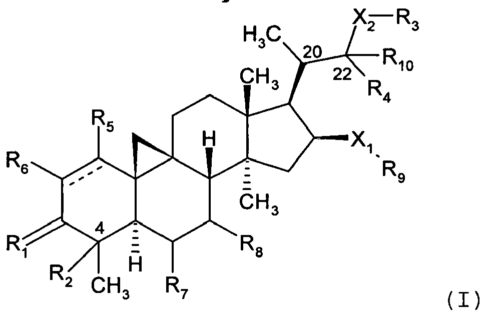 or a pharmaceutically acceptable salt thereof, wherein: 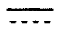 indicates a single bond or a double bond,
- X₁ and X₂ represent, independently of each other, an oxygen or sulfur atom, and notably an oxygen atom,
- R₁ represents an oxygen atom, a sulfur atom or an NOR₁₁ or N-NHCO-NH₂ group,
- R₂ represents a hydrogen atom or an OR₁₂ or SR₁₂ group,
- R₃ represents a hydrogen atom, -SO₂R₅₅, -CH₂OCH₂CH₂SiR₆₁R₆₂R₆₃ or a -CO-(C₁-C₆)alkyl, such as -COCH₃, or -CO-(C₂-C₆)alkenyl group, wherein said group is optionally substituted by a halogen atom or a COOH or NR₅₆R₅₇ group,
- R₄ represents a chain: 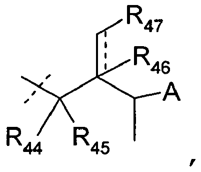 wherein:
■ 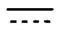 represents a single bond or a double bond,
■ R₄₄ and R₄₅ together form an =O group,
■ R₄₆ represents a hydrogen atom and R₄₇ represents a hydrogen atom, a (C₁-C₆) alkoxy group, -NH-OR₄₉ or a heterocycle with 5 or 6 members linked to the rest of the molecule via a nitrogen atom, such as an imidazolyl, when 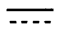 represents a single bond, or R₄₆ is absent and R₄₇ represents a hydrogen atom when 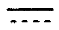 represents a double bond, and
■ A represents a -CHO, -COOH, -CH₂A₁ or -CH₂OCH₂A₁ group with :
∘ A₁ representing a hydrogen atom, a halogen atom, -OH, -OSO₂R₁₃, -N₃, (C₁-C₆) alkoxy optionally substituted by one or more -OH groups; (C₂-C₆) alkenoxy, -OCH₂OR₆₆, -Z₁C (X) R₁₄, -Z₃C (X) Z₄R₁₆, -NH-OR₁₇, -OSiR₁₉R₂₀R₂₁, -OP(O) (OR₂₂) (OR₂₃) , -NR₂₄R₂₅, a heterocycle with 5 or 6 members or a sugar residue, wherein one or more hydroxy groups of said sugar residue are optionally substituted by an acetyl or -P(O)(OH)₂ group,
∘ R₄₈ and R₄₉ representing, independently of each other, a hydrogen atom, a (C₁-C₆)alkyl, aryl or aryl-(C₁-C₆)alkyl group,
∘ R₆₆ representing a -CO-( (C₁-C₆)alkyl) or (C₁-C₆)alkyl group optionally substituted by an SiR₆₇R₆₈R₆₉ group, and
∘ R₆₇, R₆₈ and R₆₉ representing, independently of one another, a (C₁-C₆) alkyl group,
- R₅ and R₆ each represent a hydrogen atom when 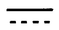 represents a double bond, or
R₅ and R₆ each represent, independently of each other, a hydrogen atom or an OR₄₈ group, such as OH, or R₅ and R₆ together form, with the carbon atoms that carry them, an epoxide ring, when 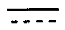 represents a single bond,
- R₇ represents a hydrogen atom or a OR₄₉ group, such as OH,
- R₈ represents a hydrogen atom, or
R₇ and R₈ together form, with the carbon atoms that carry them, an epoxide ring,
- R₉ represents a -CO- (C₁-C₆) alkyl group,
- R₁₀ represents a hydrogen atom, or
R₁₀ and R₃ together form a bond,
with:
■ R₁₁, R₂₆, R₂₈, R₃₀, R₃₁, R₃₆, R₃₇, R₄₁, R₄₂, R₄₃, R₄₈, R₄₉ and R₅₀ representing, independently of one another, a hydrogen atom or a (C₁-C₆) alkyl, (C₂-C₆) alkenyl, aryl or aryl- (C₁-C₆) alkyl group,
■ R₁₂ representing a hydrogen atom or a (C₁-C₆) alkyl or (C₂-C₆) alkenyl group, and notably a hydrogen atom,
■ R₁₃ and R₅₅ represent, independently of each other, an -OH, (C₁-C₆) alkoxy, aryl, -NR₃₀R₃₁, (C₁-C₆) alkylaryl group, or a (C₁-C₆) alkyl group optionally substituted by an -NR₃₀R₃₁ group,
■ R₁₄ representing a (C₁-C₆) alkyl, (C₂-C₆)alkenyl, aryl, (C₁-C₆) alkyl-aryl or aryl- (C₁-C₆) alkyl group, wherein said group is optionally substituted by a group selected from a halogen atom, an -NR₃₂-[(CH₂)ₐ-NR₃₃]_{d}-[(CH₂)_{b}-NR₃₄-(CH₂)c-N-R₃₅]e-R₅₂, -P(O)(OH)₂ or -COOH group, with a, b and c representing an integer between 1 and 5 and d and e each representing 0 or 1,
■ R₁₅ and R₁₆ representing, independently of each other, a hydrogen atom or a (C₁-C₆) alkyl, (C₂-C₆) alkenyl, aryl, (C₁-C₆) alkyl-aryl or aryl-(C₁-C₆)alkyl group, wherein said group is optionally substituted by a group selected from a halogen atom, an -NR₃₂-[(CH₂)ₐ-NR₃₃]_{d}-[(CH₂)_{b}-NR₃₄-(CH₂)_{c}-N-R₃₅]ₑ-R₅₂ or -COOH group, with a, b, c, d and e as defined above,
■ R₁₇ and R₁₈ representing, independently of each other, a hydrogen atom or a (C₁-C₆) alkyl, (C₂-C₆) alkenyl, aryl or aryl- (C₁-C₆) alkyl group,
■ R₁₉, R₂₀, R₂₁, R₅₈, R₅₉, R₆₀, R₆₁, R₆₂ and R₆₃ representing, independently of one another, a (C₁-C₆) alkyl, (C₂-C₆) alkenyl or aryl group,
■ R₂₂ and R₂₃, identical or different, and notably identical, representing a hydrogen atom or a (C₁-C₆) alkyl or (C₂-C₆)alkenyl group, wherein said group is optionally substituted by an -OC(O)-(C₁-C₆) alkyl, NR₃₆R₃₇ and -N⁺R₃₈R₃₉R₄₀ group,
or R₂₂ and R₂₃ together form, with the oxygen atoms that carry them and the phosphorous atom, a ring, notably with 5 or 6 members,
■ R₂₄ and R₂₅, representing, independently of each other, a hydrogen atom or a -CO-(C₁-C₆) alkyl, -CO-(C₂-C₆) alkenyl, (C₂-C₆) alkenyl, (C₃-C₇) cycloalkyl or (C₁-C₆) alkyl group optionally substituted by an NR₄₁R₄₂ group, or
R₂₄ and R₂₅ together form, with the nitrogen atom that carries them, a heterocycle with 5 or 6 members, wherein said heterocycle may comprise, in addition to the nitrogen atom carrying R₂₄ and R₂₅, one or more heteroatoms selected from nitrogen, oxygen and sulfur, and is optionally substituted by a (C₁-C₆) alkyl group,
■ R₂₇ representing an aryl, (C₁-C₆) alkyl or (C₂-C₆)alkenyl group, wherein said group is optionally substituted by one or more halogen atoms,
■ R₂₉ representing a (C₁-C₆) alkyl, (C₂-C₆) alkenyl, aryl or aryl-(C₁-C₆) alkyl group,
■ R₃₂, R₃₃, R₃₄, R₃₅, R₅₂, R₅₃, R₅₄, R₅₆ and R₅₇ representing, independently of one another, a hydrogen atom or a (C₁-C₆) alkyl, (C₂-C₆) alkenyl, -CO- (C₁-C₆) alkyl, -CO- (C₂-C₆) alkenyl, -CO₂-(C₁-C₆)alkyl or -CO₂-(C₂-C₆) alkenyl group,
■ R₃₈, R₃₉ and R₄₀ representing, independently of one another, a (C₁-C₆) alkyl or (C₂-C₆)alkenyl group,
■ X representing O, S or NR₅₀, and notably O,
■ Z₁, Z₂, Z₃ and Z₄ representing, independently of one another, O or NR₄₃, or
Z₂R₁₅ and/or Z₄R₁₆ representing, independently of each other, a heterocycle with 5 or 6 members optionally substituted by a (C₁-C₆)alkyl group, wherein the heterocycle comprises at least one nitrogen atom by which it is linked to the rest of the molecule.

2. The compound according to claim 1, **characterized in that** R₁ is selected from an oxygen atom, an -N-OH, -N-OMe, -N-OBn and -N-NHCO-NH₂ group, and notably is an oxygen atom.

3. The compound according to either claim 1 or claim 2, **characterized in that** R₂ represents a hydrogen atom.

4. The compound according to any one of claims 1 to 3, **characterized in that** R₅ and R₆ each represent a hydrogen atom and 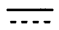 represents a double bond.

5. The compound according to any one of claims 1 to 4, **characterized in that** R₇ and R₈ each represent a hydrogen atom.

6. The compound according to any one of claims 1 to 5, **characterized in that** R₉ represents a -COCH₃ group.

7. The compound according to any one of claims 1 to 6, **characterized in that** X₂-R₃ represents an OH or OC(O)CH₃ group and R₁₀ represents a hydrogen atom or R₃ and R₁₀ together form a bond.

8. The compound according to any one of claims 1 to 7, **characterized in that** it has the following formula (Ia) or (Ib): 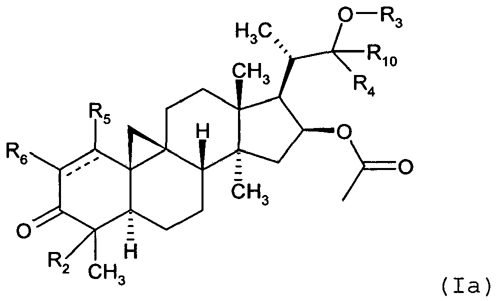 or 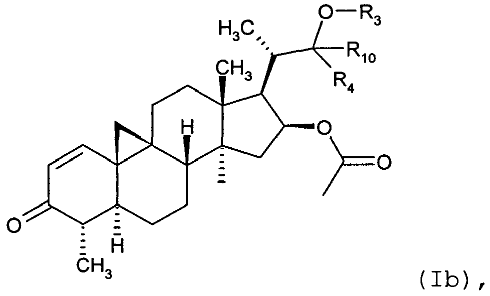 with R₂, R₃, R₄, R₅, R₆ and R₁₀ as defined in claim 1.

9. The compound according to any one of claims 1 to 8, **characterized in that** R₄ represents a chain: 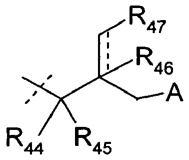 with R₄₄ to R₄₇ and A as defined in claim 1.

10. The compound according to any one of claims 1 to 9, **characterized in that** it is selected from:

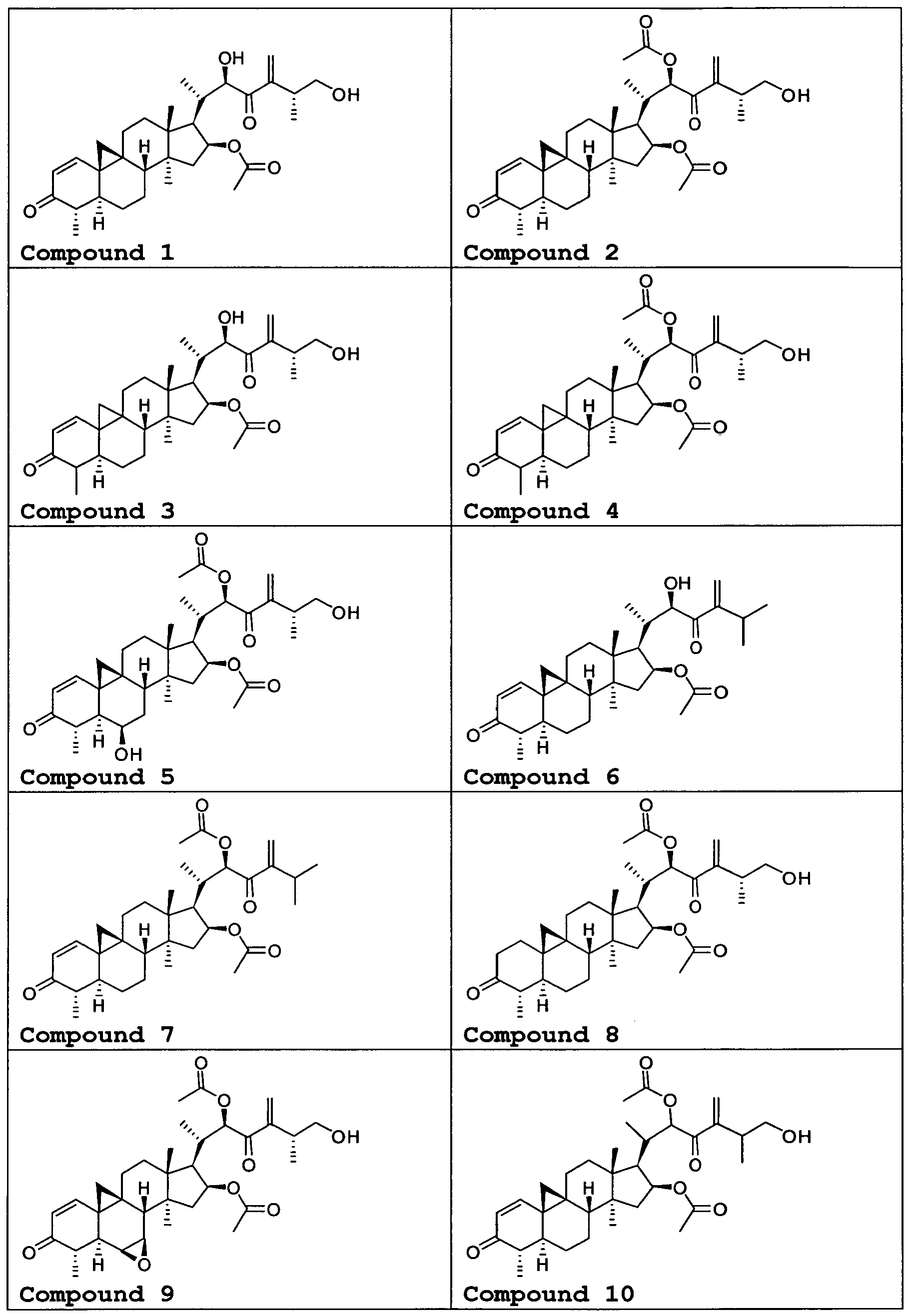

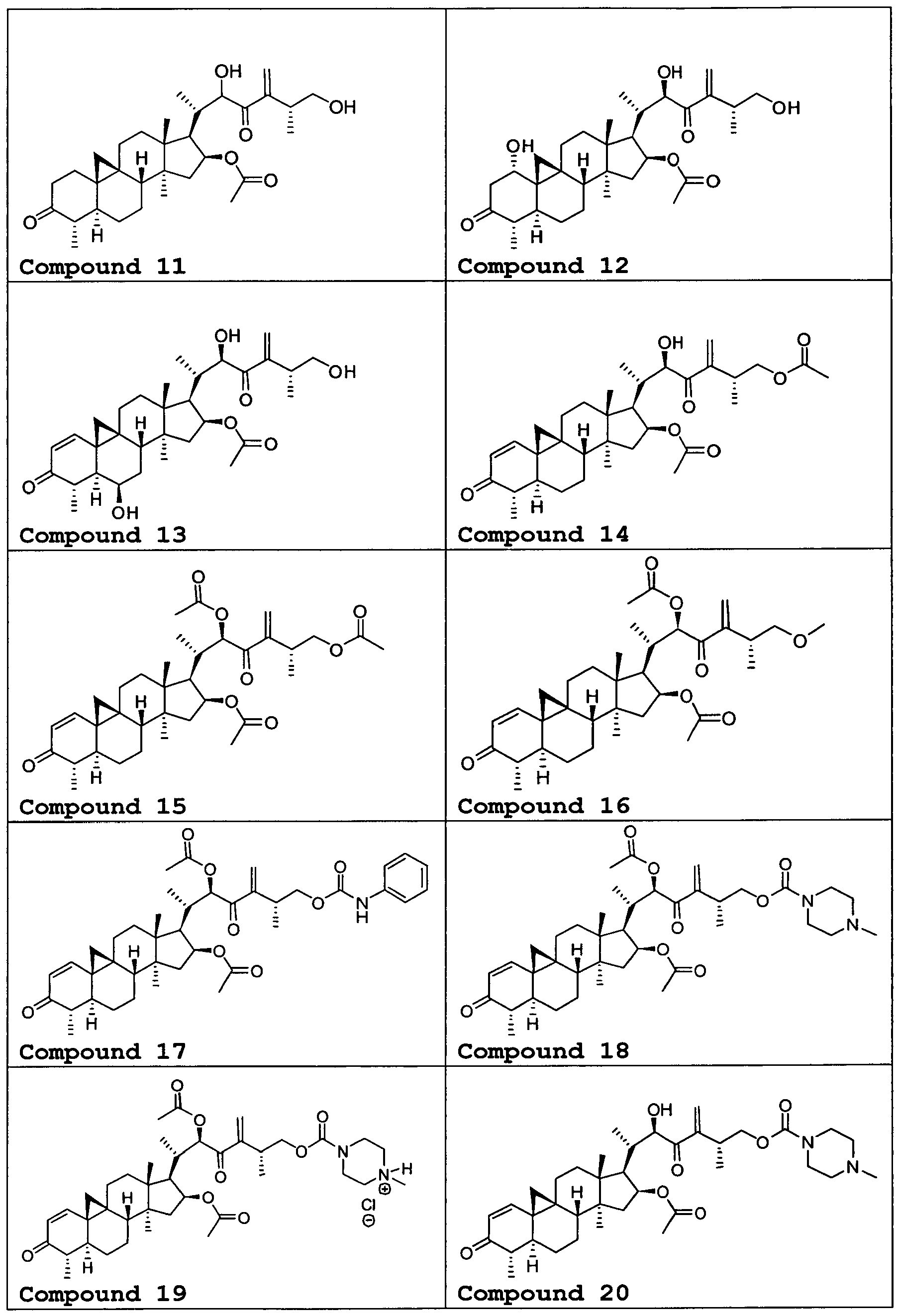

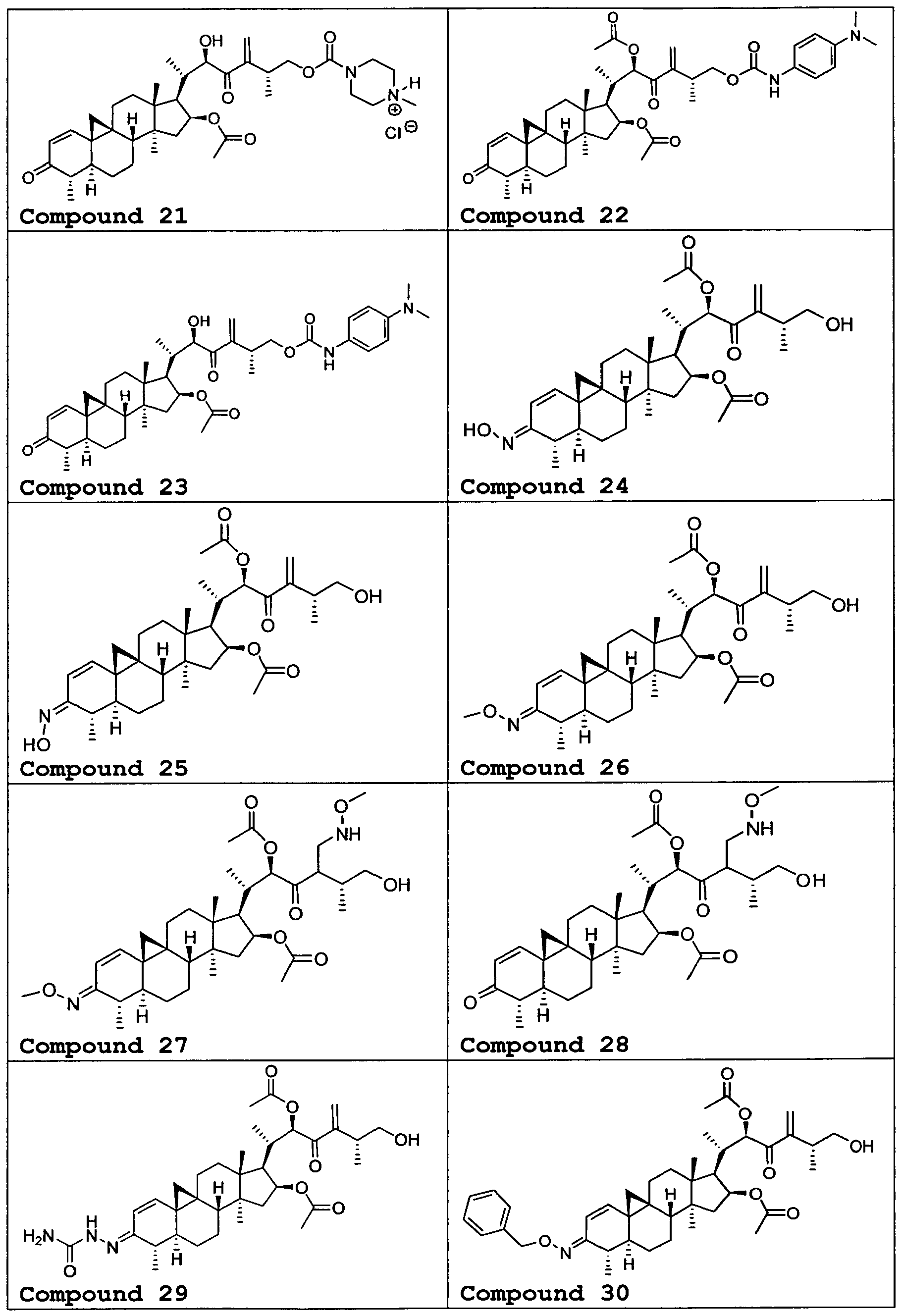

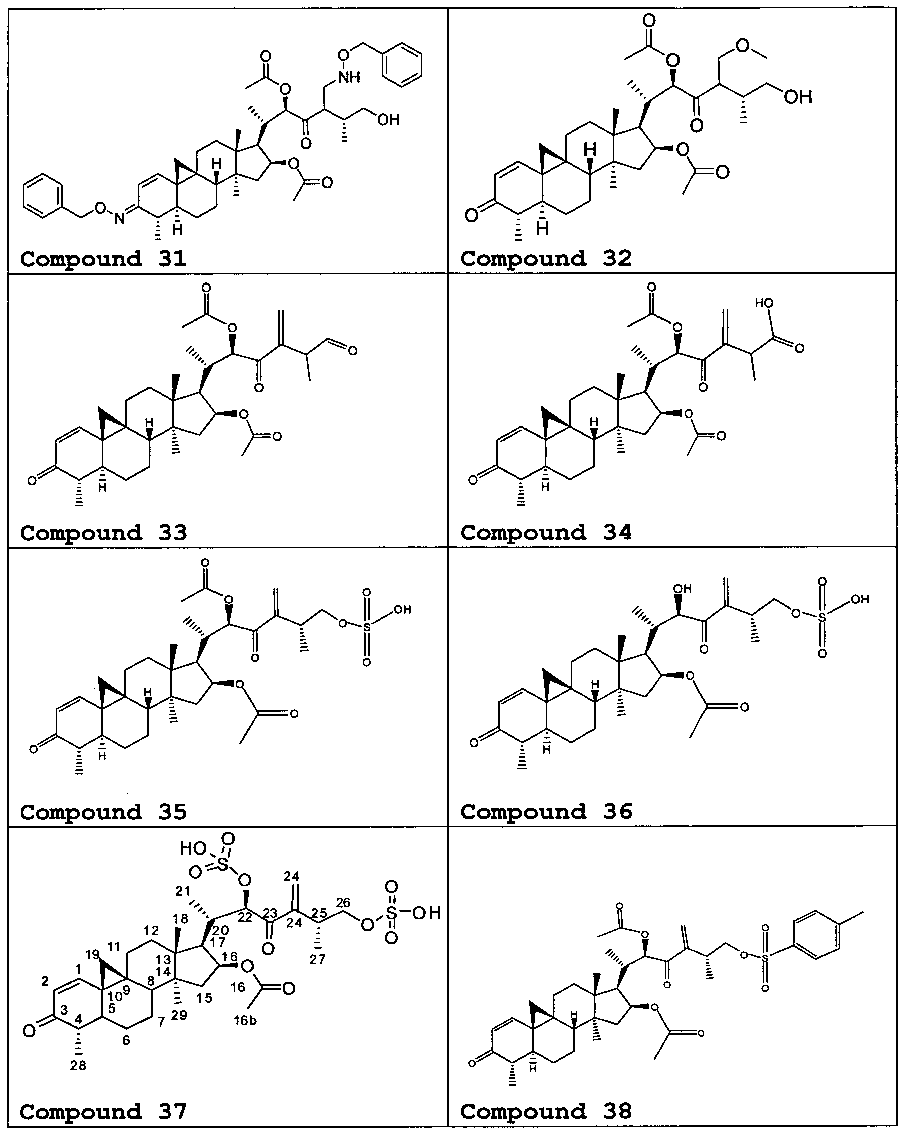

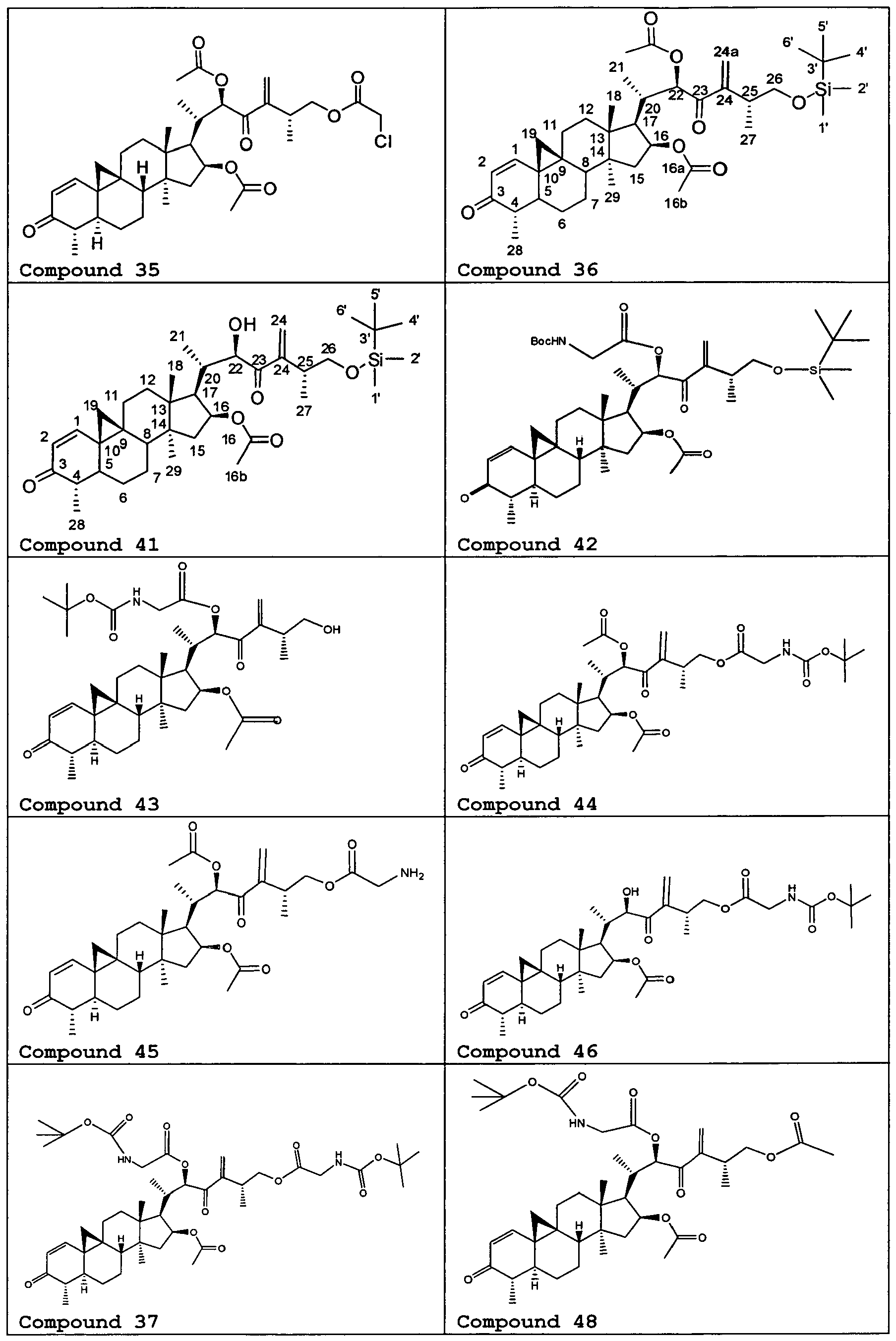

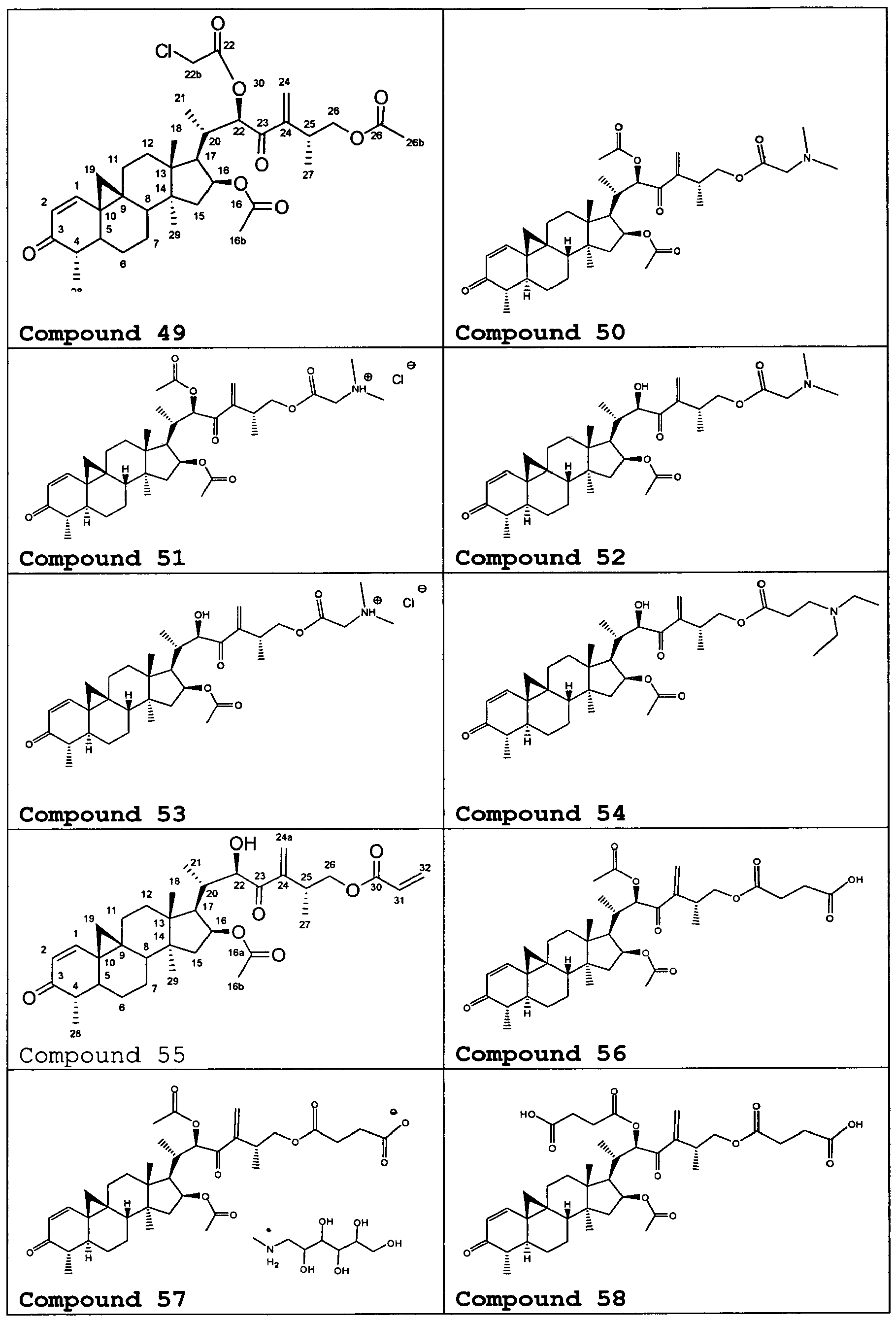

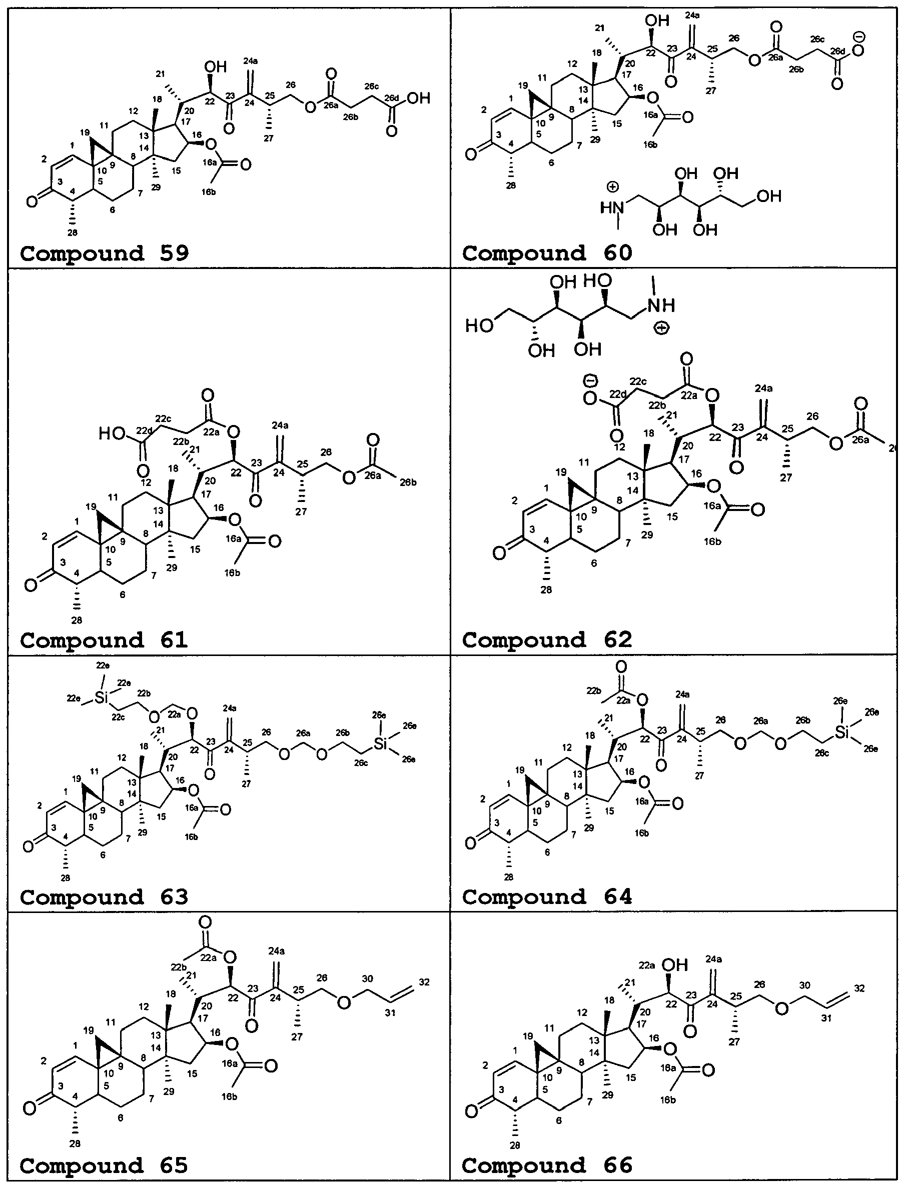

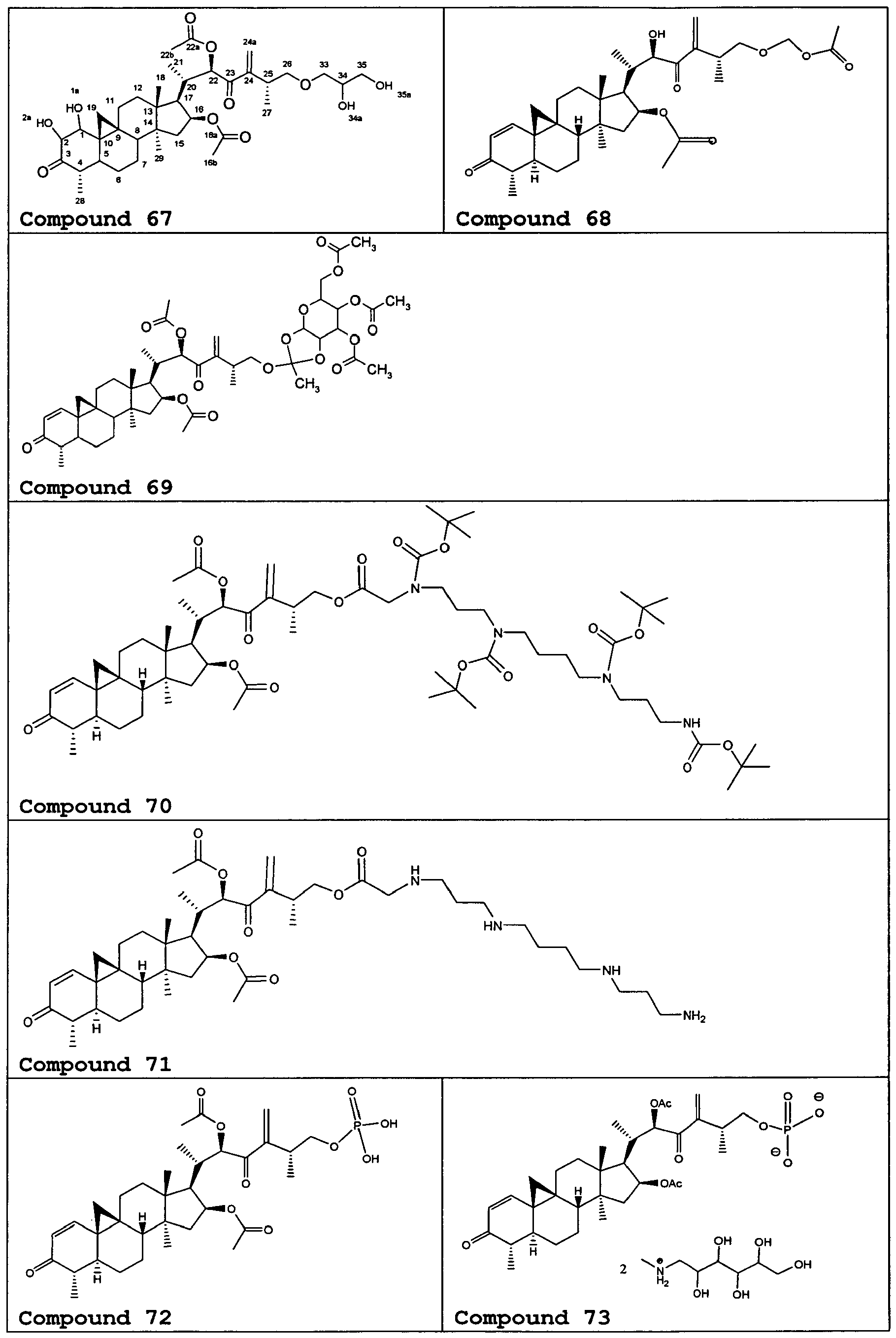

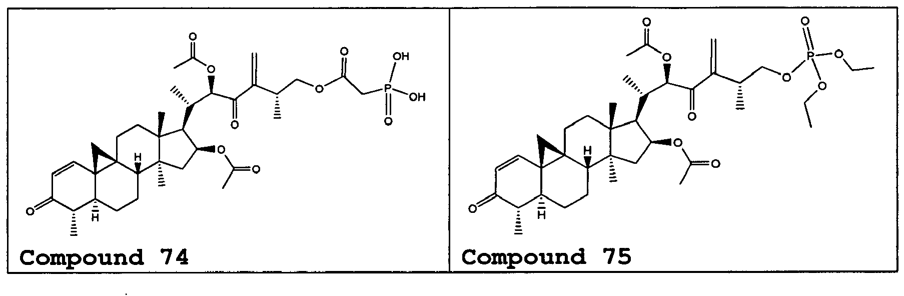

11. The compound according to any one of claims 1 to 10 for the use thereof as a drug, intended in particular for treating a proliferative disease such as cancer.

12. A pharmaceutical composition comprising at least one compound according to any one of claims 1 to 10 and at least one pharmaceutically acceptable carrier.

13. The pharmaceutical composition according to claim 12, **characterized in that** it further comprises at least one other active ingredient, such as an anticancer agent.

14. A pharmaceutical composition comprising:
(i) at least one compound according to any one of claims 1 to 10, and
(ii) at least one other active ingredient, such as an anticancer agent,
as combination products for simultaneous, separate or sequential use.

15. The pharmaceutical composition according to any one of claims 12 to 14 for the use thereof as a drug, intended in particular for treating a proliferative disease such as cancer.
